# EUROPÄISCHE PATENTSCHRIFT

(11) **EP 2 385 945 B1**
(45) Veröffentlichungstag und Bekanntmachung des Hinweises auf die Patenterteilung: **23.01.2013**
(21) Anmeldenummer: 09796639.4
(22) Anmeldetag: 29.12.2009
(51) Int. Cl.: C07D 487/04, A61K 31/519, A61P 11/00

(54) **TRIAZOLO- UND TETRAZOLOPYRIMIDIN-DERIVATE ALS HNE-INHIBITOREN ZUR BEHANDLUNG VON COPD**
TRIAZOLO AND TETRAZOLO PYRIMIDINE DERIVATIVES AS HNE INHIBITORS FOR TREATING COPD
DÉRIVÉS DE TRIAZOLO- ET TÉTRAZOLOPYRIMIDINE EN TANT QU'INHIBITEURS DE L'ÉLASTASE NEUTROPHILE HUMAINE (HNE) POUR LE TRAITEMENT DE LA MALADIE PULMONAIRE OBSTRUCTIVE CHRONIQUE (COPD)

(30) Priorität: 09.01.2009 DE 102009004197
(43) Veröffentlichungstag der Anmeldung: 16.11.2011
(73) Patentinhaber: Bayer Intellectual Property GmbH, 40789 Monheim (DE)
(72) Erfinder: VON NUSSBAUM, Franz, 40219 Düsseldorf (DE); KARTHAUS, Dagmar, 42697 Solingen (DE); ANLAUF, Sonja, 42929 Wermelskirchen (DE); DELBECK, Martina, 45239 Essen (DE); LI, Volkhart, Min-Jian, 42553 Velbert (DE); MEIBOM, Daniel, 51373 Leverkusen (DE); LUSTIG, Klemens, 42113 Wuppertal (DE)
(74) Vertreter: BIP Patents
(86) Internationale Anmeldenummer: PCT/EP2009/009296
(87) Internationale Veröffentlichungsnummer: WO 2010/078953

(56) Entgegenhaltungen:
- WO-A1-2008/003412
- WO-A1-2008/135537

## Beschreibung

Die vorliegende Anmeldung betrifft neue heterocyclisch anellierte Diaryldihydropyrimidin-Derivate, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/ oder Prävention von Erkrankungen der Lunge und des Herz-Kreislauf-Systems.

Die Humane Leukozyten-Elastase (HLE, EC 3.4.21.37), auch Humane Neutrophile Elastase (HNE, hNE) genannt, gehört zur Familie der Serinproteasen. Das proteolytische Enzym findet sich in den azurophilen Granula der potymorphkemigen Leukozyten (engl. *polymorphonuclear leukocytes, PMN leukocytes*). Die intrazelluläre Elastase nimmt eine wichtige Funktion in der Pathogenabwehr wahr, indem über Phagozytose aufgenommene Fremdpartikel abgebaut werden. Aktivierte neutrophile Zellen setzen die HNE aus den Granula in den Extrazellulärraum frei (extrazelluläre HNE), wobei ein Teil der freigesetzten HNE an der Außenseite der neutrophilen Zellmembran verbleibt (membranständige HNE). Das hochaktive Enzym ist in der Lage, eine Vielzahl von Bindegewebsproteinen abzubauen, z.B. die Proteine Elastin, Kollagen und Fibronektin. Elastin kommt in hohen Konzentrationen in allen Gewebetypen vor, die eine hohe Elastizität zeigen, z.B. in der Lunge und in Arterien. Bei einer Vielzahl von pathologischen Prozessen (z.B. Gewebeverletzungen) spielt die HNE eine Rolle beim Gewebeab- und -umbau (engl. *tissue remodeling).* Darüber hinaus ist die HNE ein wichtiger Modulator bei entzündlichen Prozessen. HNE induziert beispielsweise eine erhöhte Genexpression von Interleukin-8 (IL-8).

Es wird daher angenommen, dass die HNE bei vielen Erkrankungen, Verletzungen und pathologischen Veränderungen, deren Entstehung und/oder Progression mit einem entzündlichen Geschehen und/oder einem proliferativen und hypertrophen Gewebe- und Gefäßumbau in Zusammenhang steht, eine wichtige Rolle spielt. Dies können insbesondere Erkrankungen und/oder Schädigungen der Lunge oder des Herz-Kreislauf-Systems sein, oder es kann sich hierbei um eine Sepsis, um Krebs-Erkrankungen oder um andere entzündliche Erkrankungen handeln.

In diesem Zusammenhang zu nennende Erkrankungen und Schädigungen der Lunge sind insbesondere die chronisch-obstruktive Lungenerkrankung (engl. *chronic obstructive pulmonary disease,* COPD), das akute Atemwegssyndrom (engl. *acute respiratory distress syndrome,* ARDS), die zystische Fibrose (engl. *cystic fibrosis,* CF; auch Mukoviszidose genannt), das Lungenemphysem (engl. *lung emphysema)* und die akute Lungenschädigung (engl. *acute lung injury,* ALI). Erkrankungen und Schädigungen des Herz-Kreislauf-Systems, in denen die HNE involviert ist, sind zum Beispiel Gewebeveränderungen bei einer Herzinsuffizienz und Reperfusionsschäden nach einem Myokardinfarkt (engl. *acute myocardial infarct,* AMI), der kardiogene Schock, das akute Koronarsyndrom (engl. *acute coronary syndrome,* ACS) sowie Aneurysmen. Erkrankungen in Zusammenhang mit einer Sepsis sind beispielsweise eine systemische entzündliche Reaktion (engl. *systemic inflammatory response syndrome,* SIRS), die schwere Sepsis, der septische Schock und das multiple Organversagen (engl. *multi-organ failure,* MOF; *multi-organ dysfunction,* MODS) sowie die intravaskuläre Gerinnung (engl. *disseminated intravascular coagulation,* DIC). Beispiele für einen Gewebeab- und -umbau bei Krebsprozessen sind das Einwandern von Krebszellen in das gesunde Gewebe (Metastasenbildung) und die Neuausbildung von versorgenden Blutgefäßen (Neo-Angiogenese). Andere entzündliche Krankheiten, bei denen die HNE eine Rolle spielt, sind rheumatoide Erkrankungen, zum Beispiel die rheumatoide Arthritis, chronische Darmentzündungen (engl. *inflammatory bowel disease,* IBD; Morbus Crohn, engl. *Crohn's disease,* CD; Colitis ulcerosa, engl. *ulcerative colitis,* UC) und die Arteriosklerose.

Im Allgemeinen geht man davon aus, dass Elastase-vermittelten pathologischen Prozessen ein verschobenes Gleichgewicht zugrunde liegt zwischen der freien Elastase und dem körpereigenen Elastase-Inhibitorprotein (hauptsächlich das alpha-1-Antitrypsin, AAT) *[*Neutrophils and protease/antiprotease imbalance, Stockley, Am. J. Respir. Crit. Care Med. 160, 49-52 (1999)]. AAT liegt im Plasma im hohen Überschuss vor und neutralisiert so sehr schnell freie HNE. In verschiedenen pathologischen Prozessen ist die Konzentration an freier Elastase erhöht, so dass lokal die Balance zwischen Protease und Protease-Inhibitor zu Gunsten der Protease verschoben ist. Zudem ist die membranständige Elastase der aktivierten PMN-Zellen vor einer Inhibition durch AAT weitestgehend geschützt. Gleiches gilt für die freie Elastase, die sich in einem erschwert zugänglichen Mikrokompartiment zwischen der neutrophilen Zelle und der angrenzenden Gewebezelle (z.B. Endothelzelle) befindet. Zusätzlich herrschen stark oxidierende Bedingungen im Umfeld von aktivierten Leukozyten (engl. *oxidative burst),* wodurch AAT oxidiert wird und in der inhibitorischen Wirkung mehrere Größenordnungen verliert.

Neue Elastase-inhibierende Wirkstoffe (exogen verabreichte Inhibitoren der HNE) sollten demnach ein niedriges Molekulargewicht aufweisen, um in der Lage zu sein, auch die membranständige HNE und die im geschützten Mikrokompartiment befindliche HNE (s.o.) zu erreichen und zu inhibieren. Hierzu ist auch eine gute Stabilität der Substanzen *in vivo* notwendig (geringe *in vivo*-Clearance). Außerdem sollten diese Verbindungen stabil sein unter oxidativen Bedingungen, um im Krankheitsgeschehen nicht an inhibitorischer Potenz zu verlieren.

Die Pulmonale Arterielle Hypertonie (PAH) ist eine progrediente Lungenerkrankung, die unbehandelt durchschnittlich innerhalb von 2.8 Jahren nach Diagnosestellung zum Tode führt. Eine zunehmende Verengung der Lungenstrombahn führt zu einer Mehrbelastung des rechten Herzens, die bis zum Rechtsherzversagen gehen kann. Definitionsgemäß liegt bei einer chronischen pulmonalen Hypertonie ein pulmonal-arterieller Mitteldruck (mPAP)- von > 25 mmHg in Ruhe oder > 30 mmHg unter Belastung vor (Normalwert < 20 mmHg). Die Pathophysiologie der pulmonal-arteriellen Hypertonie ist gekennzeichnet durch Vasokonstriktion und Remodeling der Pulmonalgefäße. Bei der chronischen PAH kommt es zu einer Neomuskularisierung primär nicht muskularisierter Lungengefäße, und die Gefäßmuskulatur der bereits muskularisierten Gefäße nimmt an Umfang zu. Durch diese zunehmende Obliteration der Lungenstrombahn kommt es zu einer progredienten Belastung des rechten Herzens, die zu einer verminderten Auswurfleistung des rechten Herzens führt und letztlich in einem Rechtsherzversagen endet (M. Humbert et al., J. Am. Coll. Cardiol. 2004, 43, 13S-24S). Mit einer Prävalenz von 1-2 pro einer Million handelt es sich bei PAH um eine äußerst seltene Erkrankung. Das mittlere Alter der Patienten wurde auf 36 Jahre geschätzt, nur 10% der Patenten waren über 60 Jahre alt. Deutlich mehr Frauen als Männer sind betroffen (G.E. D'Alonzo et al., Ann. Intern. Med 1991, 115, 343-349).

Trotz aller Fortschritte in der Therapie der pulmonal-arteriellen Hypertonie gibt es bisher keine Aussicht auf Heilung dieser schwerwiegenden Erkrankung. Auf dem Markt befindliche Standardtherapien (z.B. Prostacyclin-Analoga, Endothelinrezeptor-Antagonisten, Phosphodiesterase-Inhibitoren) sind in der Lage, die Lebensqualität, die körperliche Belastbarkeit und die Prognose der Patienten zu verbessern. Hierbei handelt es sich um primär hämodynamische Therapieprinzipien, die den Gefäßtonus beeinflussen, jedoch keinen direkten Einfluss auf die pathogenen Remodeling-Prozesse haben. Darüber hinaus ist die Anwendbarkeit dieser Medikamente durch die z.T. gravierenden Nebenwirkungen und/oder aufwendigen Applikationsformen eingeschränkt. Der Zeitraum, über den unter einer spezifischen Monotherapie die klinische Situation der Patienten verbessert oder stabilisiert werden kann, ist begrenzt (z.B. aufgrund einer Toleranzentwicklung). Es erfolgt schließlich eine Therapieeskalation und somit eine Kombinationstherapie, bei der mehrere Medikamente gleichzeitig gegeben werden müssen.

Neue Kombinationstherapien sind eine der aussichtsreichsten zukünftigen Therapieoptionen zur Behandlung der pulmonalen arteriellen Hypertonie. In diesem Zusammenhang ist die Erkundung neuer pharmakologischer Mechanismen zur Behandlung der PAH von besonderem Interesse (Ghofrani et al., Herz 2005, 30, 296-302; E.B. Rosenzweig, Expert Opin. Emerging Drugs 2006, 11, 609-619; T. Ito et al., Curr. Med. Chem. 2007, 14, 719-733). Vor allem solche Therapieoptionen, die direkt in das Remodeling-Geschehen eingreifen (anti-Remodeling-Mechanismen, reverse-Remodeling-Mechanismen), könnten Grundlage einer mehr ursächlichen Behandlung sein und somit einen großen Vorteil für die Patienten bringen. Neue Therapien sollten hierbei mit den bekannten kombinierbar sein. Um in einer solchen Kombinationstherapie das Risiko für störende Wechselwirkungen zwischen den Medikamenten zu minimieren, sollten diese neuen Wirkstoffe metabolisierende P450 CYP-Enzyme nicht oder in nur sehr geringem Maße hemmen.

Heute geht man davon aus, dass die Elastase beim pathologischen Remodeling eine zentrale Rolle spielt. In Tiermodellen und in Patienten mit einem erhöhten arteriellen Lungenblutdruck (pulmonale arterielle Hypertension) konnte eine Fragmentierung des Bindegewebes (interne elastische Lamina) festgestellt werden [Rabinovitch et al., Lab. Invest. 55, 632-653 (1986)], und in Tiermodellen für die pulmonale arterielle Hypertension (hypoxisches Ratten- und Mausmodell, Monocrotalin-Rattenmodell) konnte eine erhöhte Elastase-Aktivität gezeigt werden, die mit der Fragmentierung des Bindegewebes einherging [Todorovich-Hunter et al., Am. Rev. Respir. Dis. 146, 213-223 (1992)]. Man vermutet, dass der zu beobachtende Gewebeumbau im Verlauf des Krankheitsgeschehens der pulmonalen arteriellen Hypertonie durch ein Elastase-vermitteltes Freisetzen von bindegewebsständigen Wachstumsfaktoren induziert wird, z.B. des basischen Fibroblasten-Wachstumsfaktors (engl. *basic fibroblast growth factor,* bFGF) [Rabinovitch, Am. J. Physiol. 277, L5-L12 (1999)]. Im hypoxischen Mausmodell für die pulmonale arterielle Hypertension konnte ein positiver Effekt mit einem überexprimierten Elastase-Inhibitorprotein gezeigt werden [Zaidi et al., Circulation 105, 516-521 (2002)]. Im Monocrotalin-Rattenmodell für die pulmonale arterielle Hypertension konnte eine positive Wirkung mit synthetischen, niedermolekularen Elastase-Inhibitoren gezeigt werden; hierbei war auch ein günstiger Effekt beim Gewebeumbau zu verzeichnen [Cowan et al., Nature Med. 6, 698-702 (2000)]. Alle bisher bekannten niedermolekularen Elastase-Inhibitoren sind jedoch wenig selektiv, chemisch reaktiv und/oder nur begrenzt oral verfügbar, was eine klinische Entwicklung eines oralen Elastase-Inhibitors in diesen Indikationen bisher vereitelte.

Der Begriff "pulmonale arterielle Hypertonie" umfasst bestimmte Formen der pulmonalen Hypertonie, wie sie z.B. von der Weltgesundheitsorganisation (WHO) festgelegt worden sind (Clinical Classification of Pulmonary Hypertension, Venedig 2003; G. Simonneau et al., J. Am. Coll. Cardiol. 2004, 43, 5S- 12S).

Die pulmonale arterielle Hypertonie beinhaltet nach dieser Einteilung die Idiopathische Pulmonale Arterielle Hypertonie (IPAH, früher auch als primäre pulmonale Hypertonie, PPH, bezeichnet), die Familiär bedingte Pulmonale Arterielle Hypertonie (FPAH), die persistierende pulmonale Hypertonie der Neugeborenen sowie die Assoziierte Pulmonal-Arterielle Hypertonie (APAH), welche assoziiert ist mit Kollagenosen, kongenitalen systemisch-pulmonalen Shuntvitien, portaler Hypertension, HIV-Infektionen, der Einnahme bestimmter Drogen und Medikamente (z.B. von Appetitzüglern), mit Erkrankungen mit einer signifikanten venösen/kapillären Beteiligung wie der pulmonal-venookklusiven Erkrankung und der pulmonal-kapillären Hämangiomatose, oder mit anderen Erkrankungen wie Schilddrüsenerkrankungen, Glykogenspeicherkrankheiten, Morbus Gaucher, hereditäre Teleangiektasie, Hämoglobinopathien, myeloproliferative Erkrankungen und Splenektomie.

Andere Formen der pulmonalen Hypertonie umfassen beispielsweise die mit Linksherzerkrankungen assoziierte pulmonale Hypertonie, z.B. bei ventrikulären oder valvulären Erkrankungen, die mit Erkrankungen der Atemwege und/oder der Lunge assoziierte pulmonale Hypertonie, z.B. bei chronisch-obstruktiver Lungenerkrankung, interstitieller Lungenkrankheit oder Lungenfibrose, die auf chronische thrombotische und/oder embolische Erkrankungen zurückzuführende pulmonale Hypertonie, z.B. bei thromboembolischer Obstruktion von Lungenarterien, sowie die durch allgemein entzündliche Krankheitsprozesse oder durch spezielle Ursachen hervorgerufene pulmonale Hypertonie (z.B. bei Schistosomiasis, Sarkoidose und Tumorerkrankungen).

Die chronisch-obstruktive Lungenerkrankung (COPD) ist eine langsam fortschreitende Lungenerkrankung, die durch eine Behinderung der Atemströmung charakterisiert ist, welche durch ein Lungenemphysem und/oder eine chronische Bronchitis hervorgerufen wird. Die ersten Symptome der Erkrankung zeigen sich in der Regel ab dem vierten bis fünften Lebensjahrzehnt. In den darauffolgenden Lebensjahren verschlimmert sich häufig die Kurzatmigkeit und es manifestiert sich Husten, verbunden mit einem ausgiebigen und stellenweise eitrigen Auswurf und einer Stenose-Atmung bis hin zu einer Atemnot (Dyspnoe). COPD ist in erster Linie eine Krankheit von Rauchern: Rauchen ist verantwortlich für 90% aller COPD-Fälle und 80-90% aller COPD-Todesfälle. COPD ist ein großes medizinisches Problem und stellt weltweit die sechsthäufigste Todesursache dar. Von den über 45-jährigen Menschen sind ca. 4-6% betroffen.

Obwohl die Behinderung der Atemströmung nur partiell und zeitlich befristet sein kann, ist COPD nicht heilbar. Behandlungsziel ist folglich eine Verbesserung der Lebensqualität, die Linderung der Symptome, die Verhinderung akuter Verschlechterungen und die Verlangsamung der fortschreitenden Beeinträchtigung der Lungenfunktion. Bestehende Pharmakotherapien, die sich seit den letzten zwei bis drei Jahrzehnten kaum geändert haben, sind das Verwenden von Bronchodilatoren, um blockierte Atemwege zu öffnen, und in bestimmten Situationen Kortikosteroide, um die Entzündung der Lunge einzudämmen [P.J. Barnes, N. Engl. J. Med 343, 269-280 (2000)]. Die chronische Entzündung der Lunge, hervorgerufen durch Zigarettenrauch oder andere Reizstoffe, ist die treibende Kraft der Krankheitsentwicklung. Der zugrunde liegende Mechanismus beinhaltet Immunzellen, die im Zuge der inflammatorischen Reaktion der Lunge verschiedene Chemokine ausschütten. Hierdurch werden neutrophile Zellen und im weiteren Verlauf alveolare Makrophagen zum Lungenbindegewebe und Lumen gelockt. Neutrophile Zellen sezemieren einen Protease-Cocktail, der hauptsächlich HNE und Proteinase 3 enthält. Hierdurch wird lokal die Protease/Antiprotease-Balance zu Gunsten der Proteasen verschoben, was u.a. zu einer unkontrollierten Elasase-Aktivität und in Folge hiervon zu einem überschießenden Abbau des Elastins der Alveolaren führt [J.E. Gadek et al., J. Clin. Invest. 68 889-898 (1981); Z. Werb et al., J. Invest. Dermatol. 79, 154-159 (1982); A. Janoff, Am. Rev. Respir. Dis. 132, 417-433 (1985); P.J. Barnes, N. Engl. J. Med. 343, 269-280 (2000)]. Dieser Gewebeabbau verursacht einen Kollaps der Bronchien. Dies geht einher mit einer verminderten Elastizität der Lunge, was zu einer Behinderung der Atemströmung und beeinträchtigter Atmung führt. Darüber hinaus kann eine häufige und andauernde Entzündung der Lunge zu einem Remodeling der Bronchien und in der Folge zu einer Ausbildung von Läsionen führen. Solche Läsionen tragen zum Auftreten des chronischen Hustens bei, der eine chronische Bronchitis kennzeichnet.

Alpha-1-Antitrypsin (AAT) ist ein kleines körpereigenes Protein und stellt, wie oben erwähnt, den wichtigsten endogenen Elastase-Hemmer dar. Bei Patienten mit einer genetisch bedingten Defizienz dieses Proteins (AATD) ist die Protease/Antiprotease-Balance verschoben. Der Wirkradius und die Wirkdauer der HNE ist in AATD-Patienten entsprechend um einen Faktor 2.5 bzw. 6.5 erhöht [T.G. Liou und E.J. Campbell, Biochemistry 1995, 16171-16177]. AATD-Patienten haben ein erhöhtes Risiko, ein Lungenemphysem oder COPD zu entwickeln, und bei vielen AATD-Patienten ist eine Lungentransplantation indiziert.

Unter einer Bronchiektasie versteht man eine abnorme Ausweitung des Bronchialbaums. Zwei Formen können unterschieden werden: sackförmige, lokalisierte Bronchiektasen und generalisierte, zylindrische Bronchiektasen. Bronchiektasen können in angeborener Form auftreten, sind aber meist erworben und finden sich insbesondere bei Rauchern. Durch die Ausweitung ist die Entleerung des Bronchialsekrets erschwert, und durch das retinierte Bronchialsekret werden Infektionen begünstigt. Bronchiektasen finden sich oft auch bei angeborenen Schleimhauterkrankungen wie der Mukoviszidose mit abnormer Viskosität des Bronchialsekrets und beim Syndrom der ziliären Dyskinesie. Bei diesem Syndrom (Kartagener-Syndrom) sind die Zilienarchitektur und -funktion und dadurch die Sekretdrainage gestört. Andere Ursachen von Bronchiektasen können Obstruktionen proximal der Ektasie sein, z.B. durch Tumore oder Fremdkörper. Als ursächlich werden auch rezidivierende und persistierende Infekte angenommen, die die Bronchuswände schwächen. Ferner gibt es Bronchiektasien, die nicht eindeutig mit Infektionszuständen oder exogenen Noxen in Zusammenhang gebracht werden können (idiopathische Bronchiektasien).

Die Bronchiektasie ist charakterisiert durch eine Einwanderung von Neutrophilen in das Lungengewebe. Die Patienten zeigen ein starkes Ungleichgewicht zwischen Neutrophilenaktivität und schützenden Inhibitor-Proteinen, was zu einer Schädigung des Lungengewebes durch die von den Neutrophilen sezemierten Proteasen (hauptsächlich HNE) führt [Schaaf et al., Respiration 67, 52-59 (2000)].

Die Bronchiolitis obliterans ist eine in den Bronchioli angreifende Entzündung mit Epitheldestruktion und Bildung eines fibrinreichen Exsudats in den Bronchioli und den angrenzenden Alveolen. Durch Organisation des Exsudats entstehen Bindegewebspfröpfe, die aus den Bronchioli in die Alveolen hineinreichen. Die Erkrankung ist gekennzeichnet durch eine erhöhte Anzahl von Neutrophilen in den Atemwegen und ein Ungleichgewicht zwischen der freien Elastase und dem körpereigenen Elastase-Inhibitorprotein [Elssner et al., Transpl. Infect. Dis. 3, 168-176 (2001)]. Als Ursachen werden vorangegangene Infektionen sowie Medikamente diskutiert. Die Erkrankung kann auch im Rahmen einer Abstoßungsreaktion eines Transplantats vorkommen.

Die akute Lungenschädigung (ALI) sowie die ausgeprägtere Form hiervon, das akute Lungenversagen (ARDS), sind schwerwiegende Erkrankungen, die mit einer Mortalität von 50-60% einhergehen. Nach der Definition der North American-European Consensus Conference (NAECC) von 1994 sind ALI und ARDS definiert durch eine akute auslösende Erkrankung, bilaterale radiologisch sichtbare Infiltrate, einen PaO₂FiO₂-Index von ≤ 300 mmHg (ALI) bzw. ≤ 200 mmHg (ARDS), einen pulmonal-kapillären Verschlussdruck von < 18 mmHg bzw. fehlende klinische Hinweise auf linksatriale Hypertension.

Der Entstehung einer akuten Lungenschädigung können sowohl pulmonale als auch extrapulmonale Erkrankungen vorausgehen. Als lungenspezifische prädisponierende Faktoren gelten Aspiration von Mageninhalt, Pneumonien, Rauchgasvergiftung, Lungenkontusion sowie ein Beinahe-Ertrinken. Vor allem Magensaftaspiration und Pneumonien werden häufig als Ausgangserkrankung für ALI/ARDS pulmonalen Ursprungs gesehen. Als indirekte Ereignisse kommen vor allem Polytrauma, Sepsis, mehrfache Bluttransfusionen, akute Pankreatitis und Verbrennungen vor. Die Inzidenz liegt bei 17.9 Fällen für ALI bzw. 13.5 Fällen für ARDS pro 100 000 Einwohner und Jahr [Luhr et al., Am. J. Respir. Crit. Care Med 159, 1849-1861 (1999)].

Eine zentrale Rolle für die Entstehung dieser Erkrankungen stellen die massiven entzündlichen Veränderungen in der Lunge dar, die durch ein weitverzweigtes System von Mediatoren ausgelöst werden. Eine wichtige Rolle bei der Entwicklung der Lungenschädigung spielen auch die neutrophilen Granulozyten, deren Anzahl sich mit dem Andauern des entzündlichen Prozesses ständig erhöht [Chollet-Martin et al., Am. J. Respir. Crit. Care Med 154, 594-601 (1996)]. Die Wirkung der Mediatoren bedingt eine Schädigung der alveolokapillären Membranen, hieraus resultiert eine Permeabilitätserhöhung der alveolär-kapillären Barriere. Durch die Permeabilitätserhöhung kann proteinreiche Flüssigkeit in die Alveolen und auch in das Interstitium eindringen; es bildet sich ein pulmonales Niederdrucködem aus. Charakteristisch für ALI/ARDS ist, dass es sich hierbei um ein nicht-kardiogen induziertes Ödem handelt. Die Ödemflüssigkeit enthält vor allem Fibrin, Erythrozyten, Leukozyten, hyaline Membranen und andere Proteine. Das proteinreiche Exsudat führt zusammen mit den Produkten von aktivierten Neutrophilen zu einer Dysfunktion des Surfactants. Die inflammatorischen Prozesse führen zur Schädigung und zum Verlust von Pneumozyten des Typs II, die Surfactant bilden, so dass ein Herabsinken der Surfactant-Produktion resultiert. Durch den Surfactant-Mangel erhöht sich die Oberflächenspannung in den Alveolen; Alveolen kollabieren, es bilden sich Atelektasen aus. Bei weiter bestehender Perfusion entsteht somit eine Ventilations-Perfusions-Störung, die in einer Erhöhung des pulmonalen Rechts-Links-Shunts mündet. Des Weiteren sinkt die Compliance herab, der alveoläre Totraum hingegen nimmt zu, denn es gibt auch Areale, die zwar belüftet, aber aufgrund einer pulmonalen Hypertension nicht mehr ausreichend perfundiert werden.

In der bronchoalveolaren Waschflüssigkeit von ARDS-Patienten (BALF) konnte eine erhöhte Elastase-Aktivität gemessen werden, die mit dem Schweregrad der Lungenschädigung einhergeht. In Tiermodellen, in denen die Lunge geschädigt wird (z.B. durch Gabe von LPS), kann dieser Effekt nachgestellt werden. Eine Behandlung mit Elastase-Hemmern (z.B. Sivelestat oder Elafin, s.u.) vermindert hier deutlich die Elastase-Aktivität in der BALF und verbessert die Lungenfunktion.

Zur Behandlung einer akuten Lungenschädigung, die mit SIRS assoziiert ist, ist ein Elastase-Hemmer in Japan und Südkorea zugelassen (Sivelestat, Elaspol^{®}). Die reversible, aber reaktive Verbindung besitzt nur eine relativ schwache Wirksamkeit gegenüber der HNE (Kᵢ 200 nM) und wirkt ebenfalls gegenüber der Elastase des Pankreas (IC₅₀ 5.6 µM). Der Wirkstoff wird intravenös verabreicht, eine orale Applikation ist nicht möglich.

Auch Elafin und strukturelle Analoga werden als therapeutisch nutzbare Elastase-Inhibitoren untersucht. Elafin ist ein körpereigenes kleines Protein, welches sowohl Elastase als auch Proteinase 3 hemmt. Aufgrund des proteinergen Charakters ist jedoch eine orale Verabreichung von Elafin nicht möglich.

Die Aufgabe der vorliegenden Erfindung liegt in der Bereitstellung neuer Substanzen, die als niedermolekulare, nicht-reaktive und selektive Inhibitoren der humanen neutrophilen Elastase (HNE) wirken und sich als solche zur Behandlung und/oder Prävention insbesondere von Erkrankungen der Lunge und des Herz-Kreislauf-Systems eignen.

In WO 2004/024700, WO 2004/024701, WO 2005/082863, WO 2005/082864 und WO 2008/ 003412 werden verschiedene 1,4-Diaryl-dihydropyrimidin-2-on-Derivate als HNE-Inhibitoren zur Behandlung von chronisch-obstruktiven Lungenerkrankungen, akutem Koronarsyndrom, Myokardinfarkt, Herzinsuffizienz und pulmonaler Hypertonie offenbart. In WO 2007/129060 und WO 2008/135537 werden Tetrahydropyrrolopyrimidindione als HNE-Inhibitoren beansprucht. Heterocyclisch anellierte Dihydropyrimidine werden in WO 01/40231 als Kaliumkanal-Inhibitoren zur Behandlung atrialer Arrhythmien beschrieben.

Gegenstand der vorliegenden Erfindung sind Verbindungen der allgemeinen Formel (I) 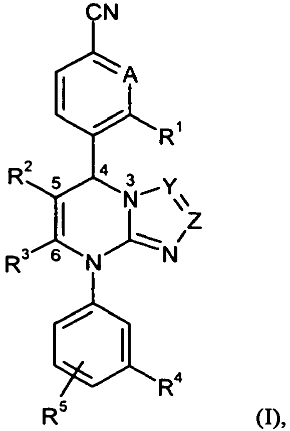 in welcher
- A: für C-R⁶ oder N steht, worin
R⁶ Wasserstoff, Fluor oder Chlor bedeutet,
- Y: für C-R⁷ oder N steht, worin
R⁷ Wasserstoff, (C₁-C₆)-Alkyl, Amino oder eine Gruppe der Formel -NH-C(=O)-R⁸ oder -NH-SO₂-R⁸ bedeutet, worin
R⁸ (C₁-C₆)-Alkyl, (C₃-C₆)-Cycloalkyl oder Phenyl darstellt,
wobei (C₁-C₆)-Alkyl mit Hydroxy, (C₁-C₄)-Alkoxy, (C₃-C₆₎-Cycloalkyl oder Phenyl und bis zu dreifach mit Fluor substituiert sein kann
und wobei
die genannten Cycloalkyl-Gruppen bis zu zweifach, gleich oder verschieden, mit (C₁-C₄)-Alkyl und bis zu zweifach mit Fluor
sowie
die genannten Phenyl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy
substituiert sein können,
- Z: für C-R⁹ oder N steht, worin
R⁹ Wasserstoff, (C₁-C₆)-Alkyl, (C₁-C₆)-Alkoxy, (C₁-C₆)-Alkoxycarbonyl, (C₃-C₆)-Cycloalkyl, Phenyl oder 5- oder 6-gliedriges Heteroaryl bedeutet,
wobei (C₁-C₆)-Alkyl mit Hydroxy, (C₁-C₄)-Alkoxy, (C₃-C₆)-Cycloalkyl oder Phenyl und bis zu dreifach mit Fluor substituiert sein kann
und wobei
die genannten Cycloalkyl-Gruppen bis zu zweifach, gleich oder verschieden, mit (C₁-C₄)-Alkyl und bis zu zweifach mit Fluor
sowie
die genannten Phenyl- und Heteroaryl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy
substituiert sein können,
oder
R⁹ eine Gruppe der Formel -SO₂-NR¹⁰R¹¹ oder -NR¹²R¹³ bedeutet, worin
R¹⁰ und R¹¹ gleich oder verschieden sind und unabhängig voneinander Wasserstoff, (C₁-C₆)-Alkyl, (C₃-C₆)-Cycloalkyl, Phenyl oder 5- oder 6-gliedriges Heteroaryl darstellen,
R¹² Wasserstoff, (C₁-C₆)-Alkyl, (C₃-C₆)-Cycloalkyl, Phenyl oder (C₁-C₆)-Alkylsulfonyl darstellt,
wobei (C₁-C₆)-Alkyl mit Cyano, Hydroxy, (C₁-C₄)-Alkoxy, Hydroxycarbonyl, (C₁-C₄)-Alkoxycarbonyl, Aminocarbonyl, Mono-(C₁-C₄)-alkylamino-carbonyl, Di-(C₁-C₄)-alkylaminocarbonyl, (C₃-C₆)-Cycloalkyl oder Phenyl und bis zu dreifach mit Fluor substituiert sein kann
und wobei
die genannten Cycloalkyl-Gruppen bis zu zweifach, gleich oder verschieden, mit (C₁-C₄)-Alkyl und bis zu zweifach mit Fluor
sowie
die genannten Phenyl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy
substituiert sein können,
R¹³ Wasserstoff, (C₁-C₆)-Alkyl oder eine Gruppe der Formel -C(=O)-R¹⁴, -C(=O)-O-R¹⁵ oder -C(=>O-NR¹⁶R¹⁷ darstellt, worin
R¹⁴ Wasserstoff, (C₁-C₈)-Alkyl, (C₃-C₆)-Cycloalkyl, 4- bis 6-gliedriges Heterocyclyl, Phenyl oder 5- oder 6-gliedriges Heteroaryl bedeutet,
wobei (C₁-C₈)-Alkyl mit Hydroxy, (C₁-C₄)-Alkoxy, Benzyloxy, Phenoxy, (C₁-C₄)-Acyloxy, Amino, Mono-(C₁-C₄)-alkylamino, Di-(C₁-C₄)-alkylamino, (C₁-C₄)-Acylamino, (C₁-C₄)-Alkoxycarbonyl-amino, (C₃-C₆)-Cycloalkyl oder Phenyl und bis zu dreifach mit Fluor substituiert sein kann und bis zu zwei CH₂-Gruppen in (C₁-C₈)-Alkyl, soweit in einer chemisch stabilen Verbindung resultierend, gegen ein Sauerstoffatom ausgetauscht sein können
und wobei
die genannten Cycloalkyl- und Heterocyclyl-Gruppen bis zu zweifach, gleich oder verschieden, mit (C₁-C₄)-Alkyl und bis zu zweifach mit Fluor
sowie
die genannten Phenyl- und Heteroaryl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy, Trifluormethoxy und Hydroxycarbonyl
substituiert sein können,
R¹⁵ (C₁-C₆)-Alkyl, welches mit (C₃-C₆)-Cycloalkyl oder Phenyl substituiert sein kann, bedeutet,
R¹⁶ und R¹⁷ gleich oder verschieden sind und unabhängig voneinander Wasserstoff oder (C₁-C₆)-Alkyl, welches mit Hydroxy oder (C₁-C₄)-Alkoxy substituiert sein kann, bedeuten, oder
R¹² und R¹³ zusammen mit dem Stickstoffatom, an das sie gebunden sind, einen 5-oder 6-gliedrigen Heterocyclus bilden, welcher ein weiteres Ring-Heteroatom aus der Reihe N, O oder S enthalten kann und der bis zu zweifach, gleich oder verschieden, mit (C₁-C₄)-Alkyl, Hydroxy, (C₁-C₄)-Alkoxy und Oxo substituiert und mit einem Phenyl-Ring anelliert sein kann, oder worin
R⁷ und R⁹, sofern beide vorhanden, miteinander verknüpft sind und zusammen mit den Kohlenstoffatomen, an die sie gebunden sind, einen anellierten Phenyl-, Pyridyl- oder Pyrimidyl-Ring bilden, welcher jeweils bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy substituiert sein kann,
- R¹: für Wasserstoff, Halogen, Cyano, Nitro, (C₁-C₆)-Alkyl, Difluormethyl, Trifluormethyl, (C₁-C₆)-Alkoxy, Difluormethoxy, Trifluormethoxy, Amino, Mono- oder Di-(C₁-C₆)-alkyl-amino
oder
für eine Gruppe der Formel -NH-C(=O)-R¹⁸, -NH-C(=O)-NHR¹⁸, -NH-SO₂-R¹⁹ oder -S(O)ₙ-R²⁰ steht, worin
R¹⁸ Wasserstoff oder (C₁-C₆)-Alkyl bedeutet,
R¹⁹ (C₁-C₆)-Alkyl bedeutet,
R²⁰ (C₁-C₆)-Alkyl, das mit Hydroxy, (C₁-C₄)-Alkoxy, Amino, Mono- oder Di-(C₁-C₄)-alkylamino, Hydroxycarbonyl, Aminocarbonyl, (C₃-C₆)-Cycloalkyl oder Phenyl und bis zu dreifach mit Fluor substituiert sein kann, oder (C₂-C₆)-Alkenyl, (C₃-C₆)-Cycloalkyl oder Phenyl bedeutet,
wobei die genannten Cycloalkyl-Gruppen bis zu zweifach, gleich oder verschieden, mit (C₁-C₄)-Alkyl, Hydroxy und (C₁-C₄)-Alkoxy
und
die genannten Phenyl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy substituiert sein können,
und
n die Zahl 0, 1 oder 2 bedeutet,
- R²: für Cyano oder eine Gruppe der Formel -C(=O)-R²¹, -C(=O)-O-R²¹ oder -C(=O)-NH-R²¹ steht, worin
R²¹ Wasserstoff, (C₁-C₆)-Alkyl, (C₃-C₆)-Alkenyl oder (C₃-C₆)-Cycloalkyl bedeutet,
wobei (C₁-C₆)-Alkyl und (C₃-C₆)-Cycloalkyl ihrerseits bis zu zweifach, gleich oder verschieden, mit Hydroxy, (C₁-C₄)-Alkoxy, Hydroxycarbonyl, (C₁-C₄)-Alkoxycarbonyl, Amino, Mono- und Di-(C₁-C₄)-alkylamino substituiert sein können und in (C₁-C₆)-Alkyl und (C₃-C₆)-Cycloalkyl jeweils eine CH₂-Gruppe, soweit in einer chemisch stabilen Verbindung resultierend, gegen ein O-Atom ausgetauscht sein kann,
- R³: für Methyl oder Ethyl steht
oder
- R² und R³: miteinander verknüpft sind und zusammen eine anellierte Gruppe der Formel 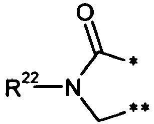 bilden, worin
* die Verknüpfungsstelle mit der in Formel (I) bezeichneten 5-Position des Dihydropyrimidin-Rings
und
** die Verknüpfungsstelle mit der in Formel (I) bezeichneten 6-Position des Dihydropyrimidin-Rings bedeuten und
R²² Wasserstoff, (C₁-C₆)-Alkyl oder (C₃-C₆)-Cycloalkyl bedeutet,
wobei (C₁-C₆)-Alkyl mit Hydroxy, (C₁-C₄)-Alkoxy, Aminocarbonyl, Aminocarbonylamino, (C₁-C₄)-Acylamino oder (C₃-C₆)-Cycloalkyl substituiert sein kann,
- R⁴: für Nitro oder Trifluormethyl steht
und
- R⁵: für Wasserstoff, Fluor oder Chlor steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachstehend aufgeführten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₈)-Alkyl, (C₁-C₆)-Alkyl und (C₁-C₄)-Alkyl stehen im Rahmen der Erfindung für einen geradkettigen oder verzweigten Alkylrest mit 1 bis 8, 1 bis 6 bzw. 1 bis 4 Kohlenstoffatomen. Bevorzugt ist ein geradkettiger oder verzweigter Alkylrest mit 1 bis 6, besonders bevorzugt mit 1 bis 4 Kohlenstoffatomen. Beispielhaft und vorzugsweise seien genannt: Methyl, Ethyl, n-Propyl, Isopropyl, n-Butyl, *iso*-Butyl, sec.-Butyl, *tert*.-Butyl, 1-Ethylpropyl, n-Pentyl, Neopentyl, n-Hexyl, *n*-Heptyl und n-Octyl.
(C₂-C₆)-Alkenyl und (C₃-C₆)-Alkenyl stehen im Rahmen der Erfindung für einen geradkettigen oder verzweigten Alkenylrest mit 2 bis 6 bzw. 3 bis 6 Kohlenstoffatomen und einer oder zwei Doppelbindungen. Bevorzugt ist ein geradkettiger oder verzweigter Alkenylrest mit 3 bis 6 Kohlenstoffatomen und einer Doppelbindung. Beispielhaft und vorzugsweise seien genannt: Allyl, Isopropenyl, *n*-But-2-en-1-yl, *n*-But-3-en-1-yl, *n*-Pent-2-en-1-yl, *n*-Pent-3-en-1-yl, *n*-Pent-4-en-1-yl, 3-Methylbut-2-en-1-yl und 4-Methylpent-3-en-1-yl.
(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, *tert.*-Butoxy, *n*-Pentoxy und n-Hexoxy.
(C₁-C₆)-Alkoxycarbonyl und (C₁-C₄)-Alkoxycarbonyl stehen im Rahmen der Erfindung für einen geradkettigen oder verzweigten Alkoxyrest mit 1 bis 6 bzw. 1 bis 4 Kohlenstoffatomen, der über eine Carbonylgruppe verknüpft ist. Bevorzugt ist eine geradkettige oder verzweigte Alkoxycarbonyl-Gruppe mit 1 bis 4 Kohlenstoffatomen im Alkoxyrest. Beispielhaft und vorzugsweise seien genannt: Methoxycarbonyl, Ethoxycarbonyl, n-Propoxycarbonyl, Isopropoxycarbonyl, *n-*Butoxycarbonyl, *tert*.-Butoxycarbonyl, n-Pentoxycarbonyl und n-Hexoxycarbonyl.
(C₁-C₄)-Alkoxycarbonylamino steht im Rahmen der Erfindung für eine Amino-Gruppe mit einem geradkettigen oder verzweigten Alkoxycarbonyl-Substituenten, der 1 bis 4 Kohlenstoffatome im Alkoxyrest aufweist und über die Carbonylgruppe mit dem N-Atom verknüpft ist. Beispielhaft und vorzugsweise seien genannt: Methoxycarbonylamino, Ethoxycarbonylamino, *n*-Propoxycarbonylamino, Isopropoxycarbonylamino, n-Butoxycarbonylamino und *tert.*-Butoxycarbonylamino.
(C₁-C₆)-Alkylsulfonyl und (C₁-C₄)-Alkylsulfonyl stehen im Rahmen der Erfindung für einen geradkettigen oder verzweigten Alkylsulfonyl-Rest mit 1 bis 6 bzw. 1 bis 4 Kohlenstoffatomen. Bevorzugt ist ein geradkettiger oder verzweigter Alkylsulfonyl-Rest mit 1 bis 4 Kohlenstoffatomen. Beispielhaft und vorzugsweise seien genannt: Methylsulfonyl, Ethylsulfonyl, *n*-Propylsulfonyl, Isopropylsulfonyl, *n*-Butylsulfonyl, *tert.*-Butylsulfonyl, *n*-Pentylsulfonyl und *n*-Hexylsulfonyl.
Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohtenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, *n*-Propylamino, Isopropylamino, n-Butylamino, *tert.*-Butylamino, *n*-Pentylamino und *n*-Hexylamino.
Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N-*Dimethylamino, *N,N-*Diethylamino, *N-*Ethyl-*N-*methylamino, *N*-Methyl-*N*-*n*-propylamino, *N*-Isopropyl-*N*-methylamino, *N*-Isopropyl-*N-n*-propylamino, *N,N-*Diisopropylamino, *N*-*n*-Butyl-*N*-methylamino, *N*-tert.-Butyl-*N-*methylamino, *N-*Methyl*-N-n-*pentylamino und *N-n-*Hexyl-*N*-methylamino.
Mono- bzw. Di-(C₁-C₄)-akylaminocarbonyl steht im Rahmen der Erfindung für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit jeweils 1 bis 4 Kohlenstoffatomen aufweist. Beispielhaft und vorzugsweise seien genannt: Methylaminocarbonyl, Ethylaminocarbonyl, *n*-Propylaminocarbonyl, Isopropylaminocarbonyl, *n*-Butylaminocarbonyl, *tert*.-Butylaminocarbonyl, *N,N-*Dimethylaminocarbonyl, *N,N-*Diethylaminocarbonyl, *N-*Ethyl-*N-*methylaminocarbonyl, *N-*Methyl-*N-n*-propylaminocarbonyl, *N-*Isopropyl-*N-*methylaminocarbonyl, *N,N*-Diisopropylaminocarbonyl, *N-n*-Butyl-*N-*methylaminocarbonyl und *N-tert.-*Butyl-*N-*methylaminocarbonyl.
(C₁-C₄)-Acyl [(C₁-C₄)-Alkanoyl] steht im Rahmen der Erfindung für einen geradkettigen oder verzweigten Alkylrest mit 1 bis 4 Kohlenstoffatomen, der in der 1-Position ein doppelt gebundenes Sauerstoffatom trägt und über die 1-Position verknüpft ist. Beispielhaft und vorzugsweise seien genannt: Formyl, Acetyl, Propionyl, *n*-Butyryl und *iso*-Butyryl.
(C₁-C₄)-Acylamino steht im Rahmen der Erfindung für eine Amino-Gruppe mit einem geradkettigen oder verzweigten Acyl-Substituenten, der 1 bis 4 Kohlenstoffatome aufweist und über die Carbonylgruppe mit dem N-Atom verknüpft ist. Beispielhaft und vorzugsweise seien genannt: Formylamino, Acetylamino, Propionylamino, *n*-Butyrylamino und *iso*-Butyrylamino.
(C₁-C₄)-Acyloxy steht im Rahmen der Erfindung für einen geradkettigen oder verzweigten Alkylrest mit 1 bis 4 Kohlenstoffatomen, der in der 1-Position ein doppelt gebundenes Sauerstoffatom trägt und in der 1-Position über ein weiteres Sauerstoffatom verknüpft ist. Beispielhaft und vorzugsweise seien genannt: Acetoxy, Propionoxy, *n*-Butyroxy und *iso*-Butyroxy.
(C₃-C₆)-Cycloalkyl steht im Rahmen der Erfindung für eine monocyclische, gesättigte Cycloalkylgruppe mit 3 bis 6 Ring-Kohlenstoffatomen. Beispielhaft und vorzugsweise seien genannt: Cyclopropyl, Cyclobutyl, Cyclopentyl und Cyclohexyl.
4- bis 6-gliedriges Heterocyclyl steht im Rahmen der Erfindung für einen monocyclischen, gesättigten Heterocyclus mit insgesamt 4 bis 6 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Bevorzugt ist ein 5- oder 6-gliedriger Heterocyclus mit ein oder zwei Ring-Heteroatomen aus der Reihe N, O und/oder S. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl und Thiomorpholinyl. Bevorzugt sind Pyrrolidinyl, Tetrahydrofuranyl, Piperidinyl, Piperazinyl, Tetrahydropyranyl und Morpholinyl.
5- oder 6-gliedriges Heteroaryl steht im Rahmen der Erfindung für einen aromatischen Heterocyclus (Heteroaromaten) mit insgesamt 5 oder 6 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Furyl, Pyrrolyl, Thienyl, Pyrazolyl, Imidazolyl, Oxazolyl, Thiazolyl, Isoxazolyl, Isothiazolyl, Pyridyl, Pyrimidinyl, Pyridazinyl und Pyrazinyl. Bevorzugt sind Thienyl, Pyrazolyl, Imidazolyl, Oxazolyl, Thiazolyl, Pyridyl und Pyrimidinyl.
Halogen schließt im Rahmen der Erfindung Fluor, Chlor, Brom und Iod ein. Bevorzugt sind Chlor, Fluor oder Brom, besonders bevorzugt Fluor oder Chlor.
Ein Oxo-Substituent steht im Rahmen der Erfindung für ein Sauerstoffatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder zwei gleichen oder verschiedenen Substituenten ist bevorzugt. Ganz besonders bevorzugt ist die Substitution mit einem Substituenten.

Bevorzugt sind Verbindungen der Formel (I), in welcher
- A: für CH oder N steht,
sowie ihre Salze, Solvate und Solvate der Salze.

Bevorzugt sind auch Verbindungen der Formel (I), in welcher
- Y: für C-R⁷
und
- Z: für N steht,
sowie ihre Salze, Solvate und Solvate der Salze.

Bevorzugt sind auch Verbindungen der Formel (I), in welcher
- Y: für N
und
- Z: für C-R⁹ steht,
sowie ihre Salze, Solvate und Solvate der Salze.

Bevorzugt sind auch Verbindungen der Formel (**I**), in welcher
Y und Z beide für N stehen,
sowie ihre Salze, Solvate und Solvate der Salze.

Bevorzugt sind auch Verbindungen der Formel (**I**), in welcher
- R¹: für Wasserstoff, Fluor, Chlor, Cyano, Nitro, (C₁-C₄)-Alkyl, Difluormethyl, Trifluormethyl, (C₁-C₄)-Alkoxy, Difluormethoxy, Trifluormethoxy oder eine Gruppe der Formel -SO₂-R²⁰ steht,
sowie ihre Salze, Solvate und Solvate der Salze.

Bevorzugt sind auch Verbindungen der Formel (I), in welcher
- R²: für Cyano oder eine Gruppe der Formel -C(=O)-R²¹, -C(=O)-O-R²¹, -C(=O)-NH₂ oder -C(=O)-NH-R²¹ steht, worin
- R²¹: (C₁-C₄)-Alkyl, das mit Hydroxy substituiert sein kann, bedeutet,
und
- R³: für Methyl steht,
sowie ihre Salze, Solvate und Solvate der Salze.

Bevorzugt sind auch Verbindungen der Formel (I), in welcher
- R⁴: für Trifluormethyl
und
- R⁵: für Wasserstoff steht,
sowie ihre Salze, Solvate und Solvate der Salze.

Besonders bevorzugt sind Verbindungen der Formel (I), in welcher
- A: für CH steht,
- Y: für C-R⁷ oder N steht,
- Z: für C-R⁹ oder N steht,
wobei mindestens eines der beiden Ringglieder Y und Z für N steht
und worin
R⁷ Wasserstoff, Amino oder eine Gruppe der Formel -NH-C(=O)-R⁸ oder -NH-SO₂-R⁸ bedeutet, worin
R⁸ (C₁-C₄)-Alkyl oder (C₃-C₆)-Cycloalkyl darstellt,
wobei (C₁-C₄)-Alkyl mit Hydroxy, (C₁-C₄)-Alkoxy oder (C₃-C₆)-Cycloalkyl und bis zu dreifach mit Fluor substituiert sein kann
und
die genannten Cycloalkyl-Gruppen bis zu zweifach, gleich oder verschieden, mit (C₁-C₄)-Alky) und bis zu zweifach mit Fluor substituiert sein können, und
R⁹ Wasserstoff, (C₁-C₄)-Alkyl, (C₁-C₄)-Alkoxy, (C₁-C₄)-Alkoxycarbonyl, (C₃-C₆)-Cycloalkyl oder 5- oder 6-gliedriges Heteroaryl bedeutet,
wobei (C₁-C₄)-Alkyl mit Hydroxy, (C₁-C₄)-Alkoxy oder (C₃-C₆)-Cycloalkyl und bis zu dreifach mit Fluor substituiert sein kann
und wobei
die genannten Cycloalkyl-Gruppen bis zu zweifach, gleich oder verschieden, mit (C₁-C₄)-Alkyl und bis zu zweifach mit Fluor
sowie
die genannte Heteroaryl-Gruppe bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy
substituiert sein können,
oder
R⁹ eine Gruppe der Formel -SO₂-NR¹⁰R¹¹ oder -NR¹²R¹³ bedeutet, worin
R¹⁰ und R¹¹ gleich oder verschieden sind und unabhängig voneinander Wasserstoff, (C₁-C₄)-Alkyl oder (C₃-C₆)-Cycloalkyl darstellen,
R¹² Wasserstoff, (C₁-C₄)-Alkyl, (C₃-C₆)-Cycloalkyl oder (C₁-C₄)-Alkylsulfonyl darstellt,
wobei (C₁-C₄)-Alkyl mit Cyano, Hydroxy, (C₁-C₄)-Alkoxy, Hydroxycarbonyl, (C₁-C₄)-Alkoxycarbonyl, Aminocarbonyl, Mono-(C₁-C₄)-alkylamino-carbonyl, Di-(C₁-C₄)-alkylaminocarbonyl oder (C₃-C₆)-Cycloalkyl und bis zu dreifach mit Fluor substituiert sein kann
und
die genannten Cycloalkyl-Gruppen bis zu zweifach, gleich oder verschieden, mit (C₁-C₄)-Alkyl und bis zu zweifach mit Fluor substituiert sein können,
R¹³ Wasserstoff, (C₁-C₄)-Alkyl oder eine Gruppe der Formel -C(=O)-R¹⁴ -C(=O)-O-R¹⁵ oder -C(=O)-NR¹⁶R¹⁷ darstellt, worin
R¹⁴ (C₁-C₆)-Alkyl, (C₃-C₆)-Cycloalkyl, 4- bis 6-gliedriges Heterocyclyl oder 5- oder 6-gliedriges Heteroaryl bedeutet,
wobei (C₁-C₆)-Alkyl mit Hydroxy, (C₁-C₄)-Alkoxy, (C₁-C₄)-Acyl-oxy, Amino, Mono-(C₁-C₄)-alkylamino, Di-(C₁-C₄)-alkylamino, (C₁-C₄)-Acylamino, (C₁-C₄)-Alkoxycarbonylamino oder (C₃-C₆)-Cycloalkyl und bis zu dreifach mit Fluor substituiert sein kann
und wobei
die genannten Cycloalkyl- und Heterocyclyl-Gruppen bis zu zweifach, gleich oder verschieden, mit (C₁-C₄)-Alkyl und bis zu zweifach mit Fluor
sowie
die genannte Heteroaryl-Gruppe bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy
substituiert sein können,
R¹⁵ (C₁-C₄)-Alkyl, welches mit (C₃-C₆)-Cycloalkyl oder Phenyl substituiert sein kann, bedeutet, und
R¹⁶ und R¹⁷ gleich oder verschieden sind und unabhängig voneinander Wasserstoff oder (C₁-C₄)-Alkyl, welches mit Hydroxy oder (C₁-C₄)-Alkoxy substituiert sein kann, bedeuten,
- R¹: für Wasserstoff, Fluor, Chlor, Nitro, Methyl, Difluormethyl, Trifluormethyl oder eine Gruppe der Formel -SO₂-R²⁰ steht, worin
R²⁰ (C₁-C₄)-Alkyl, das mit Hydroxy, Methoxy oder Ethoxy oder bis zu dreifach mit Fluor substituiert sein kann, bedeutet,
- R²: für Cyano oder eine Gruppe der Formel -C(=O)-R²¹ oder -C(=O)-O-R²¹ steht, worin
R²¹ Methyl, Ethyl oder 2-Hydroxyethyl bedeutet,
- R³: für Methyl steht,
- R⁴: für Trifluormethyl steht
und
- R⁵: für Wasserstoff steht,
sowie ihre Salze, Solvate und Solvate der Salze.

Insbesondere bevorzugt sind Verbindungen der Formel (**I**), in welcher
- A: für CH steht,
- Y: für C-R⁷ oder N steht,
- Z: für C-R⁹ oder N steht,
wobei entweder Y für C-R⁷ und Z für N oder Y für N und Z für C-R⁹ stehen
und worin
R⁷ Amino oder eine Gruppe der Formel -NH-C(=O)-R⁸ bedeutet, worin
R⁸ (C₁-C₄)-Alkyl oder (C₃-C₆)-Cycloalkyl darstellt, wobei (C₁-C₄)-Alkyl mit Hydroxy, Methoxy, Ethoxy oder (C₃-C₆)-Cycloalkyl und bis zu dreifach mit Fluor substituiert sein kann
und
die genannten Cycloalkyl-Gruppen bis zu zweifach mit Methyl und bis zu zweifach mit Fluor substituiert sein können, und
R⁹ (C₁-C₄)-Alkyl, (C₁-C₄)-Alkoxy oder (C₃-C₆)-Cycloalkyl bedeutet,
wobei (C₁-C₄)-Alkyl mit Hydroxy, Methoxy, Ethoxy oder (C₃-C₆)-Cycloalkyl und bis zu dreifach mit Fluor substituiert sein kann
und
die genannten Cycloalkyl-Gruppen bis zu zweifach mit Methyl und bis zu zweifach mit Fluor substituiert sein können,
oder
R⁹ eine Gruppe der Formel -SO₂-NR¹⁰R¹¹ oder -NR¹²R¹³ bedeutet, worin
R¹⁰ und R¹¹ gleich oder verschieden sind und unabhängig voneinander Wasserstoff, Methyl, Ethyl oder Cyclopropyl darstellen,
R¹² Wasserstoff, (C₁-C₄)-Alkyl, (C₃-C₆)-Cycloalkyl oder (C₁-C₄)-Alkylsulfonyl darstellt,
wobei (C₁-C₄)-Alkyl mit Cyano, Hydroxy, (C₁-C₄)-Alkoxy, Hydroxycarbonyl, (C₁-C₄)-Alkoxycarbonyl, Aminocarbonyl, Mono-(C₁-C₄)-alkylamino-carbonyl, Di-(C₁-C₄)-alkylaminocarbonyl oder (C₃-C₆)-Cycloalkyl und bis zu dreifach mit Fluor substituiert sein kann
und
die genannten Cycloalkyl-Gruppen bis zu zweifach mit Methyl und bis zu zweifach mit Fluor substituiert sein können,
R¹³ Wasserstoff oder eine Gruppe der Formel -C(=O)-R¹⁴ oder -C(=O)-NR¹⁶R¹⁷ darstellt, worin
R¹⁴ (C₁-C₄)-Alkyl, (C₃-C₆)-Cycloalkyl oder 5- oder 6-gliedriges Heteroaryl bedeutet,
wobei (C₁-C₄)-Alkyl mit Hydroxy, (C₁-C₄)-Alkoxy, Amino, Mono-(C₁-C₄)-alkylamino, Di-(C₁-C₄)-alkylamino, (C₁-C₄)-Acylamino oder (C₃-C₆)-Cycloalkyl und bis zu dreifach mit Fluor substituiert sein kann
und wobei
die genannten Cycloalkyl-Gruppen bis zu zweifach mit Methyl und bis zu zweifach mit Fluor
sowie
die genannte Heteroaryl-Gruppe bis zu zweifach, gleich oder verschieden, mit Fluor, Cyano, Methyl, Difluormethyl, Trifluormethyl, Methoxy und Trifluormethoxy
substituiert sein können, und
R¹⁶ und R¹⁷ gleich oder verschieden sind und unabhängig voneinander Wasserstoff oder (C₁-C₄)-Alkyl, welches mit Hydroxy, Methoxy oder Ethoxy substituiert sein kann, bedeuten,
- R¹: für Wasserstoff, Nitro, Trifluormethyl, Methylsulfonyl oder Trifluormethylsulfonyl steht,
- R²: für Cyano, Acetyl, Ethoxycarbonyl oder (2-Hydroxyethoxy)carbonyl steht,
- R³: für Methyl steht,
- R⁴: für Trifluormethyl steht
und
- R⁵: für Wasserstoff steht,
sowie ihre Salze, Solvate und Solvate der Salze.
Ganz besonders bevorzugt sind Verbindungen der Formel (I), in welcher
- A: für CH steht,
- Y: für N steht,
- Z: für C-R⁹ steht, worin
R⁹ eine Gruppe der Formel -NHR¹² oder -NH-C(=O)-R¹⁴ bedeutet, worin
R¹² eine Gruppe der Formel -CH₂-C(=O)-OH oder -CH₂-C(=O)-NH₂ darstellt
und
R¹⁴ (C₃-C₆)-Cycloalkyl, das bis zu zweifach mit Methyl und bis zu zweifach mit Fluor substituiert sein kann, oder (C₁-C₄)-Alkyl darstellt,
- R¹: für Wasserstoff oder Methylsulfonyl steht,
- R²: für Cyano steht,
- R³: für Methyl steht,
- R⁴: für Trifluormethyl steht
und
- R⁵: für Wasserstoff steht,
sowie ihre Salze, Solvate und Solvate der Salze.

Von besonderer Bedeutung sind Verbindungen gemäß Formel (I) mit der in Formel (I*-ent*) wiedergegebenen Konfiguration an der 4-Position des Dihydropyrimidin-Rings 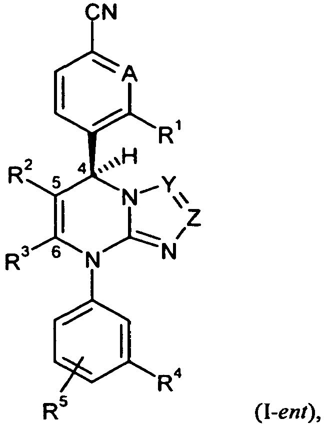 worin A, Y, Z, R¹, R², R³, R⁴ und R⁵ jeweils die oben angegebenen Bedeutungen haben,
und ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man zunächst eine Verbindung der Formel (II) 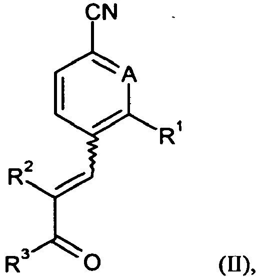 in welcher A, R¹, R² und R³ jeweils die oben angegebenen Bedeutungen haben,
in Gegenwart einer Base mit einer Verbindung der Formel (III) 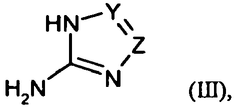 in welcher Y und Z die oben angegebenen Bedeutungen haben,
zu einem Intermediat der Formel (IV) 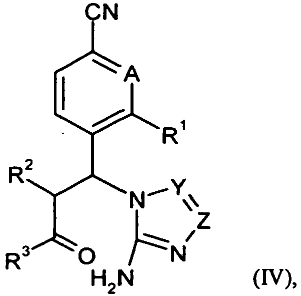 in welcher A, Y, Z, R¹, R² und R³ jeweils die oben angegebenen Bedeutungen haben,
umsetzt, dieses dann *in situ* oder in einem separaten, säurekatalysierten Reaktionsschritt zu einer Verbindung der Formel (V) 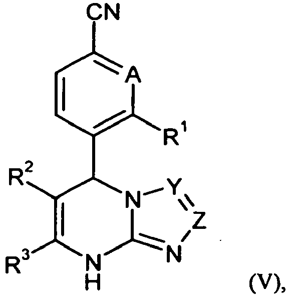 in welcher A, Y, Z, R', R² und R³ jeweils die oben angegebenen Bedeutungen haben,
cyclisiert und die Verbindung (V) anschließen in Gegenwart eines Kupfer(II)-Katalysators und einer Base mit einer Phenylboronsäure der Formel (VI) 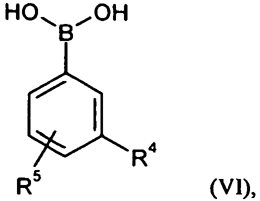 in welcher R⁴ und R⁵ die oben angegebenen Bedeutungen haben,
zu einer Verbindung der Formel (I) kuppelt
und gegebenenfalls die so erhaltenen Verbindungen der Formel (I) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

Für den Verfahrensschritt (II) + (III) → (IV) geeignete Lösungsmittel sind übliche organische Lösungsmittel, die sich unter den Reaktionsbedingungen nicht verändern. Dazu zählen insbesondere Halogenkohlenwasserstoffe wie Dichlormethan, Trichlormethan, Tetrachlormethan, 1,2-Dichlorethan oder Chlorbenzol, oder Lösungsmittel wie Pyridin, Dimethylsulfoxid (DMSO), *N,N-*Dimethylformamid (DMF), *N,N-*Diethylformamid, *N,N-*Dimethylacetamid (DMA), *N,N'*-Dimethylpropylenharnstoff (DMPU) oder *N*-Methylpyrrolidinon (NMP). Ebenso ist es möglich, Gemische der genannten Lösungsmittel einzusetzen. Bevorzugt werden Dichlormethan, Pyridin, *N,N-*Dimethylformamid oder Gemische hiervon verwendet.

Als Base für den Verfahrensschritt (II) + (III) → (IV) eignen sich insbesondere Prolin (in racemischer oder enantiomerenreiner Form) sowie übliche organische Aminbasen wie beispielsweise Triethylamin, *N,N-*Düsopropylethylamin, Piperidin, *N*-Methylpiperidin oder *N*-Methylmorpholin. Bevorzugt wird D,L-Prolin eingesetzt.

Der Verfahrensschritt (II) + (III) → (IV) wird im Allgemeinen in einem Temperaturbereich von 0°C bis +1 00°C, bevorzugt bei +20°C bis +50°C durchgeführt. Die Umsetzung kann bei normalem, erhöhtem oder erniedrigtem Druck erfolgen (z.B. von 0.5 bis 5 bar); in der Regel arbeitet man bei Normaldruck.

Die Cyclisierung im Verfahrensschritt (IV) → (V) wird bevorzugt in Lösungsmitteln wie Benzol, Toluol, Xylol oder Pyridin oder in Gemischen hiervon bei der jeweiligen Rückfluss-Temperatur durchgeführt. Gegebenenfalls kann eine Reaktionsführung unter Mikrowellen-Bestrahlung von Vorteil sein.

Als Säure-Katalysator für diese Reaktion eignen sich übliche anorganische oder organische Säuren wie beispielsweise Schwefelsäure, Chlorwasserstoff/Salzsäure, Trifluoressigsäure, Methansulfonsäure, Trifluormethansulfonsäure, Camphersulfonsäure, Benzolsulfonsäure, *p*-Toluolsulfonsäure oder Pyridinium-*p*-toluolsulfonat. Bevorzugt wird *p*-Toluolsulfonsäure verwendet.

Bei ausreichend elektronenreichen Verbindungen der Formel (III) kann die Reaktionssequenz (II) + (III) → (IV) → (V) auch in einem Schritt erfolgen. Als Base für eine solche einstufige Reaktionsführung sind insbesondere schwache Basen wie Natrium- oder Kaliumhydrogencarbonat oder Natrium- oder Kaliumhydrogenphosphat geeignet. Bevorzugt wird Natriumhydrogencarbonat eingesetzt.

Die Umsetzung wird vorzugsweise in dipolar-aprotischen Lösungsmitteln wie Dimethylsulfoxid (DMSO), *N,N-*Dimethylformamid (DMF), *N,N-*Diethylformamid, *N,N-*Dimethylacetamid (DMA), *N,N'*-Dimethylpropylenharnstoff (DMPU) oder *N*-Methylpyrrolidinon (NMP) in einem Temperaturbereich von +50°C bis +80°C durchgeführt. Bevorzugt wird *N,N-*Dimethylformamid verwendet. Die Reaktion kann bei normalem, erhöhtem oder erniedrigtem Druck erfolgen (z.B. von 0.5 bis 5 bar); im Allgemeinen arbeitet man bei Normaldruck.

Inerte Lösungsmittel für den Verfahrensschritt (V) + (VI) → (I) sind beispielsweise Ether wie Diethylether, Düsopropylether, Methyl-*tert*.-butylether, 1,2-Dimethoxyethan, Dioxan oder Tetrahydrofuran, Kohlenwasserstoffe wie Pentan, Hexan, Cyclohexan, Benzol, Toluol oder Xylol, Halogenkohlenwasserstoffe wie Dichlormethan, 1,2-Dichlorethan oder Trichlormethan, oder andere Lösungsmittel wie Pyridin, Acetonitril, Dimethylsulfoxid (DMSO), *N,N*-Dimethylformamid (DMF), *N,N'*-Dimethylpropylenharnstoff (DMPU) oder *N*-Methylpyrrolidinon (NMP). Ebenso ist es möglich, Gemische der genannten Lösungsmittel einzusetzen. Bevorzugt wird Dichlormethan oder ein Gemisch aus Dichlormethan und Pyridin verwendet.

Als Base für den Verfahrensschritt (V) + (VI) → (I) eignen sich übliche anorganische oder organische Basen. Hierzu gehören insbesondere Alkalihydrogencarbonate wie Natrium- oder Kaliumhydrogencarbonat, Alkali- oder Erdalkalicarbonate wie Lithium-, Natrium-, Kalium-, Calcium-oder Cäsiumcarbonat, Alkalihydrogenphosphate wie Dinatrium- oder Dikaliumhydrogenphosphat, Alkali- oder Erdalkalihydroxide wie Lithium-, Natrium-, Kalium- oder Bariumhydroxid, Amide wie Lithium- oder Kalium-bis(trimethylsilyl)amid oder Lithiumdiisopropylamid (LDA), oder organische Aminbasen wie Triethylamin, *N*-Methylmorpholin, *N*-Methylpiperidin, *N,N-*Diisopropylethylamin, 1,5-Diazabicyclo[4.3.0]non-5-en (DBN), 1,8-Diazabicyclo[5.4.0]undec-7-en (DBU), Pyridin oder 2,6-Lutidin. Bevorzugt wird Triethylamin, gegebenenfalls in Kombination mit Pyridin, verwendet.

Als Katalysator für die Übergangsmetall-vermittelte N-Arylierungsreaktion (V) + (VI) → (I) werden vorzugsweise Kupfer(II)-Salze wie Kupfer(II)acetat, -carbonat, -chlorid oder -sulfat, optional in Verbindung mit aktiviertem elementaren Kupfer, eingesetzt. Alternativ können auch Palladium(0)- oder Palladium(II)-Verbindungen wie beispielsweise Palladium-schwarz, Tetrakis(triphenylphosphin)palladium(0), Bis(dibenzylidenaceton)palladium(0), Palladium(II)chlorid, Palladium(11)acetat oder Palladium(II)trifluoracetat verwendet werden, gegebenenfalls in Kombination mit Komplexliganden wie Acetonitril, Benzonitril, Tri-n-butylphosphin, Tri-*tert*.-butylphosphin, Triphenylphosphin, Tri-o-tolylphosphin, 1,2-Bis(diphenylphosphino)ethan, 1,1'-Bis(diphenylphosphino)ferrocen oder Dicyclohexyl-(2',4',6'-trüsopropylbiphenyl-2-yl)-phosphin. Auch Palladium-Kupfer-Mischsysteme sind geeignet. Bevorzugt wird Kupfer(II)acetat allein oder in Kombination mit aktiviertem Kupfer, als Katalysator eingesetzt.

Die Kupplungsreaktion (V) + (VI) → (I) wird im Allgemeinen in einem Temperaturbereich von +20°C bis +150°C, bevorzugt bei +20°C bis +60°C durchgeführt. Eine Umsetzung unter gleichzeitiger Mikrowellen-Bestrahlung kann gegebenenfalls von Vorteil sein.

Im Falle, dass in der Verbindung der Formel (V) Z für C-R⁹ steht, worin R⁹ Amino bedeutet, ist es erforderlich, diese Amino-Gruppe vor der Kupplungsreaktion mit der Phenylboronsäure (VI) durch eine Schutzgruppe zu desaktivieren. Hierfür kommen übliche Amino-Schutzgruppen in Betracht, wie beispielsweise eine Phthalimid-, Trityl-, Benzyliden-, Diphenylmethylen-, Trifluoracetyl-, Benzyloxycarbonyl- oder *tert.*-Butoxycarbonyl-Gruppe. Einführung und Entfernung dieser Schutzgruppen erfolgen nach bekannten, dem Fachmann geläufigen Methoden [siehe z.B. T.W. Greene und P.G.M. Wuts, Protective Groups in Organic Synthesis, Wiley, New York, 1999]. Bevorzugt wird eine Phthalimid- oder eine Trifluoracetyl-Gruppe verwendet.

Einige der nach Kupplung mit der Phenylboronsäure (VI) resultierenden, solcherart N-geschützten Verbindungen zeigen ebenfalls signifikante HNE-inhibitorische Wirkung und sind insofern auch vom Bedeutungsumfang der vorliegenden Erfindung, d.h. den Verbindungen der Formel (I) umfasst.

Anstelle der Phenylboronsäure (VI) können bei diesen N-Arylierungsreaktionen auch entsprechende Phenylbromide, -iodide oder -trifluormethansulfonate als Kupplungspartner eingesetzt werden. Die oben beschriebenen Reaktionsparameter, wie Lösungsmittel, Basen, Katalysatoren, Reaktionstemperaturen und etwaige Schutzgruppen, finden hierbei in analoger Weise Anwendung.

Die Verbindungen der Formel (II) sind nach literaturbekannten Verfahren durch Säure- und/oder Base-katalysierte Kondensation eines Aldehyds der Formel (VII) mit einer Keto-Verbindung der Formel (VIII) 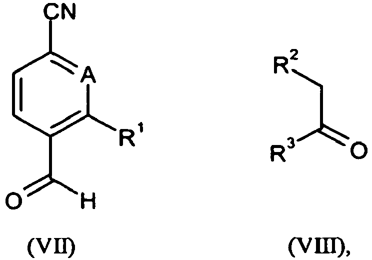 in welchen A und R¹ bzw. R² und R³ die oben angegebenen Bedeutungen haben,
zugänglich [vgl. auch die nachfolgenden Reaktionsschemata 4, 5 und 7].

Erfindungsgemäße Verbindungen der Formel (I), in welcher R² und R³ miteinander verbunden sind und zusammen eine anellierte Gruppe der Formel 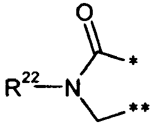 bilden,
worin * und ** die oben beschriebenen Verknüpfungspunkte darstellen und R²² die oben angegebene Bedeutung hat,
können auch dadurch hergestellt werden, dass man eine Verbindung der Formel (I-A) 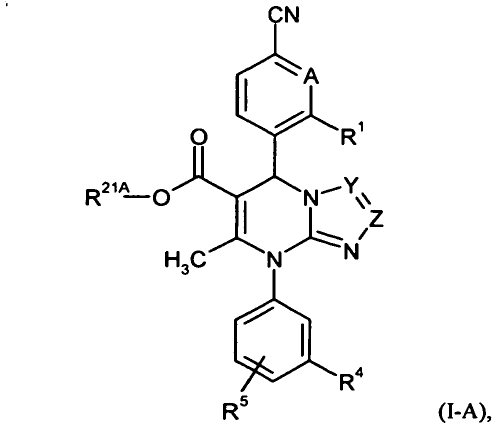 in welcher A, Y, Z, R¹, R⁴ und R⁵ jeweils die oben angegebenen Bedeutungen haben
und
R^{21A} für (C₁-C₆)-Alkyl, (C₃-C₆)-Alkenyl oder (C₃-C₆)-Cycloalkyl steht,
in einem inerten Lösungsmittel zu einer Verbindung der Formel (IX) 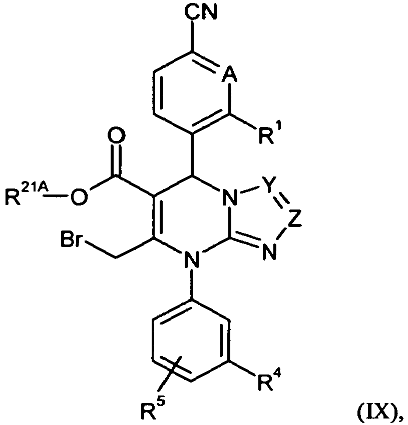 in welcher A, Y, Z, R¹, R⁴, R⁵ und R^{21A} jeweils die oben angegebenen Bedeutungen haben,
bromiert und dann mit einer Verbindung der Formel (X)

R²²-NH₂ (X),

in welcher R²² die oben angegebene Bedeutung hat,
unter Cyclisierung zu einer Verbindung der Formel (I-B) 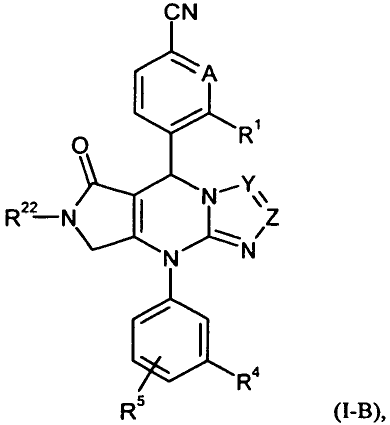 in welcher A, Y, Z, R¹, R⁴, R⁵ und R²² jeweils die oben angegebenen Bedeutungen haben,
umsetzt.

Die Bromierung im Verfahrensschritt (I-A) → (IX) wird bevorzugt mit elementarem Brom in einem üblichen inerten Lösungsmittel wie Chloroform bei einer Temperatur von -20°C bis +40°C durchgeführt. Eine im Rest R^{21A} gegebenenfalls vorhandene CC-Doppelbindung [R^{21A} = (C₃-C₆)-Alkenyl] kann unter diesen Reaktionsbedingungen gleichfalls bromiert werden, dies hat jedoch keine störende Einwirkung bei der nachfolgenden Ringschluss-Reaktion mit der Verbindung (X).

Die Lactam-Bildung im Verfahrensschritt (IX) + (X) → (I-B) wird vorzugsweise in Aceton oder einem Ether wie Tetrahydrofuran oder Dioxan als inertem Lösungsmittel bei einer Temperatur von -20°C bis +60°C durchgeführt. Gegebenenfalls kann die Verwendung eines tertiären Amins, wie Triethylamin, *N*-Methylmorpholin, *N*-Methylpiperidin oder *N,N-*Diisopropylethylamin, als Hilfsbase von Vorteil sein.

Die Verbindung der Formel (I-A) ihrerseits ist analog zu den zuvor beschriebenen Umsetzungen (II) + (III) → (IV) → (V) und (V) + (VI) → (I) erhältlich.

Erfindungsgemäße Verbindungen der Formel (I), in welcher Y für C-R⁷ steht, worin R⁷ Amino bedeutet, und Z für N steht, können hergestellt werden, indem man eine Verbindung der Formel (XI) 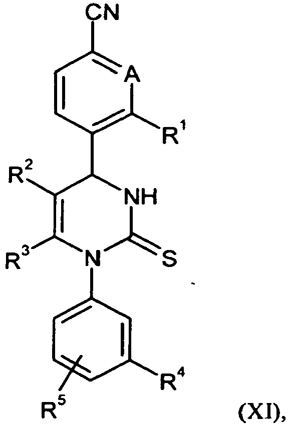 in welcher A, R¹, R², R³, R⁴ und R⁵ jeweils die oben angegebenen Bedeutungen haben,
mit Hydrazinhydrat in Gegenwart eines geeigneten Oxidationsmittels in eine Verbindung der Formel (XII) 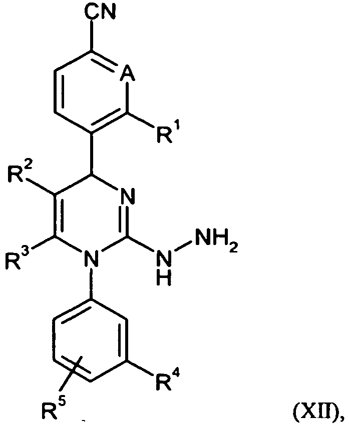 in welcher A, R¹, R², R³, R⁴ und R⁵ jeweils die oben angegebenen Bedeutungen haben,
überführt und diese dann mit Bromcyan unter Cyclisierung zu einer Verbindung der Formel (I-C) 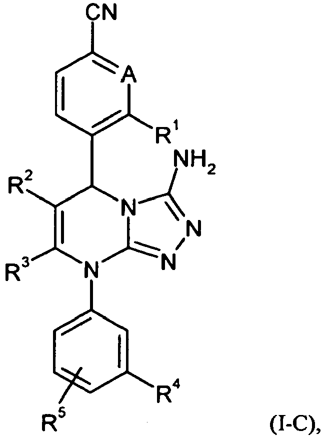 in welcher A, R¹, R², R³, R⁴ und R⁵ jeweils die oben angegebenen Bedeutungen haben,
umsetzt.

Die Entschwefelung im Zuge des Verfahrensschritts (XI) → (XII) wird vorzugsweise unter oxidativen Bedingungen durchgeführt. Als Oxidationsmittel eignet sich insbesondere tert.-Butylhydroperoxid (TBHP); alternativ kann beispielsweise auch Wasserstoffperoxid oder m-Chlorperbenzoesäure verwendet werden.

Als inerte Lösungsmittel für diese Reaktion eignen sich insbesondere Alkohole wie Methanol, Ethanol, *n*-Propanol, Isopropanol, *n*-Butanol oder *tert*.-Butanol, oder Kohlenwasserstoffe wie Pentan, Hexan, Nonan, Decan, Cyclohexan, Benzol, Toluol oder Xylol, oder auch Wasser. Ebenso ist es möglich, Gemische solcher Lösungsmittel einzusetzen. Bevorzugt wird Toluol im Gemisch mit Methanol oder Ethanol verwendet.

Die Transformation (XI) → (XII) wird in der Regel in einem Temperaturbereich von -20°C bis +100°C, bevorzugt bei 0°C bis +60°C durchgeführt. Eine Reaktionsführung unter Mikrowellen-Bestrahlung kann gegebenenfalls von Vorteil sein.

Inerte Lösungsmittel für die Umsetzung mit Bromcyan im Verfahrensschritt (XII) → (I-C) sind beispielsweise Alkohole wie Methanol, Ethanol, *n*-Propanol, Isopropanol, *n*-Butanol, *tert*.-Butanol oder 2-Methoxyethanol, Ether wie Diethylether, Diisopropylether, Methyl-*tert*.-butylether, 1,2-Dimethoxyethan, Dioxan oder Tetrahydrofuran, Halogenkohlenwasserstoffe wie Dichlormethan, 1,2-Dichlorethan oder Trichlormethan, oder dipolar-aprotische Lösungsmittel wie Aceton, Acetonitril, Dimethylsulfoxid (DMSO), *N,N-*Dimethylformamid (DMF), *N,N'*-Dimethylpropylenharnstoff (DMPU) oder *N*-Methylpyrrolidinon (NMP), sowie Gemische dieser Lösungsmittel. Bevorzugt wird Methanol eingesetzt.

Die Reaktion mit Bromcyan und die nachfolgende Cyclisierung zur Verbindung (I-C) können ohne Zusatz einer Hilfsbase durchgeführt werden; gegebenenfalls kann es jedoch vorteilhaft sein, eine übliche anorganische oder organische Base einzusetzen, wie beispielsweise Natrium- oder Kaliumcarbonat, Natriumacetat, Natrium- oder Kaliumhydroxid, Lithium- oder Kalium-bis(trimethylsilyl)amid, Triethylamin, *N*-Methylmorpholin, *N*-Methylpiperidin oder *N,N-*Diisopropylethylamin.

Die Transformation (XII) → (I-C) wird im Allgemeinen in einem Temperaturbereich von -20°C bis +80°C, bevorzugt bei 0°C bis +40°C durchgeführt. Auch hier kann eine Reaktionsführung unter Mikrowellen-Bestrahlung von Vorteil sein.

Die Verbindungen der Formel (XI) ihrerseits können in Analogie zu literaturbekannten Verfahren beispielsweise durch Polyphosphorsäureester-katalysierte Kondensation eines Aldehyds der Formel (VII) mit einer Keto-Verbindung der Formel (VIII) und einem Thioharnstoff-Derivat der Formel (XIII) 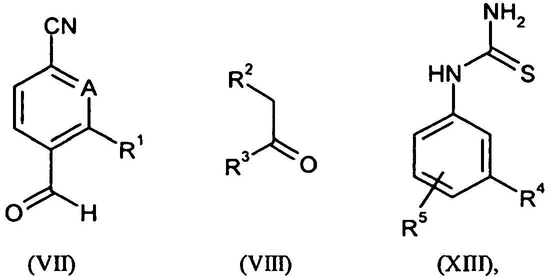 in welchen A, R¹, R², R³, R⁴ und R⁵ jeweils die oben angegebenen Bedeutungen haben,
hergestellt werden [vgl. auch das nachfolgende Reaktionsschema 8 sowie die in WO 2004/024701 beschriebenen Verfahren].

Weitere erfindungsgemäße Verbindungen der Formel (I) können, falls zweckmäßig, auch durch Umwandlungen von funktionellen Gruppen einzelner Substituenten, insbesondere den unter R¹, R², R⁷ und R⁹ aufgeführten, ausgehend von anderen, nach obigem Verfahren erhaltenen Verbindungen der Formel (I) hergestellt werden. Diese Umwandlungen werden nach üblichen, dem Fachmann geläufigen Methoden durchgeführt und umfassen beispielsweise Reaktionen wie nukleophile oder elektrophile Substitutionsreaktionen, Übergangsmetall-vermittelte Kupplungsreaktionen (z.B. Suzuki- oder Heck-Reaktion), Oxidation, Reduktion, Hydrierung, Alkylierung, Acylierung, Aminierung, Hydroxylierung, Veretherung, Veresterung, Esterspaltung und -hydrolyse, Bildung von Nitrilen, Carbonamiden, Sulfonamiden, Carbamaten und Harnstoffen, sowie die Einführung und Entfernung temporärer Schutzgruppen [vgl. auch die nachfolgenden Reaktionsschemata 6 und 8 sowie die Ausführungsbeispiele].

So können beispielsweise Verbindungen der Formel (I), in welcher Y für N und Z für C-R⁹ steht, worin R⁹ die Gruppe -NR¹²R¹³ darstellt und R¹² und R¹³ wiederum die oben angegebenen Bedeutungen haben, durch entsprechende N-Alkylierungs- oder -Acylierungsreaktionen ausgehend von einer Verbindung der Formel (I-D) 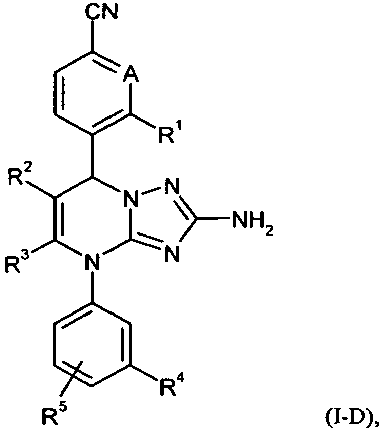 in welcher A, R¹, R², R³, R⁴ und R⁵ jeweils die oben angegebenen Bedeutungen haben,
erhalten werden, wobei die Verbindung (I-D) ihrerseits nach dem oben beschriebenen allgemeinen Verfahren zugänglich ist [vgl. auch die nachfolgenden Reaktionsschemata 5 und 6].

Eine Trennung der erfindungsgemäßen Verbindungen in die entsprechenden Enantiomere und/oder Diastereomere kann, je nach Zweckmäßigkeit, auf der Stufe der Verbindungen (I), (I-A), (I-B), (I-C) bzw. (I-D) oder auch bereits auf der Stufe der Verbindungen (V), (XI) oder (XII) erfolgen, wobei letztere dann in separierter Form entsprechend den zuvor beschriebenen Verfahrensschritten weiter umgesetzt werden. Eine solche Auftrennung der Stereoisomeren läßt sich nach üblichen, dem Fachmann bekannten Methoden durchführen; vorzugsweise werden chromatographische Verfahren, insbesondere die HPLC-Chromatographie an chiraler Phase, verwendet.

Die Verbindungen der Formeln (III), (VI), (VIII), (X) und (XIII) sind kommerziell erhältlich oder als solche literaturbekannt, oder sie können nach gängigen, in der Literatur beschriebenen Methoden hergestellt werden.

Die Verbindungen der Formel (VII) sind zum Teil kommerziell erhältlich oder literaturbekannt, oder sie können in Analogie zu in der Literatur beschriebenen Verfahren hergestellt werden [vgl. auch die nachfolgenden Reaktionsschemata 1-3 und dort angegebene Literatur].

Die oben beschriebenen Verfahren können durch die folgenden Reaktionsschemata beispielhaft veranschaulicht werden: 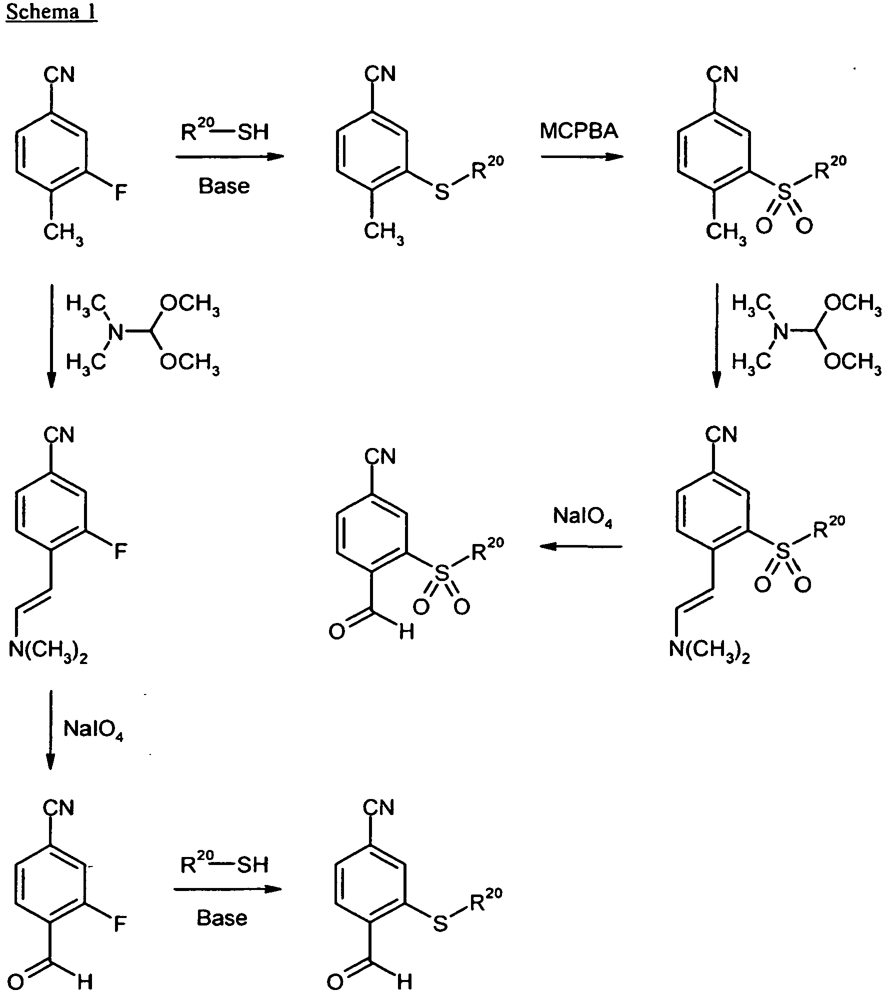 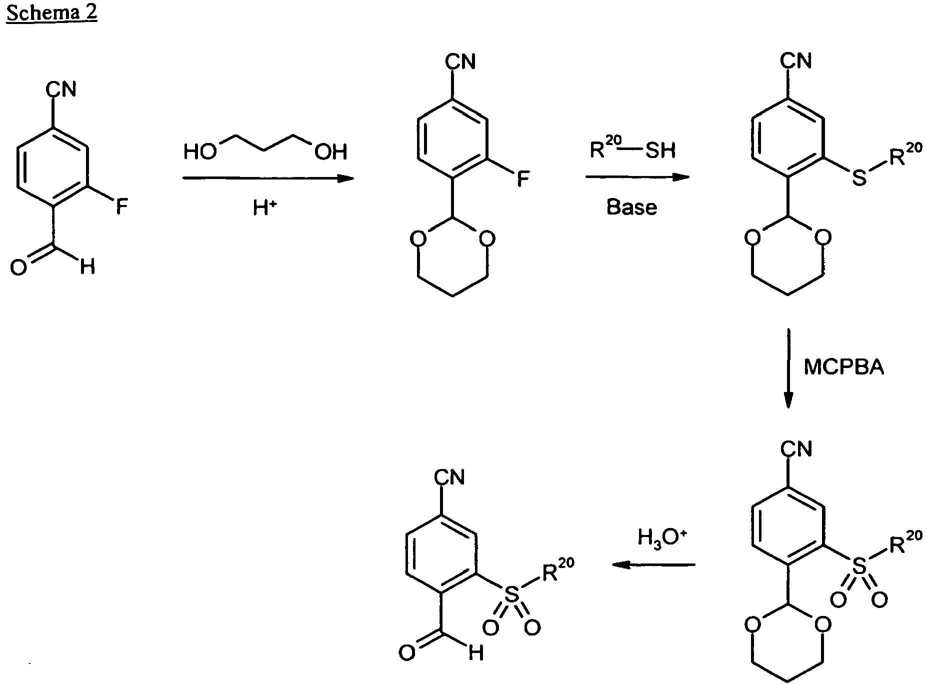 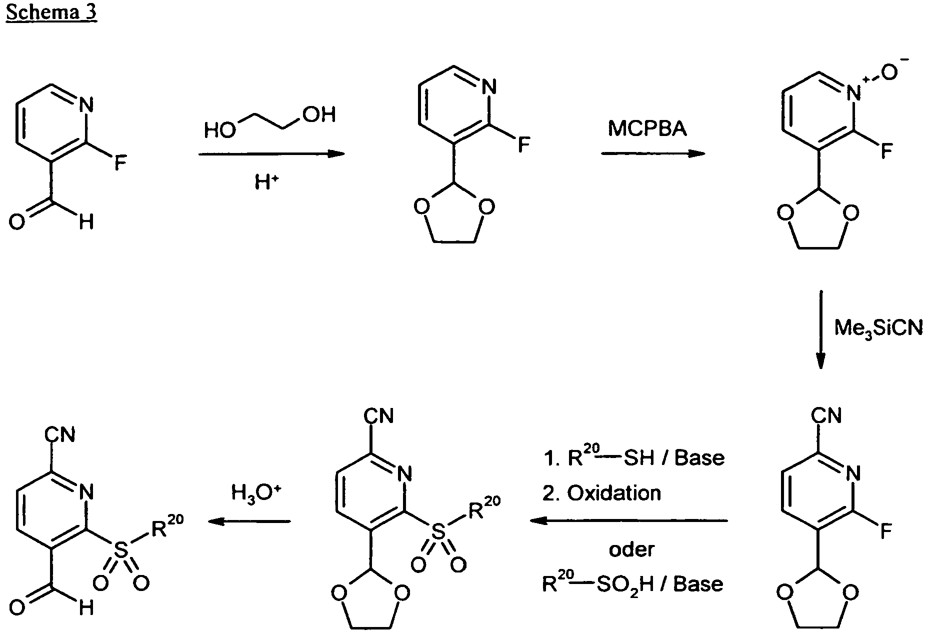 [vgl. z.B. W.K. Fife, J. Org. Chem. 48, 1375 (1983); H. Vorbrüggen und K. Krolikiewicz, Synthesis, 316 (1983); R.T. Shuman et al., J. Org. Chem. 55, 738 (1990); C.S. Burgey et al., J. Med. Chem. 46 (4), 461 (2003); J.J. Li et al., J. Med. Chem. 39, 1846 (1996); K.N. Dack et al., Bioorg. Med. Chem. Lett. 8 (16), 2061 (1998)].

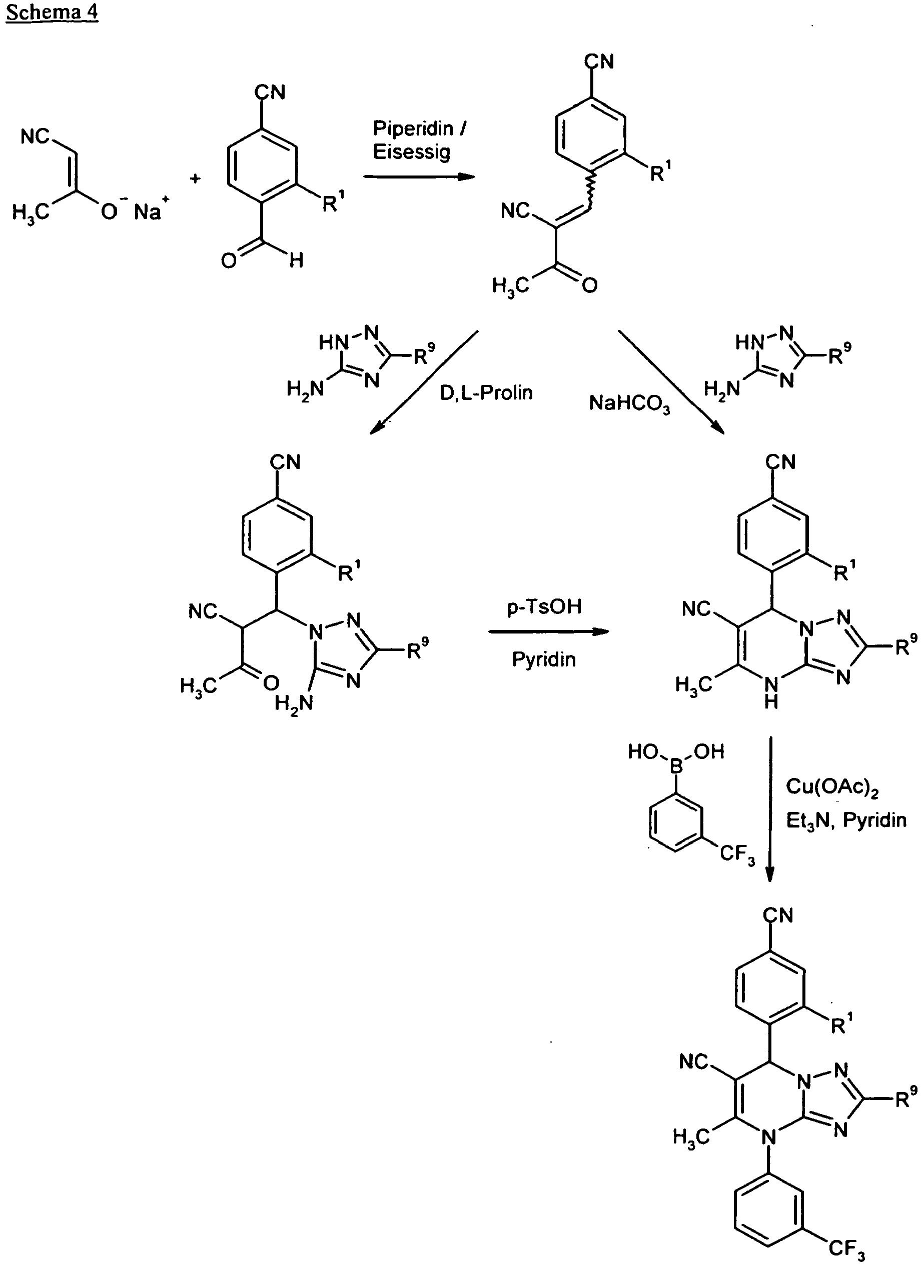

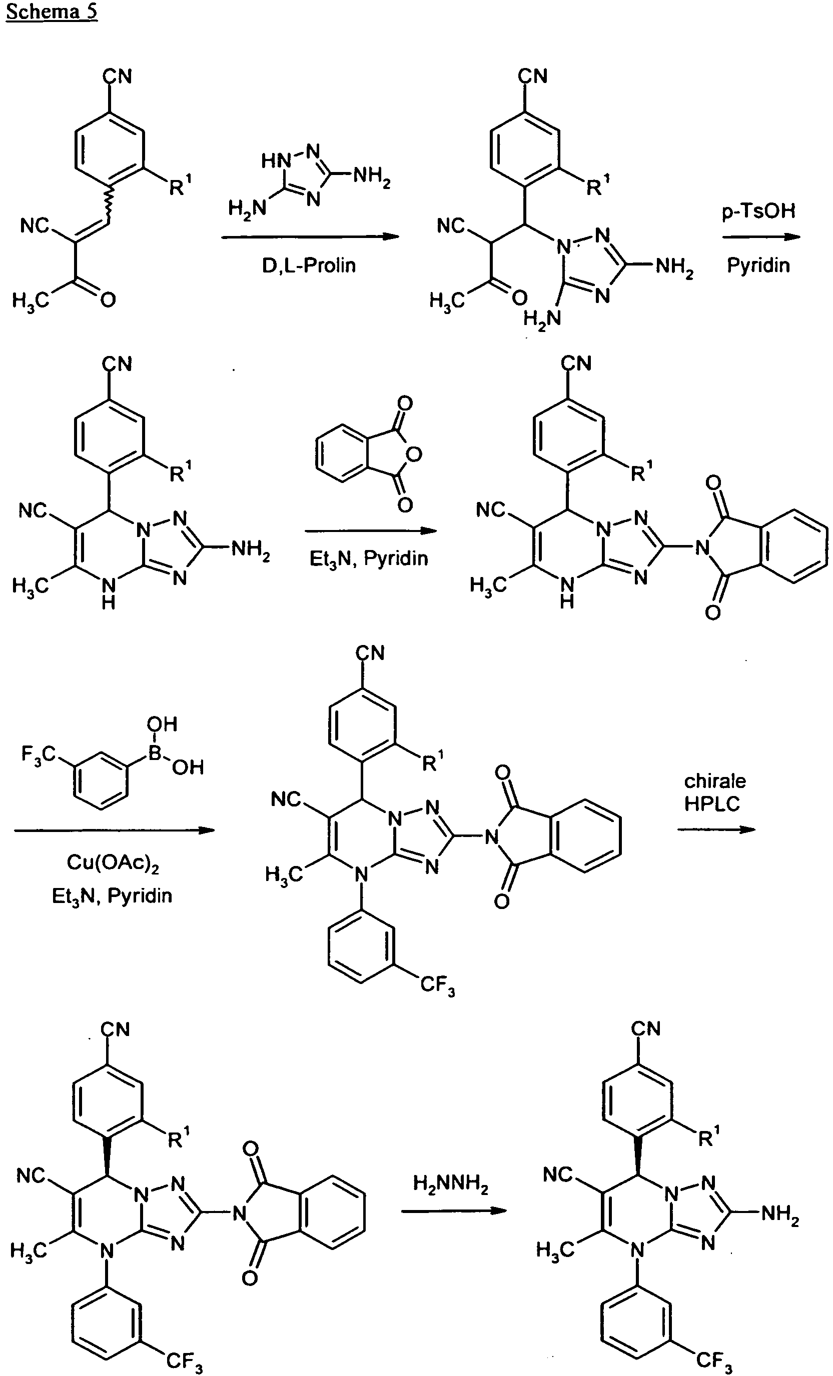

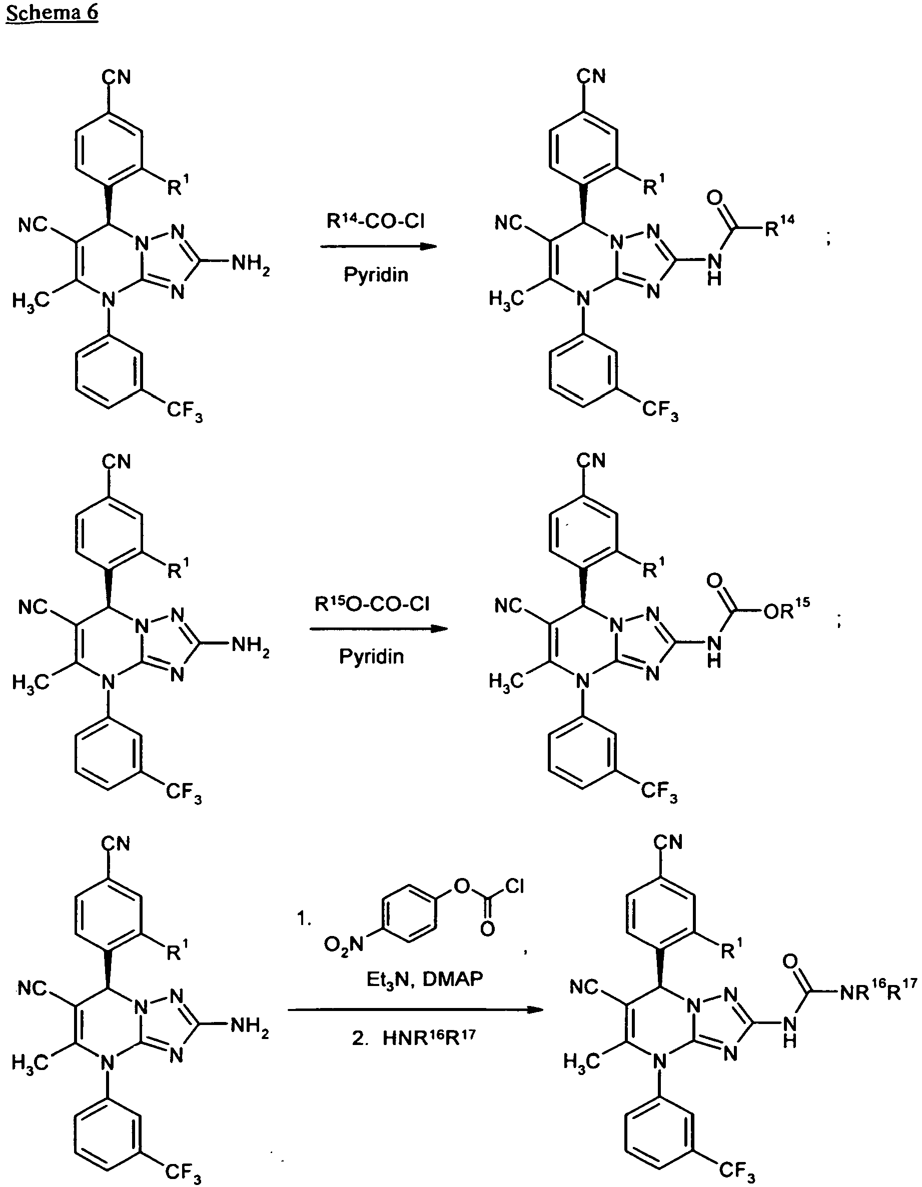

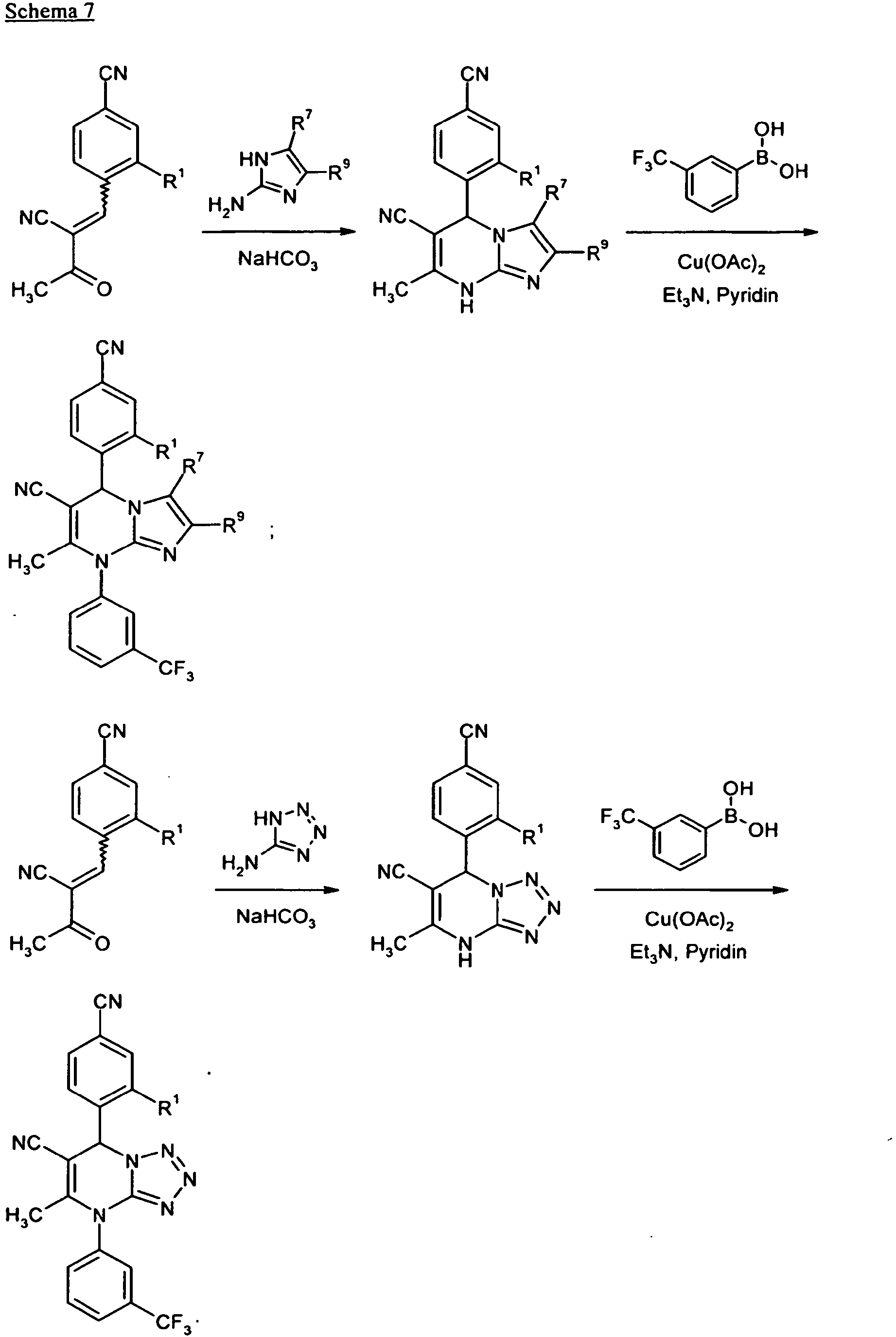

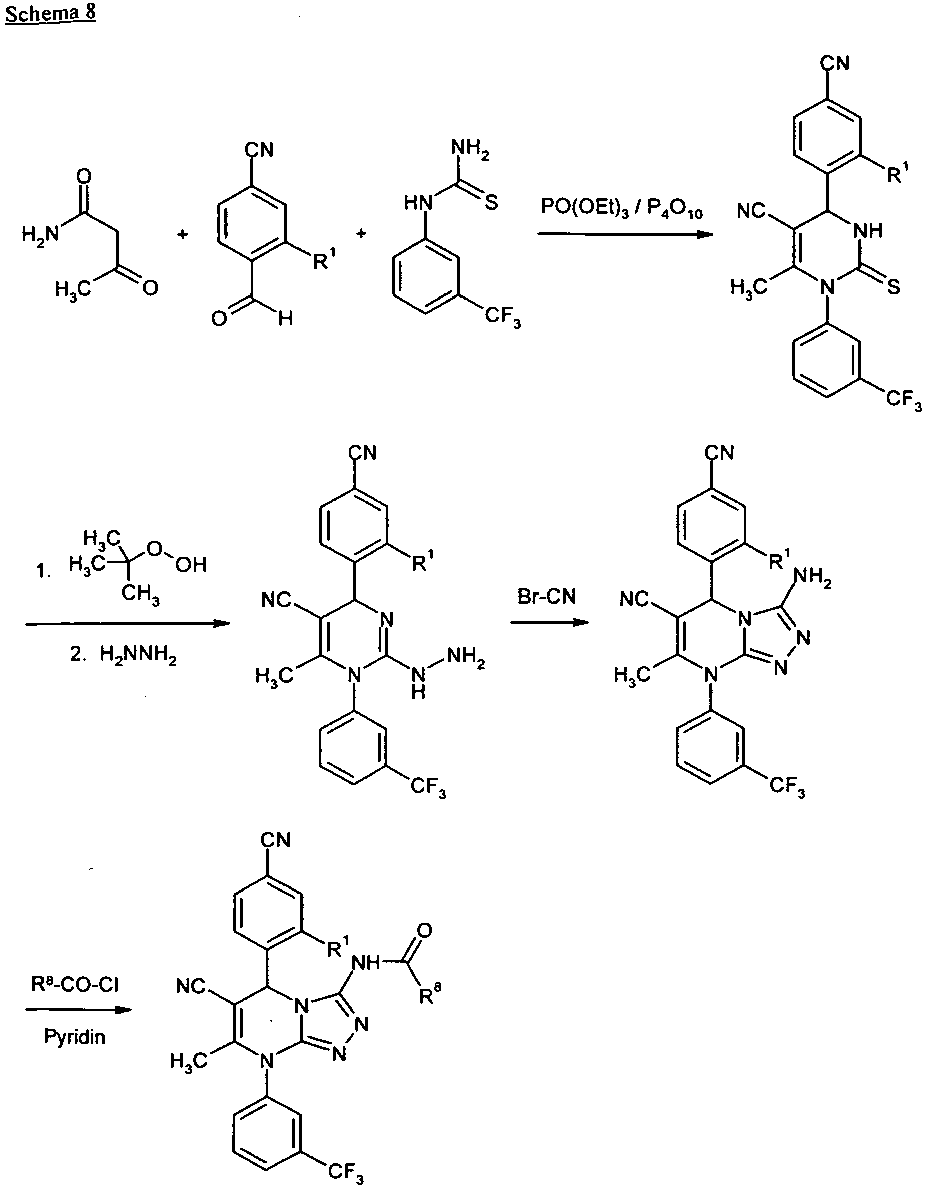

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente niedermolekulare, nicht-reaktive und selektive Inhibitoren der humanen neutrophilen Elastase (HNE) dar und eignen sich daher zur Behandlung und/oder Prävention insbesondere solcher Erkrankungen und pathologischen Prozesse, bei denen im Zuge eines Entzündungsgeschehens und/oder eines Gewebe- oder Gefäßumbaus die neutrophile Elastase involviert ist.

Dazu zählen im Sinne der vorliegenden Erfindung insbesondere Erkrankungen wie die pulmonale arterielle Hypertonie (PAH) und andere Formen der pulmonalen Hypertonie (PH), die chronisch-obstruktive Lungenerkrankung (COPD), das akute Atemwegssyndrom (ARDS), akute Lungenschädigung (ALI), alpha-1-Antitrypsin-Defizienz (AATD), Lungenfibrose, Lungenemphysem, zystische Fibrose (CF), akutes Koronarsyndrom (ACS), Herzmuskelentzündungen (Myokarditis) und andere autoimmune Herzerkrankungen (Perikarditis, Endokarditis, Valvolitis, Aortitis, Kardiomyopathien), Myokardinfarkt, kardiogener Schock, Herzinsuffizienz, Aneurysmen, Sepsis (SIRS), multiples Organversagen (MODS, MOF), Arteriosklerose, entzündliche Erkrankungen der Niere, chronische Darmentzündungen (IBD, CD, UC), Pankreatitis, Peritonitis, rheumatoide Erkrankungen, entzündliche Hauterkrankungen sowie entzündliche Augenerkrankungen.

Die erfindungsgemäßen Verbindungen können weiterhin verwendet werden zur Behandlung und/ oder Prävention von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiolitis obliterans, Bronchiektasie, Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Darüber hinaus können die erfindungsgemäßen Verbindungen auch eingesetzt werden zur Behandlung und/oder Prävention von mikro- und makrovaskulären Schädigungen (Vasculitis), Reperfusionsschäden, arteriellen sowie venösen Thrombosen, diabetischer und nicht-diabetischer Nephropathie, der Glomerulonephritis, der Glomerulosklerose, des nephrotischen Syndroms, der hypertensiven Nephrosklerose, der Mikroalbuminurie, der akuten und chronischen Niereninsuffizienz, des akuten und chronischen Nierenversagens, Cystitis, Urethritis, Prostatitis, Epidymititis, Oophoritis, Salpingitis, Vulvovaginitis, erektiler Dysfunktion, Hunner-Geschwür, Peyronie-Krankheit, arterieller Hypertonie, Schock, atrialen und ventrikulären Arrhythmien, transitorischen und ischämischen Attacken, Herzmuskelschwäche, Himschlag, endothelialer Dysfunktion, peripheren und kardialen Gefäßerkrankungen, peripheren Durchblutungsstörungen, Ödembildung wie zum Beispiel pulmonales Ödem, Himödem, renales Ödem und Herzinsuffizienz-bedingtes Ödem, Restenosen wie nach Thrombolysetherapien, percutan-transluminalen Angioplastien (PTA), transluminalen Koronarangioplastien (PTCA), Herztransplantationen und Bypass-Operationen, bei erhöhtem Spiegel von Fibrinogen und von LDL geringer Dichte sowie bei erhöhten Konzentrationen von Plasminogenaktivator-Inhibitor 1 (PAI-1), von Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien) sowie metabolischen Erkrankungen (Metabolisches Syndrom, Hyperglykämie, Insulin-abhängiger Diabetes, Nicht-Insulin-abhängiger Diabetes, Gestationsdiabetes, Hyperinsulinämie, Insulinresistenz, Glukose-Intoleranz, Fettsucht (Adipositas) und diabetische Spätfolgen wie Retinopathie, Nephropathie und Neuropathie), Krebs-erkrankungen (Hautkrebs, Hirntumore, Brustkrebs, Knochenmarktumore, Leukämien, Liposarcome, Karzinome des Magen-Darm-Traktes, der Leber, Bauchspeicheldrüse, Lunge, Niere, Hamleiter, Prostata und des Genitaltraktes sowie bösartige Tumore des lymphoproliferativen Systems wie z.B. Hodgkin's und Non-Hodgkin's Lymphom), von Erkrankungen des Gastrointestinaltraktes und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, Leberfibrose, Leberzirrhose, Pankreatitis und Cholecystitis), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), Immunerkrankungen, Schilddrüsenerkrankungen (Hyperthyreose), Hauterkrankungen (Psoriasis, Akne, Ekzeme, Neurodermitis, vielflätige Formen der Dermatitis wie z.B. Dermatitis abacribus, Dermatitis actinica, Dermatitis allergica, Dermatitis ammoniacalis, Dermatitis artefacta, Dermatitis autogenica, Dermatitis atrophicans, Dermatitis calorica, Dermatitis combustionis, Dermatitis congelationis, Dermatitis cosmetica, Dermatitis escharotica, Dermatitis exfoliativa, Dermatitis gangraenose, Dermatitis haemostatica, Dermatitis herpetiformis, Dermatitis lichenoides, Dermatitis linearis, Dermatitis maligna, Dermatitis medimencatosa, Dermatitis palmaris et plantaris, Dermatitis parasitaria, Dermatitis photoallergica, Dermatitis phototoxica, Dermatitis pustularis, Dermatitis seborrhoica, Dermatitis solaris, Dermatitis toxica, Dermatitis ulcerosa, Dermatitis veneata, infektiöse Dermatitis, pyogene Dermatitis und Rosazea-artige Dermatitis, sowie Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), von entzündlichen Augenerkrankungen (Saccoidosis, Blepharitis, Conjunctivitis, Iritis, Uveitis, Chorioiditis, Ophthalmitis), viralen Erkrankungen (durch Influenza-, Adeno- und Coronaviren, wie z.B. HPV, HCMV, HIV, SARS), von Erkrankungen des Skelettknochens und der Gelenke sowie der Skelettmuskel (vielfältige Formen der Arthritis wie z.B. Arthritis alcaptonurica, Arthritis ankylosans, Arthritis dysenterica, Arthritis exsudativa, Arthritis fungosa, Arthritis gonorrhoica, Arthritis mutilans, Arthritis psoriatica, Arthritis purulenta, Arthritis rheumatica, Arthritis serosa, Arthritis syphilitica, Arthritis tuberculosa, Arthritis urica, Arthritis villonodularis pigmentosa, atypische Arthritis, hämophile Arthritis, juvenile chronische Arthritis, rheumatoide Arthritis und metastatische Arthritis, des weiteren das Still-Syndrom, Felty-Syndrom, Sjörgen-Syndrom, Clutton-Syndrom, Poncet-Syndrom, Pott-Syndrom und Reiter-Syndrom, vielfältige Formen der Arthropathien wie z.B. Arthropathie deformans, Arthropathie neuropathica, Arthropathie ovaripriva, Arthropathie psoriatica und Arthropathie tabica, systemische Sklerosen, vielfältige Formen der entzündlichen Myopathien wie z.B. Myopathie epidemica, Myopathie fibrosa, Myopathie myoglobinurica, Myopathie ossificans, Myopathie ossificans neurotica, Myopathie ossificans progressiva multiplex, Myopathie purulenta, Myopathie rheumatica, Myopathie trichinosa, Myopathie tropica und Myopathie typhosa, sowie das Günther-Syndrom und das Münchmeyer-Syndrom), von entzündlichen Arterienveränderungen (vielfältige Formen der Arteritis wie z.B. Endarteritis, Mesarteritis, Periarteritis, Panarteritis, Arteritis rheumatica, Arteritis deformans, Arteritis temporalis, Arteritis cranialis, Arteritis gigantocellularis und Arteritis granulomatosa, sowie das Horton-Syndrom, Churg-Strauss-Syndrom und die Takayasu-Arteritis), des Muckle-Well-Syndroms, der Kikuchi-Krankheit, von Polychondritis, Sklerodermia sowie von weiteren Erkrankungen mit einer entzündlichen oder immunologischen Komponente, wie beispielsweise Katarakt, Kachexie, Osteoporose, Gicht, Inkontinenz, Lepra, Sezary-Syndrom und paraneoplastisches Syndrom, bei Abstossungsreaktionen nach Organtransplantationen und zur Wundheilung und Angiogenese insbesondere bei chronischen Wunden.

Aufgrund ihres Eigenschaftsprofils eignen sich die erfindungsgemäßen Verbindungen insbesondere zur Behandlung und/oder Prävention von pulmonaler arterieller Hypertonie (PAH) und anderen Formen der pulmonalen Hypertonie (PH), chronisch-obstruktiven Lungenerkrankungen (COPD), akuter Lungenschädigung (ALI), akutem Atemwegssyndrom (ARDS), Bronchiektasie, Bronchiolitis obliterans, Lungenemphysem, alpha-1-Antitrypsin-Defizienz (AATD), zystischer Fibrose (CF), Sepsis und systemisch-inflammatorischem Response-Syndrom (SIRS), multiplem Organversagen (MOF, MODS), entzündlichen Darmerkrankungen (IBD, Morbus Crohn, Colitis), chronischer Bronchitis, Asthma, Rhinitis, rheumatoider Arthritis, entzündlichen Haut- und Augenkrankheiten, Arteriosklerose und Krebserkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
**●** die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren;
● Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
● Verbindungen, die die Bindung von Serotonin an dessen Rezeptor blockieren, beispielhaft und vorzugsweise Antagonisten des 5-HT_{2b}-Rezeptors;
● organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
● NO-unabhängige, jedoch Häm-abhängige Stimulatoren der löslichen Guanylatcyclase, wie insbesondere die in WO 00/06568, WO 00/06569, WO 02/42301 und WO 03/095451 beschriebenen Verbindungen;
● NO- und Häm-unabhängige Aktivatoren der löslichen Guanylatcyclase, wie insbesondere die in WO 01/19355, WO 01/19776, WO 01/19778, WO 01/19780, WO 02/070462 und WO 02/070510 beschriebenen Verbindungen;
● Prostacyclin-Analoga, wie beispielhaft und vorzugsweise Iloprost, Beraprost, Treprostinil oder Epoprostenol;
● Verbindungen, die die lösliche Epoxidhydrolase (sEH) inhibieren, wie beispielsweise *N,N'*-Dicyclohexylharnstoff, 12-(3-Adamantan-1-yl-ureido)-dodecansäure oder 1-Adamantan-1-yl-3-{5-[2-(2-ethoxyethoxy)ethoxy]pentyl)-hamstoff;
● den Energiestoffwechsel des Herzens beeinflussende Verbindungen, wie beispielhaft und vorzugsweise Etomoxir, Dichloracetat, Ranolazine oder Trimetazidine;
● Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil;
● bronchodilatorisch wirkende Mittel, beispielhaft und vorzugsweise aus der Gruppe der betaadrenergen Rezeptor-Agonisten, wie insbesondere Albuterol, Isoproterenol, Metaproterenol, Terbutalin, Formoterol oder Salmeterol, oder aus der Gruppe der Anticholinergika, wie insbesondere Ipratropiumbromid;
● anti-inflammatorisch wirkende Mittel, beispielhaft und vorzugsweise aus der Gruppe der Glucocorticoide, wie insbesondere Prednison, Prednisolon, Methylprednisolon, Triamcinolon, Dexamethason, Beclomethason, Betamethason, Flunisolid, Budesonid oder Fluticason;
● antithrombotisch wirkende Mittel, beispielhaft und vorzugsweise aus der Gruppe der Thrombozytenaggregationshemmer, der Antikoagulantien oder der profibrinolytischen Substanzen;
● den Blutdruck senkende Wirkstoffe, beispielhaft und vorzugsweise aus der Gruppe der Calcium-Antagonisten, Angiotensin AII-Antagonisten, ACE-Inhibitoren, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika; und/oder
● den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Kinase-Inhibitor, wie beispielhaft und vorzugsweise Bortezomib, Canertinib, Erlotinib, Gefitinib, Imatinib, Lapatinib, Lestaurtinib, Lonafamib, Pegaptinib, Pelitinib, Semaxanib, Sorafenib, Sunitinib, Tandutinib, Tipifamib, Vatalanib, Fasudil, Lonidamin, Leflunomid, BMS-3354825 oder Y-27632, eingesetzt.

Bei einer bevorzugten Ausführungsform der Erfindung werden die erfindungsgemäßen Verbindungen in Kombination mit einem Serotonin-Rezeptor-Antagonisten, wie beispielhaft und vorzugsweise PRX-08066, eingesetzt.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095, SB-772077, GSK-269962A oder BA-1049, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen, enthalten sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster.Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Aerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale, die intravenöse und die inhalative Applikation.

Die erfindungsgemäßen Verbindungen können in die angeführten Applikationsformen überführt werden. Dies kann in an sich bekannter Weise durch Mischen mit inerten, nicht-toxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich, sofern nicht anders angegeben, jeweils auf das Volumen.

### A. Beispiele

### Abkürzungen:

- abs.: absolut
- Ac: Acetyl
- aq.: wässrig, wässrige Lösung
- ber.: berechnet
- Boc: *tert*.-Butoxycarbonyl
- c: Konzentration
- cat.: katalytisch
- CDI: *N,N'*-Carbonyldiimidazol
- d: Tag(e)
- DC: Dünnschichtchromatographie
- dest.: destilliert
- DIEA: *N,N-*Diisopropylethylamin
- DMAP: 4-*NN*-Dimethylaminopyridin
- DMF: *N,N-*Dimethylformamid
- DMSO: Dimethylsulfoxid
- d. Th.: der Theorie (bei Ausbeute)
- ee: Enantiomerenüberschuss
- ent: enantiomerenrein, Enantiomer
- eq.: Äquivalent(e)
- ESI: Elektrospray-lonisation (bei MS)
- Et: Ethyl
- GC-MS: Gaschromatographie-gekoppelte Massenspektrometrie
- gef.: gefunden
- Gly: Glycin
- h: Stunde(n)
- HATU: *O*-(7-Azabenzotriazol-1-yl)-*N,N,N;N'*-tetramethyluronium-Hexafluorophosphat
- HOAc: Essigsäure
- HPLC: Hochdruck-, Hochleistungsflüssigchromatographie
- HR-MS: hochauflösende Massenspektrometrie
- konz.: konzentriert
- LC-MS: Flüssigchromatographie-gekoppelte Massenspektrometrie
- MCPBA: *meta*-Chlorperbenzoesäure
- Me: Methyl
- min: Minute(n)
- MPLC: Mitteldruckflüssigchromatographie
- MS: Massenspektrometrie
- MTBE: Methyl-*tert*.-butylether
- NMM: *N*-Methylmorpholin
- NMR: Kernresonanzspektrometrie
- *p*: para
- Ph: Phenyl
- quant.: quantitativ (bei Ausbeute)
- rac: racemisch, Racemat
- RT: Raumtemperatur
- R*ₜ*: Retentionszeit (bei HPLC)
- Schmp.: Schmelzpunkt
- tBu: *tert.-*Butyl
- TFA: Trifluoressigsäure
- TFAA: Trifluoressigsäureanhydrid
- THF: Tetrahydrofuran
- TsOH: *p*-Toluolsulfonsäure
- UV: Ultraviolett-Spektrometrie
- v/v: Volumen zu Volumen-Verhältnis (einer Lösung)

### HPLC-, LC-MS- und GC-MS-Methoden:

### Methode 1 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min-3.0 min-4.5 min 2 ml/min; Ofen: 50°C; UV-Detektion: 210 nm.

### Methode 2 (LC-MS):

Gerätetyp MS: Micromass ZQ; Gerätetyp HPLC: HP 1100 Series; UV DAD; Säule: Phenomenex Gemini 3µ 30 mm x 3.0 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min-3.0 min-4.5 min 2 ml/min; Ofen: 50°C; UV-Detektion: 210 nm.

### Methode 3 (LC-MS):

Instrument: Micromass Quattro LCZ mit HPLC Agilent Serie 1100; Säule: Phenomenex Onyx Monolithic C18, 100 mm x 3 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 4 (LC-MS):

Gerätetyp MS: Waters ZQ; Gerätetyp HPLC: Waters Alliance 2795; Säule: Phenomenex Onyx Monolithic C 18, 100 mm x 3 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 5 (LC-MS):

Instrument: Micromass QuattroPremier mit Waters UPLC Acquity; Säule: Thermo Hypersil GOLD 1.9µ 50 mm x 1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 6 (LC-MS):

Instrument: Micromass Platform LCZ mit HPLC Agilent Serie 1100; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 0.2 min 100% A → 2.9 min 30% A → 3.1 min 10% A → 5.5 min 10% A; Fluss: 0.8 ml/min; Ofen: 50°C; UV-Detektion: 210 nm.

### Methode 7 (LC-MS):

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 8 (LC-MS):

Instrument: Micromass Quattro LCZ mit HPLC Agilent Serie 1100; Säule: Phenomenex Synergi 2.5µ MAX-RP 100A Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 l min 90% A → 3.0 min 5% A → 4.0 min 5% A → 4.1 min 90% A; Fluss: 2 ml/min; Ofen: 50°C; UV-Detektion: 208-400 nm.

### Methode 9 (LC-MS):

Instrument: Waters Acquity SQD UPLC System; Säule: Waters Acquity UPLC HSS T3 1.8µ 50 mm x 1 mm; Eluent A: 1 l Wasser + 0.25 ml 99%-ige Ameisensäure, Eluent B: 1 l Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 10 (HR-MS):

Instrument: Agilent 1100 HPLC-System mit LTQ Orbitrap-Massenspektrometer (Bremen, Deutschland) und einer APCI-Ionenquelle. Das Gerät wird im positiven lonenmodus betrieben. Zur Kalibrierung wird das vom Hersteller zur Verfügung gestellte Gemisch verwendet: Koffein, L-Methionylarginylphenylalanylalanin-acetat (MRFA) und Ultramark 1621 in einer Acetonitril/ Methanol/Wasser-Lösung mit 1% Essigsäure. Das Massenspektrometer wird bei einer Auflösung von 60.000 *(m*/*z* = 400) betrieben (full scan-Modus, Xcalibur 2.0-Software; ThermoScientific, Bremen, Deutschland).

### Methode 11 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min-3.0 min-4.5 min 2 ml/min; Ofen: 50°C; UV-Detektion: 208-400 nm.

### Methode 12 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Ausgangsverbindungen und Intermediate:

### Beispiel 1A

### 4-(2-Cyano-3-oxobut-1-en-1-yl)benzonitril

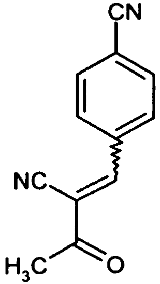

4-Cyanobenzaldehyd (360.0 g, 2.75 mol) und Natrium-1-cyanoprop-1-en-2-olat (288.5 g, 2.75 mol, 1 eq.; zur Herstellung vgl. R. Troschütz, Archiv der Pharmazie 1984, 317, 709-713) wurden in Dichlormethan (20 Liter) vorgelegt. Dann wurden Eisessig (196.5 ml, 3.43 mol, 1.25 eq.) und Piperidin (27.2 ml, 0.274 mol, 0.1 eq.) zugegeben, und die Mischung wurde am Wasserabscheider unter Rückfluss erhitzt (18 h). Die Reaktionslösung wurde danach mit gesättigter Natriumhydrogencarbonat-Lösung (5 Liter) gewaschen, über Natriumsulfat getrocknet und am Rotationsverdampfer eingeengt. Der feste Rückstand wurde in der sechsfachen Menge Ethanol ausgerührt, dann abgesaugt, und die Kristalle mit Ethanol nachgewaschen und im Hochvakuum getrocknet. Es wurden 427 g (79% d. Th.) der Titelverbindung erhalten.

LC-MS (Methode 5): R*ₜ* = 0.91 min; MS (ESIpos): *m*/*z* (%) = 280.3 (100) [M+H]⁺; MS (ESIneg): *m*/*z* (%) = 197.3 (100), 278.3 (25) [M-H]⁻.

¹H-NMR (400 MHz, DMSO-d₆): δ = 8.05 (m, 2H), 8.15 (m, 2H), 8.45 (s, 1H) (Signal der Methylgruppe vom DMSO-Peak überlagert).

### Beispiel 2A

### (rac)-2-Amino-7-(4-cyanophenyl)-5-methyl-4,7-dihydro[1,2,4]triazolo[1,5-a]pyrimidin-6-carbonitril

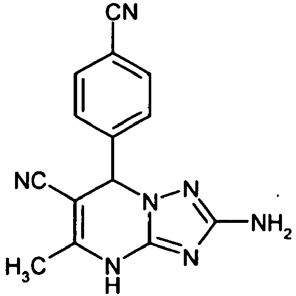

4-(2-Cyano-3-oxobut-1-en-1-yl)benzonitril (15.0 g, 76.45 mmol), 3,5-Diaminotriazol (15.15 g, 152.90 mmol, 2.0 eq.), D,L-Prolin (7.04 g, 61.16 mmol, 0.8 eq.) und Molekularsieb (4 Å, 5 g) wurden unter Argon in einer Mischung aus Dichlormethan (800 ml), Pyridin (800 ml) und DMF (1600 ml) suspendiert und 3 d lang gerührt. Danach wurden nochmals 3,5-Diaminotriazol (4.0 g, 40.37 mmol, 0.5 eq.) und D,L-Prolin (4.0 g, 34.74 mmol, 0.45 eq.) zugegeben und die Mischung weitere 5 d lang gerührt. Das Reaktionsgemisch wurde dann filtriert und das Filtrat im Vakuum eingeengt (Zwischenprodukt 4-[2-Cyano-1-(3,5-diamino-1*H*-1,2,4-triazol-1-yl)-3-oxobutyl]benzonitril).

Das Zwischenprodukt wurde mit Toluol (500 ml), Pyridin (300 ml) und 4-ToluolsulfonsäureMonohydrat (1.1 g, 5.78 mmol, 0.08 eq.) versetzt und unter Rückfluss erhitzt (1 h). Das Reaktionsgemisch wurde anschließend filtriert, das Filtrat im Vakuum eingeengt und der Rückstand im Hochvakuum getrocknet. Der Rückstand wurde mit Ethanol (300 ml) versetzt, im Ultraschall-Bad suspendiert, gerührt und der Feststoff dann abfiltriert. Es wurde nochmals mit Ethanol (300 ml) ausgerührt, abfiltriert und zuletzt im Hochvakuum getrocknet. Es wurden 16.70 g (78% d. Th.) der Titelverbindung erhalten.

LC-MS (Methode 7): R*ₜ* = 0.98 min; MS (ESIpos): *m*/*z* (%) = 278.3 (100) [M+H]⁺; MS (ESIneg): *m*/*z* (%) = 276.4 (85) [M-H]⁻.

¹H-NMR (400 MHz, DMSO-d₆): δ = 2.15 (s, 3H), 5.35 (s, 2H), 6.05 (s, 1H), 7.45 (m, 2H), 7.85 (m, 2H), 10.95 (s, 1H).

### Beispiel 3A

### (rac)-7-(4-Cyanophenyl)-2-(1,3-dioxo-1,3-dihydro-2H-isoindol-2-yl)-5-methyl-4,7-dihydro[1,2,4]-triazolo[1,5-a]pyrimidin-6-carbonitril

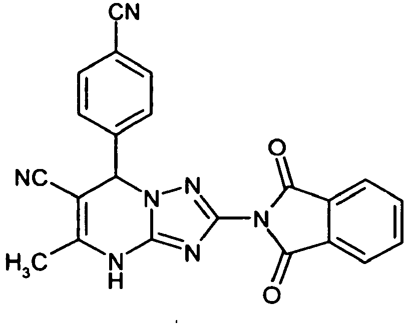

(*rac*)-2-Amino-7-(4-cyanophenyl)-5-methyl-4,7-dihydro[1,2,4]triazolo[1,5-a]pyrimidin-6-carbonitril (15 g, 54.1 mmol) wurde unter Argon zusammen mit Phthalsäureanhydrid (11.22 g, 75.7 mmol, 1.4 eq.) in einem Gemisch aus Toluol (500 ml) und Pyridin (600 ml) suspendiert, mit Triethylamin (1.51 ml, 10.8 mmol, 0.2 eq.) versetzt und über Nacht unter Rückfluss erhitzt. Danach wurde weiteres Pyridin (200 ml), Phthalsäureanhydrid (8 g, 54.0 mmol, 1.0 eq.) sowie Triethylamin (1.51 ml, 10.8 mmol, 0.2 eq.) zugegeben und die Mischung nochmals 12 h unter Rückfluss erhitzt. Das Reaktionsgemisch wurde dann im Vakuum eingeengt und der Rückstand im Hochvakuum getrocknet (12 h). Das Rohprodukt wurde dreimal nacheinander in Ethanol (100 ml) suspendiert, für 1 h gerührt, abfiltriert, mit Pentan gewaschen und im Hochvakuum getrocknet. Als Produkt wurde ein hellbeiger Feststoff erhalten (20 g), der aus DMF/Methanol umkristallisiert wurde. Hierzu wurde der Feststoff zunächst in heissem DMF (250 ml, 150°C) gelöst und die erkaltete DMF-Lösung dann langsam unter Rühren in Methanol (1.3 Liter) gegossen, wobei das Produkt ausfiel. Der Niederschlag wurde abfiltriert und im Hochvakuum getrocknet. Nach erneutem Umfällen aus DMF/Methanol wurden 15.91 g (99% Reinheit, 71.5% d. Th.) der Titelverbindung erhalten.

LC-MS (Methode 5): Rₜ = 0.98 min; MS (ESIpos): *m*/*z* (%) = 408.2 (100) [M+H]⁺; MS (ESIneg): *m*/*z* (%) = 406.4 (100) [M-H]⁻.

¹H-NMR (400 MHz, DMSO-d₆): δ = 2.25 (s, 3H), 6.55 (s, 1H), 7.6 (m, 2H), 7.95 (br. m, 6H), 11.55 (s, 1H).

### Beispiel 4A

### 4-Methyl-3-(methylsulfanyl)benzonitril

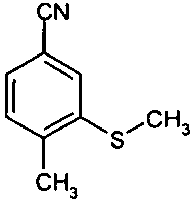

### Methode A:

Die Reaktion wurde unter Argon durchgeführt. 3-Fluor-4-methylbenzonitril (3000 mg, 22.2 mmol) und Natriummethanthiolat (1572 mg, 20.2 mmol) wurden in DMF (30 ml) vorgelegt, mit Kaliumcarbonat (6973 mg, 50.5 mmol) versetzt und über Nacht unter Rückfluss gerührt. Der Ansatz wurde danach eingeengt, der Rückstand mit Methylenchlorid/Methanol (10:1) aufgeschlämmt und das darin unlösliche Kaliumcarbonat abfiltriert. Das Filtrat wurde wieder eingeengt und der Rückstand an Kieselgel chromatographiert (Laufmittel: Cyclohexan/Essigsäureethylester 10:1). Man erhielt 2.51 g (64% d. Th.) der gewünschten Verbindung.

### Methode B:

Die Reaktion wurde unter Zuhilfenahme eines Chlorlaugenwäschers durchgeführt. 3-Fluor-4-methylbenzonitril (200 g, 1479.9 mmol) wurde in DMF (1.5 Liter) vorgelegt, auf 40°C erwärmt und portionsweise (je ca. 25 g) mit Natriummethanthiolat (insgesamt 126.8 g, 1627.9 mmol) versetzt. Während der Zugabe stieg die Temperatur auf 100°C an. Das Reaktionsgemisch wurde zunächst 1.5 h bei 175°C Badtemperatur und dann über Nacht bei Raumtemperatur gerührt. Der Ansatz wurde danach auf Wasser (7.5 Liter) gegossen und zweimal mit Essigsäureethylester (jeweils 1875 ml) extrahiert. Die vereinigten organischen Phasen wurden mit gesättigter Natriumchlorid-Lösung (1875 ml) gewaschen, am Rotationsverdampfer eingeengt und der Rückstand an Kieselgel chromatographiert (Laufmittel: Petrolether/Essigsäureethylester 95:5, ca. 30 Liter). Nach Abziehen des Lösungsmittels am Rotationsverdampfer und Trocknen im Hochvakuum erhielt man 172 g (71% d. Th.) der gewünschten Verbindung.

GC-MS (Methode 12): Rₜ = 5.25 min; MS (ESIpos): *m*/*z* (%) = 163.0 (100) [M]⁺.

¹H-NMR (400 MHz, DMSO-d₆): δ = 2.30 (s, 3H), 2.54 (s, 3H), 7.38 (d, 1H), 7.52 (dd, 1H), 7.58 (br. s, 1H).

### Beispiel 5A

### 4-Methyl-3-(methylsulfonyl)benzonitril

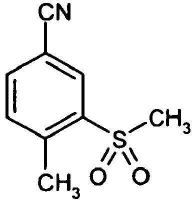

### Methode A:

4-Methyl-3-(methylsulfanyl)benzonitril (14050 mg, 80.1 mmol) wurde in Dichlormethan (700 ml) gelöst, auf 0°C gekühlt und langsam mit 3-Chlorperbenzoesäure (50923 mg, 206.6 mmol) versetzt. Anschließend wurde zunächst 40 min bei 0°C und dann über Nacht bei Raumtemperatur gerührt. Die ausgefallene 3-Chlorbenzoesäure wurde abfiltriert, das Filtrat mit 1 N Natronlauge gewaschen, die organische Phase über Natriumsulfat getrocknet und eingeengt. Der Rückstand wurde mittels Kieselgel-Chromatographie gereinigt (Laufmittel: Cyclohexan/Essigsäureethylester 1:1, 1:2). Man erhielt 13.65 g (81% d. Th.) der gewünschten Verbindung.

### Methode B:

3-Chlorperbenzoesäure (2501 g, 10144.4 mmol) wurde in 27.2 Liter Dichlormethan gelöst, auf 10°C gekühlt und portionsweise mit 4-Methyl-3-(methylsulfanyl)benzonitril (552 g, 3381.5 mmol) versetzt. Nach vollständiger Zugabe wurde 5 h bei RT gerührt. Die ausgefallene 3-Chlorbenzoesäure wurde abgesaugt und der Feststoff mit Dichlormethan (3 Liter) nachgewaschen. Die vereinigten Filtrate wurden mit 1 N Natronlauge (15 Liter) gerührt, das Gemisch filtriert und die organische Phase abgetrennt. Diese wurde nochmals mit 1 N Natronlauge (15 Liter) gerührt, von der Natronlauge abgetrennt, getrocknet und am Rotationsverdampfer eingeengt. Der Rückstand wurde in Diethylether (4 Liter) aufgeschlämmt, 10 min gerührt und dann filtriert. Der Feststoff wurde mit etwas Diethylether nachgewaschen und im Hochvakuum getrocknet. Man erhielt 613 g (93% d. Th.) der gewünschten Verbindung.

GC-MS (Methode 12): Rₜ = 6.59 min; MS (ESIpos): *m*/*z* (%) = 195.0 (100) [M]⁺.

¹H-NMR (400 MHz, DMSO-d₆): δ = 2.30 (s, 3H), 2.54 (s, 3H), 7.38 (d, 1H), 7.52 (dd, 1H), 7.58 (br. s, 1H).

### Beispiel 6A

### 4-[2-(Dimethylamino)ethenyl]-3-(methylsulfonyl)benzonitril

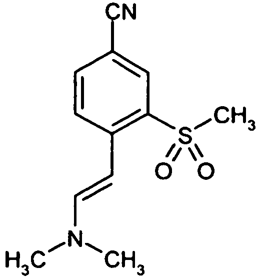

### Methode A:

Die Reaktion wurde unter Argon durchgeführt. 4-Methyl-3-(methylsulfonyl)benzonitril (13.00 g, 66.6 mmol) und 1,1 -Dimethoxy-*N,N*-dimethylmethanamin (10.315 g, 86.6 mmol) wurden 14 h bei 140°C in DMF (200 ml) gerührt. Zur Vervollständigung der Reaktion wurde danach erneut 1,1-Dimethoxy-*N,N*-dimethylmethanamin (3.967 g, 33.3 mmol) zugegeben und weitere 24 h bei 140°C gerührt. Das DMF wurde dann am Rotationsverdampfer abgezogen und der Rückstand ohne weitere Aufreinigung in der Folgestufe umgesetzt.

### Methode B:

Die Reaktion wurde unter Argon durchgeführt. 4-Methyl-3-(methylsulfonyl)benzonitril (612 g, 3134.6 mmol) wurde in DMF (6.12 Liter) vorgelegt, mit 1,1-Dimethoxy-*N,N*-dimethylmethanamin (859 g, 7209.5 mmol) versetzt und 7 h bei 140°C gerührt. Die Reaktionsmischung wurde dann auf 35 Liter 10%-ige Natriumchlorid-Lösung gegossen und zweimal mit jeweils 10 Liter Essigsäureethylester extrahiert. Die vereinigten organischen Phasen wurden mit gesättigter Natriumchlorid-Lösung (5 Liter) gewaschen, getrocknet, am Rotationsverdampfer eingeengt und der Rückstand über Nacht im Hochvakuum getrocknet. Auf diese Weise erhielt man 1098 g (98% d. Th.) der gewünschten Verbindung.

GC-MS (Methode 12): R*ₜ* = 8.95 min; MS (ESIpos): *m*/*z* (%) = 250.0 (10) [M]⁺.

### Beispiel 7A

### 4-Formyl-3-(methylsulfonyl)benzonitril

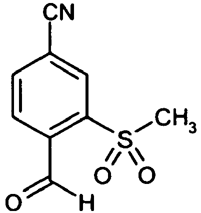

### Methode A:

4-[2-(Dimethylamino)ethenyl]-3-(methylsulfonyl)benzonitril (16.666 g, 66.6 mmol) wurde in Wasser/THF (1:1, 500 ml) vorgelegt, mit Natriumperiodat (42.722 g, 199.7 mmol) versetzt und über Nacht bei Raumtemperatur gerührt. Der ausgefallene Feststoff wurde abfiltriert und mit Essigsäureethylester nachgewaschen. Die vereinigten organischen Phasen wurden mit gesättigter Natriumhydrogencarbonat- und gesättigter Natriumchlorid-Lösung gewaschen, über Natriumsulfat getrocknet, filtriert und eingeengt. Der Rückstand wurde per Kieselgel-Chromatographie gereinigt (Laufmittel: Cyclohexan/Essigsäureethylester 1:1). Man erhielt 4.6 g (33% d. Th.) der gewünschten Verbindung.

### Methode B:

4-[2-(Dimethylamino)ethenyl]-3-(methylsulfonyl)benzonitril (1098 g, 3070.5 mmol) wurde in THF/Wasser (1:1, 13.8 Liter) vorgelegt, mit Natriumperiodat (1970 g, 9211.4 mmol) versetzt und 1 h bei Raumtemperatur gerührt. Der ausgefallene Feststoff wurde abgesaugt und mit Essigsäureethylester (17 Liter) nachgewaschen. Die vereinigten Filtrate wurden mit Wasser (17 Liter) versetzt, und nach erfolgter Extraktion wurde die wässrige Phase abgetrennt. Die organische Phase wurde mit gesättigter Natriumhydrogencarbonat-Lösung (8.5 Liter) und gesättigter Natriumchlorid-Lösung (8.5 Liter) gewaschen, dann getrocknet und am Rotationsverdampfer eingeengt. Die Reinigung des Rückstands erfolgte mittels Kieselgel-Chromatographie (Laufmittel: Dichlormethan/Essigsäureethylester 9:1, 60 Liter). Die Produktfraktionen wurden eingeengt, der Rückstand in Petrolether aufgeschlämmt, dann abgesaugt und der Feststoff im Hochvakuum über Nacht getrocknet. Auf diese Weise erhielt man 436 g (65% d. Th.) der gewünschten Verbindung.

GC-MS (Methode 12): R*ₜ* = 6.89 min; MS (ESIpos): *m*/*z* (%) = 191.1 (15) [M-18]⁺, 161.0 (100).

¹H-NMR (400 MHz, DMSO-d₆): δ = 3.57 (s, 3H), 8.10 (d, 1H), 8.39 (dd, 1H), 8.45 (d, 1H), 10.63 (s, 1H).

### Beispiel 8A

### 4-(2-Cyano-3-oxobut-1-en-1-yl)-3-(methylsulfonyl)benzonitril

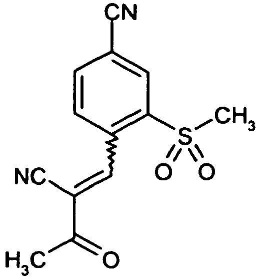

4-Formyl-3-(methylsulfonyl)benzonitril (3.0 g, 14.34 mmol) und Natrium-1-cyanoprop-1-en-2-olat (1.66 g, 15.8 mmol, 1.1 eq.; vgl. R. Troschütz, Archiv der Pharmazie 1984, 317, 709-713) wurden in Dichlormethan (180 ml) vorgelegt. Dann wurden Eisessig (1.03 ml, 18 mmol, 1.25 eq.) und Piperidin (142 µl, 1.43 mmol, 0.1 eq.) zugegeben, und die Mischung wurde am Wasserabscheider unter Rückfluss erhitzt (18 h). Die Reaktionslösung wurde dann einmal mit Wasser (50 ml) und zweimal mit gesättigter Natriumhydrogencarbonat-Lösung (je 100 ml) gewaschen, über Natriumsulfat getrocknet und eingeengt. Der feste Rückstand wurde in der sechsfachen Menge Ethanol suspendiert, wieder abgesaugt, und die Kristalle mit Ethanol nachgewaschen und im Hochvakuum getrocknet. Es wurden 2.7 g (70% Reinheit, 48% d. Th.) der Titelverbindung erhalten.

LC-MS (Methode 7): Rₜ = 1.29 min; MS (ESIneg): *m*/*z* (%) = 209.4 (100), 273.3 (45) [M-H]⁻.

¹H-NMR (400 MHz, DMSO-d₆): δ = 2.55 (s, 3H), 3.4 (s, 3H), 8.05 (m, 1H), 8.4 (m, 1H), 8.5 (s, 1H), 8.9 (s, 1H).

### Beispiel 9A

### (rac)-2-Amino-7-[4-cyano-2-(methylsulfonyl)phenyl]-5-methyl-4,7-dihydro[1,2,4]triazolo[1,2,4-a]-pyrimidin-6-carbonitril

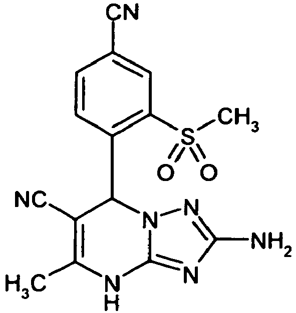

4-(2-Cyano-3-oxobut-1-en-1-y1)-3-(methylsulfonyl)benzonitril (1.0 g, 3.65 mmol), 3,5-Diaminotriazol (1.08 g, 10.94 mmol, 3.0 eq.), D,L-Prolin (214 mg, 2.55 mmol, 0.7 eq.) und Molekularsieb (4 Å, 0.5 g) wurden unter Argon in einer Mischung aus Pyridin (20 ml) und DMF (30 ml) suspendiert und 3 d lang gerührt. Danach wurde nochmals 3,5-Diaminotriazol (361 mg, 3.64 mmol, 1 eq.) zugegeben und die Mischung weitere 5 d lang gerührt. Das Reaktionsgemisch wurde dann filtriert und das Filtrat im Vakuum eingeengt (Zwischenprodukt 4-[2-Cyano-1-(3,5-diamino-1*H-*1,2,4-triazol-1-yl)-3-oxobutyl]-3-(methylsulfonyl)benzonitril).

Das Zwischenprodukt wurde mit Toluol (68 ml), Pyridin (55 ml) und 4-Toluolsulfonsäure-Monohydrat (100 mg, 0.53 mmol, 0.14 eq.) versetzt und unter Rückfluss erhitzt (1 h). Das Reaktionsgemisch wurde anschließend im Vakuum eingeengt und der Rückstand im Hochvakuum getrocknet. Der Rückstand wurde mit Ethanol (30 ml) versetzt, im Ultraschall-Bad suspendiert, gerührt, der Feststoff dann abfiltriert und zuletzt im Hochvakuum getrocknet. Es wurden 673 mg (50% d. Th.) der Titelverbindung erhalten.

LC-MS (Methode 7): Rₜ = 1.00 min; MS (ESIpos): *m*/*z* (%) = 356.1 (100) [M+H]⁺; MS (ESIneg): *m*/*z* (%) = 354.2 (100) [M-H]⁻.

¹H-NMR (400 MHz, DMSO-d₆): δ = 2.15 (s, 3H), 3.55 (s, 3H), 5.40 (m, 2H), 7.05 (s, 1H), 7.75 (m, 1H), 8.25 (m, 1H), 8.35 (s, 1H), 11.05 (s, 1H).

### Beispiel 10A

### (rac)-7-[4-Cyano-2-(methylsulfonyl)phenyl]-2-(1,3-dioxo-1,3-dihydro-2H-isoindol-2-yl)-5-methyl-4,7-dihydro[1,2,4]triazolo[1,5-a]pyrimidin-6-carbonitril

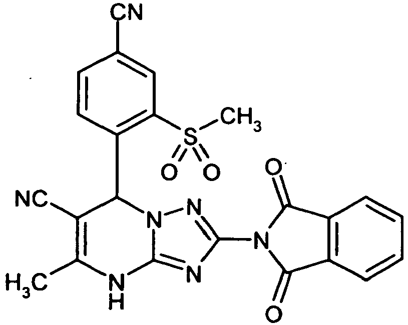

*(rac*)-2-Amino-7-[4-cyano-2-(methylsulfonyl)phenyl]-5-methyl-4,7-dihydro[1,2,4]triazolo[1,5-a]-pyrimidin-6-carbonitril (60 mg, 0.17 mmol) wurde unter Argon zusammen mit Phthalsäureanhydrid (50.02 mg, 0.34 mmol, 2.0 eq.) in einem Gemisch aus Toluol (4 ml) und Pyridin (2 ml) suspendiert, mit Triethylamin (4.7 µl, 0.034 mmol, 0.2 eq.) versetzt und 2 h unter Rückfluss erhitzt. Das Reaktionsgemisch wurde anschließend im Vakuum eingeengt. Das Produkt wurde aus dem Rückstand durch Zugabe von Ethanol (2 ml) im Ultraschall-Bad ausgefällt. Der Niederschlag wurde abfiltriert und im Hochvakuum getrocknet. Es wurden 67 mg (75.6% d. Th.) der Titelverbindung erhalten.

LC-MS (Methode 5): Rₜ = 0.97 min; MS (ESIpos): *m*/*z* (%) = 486.0 (100) [M+H]⁺, 507.9 (30) [M+Na]⁺; MS (ESIneg): *m*/*z* (%) = 484.4 (100) [M-H]⁻.

¹H-NMR (400 MHz, DMSO-d₆): δ = 2.25 (s, 3H), 3.55 (s, 3H), 7.45 (s, 1H), 7.95 (m, 4H), 8.05 (m, 1H), 8.35 (m, 1H), 8.45 (s, 1H), 11.65 (s, 1H).

### Beispiel 11A

### Ethyl 2-amino-7-(4-cyanophenyl)-5-methyl-4,7-dihydro[1,2,4]triazolo[1,5-a]pyrimidin-6-carboxylat

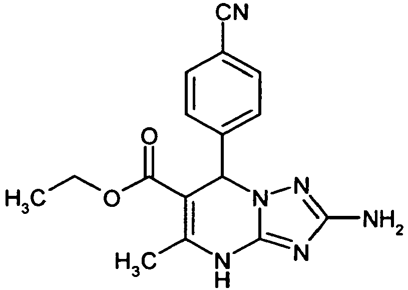

Ethyl 2-(4-cyanobenzyliden)-3-oxobutanoat (12.2 g, 50.1 mmol; Herstellung siehe WO 2004/ 020410-A2, Example 32A) und 1*H-*1,2,4-Triazol-3,5-diamin (6.0 g, 60.5 mmol, 1.2 eq.) wurden unter einer Argonatmosphäre in DMF (150 ml) gelöst. Festes Natriumhydrogencarbonat (30.7 g, 365.6 mmol, 6 eq.) wurde zugegeben und das Gemisch 12 h bei 63°C gerührt. Danach wurde filtriert und das DMF aus dem Filtrat im Vakuum abdestilliert. Der Rückstand wurde in Ethanol suspendiert und gerührt, das Produkt dann abfiltriert und im Hochvakuum getrocknet. Es wurden 12.45 g (76% d. Th.) der Titelverbindung erhalten.

LC-MS (Methode 4): R, = 2.29 min; MS (ESIpos): *m*/*z* (%) = 325.3 (100) [M+H]⁺; MS (ESIneg): *m*/*z* (%) = 323.3 (100) [M-H]⁻.

¹H-NMR (400 MHz, DMSO-d₆): δ = 1.00 (t, 3H), 2.40 (s, 3H), 3.95 (m, 2H), 5.25 (s, 2H), 6.05 (s, 1H), 7.35 (m, 2H), 7.75 (m, 2H), 10.6 (s, 1H).

### Beispiel 12A

### Ethyl 7-(4-cyanopheny)-2-(1,3-dioxo-1,3-dihydro-2H-isoindol-2-yl)-5-methyl-4,7-dihydro[1,2,4]-triazolo[1,5-a]pyrimidin-6-carboxylat

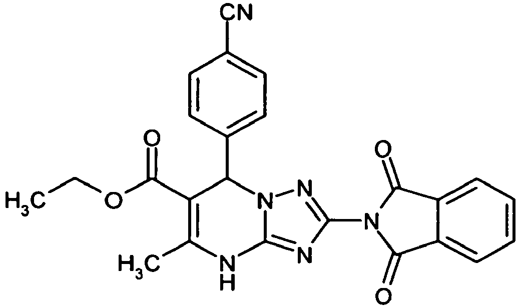

Ethyl 2-amino-7-(4-cyanophenyl)-5-methyl-4,7-dihydro[1,2,4]triazolo[1,5-a]pyrimidin-6-carboxylat (5.0 g, 15.4 mmol) und Phthalsäureanhydrid (3.4 g, 23.1 mmol, 1.5 eq.) wurden unter Argon in einem Gemisch aus Toluol (400 ml) und Pyridin (200 ml) gelöst, mit Triethylamin (42 µl, 0.3 mmol, 0.2 eq.) versetzt und 12 h unter Rückfluss erhitzt. Das Reaktionsgemisch wurde danach im Vakuum eingeengt. Der Rückstand wurde in Essigsäureethylester aufgenommen (600 ml) und zweimal mit gesättigter Natriumhydrogencarbonat-Lösung (je 150 ml) extrahiert. Die organische Phase wurde mit gesättigter Kochsalz-Lösung gewaschen, über Natriumsulfat getrocknet und im Vakuum eingeengt. Das Rohprodukt wurde in Ethanol (100 ml) suspendiert, für 12 h gerührt, der Feststoff dann abfiltriert und im Hochvakuum getrocknet. Es wurden 6.67 g (95% d. Th.) der Titelverbindung erhalten.

LC-MS (Methode 4): Rₜ = 3.11 min; MS (ESIpos): *m*/*z* (%) = 455.2 (100) [M+H]⁺; MS (ESIneg): *m*/*z* (%) = 453.2 (100) [M-H]⁻.

¹H-NMR (400 MHz, DMSO-d₆): δ = 1.0 (t, 3H), 2.4 (s, 3H), 3.95 (m, 2H), 6.5 (s, 1H), 7.55 (m, 2H), 7.85 (m, 2H), 7.95 (m, 4H), 11.15 (s, 1H).

### Beispiel 13A

### (rac)-7-(4-Cyanophenyl)-5-methyl-2-[(trifluoracetyl)amino]-4,7-dihydro[1,2,4]triazolo[1,5-a]-pyrimidin-6-carbonsäureethylester

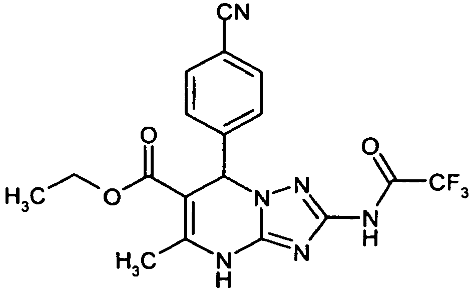

Ethyl 2-amino-7-(4-cyanophenyl)-5-methyl-4,7-dihydro[1,2,4]triazolo[1,5-a]pyrimidin-6-carboxylat (50 mg, 0.15 mmol) wurde unter einer Argon in Pyridin (1 ml) gelöst und mit Trifluoressigsäureanhydrid (35 µl, 0.25 mmol, 1.6 eq.) versetzt. Nach 1 h Rühren wurde das Lösungsmittel im Vakuum abdestilliert. Der Rückstand wurde mittels präparativer HPLC gereinigt (Gromsil C18-Säule, 30 x 250 mm; Eluent: Acetonitril-Wasser-0.1% TFA). Das Produkt wurde nach Lyophilisation als Feststoff erhalten (60.6 mg, 93% d. Th.).

LC-MS (Methode 4): Rₜ = 3.02 min; MS (ESIpos): *m*/*z* (%) = 421.3 (100) [M+H]⁺; MS (ESIneg): *m*/*z* (%) = 419.3 (100) [M-H]⁻.

¹H-NMR (400 MHz, DMSO-d₆): δ = 1.0 (t, 3H), 2.45 (s, 3H), 3.95 (m, 2H), 6.35 (s, 1H), 7.5 (m, 2H), 7.85 (m, 2H), 11.1 (s, 1H), 12.0 (s, 1H).

### Beispiel 14A

### Benzyl 4-nitrophenyl {(7R)-6-cyano-7-(4-cyanophenyl)-5-methyl-4-[3-(trifluormethyl)phenyl]-4,7-dihydro[1,2,4]triazolo[1,5-a]pyrimidin-2-yl}imidodicarbonat

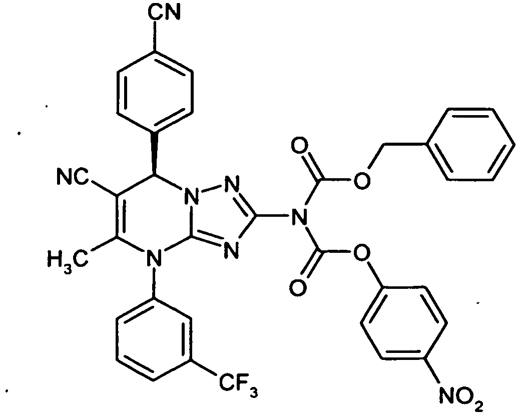

Unter einer Argonschutzgasatmosphäre wurden zu einer Lösung von Benzyl {(7R)-6-cyano-7-(4-cyanophenyl)-5-methyl-4-[3-(trifluormethyl)phenyl]-4,7-dihydro[1,2,4]triazolo[1,5-a]pyrimidin-2-yl}carbamat (30 mg, 54 µmol; Beispiel 38) in trockenem Dichlormethan (2 ml) bei 0°C nacheinander Chlorameisensäure-4-nitrophenylester (27.2 mg, 135 µmol, 2.5 eq.), Triethylamin (11.1 mg, 140 µmol, 2.6 eq.) sowie DMAP (0.7 mg, 5.4 µmol, 0.1 eq.) gegeben und die Mischung dann 5 h gerührt. Diese Reaktionslösung wurde als solche ohne weitere Aufreinigung für Folgereaktionen eingesetzt.

LC-MS (Methode 7): Rₜ = 2.50 min; MS (ESIpos): *m*/*z* (%) = 721.4 (100) [M+H]⁺; MS (ESIneg): *m*/*z* (%) = 719.4 (100) [M-H]⁻.

### Beispiel 15A

### (rac)-7-(4-Cyanophenyl)-2-(methoxymethyl)-5-methyl-4,7-dihydro[1,2,4]triazolo[1,5-a]pyrimidin-6-carbonitril

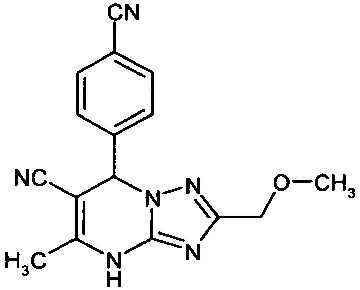

3-(Methoxymethyl)-1*H*-1,2,4-triazol-5-amin (244 mg, 1.9 mmol) und 4-(2-Cyano-3-oxobut-1-en-1-yl)benzonitril (300 mg, 1.5 mmol, 0.8 eq.) wurden unter Argonatmosphäre in DMF (3 ml) gelöst und mit festem Natriumhydrogencarbonat (803 mg, 9.6 mmol, 5 eq.) versetzt. Das Gemisch wurde 12 h lang bei 55°C gerührt. Danach wurde filtriert und das DMF aus dem Filtrat im Vakuum abdestilliert. Der Rückstand wurde mit 1 N Salzsäure angesäuert und dann mittels präparativer HPLC gereinigt (Kromasil C18-Säule, 30 x 250 mm; Eluent: Acetonitril-Wasser-0.1% TFA). Das Produkt wurde als Feststoff erhalten (38.2 mg, 80% Reinheit, 7% d. Th.).

LC-MS (Methode 2): Rₜ = 2.18 min; MS (ESIpos): *m*/*z* (%) = 307.0 (100) [M+H]⁺; MS (ESIneg): *m*/*z* (%) = 305.0 (100) [M-H]⁻.

¹H-NMR (400 MHz, DMSO-d₆): δ = 2.2 (s, 3H), 3.2 (s, 3H), 4.2 (s, 2H), 6.4 (s, 1H), 7.5 (m, 2H), 7.9 (m, 2H), 11.3 (s, 1H).

### Beispiel 16A

### (rac)-7-(4-Cyanophenyl)-5-methyl-2-thiophen-2-yl-4,7-dihydro[1,2,4]triazolo[1,5-a]pyrimidin-6-carbonitril

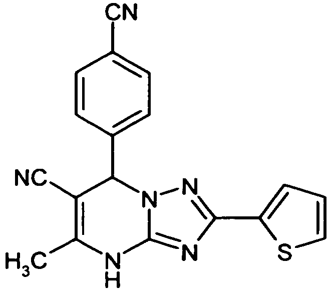

3-(2-Thienyl)-1*H-*1,2,4-triazol-5-amin (318 mg, 1.9 mmol) und 4-(2-Cyano-3-oxobut-1-en-1-yl)-benzonitril (300 mg, 1.5 mmol, 0.8 eq.) wurden unter Argonatmosphäre in DMF (3 ml) gelöst und mit festem Natriumhydrogencarbonat (803 mg, 9.6 mmol, 5 eq.) versetzt. Das Gemisch wurde 12 h lang bei 55°C gerührt. Danach wurde filtriert und das DMF aus dem Filtrat im Vakuum abdestilliert. Der Rückstand wurde mittels präparativer HPLC gereinigt (Kromasil C18-Säule, 30 x 250 mm; Eluent: Acetonitril-Wasser-0.1% TFA). Das Produkt wurde als Feststoff erhalten (73.7 mg, 91% Reinheit, 12% d. Th.).

LC-MS (Methode 2): Rₜ = 2.18 min; MS (ESIpos): *m*/*z* (%) = 344.9 (100) [M+H]⁺; MS (ESIneg): *m*/*z* (%) = 343.0 (100) [M-H]⁻.

¹H-NMR (400 MHz, DMSO-d₆): δ = 2.2 (s, 3H), 6.45 (s, 1H), 7.1 (m, 1H), 7.5 (m, 1H), 7.55 (m, 2H), 7.6 (m, 1H), 7.9 (m, 2H), 11.4 (s, 1H).

### Beispiel 17A

### (rac)-Ethyl 7-(4-cyanophenyl)-5-methyl-4,7-dihydro[1,2,4]triazolo[1,5-a]pyrimidin-6-carboxylat

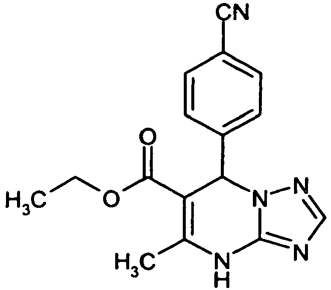

Ethyl 2-(4-cyanobenzyliden)-3-oxobutanoat (300 mg, 1.2 mmol) und 1*H*-1,2,4-Triazol-3-amin (130 mg, 1.5 mmol, 1.2 eq.) wurden unter Argonatmosphäre in DMF (3 ml) gelöst und mit festem Natriumhydrogencarbonat (518 mg, 6.2 mmol, 5 eq.) versetzt. Das Gemisch wurde 12 h lang bei 65°C gerührt. Danach wurde filtriert und das DMF aus dem Filtrat im Vakuum abdestilliert. Der Rückstand wurde mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (233 mg, 50% d. Th.).

LC-MS (Methode 1): Rₜ = 1.67 min; MS (ESIpos): *m*/*z* (%) = 310.3 (100) [M+H]⁺; MS (ESIneg): *m*/*z* (%) = 308.3 (100) [M-H]⁻.

¹H-NMR (400 MHz, DMSO-d₆): δ = 1.0 (t, 3H), 2.45 (s, 3H), 3.95 (m, 2H), 6.35 (s, 1H), 7.45 (m, 2H), 7.7 (s, 1H), 7.8 (m, 2H) 10.95 (s, 1H).

### Beispiel 18A

### (rac)-Ethyl 7-(4-cyanophenyl)-2-methoxy-5-methyl-4,7-dihydro[1,2,4]triazolo[1,5-a]pyrimidin-6-carboxylat

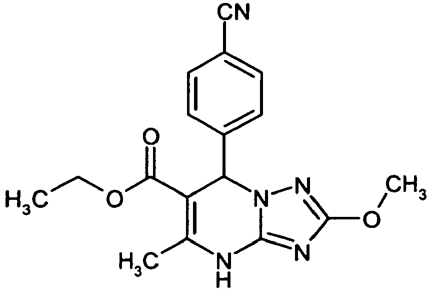

Ethyl 2-(4-cyanobenzyliden)-3-oxobutanoat (297 mg, 1.2 mmol) und 5-Methoxy-1*H*-1,2,4-triazol-3-amin (153 mg, 1.3 mmol, 1.1 eq.) wurden unter Argonatmosphäre in DMF (2.5 ml) gelöst und mit festem Natriumhydrogencarbonat (513 mg, 6.1 mmol, 5 eq.) versetzt. Das Gemisch wurde 12 h lang bei 65°C gerührt. Danach wurde filtriert und das DMF aus dem Filtrat im Vakuum abdestilliert. Der Rückstand wurde mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (191 mg, 42% d. Th.).

LC-MS (Methode 1): Rₜ = 1.77 min; MS (ESIpos): *m*/*z* (%) = 340.3 (100) [M+H]⁺; MS (ESIneg): *m*/*z* (%) = 338.3 (100) [M-H]⁻.

¹H-NMR (400 MHz, DMSO-d₆): δ = 1.0 (t, 3H), 2.35 (s, 3H), 3.7 (s, 3H), 3.95 (m, 2H), 6.15 (s, 1H), 7.4 (m, 2H), 7.8 (m, 2H), 10.85 (s, 1H).

### Beispiel 19A

### (rac)-Diethyl 7-(4-cyanophenyl)-5-methyl-4,7-dihydro[1,2,4]triazolo[1,5-a]pyrimidin-2,6-di-carboxylat

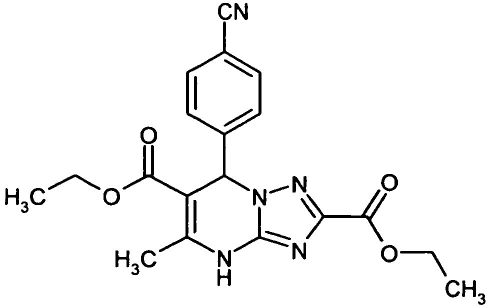

3-Amino-1*H*-1,2,4-triazol-5-carbonsäureethylester (700 mg, 4.4 mmol) und Ethyl 2-(4-cyanobenzyliden)-3-oxobutanoat (1.18 g, 4.9 mmol, 1.1 eq.) wurden unter Argonatmosphäre in DMF (5 ml) gelöst und mit festem Natriumhydrogencarbonat (1.86 g, 22.1 mmol, 5 eq.) versetzt. Das Gemisch wurde 6 h lang bei 55°C gerührt. Danach wurde filtriert und das DMF aus dem Filtrat im Vakuum abdestilliert. Der Rückstand wurde mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (1.3 g, 80% d. Th.).

LC-MS (Methode 3): Rₜ = 3.07 min; MS (ESIpos): *m*/*z* (%) = 382.3 (100) [M+H]⁺; MS (ESIneg): *m*/*z* (%) = 380.2 (100) [M-H]⁻.

¹H-NMR (400 MHz, DMSO-d₆): δ = 1.0 (t, 3H), 1.25 (t, 3H), 2.45 (s, 3H), 3.95 (m, 2H), 4.25 (m, 2H), 6.45 (s, 1H), 7.5 (m, 2H), 7.8 (m, 2H), 11.1 (s, 1H).

### Beispiel 20A

### (rac)-Ethyl 7-(4-cyanophenyl)-2,5-dimethyl-4,7-dihydro[1,2,4]triazolo[1,5-a]pyrimidin-6-carboxylat

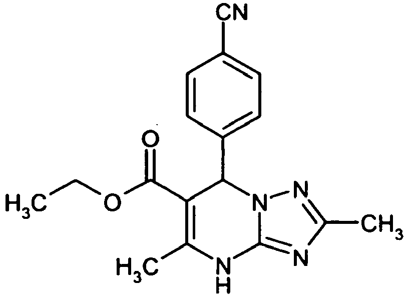

Ethyl 2-(4-cyanobenzyliden)-3-oxobutanoat (381 mg, 1.6 mmol) und 5-Methyl-4*H*-1,2,4-triazol-3-amin (200 mg, 2.0 mmol, 1.3 eq.) wurden unter Argonatmosphäre in DMF (4 ml) gelöst und mit festem Natriumhydrogencarbonat (659 mg, 7.8 mmol, 5 eq.) versetzt. Das Gemisch wurde 12 h lang bei 65°C gerührt. Danach wurde filtriert und das DMF aus dem Filtrat im Vakuum abdestilliert. Der Rückstand wurde mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (300 mg, 46% d. Th.).

LC-MS (Methode 2): Rₜ = 1.92 min; MS (ESIpos): *m*/*z* (%) = 324.1 (100) [M+H]⁺; MS (ESIneg): *m*/*z* (%) = 322.1 (100) [M-H]⁻.

¹H-NMR (400 MHz, DMSO-d₆): δ = 1.0 (t, 3H), 2.1 (t, 3H), 2.4 (s, 3H), 3.4 (m, 2H), 6.25 (s, 1H), 7.4 (m, 2H), 7.8 (m, 2H), 10.75 (s, 1H).

### Beispiel 21A

### (rac)-7-(4-Cyanophenyl)-2-methoxy-5-methyl-4,7-dihydro[1,2,4]triazolo[1,5-a]pyrimidin-6-carbonitril

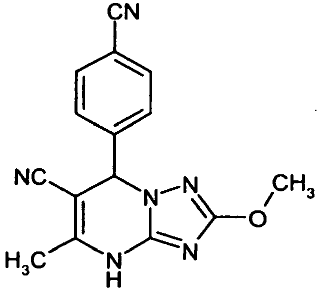

4-(2-Cyano-3-oxobut-1-en-1-yl)benzonitril (500 mg, 2.55 mmol), 3-Methoxy-1*H*-1,2,4-triazol-5-amin (291 mg, 2.55 mmol, 1.0 eq.), D,L-Prolin (151 mg, 1.27 mmol, 0.5 eq.) und Molekularsieb (4 Å, 0.5 g) wurden unter Argon in einer Mischung aus Pyridin (2 ml) und DMF (6 ml) gerührt (4 d). Die Lösungsmittel wurden danach im Vakuum abdestilliert und der Rückstand 12 h lang im Hochvakuum getrocknet (Zwischenprodukt 4-[1-(5-Amino-3-methoxy-1*H*-1,2,4-triazol-1-yl)-2-cyano-3-oxobutyl]benzonitril).

Das Zwischenprodukt wurde mit Toluol (30 ml), Pyridin (20 ml) und 4-Toluolsulfonsäure-Monohydrat (31 mg, 161 µmol, 0.1 eq.) versetzt und unter Rückfluss erhitzt (1 h). Das Reaktionsgemisch wurde anschließend filtriert, das Filtrat im Vakuum eingeengt und der Rückstand im Hochvakuum getrocknet. Der Rückstand wurde mit Ethanol (10 ml) versetzt, im Ultraschall-Bad suspendiert, gerührt und das Produkt dann abfiltriert. Es wurde nochmals mit Ethanol (10 ml) ausgerührt, abfiltriert und zuletzt im Hochvakuum getrocknet. Es wurden 179 mg (37% d. Th.) der Titelverbindung erhalten.

LC-MS (Methode 2): Rₜ = 1.69 min; MS (ESIpos): *m*/*z* (%) = 293.1 (100) [M+H]⁺; MS (ESIneg): *m*/*z* (%) = 291.2 (100) [M-H]⁻.

¹H-NMR (400 MHz, DMSO-d₆): δ = 2.15 (s, 3H), 3.7 (s, 3H), 6.2 (s, 1H), 7.5 (m, 2H), 7.9 (m, 2H), 11.25 (s, 1H).

### Beispiel 22A

### (rac)-Ethyl 6-(4-cyanophenyl)-8-methyl-6,9-dihydropyrimido[2,1-f]purin-7-carboxylat

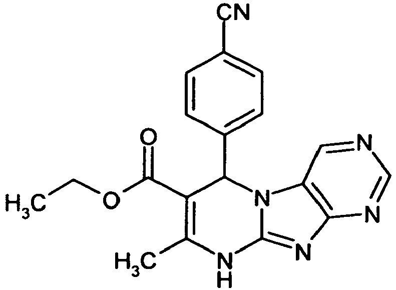

7H-Purin-8-amin (300 mg, 2.2 mmol) und Ethyl 2-(4-cyanobenzyliden)-3-oxobutanoat (702 mg, 2.9 mmol, 1.3 eq.) wurden unter einer Argonatmosphäre in DMF (5 ml) gelöst und mit festem Natriumhydrogencarbonat (932 mg, 11.1 mmol, 5 eq.) versetzt. Das Gemisch wurde 12 h lang bei 55°C gerührt. Danach wurde filtriert und das DMF aus dem Filtrat im Vakuum abdestilliert. Der Rückstand wurde mittels präparativer HPLC gereinigt (Gromsil C18-Säule, 30 x 250 mm; Eluent: Acetonitril-Wasser-0.1% TFA 10:90 → 90:10). Die Titelverbindung wurde nach Lyophilisation der entsprechenden Fraktionen als Feststoff erhalten (430 mg, 54% d. Th.). Als Nebenprodukt fiel die isomere Verbindung (rac)-Ethyl 9-(4-cyanophenyl)-7-methyl-6,9-dihydropyrimido[1,2-e]purin-8-carboxylat an (siehe Beispiel 23A).

LC-MS (Methode 2): Rₜ = 1.72 min; MS (ESIpos): *m*/*z* (%)=361.1 (100) [M+H]⁺; MS (ESIneg): *m*/*z* (%) = 359.2 (100) [M-H]⁻.

¹H-NMR (400 MHz, DMSO-d₆): δ = 1.1 (t, 3H), 2.5 (s, 3H), 4.0 (m, 2H), 6.7 (s, 1H), 7.7 (m, 2H), 7.8 (m, 2H), 8.65 (s, 1H), 8.75 (s, 1H), 11.6 (s, 1H).

### Beispiel 23A

### (rac)-Ethyl 9-(4-cyanophenyl)-7-methyl-6,9-dihydropyrimido[1,2-e]purin-8-carboxylat

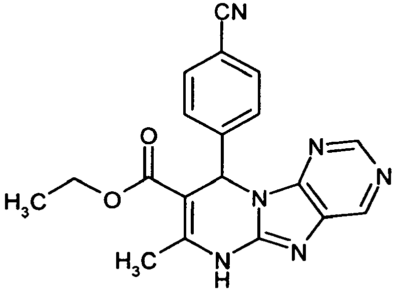

Die Titelverbindung wurde als Nebenprodukt bei der Herstellung der Verbindung aus Beispiel 22A erhalten und im Zuge der dort beschriebenen HPLC-Aufreinigung abgetrennt. Nach Lyophilisation der entsprechenden Fraktionen wurden 170 mg (2 1 % d. Th.) als Feststoff erhalten.

LC-MS (Methode 2): Rₜ = 1.87 min; MS (ESIpos): *m*/*z* (%) = 361.1 (100) [M+H]⁺; MS (ESIneg): *m*/*z* (%) = 359.2 (100) [M-H]⁻.

¹H-NMR (400 MHz, DMSO-d₆): δ = 1.0 (t, 3H), 2.5 (s, 3H), 4.0 (m, 2H), 6.55 (s, 1H), 7.55 (m, 2H), 7.75 (m, 2H), 8.65 (s, 1H), 8.75 (s, 1H), 11.3 (s, 1H).

### Beispiel 24A

### 7-(4-Cyanophenyl)-5-methyl-2-(propan-2-yl)-4,7-dihydro[1,2,4]triazolo[1,5-a]pyrimidin-6-carbonitril

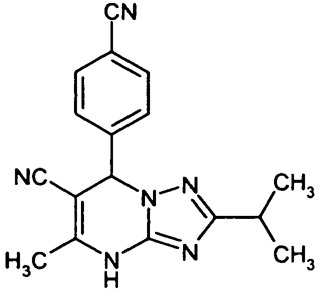

3-Isopropyl-1*H*-1,2,4-triazol-5-amin (241 mg, 1.9 mmol) und 4-(2-Cyano-3-oxobut-1-en-1-yl)-benzonitril (300 mg, 1.5 mmol, 0.8 eq.) wurden unter Argonatmosphäre in DMF (3 ml) gelöst und mit festem Natriumhydrogencarbonat (803 mg, 9.6 mmol, 5 eq.) versetzt. Das Gemisch wurde über Nacht bei 55°C gerührt. Danach wurde filtriert und das DMF aus dem Filtrat im Vakuum abdestilliert. Der Rückstand wurde in Methanol (2 ml) suspendiert, der Feststoff dann abfiltriert und im Hochvakuum getrocknet (34 mg, 7% d.Th.). Das Filtrat wurde mit 1 N Salzsäure angesäuert und mittels präparativer HPLC gereinigt (Kromasil C18-Säule, 30 x 250 mm; Eluent: Acetonitril-Wasser-0.1% TFA), wodurch eine zweite Produktfraktion (67 mg, 13% d. Th.) als Feststoff erhalten wurde.

LC-MS (Methode 2): Rₜ = 2.00 min; MS (ESIpos): *m*/*z* (%) = 305.1 (100) [M+H]⁺; MS (ESIneg): *m*/*z* (%) = 303.2 (100) [M-H]⁻.

¹H-NMR (400 MHz, DMSO-d₆): δ = 1.15 (dd, 6H), 2.15 (s, 3H), 2.8 (m, 1H), 6.35 (s, 1H), 7.45 (m, 2H), 7.9 (m, 2H), 11.2 (s, 1H).

### Beispiel 25A

### (rac)-7-(4-Cyanophenyl)-5-methyl-4,7-dihydrotetrazolo[1,5-a]pyrimidin-6-carbonsäureethylester

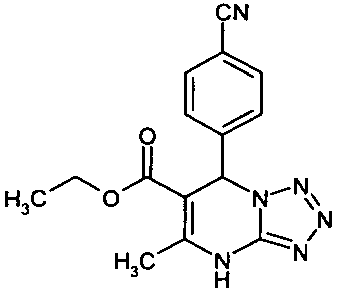

5-Amino-1,2,3,4-tetrazol-Hydrat (1.0 g, 9.7 mmol) und Ethyl 2-(4-cyanobenzyliden)-3-oxo-butanoat (2.6 g, 10.7 mmol, 1.1 eq.) wurden unter einer Argonatmosphäre in DMF (50 ml) gelöst und mit festem Natriumhydrogencarbonat (4.1 g, 48.5 mmol, 5 eq.) versetzt. Das Gemisch wurde 12 h lang bei 55°C gerührt. Danach wurde filtriert und das DMF aus dem Filtrat im Vakuum abdestilliert. Der Rückstand wurde in Ethanol suspendiert und über Nacht gerührt. Das Produkt wurde abfiltriert und im Hochvakuum getrocknet. Es wurden 950 mg (32% d. Th.) der Titelverbindung erhalten.

LC-MS (Methode 4): Rₜ = 2.58 min; MS (ESIpos): *m*/*z* (%) = 311.3 (100) [M+H]⁺; MS (ESIneg): *m*/*z* (%) = 309.3 (100) [M-H]⁻.

¹H-NMR (400 MHz, DMSO-d₆): δ = 1.00 (t, 3H), 3.30 (s, 3H), 3.95 (m, 2H), 6.80 (s, 1H), 7.50 (m, 2H), 7.85 (m, 2H), 11.40 (s, 1H).

### Beispiel 26A

### (rac)-4-(6-Acetyl-5-methyl-4,7-dihydrotetrazolo[1,5-a]pyrimidin-7-yl)benzonitril

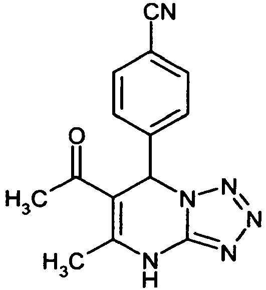

5-Amino-1,2,3,4-tetrazol-Hydrat (300 mg, 2.9 mmol) und 4-(2-Acetyl-3-oxobut-1-en-1-yl)benzonitril (2.6 g, 10.7 mmol, 1.1 eq.; Herstellung siehe WO 2005/080372-A1, Example 4A) wurden unter einer Argonatmosphäre in DMF (15 ml) gelöst und mit festem Natriumhydrogencarbonat (1.2 g, 14.5 mmol, 5 eq.) versetzt. Das Gemisch wurde über Nacht bei 62°C gerührt. Danach wurde filtriert und das DMF aus dem Filtrat im Vakuum abdestilliert. Der Rückstand wurde mit 1 N Salzsäure angesäuert und dann mittels präparativer HPLC gereinigt (Gromsil C18-Säule, 30 x 250 mm; Eluent: Acetonitril-Wasser-0.1% TFA). Die lyophilisierte Produktfraktion wurde in Ethanol (2 ml) suspendiert, der Feststoff dann abfiltriert und im Hochvakuum getrocknet. Es wurden 150 mg (18.4% d. Th.) der Titelverbindung erhalten.

LC-MS (Methode 4): Rₜ = 1.79 min; MS (ESIpos): *m*/*z* (%) = 281.3 (100) [M+H]⁺; MS (ESIneg): *m*/*z* (%) = 279.3 (100) [M-H]⁻.

¹H-NMR (400 MHz, DMSO-d₆): δ = 2.20 (s, 3H), 6.90 (s, 1H), 7.55 (m, 2H), 7.85 (m, 2H), 11.40 (s, 1H) (Signal einer Methylgruppe vom DMSO-Peak überlagert).

### Beispiel 27A

### Ethyl 5-(brommethyl)-7-(4-cyanophenyl)-4-[3-(trifluormethyl)phenyl]-4,7-dihydrotetrazolo[1,5-a]-pyrimidin-6-carboxylat

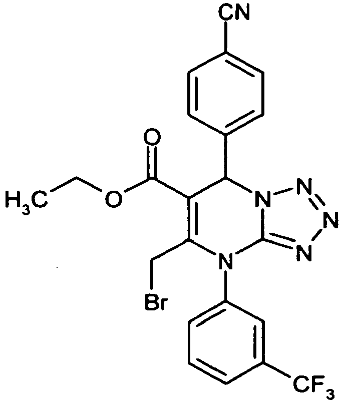

Ethyl 7-(4-cyanophenyl)-5-methyl-4-[3-(trifluormethyl)phenyl]-4,7-dihydrotetrazolo[1,5-a]pyrimidin-6-carboxylat (30 mg, 66.1 µmol; Beispiel 70) wurde in Chloroform (1 ml) gelöst und bei 0°C portionsweise mit einer Lösung von Brom (3 x 11.6 mg, 3 x 72.6 µmol, 3.3 eq.) in Chloroform versetzt. Die Mischung wurde anschließend für 12 h unter langsamen Erwärmen auf RT gerührt. Das Gemisch wurde dann mit Chloroform verdünnt und mit 10%-iger Natriumthiosulfat-Lösung gewaschen. Die organische Phase wurde abgetrennt, über Natriumsulfat getrocknet und nach Filtration am Rotationsverdampfer eingeengt. Das Rohprodukt wurde ohne weitere Aufreinigung weiter umgesetzt (35.3 mg, 87% Reinheit laut LC-MS, 87% d. Th.).

LC-MS (Methode 4): Rₜ = 3.78 min; MS (ESIpos): *m*/*z* (%) = 535.1 (100) [M+H]⁺.

### Beispiel 28A

### Ethyl 5-(4-cyanophenyl)-7-methyl-5,8-dihydroimidazo[1,2-a]pyrimidin-6-carboxylat

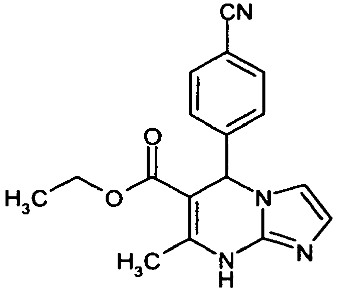

Ethyl 2-(4-cyanobenzyliden)-3-oxobutanoat (1.42 g, 5.82 mmol) und 1H-Imidazol-2-amin-Sulfat (2:1) (1000 mg, 3.78 mmol, 0.65 eq.) wurden unter Argonatmosphäre in DMF (10 ml) gelöst und mit festem Natriumhydrogencarbonat (1.96 g, 23.3 mmol, 4 eq.) versetzt. Das Gemisch wurde 12 h lang bei 65°C gerührt. Danach wurde über Kieselgur filtriert und das DMF aus dem Filtrat im Vakuum abdestilliert. Der Rückstand wurde mit Acetonitril (10 ml) versetzt. Das ausgefallene Produkt wurde abfiltriert, mit Ethanol gewaschen und im Hochvakuum getrocknet, wodurch eine erste Produktfraktion (503 mg) erhalten wurde. Das eingeengte Filtrat wurde mittels präparativer HPLC gereinigt (Gromsil C18-Säule, 30 x 250 mm; Eluent: Acetonitril-Wasser-0.1% TFA) und lyophilisiert. Auf diese Weise wurden 778 mg als weitere Produktfraktion erhalten (Gesamtausbeute: 71% d. Th.).

LC-MS (Methode 6): Rₜ = 2.66 min; MS (ESIpos): *m*/*z* (%) = 309.1 (100) [M+H]⁺; MS (ESIneg): *m*/*z* (%) = 307.1 (100) [M-H]⁻.

¹H-NMR (400 MHz, DMSO-d₆): δ = 1.05 (t, 3H), 2.44 (s, 3H), 4.0 (m, 2H), 6.40 (s, 1H), 7.05 (m, 2H), 7.55 (m, 2H), 7.85 (m, 2H), 11.45 (br. s, 1H).

### Beispiel 29A

### (7R)-6-Cyano-7-(4-cyanophenyl)-5-methyl-4-[3-(trifluormethyl)phenyl]-4,7-dihydro[1,2,4]triazolo-[1,5-a]pyrimidin-2-sulfonylchlorid

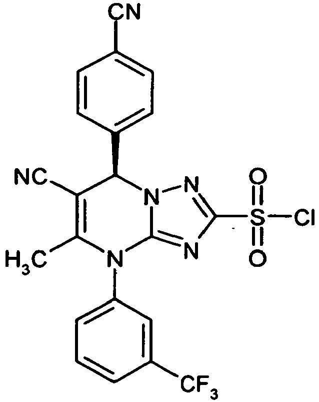

(7R)-2-Amino-7-(4-cyanophenyl)-5-methyl-4-[3-(trifluormethyl)phenyl]-4,7-dihydro[1,2,4]triazolo[1,5-a]pyrimidin-6-carbonitril (400 mg, 1.0 mmol; Beispiel 7, freie Base) wurde in einem Gemisch aus Essigsäure, konz. Salzsäure und Wasser (2:1:1, 10 ml) bei RT gelöst. Bei -10°C wurde langsam eine Lösung von Natriumnitrit (72 mg, 1.0 mmol, 1.1 eq.) in Wasser (1.1 ml) zum Reaktionsgemisch getropft. Man ließ langsam auf 0-2°C erwärmen und kühlte dann wieder auf -10°C ab. Diese Lösung wurde zu einer auf -10°C vortemperierten, mit Schwefeldioxid-Gas gesättigten und mit Kupfer(I)chlorid (18.8 mg, 0.019 mmol, 0.2 eq.) versetzten Eisessig-Lösung (11 ml) gegeben. Es trat kurzzeitig eine heftige Reaktion ein. Der Ansatz wurde 1 h bei -10°C gerührt, dann auf 15°C erwärmt und nochmals 1 h nachgerührt. Die Reaktionslösung wurde danach erneut auf 0°C abgekühlt. Das Produkt wurde ausgefällt, indem diese Reaktionslösung unter Rühren in Eiswasser (60 ml) getropft wurde. Der beigefarbene Niederschlag wurde abfiltriert, in Essigsäureethylester aufgenommen, mit gesättigter Natriumchlorid-Lösung gewaschen und anschließend über Natriumsulfat getrocknet. Nach Filtration wurde im Vakuum eingeengt und der Rückstand im Hochvakuum getrocknet. Das Produkt wurde als Feststoff (415 mg, 63% Reinheit, 55% d. Th.) erhalten.

LC-MS (Methode 5): Rₜ = 1.39 min; MS (ESIpos): *m*/*z* (%) = 505.1 (100) [M+H]⁺; MS (ESIneg): *m*/*z* (%) = 503.1 (90) [M-H]⁻.

¹H-NMR (400 MHz, DMSO-d₆): δ = 2.0 (s, 3H), 6.5 (s, 1H), 7.7-8.05 (m, 7H), 8.15 (br. s, 1H).

### Beispiel 30A

### (rac)-4-(4-Cyanophenyl)-6-methyl-2-thioxo-1-[3-(trifluormethyl)phenyl]-1,2,3,4-tetrahydro-pyrimidin-5-carbonitril

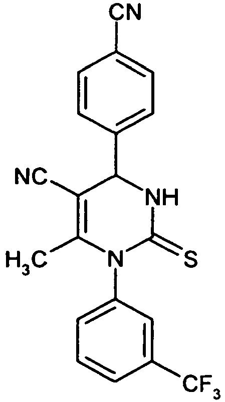

Unter einer Argonatmosphäre wurde Phosphorsäuretriethylester (12.4 ml, 73.24 mmol, 8 eq.) zusammen mit Diphosphorpentoxid (6.94 g, 48.86 mmol, 5.4 eq.) 4 h lang bei 50°C gerührt. Dann wurden abs. THF (60 ml), 3-(Trifluormethyl)phenylthioharnstoff (2.0 g, 9.08 mmol), 4-Cyanobenzaldehyd (2.0 g, 15.26 mmol, 1.7 eq.) sowie Acetessigsäureamid (1.54 g, 15.26 mmol, 1.7 eq.) zugegeben und das Gemisch 12 h unter Rückfluss erhitzt. Danach wurde die Reaktionsmischung im Vakuum eingeengt und der Rückstand direkt mittels präparativer HPLC gereinigt (Gromsil C18-Säule, 30 x 250 mm; Eluent: Acetonitril-Wasser). Nach Lyophilisation erhielt man die Titelverbindung als Feststoff (680 mg, 18% d. Th.).

LC-MS (Methode 5): Rₜ = 1.27 min; MS (ESIpos): m/z (%) = 399.2 (100) [M+H]⁺; MS (ESIneg): m/z (%) = 397.1 (20) [M-H]⁻.

¹H-NMR (400 MHz, DMSO-d₆): δ = 1.8 (s, 3H), 5.45 (s, 1H), 7.55-7.9 (m, 6H), 8.0 (m, 2H), 11.25 (br. m, 1H).

### Beispiel 31A

### (rac)-4-(4-Cyanophenyl)-2-hydrazinyl-6-methyl-1-[3-(trifluormethyl)phenyl]-1,4-dihydropyrimidin-5-carbonitril-Trifluoracetat

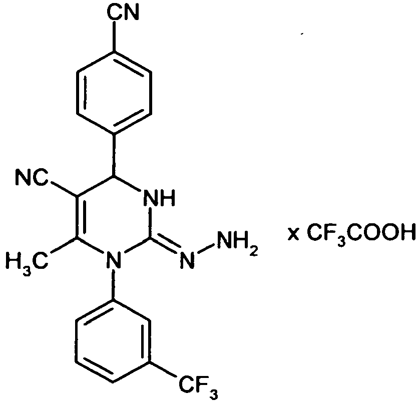

4-(4-Cyanophenyl)-6-methyl-2-thioxo-1-[3-(trifluormethyl)phenyl]-1,2,3,4-tetrahydropyrimidin-5-carbonitril (100 mg, 0.25 mmol) wurde in einem Gemisch aus Ethanol (10 ml) und Toluol (5 ml) gelöst. Bei 0°C wurde *tert*.-Butylhydroperoxid (3 M Lösung in Toluol, 602 µl, 1.81 mmol, 7.2 eq.) zugetropft. Die Mischung wurde 3 h bei RT gerührt und dann mit Hydrazinhydrat (183 µl, 3.76 mmol, 15 eq.) versetzt. Nach 12 h Rühren wurden nochmals *tert.*-Butylhydroperoxid-Lösung (3 M in Toluol, 167 µl, 0.50 mmol, 2 eq.) und Hydrazinhydrat (49 µl, 1.00 mmol, 4 eq.) nachgegeben und die Mischung für weitere 3 h gerührt. Das Gemisch wurde dann im Vakuum weitgehend eingeengt, mit Wasser (2 ml) versetzt und die restlichen organischen Lösungsmittel abdestilliert. Der wässrige Rückstand wurde mittels präparativer HPLC gereinigt (Gromsil C18-Säule, 30 x 250 mm; Eluent: Acetonitril-Wasser-0.1% TFA). Nach Lyophilisation erhielt man die Titelverbindung als Feststoff(13.7 mg, 14% d. Th.).

LC-MS (Methode 2): Rₜ = 1.45 min; MS (ESIpos): *m*/*z* (%) = 397.1 (100) [M+H]⁺; MS (ESIneg): *m*/*z* (%) = 395.2 (100) [M-H]⁻.

¹H-NMR (400 MHz, DMSO-d₆): δ = 1.8 (br. s, 3H), 5.6 (br. s, 1H), 7.55-7.95 (br. m, 7H), 8.0 (m, 2H), 8.15 (br. s, 1H), 9.3 (br. s, 1H).

### Ausführungsbeispiele:

### Beispiel 1

### (rac)-7-(4-Cyanophenyl)-2-(1,3-dioxo-1,3-dihydro-2H-isoindol-2-yl)-5-methyl-4-[3-(trifluormethyl)phenyl]-4,7-dihydro[1,2,4]triazolo[1,5-a]pyrimidin-6-carbonsäureethylester

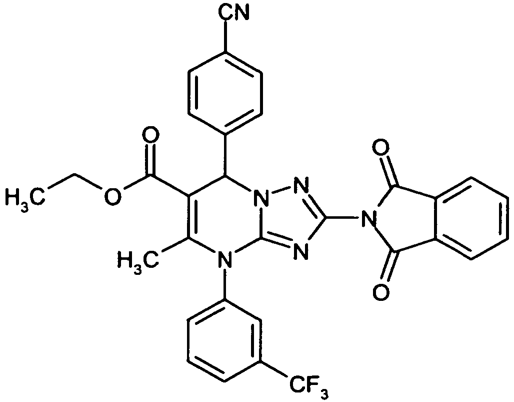

Ethyl 7-(4-cyanophenyl)-2-(1,3-dioxo-1,3-dihydro-2H-isoindol-2-yl)-5-methyl-4,7-dihydro[1,2,4]-triazolo[1,5-a]pyrimidin-6-carboxylat (50 mg, 0.11 mmol), 3-(Trifluormethyl)phenylboronsäure (63 mg, 0.33 mmol, 3 eq.), wasserfreies Kupfer(II)acetat (60 mg, 0.33 mmol, 3 eq.) und Molekularsieb (50 mg, 4 Å) wurden vorgelegt. Unter Argonschutzgasatmosphäre wurden abs. Dichlormethan (2.5 ml), Pyridin (71 µl, 0.88 mmol) und Triethylamin (46 µl, 0.33 mmol, 3 eq.) zugegeben. Nach 12 h Rühren wurden nochmals 3-(Trifluormethyl)phenylboronsäure (21 mg, 0.11 mmol, 1 eq.), wasserfreies Kupfer(II)acetat (20 mg, 0.11 mmol, 1 eq.) und Pyridin (36 µl, 0.44 mmol) hinzugefügt. Nach weiteren 24 h Rühren wurde über Kieselgur filtriert, mit Dichlormethan und Methanol nachgewaschen und das Filtrat im Vakuum eingeengt. Der Rückstand wurde mittels präparativer HPLC gereinigt (Gromsil C18-Säule, 30 x 250 mm; Eluent: Acetonitril-Wasser-0.1% TFA). Das Produkt wurde nach Lyophilisation als Feststoff erhalten (23.3 mg, 35% d. Th.).
LC-MS (Methode 4): Rₜ = 3.95 min; MS (ESIpos): *m*/*z* (%) = 599.2 (100) [M+H]⁺; MS (ESIneg): *m*/*z* (%) = 597.3 (20) [M-H]⁻.
¹H-NMR (400 MHz, DMSO-d₆): δ = 1.0 (t, 3H), 2.2 (s, 3H), 4.0 (m, 2H), 6.6 (s, 1H), 7.80-8.05 (m, 11H), 8.25 (br. s, 1H).

### Beispiel 2

### (rac)-7-(4-Cyanophenyl)-5-methyl-2-[(trifluoracetyl)amino]-4-[3-(trifluormethyl)phenyl]-4,7-dihydro[1,2,4]triazolo[1,5-a]pyrimidin-6-carbonsäureethylester

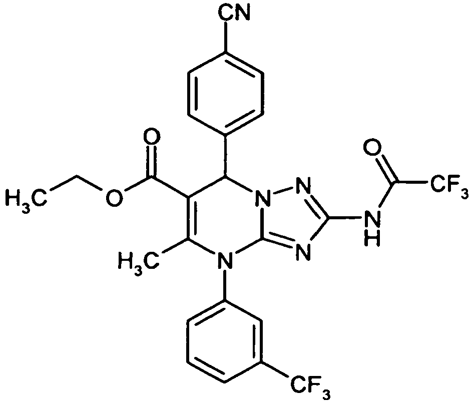

Ethyl 7-(4-cyanophenyl)-5-methyl-2-[(trifluoracetyl)amino]-4,7-dihydro[1,2,4]triazolo[1,5-a]pyrimidin-6-carboxylat (45 mg, 107 µmol) und 3-(Trifluormethyl)phenylboronsäure (61 mg, 321 µmol, 3 eq.) wurden zusammen mit wasserfreiem Kupfer(II)acetat (58 mg, 321 µmol, 3 eq.) und Molekularsieb (0.1 g, 4 Å) vorgelegt und unter Argonschutzgasatmosphäre in abs. Dichlormethan (3 ml) suspendiert. Dann wurden abs. Pyridin (69 µl, 856 µmol, 8 eq.) und Triethylamin (45 µl, 321 µmol, 3 eq.) zugegeben. Nach 12 h Rühren wurden nochmals 3-(Trifluormethyl)phenylboronsäure (20 mg, 107 µmol, 1 eq.), wasserfreies Kupfer(II)acetat (19 mg, 321 µmol, 1 eq.) und Pyridin (35 µl, 428 µmol, 4 eq.) hinzugefügt und die Mischung weitere 24 h gerührt. Anschließend wurde über Kieselgur filtriert, mit Dichlormethan und Methanol nachgewaschen und das Filtrat im Vakuum eingeengt. Der Rückstand wurde mittels präparativer HPLC gereinigt (Gromsil C18-Säule, 10 µm, 30 x 250 mm; Eluent: Acetonitril-Wasser-0.1% TFA). Das Produkt wurde nach Lyophilisation als Feststoff erhalten (10.3 mg, 17% d. Th.).

LC-MS (Methode 4): Rₜ = 3.80 min; MS (ESIpos): *m*/*z* (%) = 565.2 (100) [M+H]⁺; MS (ESIneg): *m*/*z* (%) = 563.2 (100) [M-H]⁻.

¹H-NMR (400 MHz, DMSO-d₆): δ = 1.05 (t, 3H), 2.2 (s, 3H), 4.0 (q, 2H), 6.5 (s, 1H), 7.7 (m, 2H), 7.75-8.00 (m, 5H), 8.2 (br. s, 1H), 12.05 (s, 1H).

### Beispiel 3

### (rac)-7-[4-Cyanophenyl]-2-(1,3-dioxo-1,3-dihydro-2H-isoindol-2-yl)-5-methyl-4-[3-(trifluormethyl)phenyl]-4,7-dihydro[1,2,4]triazolo[1,5-a]pyrimidin-6-carbonitril

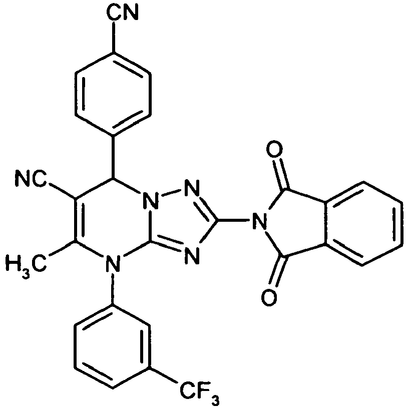

(*rac*)-7-[4-Cyanophenyl]-2-(1,3-dioxo-1,3-dihydro-2H-isoindol-2-yl)-5-methyl-4,7-dihydro[1,2,4]-triazolo[1,5-a]pyrimidin-6-carbonitril (12 g, 29.5 mmol) wurde unter Argonschutzgasatmosphäre in abs. Dichlormethan (600 ml) mit Molekularsieb (20 g, 4 Å) für 1 h gerührt. 3-(Trifluormethyl)-phenylboronsäure (16.78 g, 88.4 mmol, 3 eq.) wurde zusammen mit wasserfreiem Kupfer(II)acetat (16.05 g, 88.4 mmol, 3 eq.) zugegeben. Dann wurden abs. Pyridin (400 ml) und Triethylamin (12.32 ml, 88.4 mmol, 3 eq.) hinzugefügt. Es wurde kurz gerührt (5 min), dann erneut abs. Pyridin (440 ml) zugegeben und die Mischung 36 h lang gerührt. Da hiernach noch Edukt detektiert werden konnte (HPLC-Kontrolle), wurden 2,6-Lutidin (3.43 ml, 29.6 mmol, 1 eq.) und eine katalytische Menge (Spatelspitze) aktiviertes Kupfer zugegeben und unter einer Atmosphäre aus trockener Luft weitere 36 h gerührt. Danach wurden nochmals 3-(Trifluormethyl)phenylboronsäure (5.59 g, 29.5 mmol, 1 eq.), wasserfreies Kupfer(II)acetat (5.35 g, 29.5 mmol, 1 eq.) und Triethylamin (4.11 ml, 29.5 mmol, 1 eq.) nachdosiert und die Mischung dann über 5 d weiter gerührt. Anschließend wurde das Reaktionsgemisch bis zur Trockene eingeengt, der Rückstand mit Dichlormethan (∼500 ml) auf Kieselgel aufgezogen und mittels Flash-Chromatographie gereinigt (Kieselgel; Eluent: Cyclohexan/Essigsäureethylester 1:2). Das Produkt wurde als Feststoff erhalten (3.07 g, 18.1% d. Th.).

LC-MS (Methode 5): Rₜ = 1.34 min; MS (ESIpos): *m*/*z* (%) = 552.2 (100) [M+H]⁺; MS (ESIneg): *m*/*z* (%) = 550.1 (100) [M-H]⁻.

¹H-NMR (400 MHz, DMSO-d₆): δ = 2.05 (s, 3H), 6.65 (s, 1H), 7.80-8.05 (m, 11H), 8.25 (br. s, 1H).

### Beispiel 4

### (7R)-7-(4-Cyanophenyl)-2-(1,3-dioxo-1,3-dihydro-2H-isoindol-2-yl)-5-methyl-4-[3-(trifluormethyl)phenyl]-4,7-dihydro[1,2,4]triazolo[1,5-a]pyrimidin-6-carbonitril

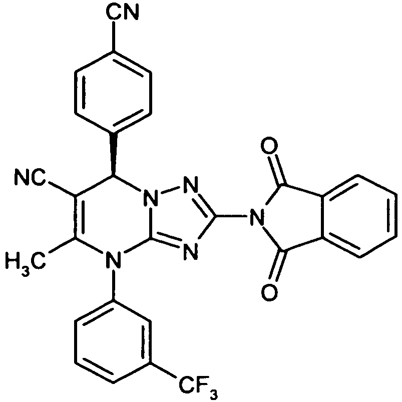

(*rac*)-7-(4-Cyanophenyl)-2-(1,3-dioxo-1,3-dihydro-2*H*-isoindol-2-yl)-5-methyl-4-[3-(trifluormethyl)phenyl]-4,7-dihydro[1,2,4]triazolo[1,5-a]pyrimidin-6-carbonitril (6.4 g) wurde durch präparative HPLC-Chromatographie an chiraler Phase in die Enantiomere aufgetrennt [stationäre Phase: chirale Kieselgel-Phase basierend auf dem Selektor Poly(*N-*methacryloyl-L-isoleucin-3-pentylamid), 430 x 40 mm; Probenvorbereitung: Lösung in 192 ml Essigsäureethylester/Isohexan 1:1; Fluss: 50 ml/min; Detektion: 260 nm; Injektionsvolumen: 5 ml; Temperatur: 24°C; Eluent: Essigsäureethylester/Isohexan 1:1]. Die Titelverbindung wurde als Feststoff erhalten (3.2 g, 100% d. Th.). Der Enantiomerenüberschuss (ee-Wert) wurde chromatographisch bestimmt [Säule: chirale Kieselgel-Phase basierend auf dem Selektor Poly(*N*-methacryloyl-L-isoleucin-3-pentyl-amid), 250 mm x 4.6 mm; Eluent: Essigsäureethylester/Isohexan 1:1; Fluss: 2 ml/min; Temperatur: 24°C; Detektion: 265 nm; Rₜ = 2.11 min; ee >99.5%].

LC-MS (Methode 2): Rₜ = 2.63 min; MS (ESIpos): *m*/*z* (%) = 552.2 (100) [M+H]⁺; MS (ESIneg): *m*/*z* (%) = 550.2 (100) [M-H]⁻.

¹H-NMR (400 MHz, DMSO-d₆): δ = 2.00 (s, 3H), 6.65.(s, 1H), 7.80-8.00 (m, 11H), 8.25 (br. s, 1H).

### Beispiel 5

### (rac)-7-[4-Cyano-2-(methylsulfonyl)phenyl]-2-(1,3-dioxo-1,3-dihydro-2H-isoindol-2-yl)-5-methyl-4-[3-(trifluormethyl)phenyl]-4,7-dihydro[1,2,4]triazolo[1,5-a]pyrimidin-6-carbonitril

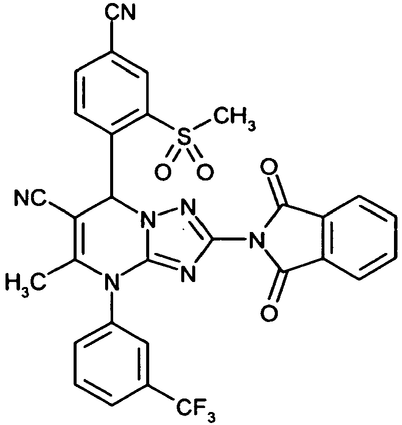

(*rac*)-7-[4-Cyano-2-(methylsulfonyl)phenyl]-2-(1,3-dioxo-1,3-dihydro-2*H*-isoindol-2-yl)-5-methyl-4,7-dihydro[1,2,4]triazolo[1,5-a]pyrimidin-6-carbonitril (600 mg, 1.2 mmol) wurde unter Argonschutzgasatmosphäre in abs. Dichlormethan (50 ml) mit Molekularsieb (0.5 g, 4 Å) für 15 min gerührt. 3-(Trifluormethyl)phenylboronsäure (704 mg, 3.7 mmol, 3 eq.) wurde zusammen mit wasserfreiem Kupfer(II)acetat (673 mg, 3.7 mmol, 3 eq.) hinzugefügt. Dann wurden abs. Pyridin (60 ml) und Triethylamin (571 µl, 3.7 mmol, 3 eq.) zugegeben und die Mischung 12 h lang gerührt. Anschließend wurde 2,6-Lutidin (864 µl, 7.4 mmol, 6 eq.) zugegeben und das Reaktionsgemisch zunächst weitere 12 h unter einer Atmosphäre aus trockener Luft gerührt und dann 3 d unter Argonatmosphäre belassen. Danach wurden nochmals 3-(Trifluormethyl)phenylboronsäure (235 mg, 1.2 mmol, 1 eq.), wasserfreies Kupfer(II)acetat (225 mg, 1.2 mmol, 1 eq.), 2,6-Lutidin (432 µl, 3.7 mmol, 3 eq.) sowie eine katalytische Menge (Spatelspitze) aktiviertes Kupfer hinzugefügt und das Gemisch 5 d lang unter einer Atmosphäre aus trockener Luft gerührt. Anschließend wurde das Reaktionsgemisch bis zur Trockene eingeengt, der Rückstand mit Dichlormethan (~50 ml) auf Kieselgel aufgezogen und mittels Flash-Chromatographie gereinigt (Kieselgel; Eluent: Cyclohexan/Essigsäureethylester 1:2). Das Produkt wurde als Feststoff erhalten (102 mg, 13.1% d. Th.).

LC-MS (Methode 2): Rₜ = 2.56 min; MS (ESIpos): *m*/*z* (%) = 630.0 (100) [M+H]⁺; MS (ESIneg): *m*/*z* (%) = 628.0 (100) [M-H]⁻.

### Beispiel 6

### (7S)-7-[4-Cyano-2-(methylsulfonyl)phenyl]-2-(1,3-dioxo-1,3-dihydro-2H-isoindol-2-yl)-5-methyl-4-[3-(trifluormethyl)phenyl]-4,7-dihydro[1,2,4]triazolo[1,5-a]pyrimidin-6-carbonitril

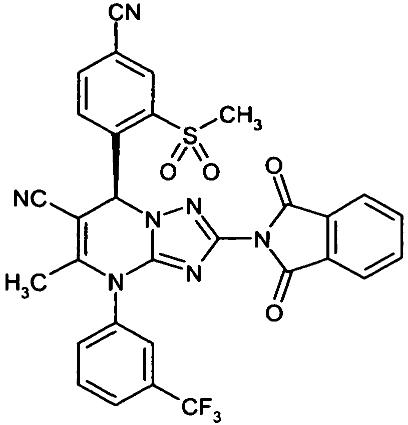

(*rac*)-7-[4-Cyano-2-(methylsulfonyl)phenyl]-2-(1,3-dioxo-1,3-dihydro-2*H*-isoindol-2-yl)-5-methyl-4-[3-(trifluormethyl)phenyl]-4,7-dihydro[1,2,4]triazolo[1,5-a]pyrimidin-6-carbonitril (100 mg) wurde durch präparative HPLC-Chromatographie an chiraler Phase in die Enantiomere aufgetrennt [stationäre Phase: chirale Kieselgel-Phase basierend auf dem Selektor Poly(N-methacryloyl-L-isoleucin-3-pentylamid), 250 x 20 mm; Probenvorbereitung: Lösung in 10 ml Essigsäureethylester; Fluss: 25 ml/min; Detektion: 260 nm; Injektionsvolumen: 0.5 ml; Temperatur: 24°C; Eluent: Essigsäureethylester/Isohexan 1:3]. Die Titelverbindung wurde als Feststoff erhalten (43 mg, 86% d. Th.). Der Enantiomerenüberschuss (ee-Wert) wurde chromatographisch bestimmt [Säule: chirale Kieselgel-Phase basierend auf dem Selektor Poly(*N*-methacryloyl-L-isoleucin-3-pentyl-amid), 250 mm x 4.6 mm; Eluent: Essigsäureethylester/Isohexan 1:3; Fluss: 2 ml/min; Temperatur: 24°C; Detektion: 265 nm; Rₜ = 11.19 min; ee >99.5%].

Für weitere analytische Daten siehe die racemische Verbindung (Beispiel 5).

### Beispiel 7

### (7R)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid

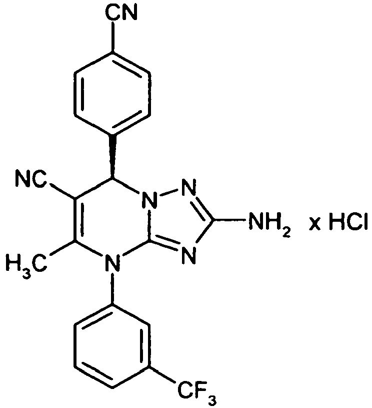

(7*R*)-7-(4-Cyanophenyl)-2-(1,3-dioxo-1,3-dihydro-2*H*-isoindol-2-yl)-5-methyl-4-[3-(trifluormethyl)phenyl]-4,7-dihydro[1,2,4]triazolo[1,5-a]pyrimidin-6-carbonitril (1.39 g, 2.5 mmol) wurde unter Argonschutzgasatmosphäre in abs. Ethanol (35 ml) vorgelegt. Hydrazinhydrat (208 µl, 4.3 mmol, 1.7 eq.) wurde zugegeben und das Gemisch für 1 h auf 85°C erhitzt. Die Reaktionsmischung wurde eingeengt, der Rückstand in DMF (30 ml) und 1 N Salzsäure (5 ml) gelöst und dann mittels präparativer HPLC gereinigt (Gromsil C18-Säule, 30 x 200 mm; Eluent: Acetonitril-Wasser-0.1 % TFA). Nach Lyophilisation erhielt man die freie Base der Titelverbindung als Feststoff (1.03 g, 97% d. Th.). Das Lyophilisat wurde in einer Lösung von Chlorwasserstoff in Dioxan (4 N, 20 ml) aufgenommen und wieder zur Trockene eingeengt. Dieser Vorgang wurde noch einmal wiederholt. Der Rückstand wurde in Wasser (25 ml) und Acetonitril (5 ml) suspendiert und erneut lyophilisiert.

LC-MS (Methode 2): Rₜ = 2.20 min; MS (ESIpos): *m*/*z* (%) = 422.1 (100) [M+H]⁺; MS (ESIneg): *m*/*z* (%) = 420.1 (100) [M-H]⁻.

¹H-NMR (400 MHz, DMSO-d₆): δ = 1.95 (s, 3H), 5.45 (s, 2H), 6.20 (s, 1H), 7.70-7.95 (m, 7H), 8.15 (br.s, 1H).

### Beispiel 8

### (rac)-2-Amino-7-[4-cyano-2-(methylsulfonyl)phenyl]-5-methyl-4-[3-(trifluormethyl)phenyl]-4,7-dihydro[1,2,4]triazolo[1,5-a]pyrimidin-6-carbonitril

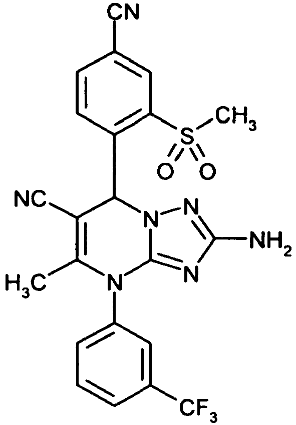

(*rac*)-7-[4-Cyano-2-(methylsulfonyl)phenyl]-2-(1,3-dioxo-1,3-dihydro-2*H*-isoindol-2-yl)-5-methyl-4-[3-(trifluormethyl)phenyl]-4,7-dihydro[1,2,4]triazolo[1,5-a]pyrimidin-6-carbonitril (10 mg, 16 µmol) wurde in einem druckfesten Glasrohr in abs. Ethanol (3 ml) vorgelegt. Hydrazinhydrat (1.3 µl, 27 µmol, 1.7 eq.) wurde zugegeben und die Mischung für 1 h auf 85°C erhitzt. Das Reaktionsgemisch wurde im Vakuum eingeengt, der Rückstand in DMF (1 ml) aufgenommen, mit 1 N Salzsäure (32 µl, 2 eq.) versetzt und mittels präparativer HPLC gereinigt (Reprosil C18-Säule, 10 µm, 30 x 250 mm; Eluent: Acetonitril-Wasser-0.1% TFA). Das Produkt wurde nach Lyophilisation als Feststoff erhalten (7.9 mg, quant.).

LC-MS (Methode 5): Rₜ = 1.11 min; MS (ESIpos): *m*/*z* (%) = 500.1 (100) [M+H]⁺; MS (ESIneg): *m*/*z* (%) = 498.4 (100) [M-H]⁻.

### Beispiel 9

### (7S)-2-Amino-7-[4-cyano-2-(methylsulfonyl)phenyl]-5-methyl-4-[3-(trifluormethyl)phenyl]-4,7-dihydro[1,2,4]triazolo[1,5-a]pyrimidin-6-carbonitril-Hydrochlorid

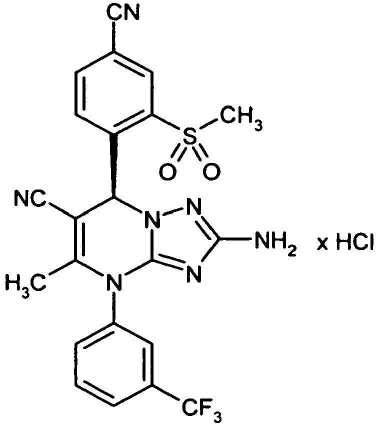

(7S)-7-[4-Cyano-2-(methylsulfonyl)phenyl]-2-(1,3-dioxo-1,3-dihydro-2H-isoindol-2-yl)-5-methyl-4-[3-(trifluormethyl)phenyl]-4,7-dihydro[1,2,4]triazolo[1,5-a]pyrimidin-6-carbonitril (45 mg, 71.5 µmol) wurde unter Argonschutzgasatmosphäre in abs. Ethanol (2.5 ml) vorgelegt. Hydrazinhydrat (6 µl, 121.5 mmol, 1.7 eq.) wurde zugegeben und das Gemisch für 1 h auf 85°C erhitzt. Die Reaktionsmischung wurde eingeengt, der Rückstand in DMF (1 ml) und 1 N Salzsäure (0.143 ml) gelöst und dann mittels präparativer HPLC gereinigt (Reprosil C18-Säule, 30 x 250 mm; Eluent: Acetonitril-Wasser-0.1% TFA). Nach Lyophilisation erhielt man die freie Base der Titelverbindung als Feststoff (30 mg, 84% d. Th.). Das Lyophilisat wurde in einer Lösung von Chlorwasserstoff in Dioxan (4 N, 2 ml) aufgenommen und wieder zur Trockene eingeengt. Dieser Vorgang wurde noch einmal wiederholt. Der Rückstand wurde in Wasser (2 ml) und Acetonitril (0.5 ml) suspendiert und erneut lyophilisiert.

LC-MS (Methode 5): Rₜ = 1.12 min; MS (ESIpos): *m*/*z* (%) = 500.0 (100) [M+H]⁺; MS (ESIneg): *m*/*z* (%) = 498.7 (100) [M-H]⁻.

¹H-NMR (400 MHz, DMSO-d₆): δ = 2.0 (s, 3H), 3.6 (s, 3H), 5.55 (br. s, 2H), 7.20 (s, 1H), 7.70-8.45 (m, 7H).

### Beispiel 10

### Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}(methylsulfonyl)carbamat

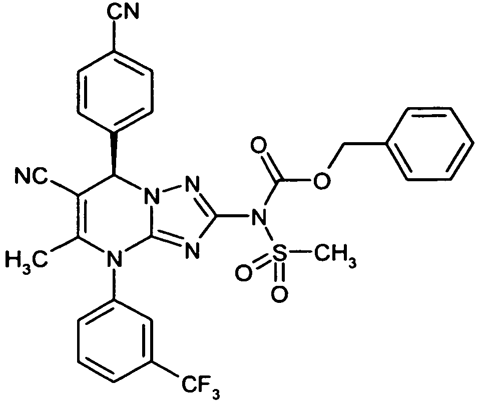

Unter Argonschutzgasatmosphäre wurden bei Raumtemperatur zu einer Lösung von Benzyl {(7R)-6-cyano-7-(4-cyanophenyl)-5-methyl-4-[3-(trifluormethyl)phenyl]-4,7-dihydro[1,2,4]triazolo[1,5-a]pyrimidin-2-yl}carbamat (20 mg, 36 µmol; Beispiel 38) in trockenem Pyridin (2 ml) und THF (2 ml) Triethylamin (11.1 mg, 140 µmol, 2.6 eq.) sowie Methansulfonylchlorid (41 mg, 360 µmol, 10 eq.) gegeben und die Mischung 12 h lang gerührt. Das Reaktionsgemisch wurde dann im Vakuum eingeengt und direkt mittels präparativer HPLC gereinigt (Reprosil C18-Säule, 30 x 200 mm; Eluent: Acetonitril-Wasser-0.1% TFA). Nach Lyophilisation erhielt man das Produkt als Feststoff (17.4 mg, 72% d. Th.).

LC-MS (Methode 2): Rₜ = 2.71 min; MS (ESIpos): *m*/*z* (%) = 634.2 (100) [M+H]⁺; MS (ESIneg): *m*/*z* (%) = 632.2 (100) [M-H]⁻.

¹H-NMR (400 MHz, DMSO-d₆): δ = 1.95 (s, 3H), 3.40 (s, 3H), 5.15 (d, 1H), 5.25 (d, 1H), 6.55 (s, 1H), 7.15 (m, 2H), 7.35 (m, 4H), 7.65 (m, 1H), 7.80-7.95 (m, 5H), 8.15 (br. s, 1H).

### Beispiel 11

### 2-({7-(4-Cyanopheny)-6-(ethoxycarbony)-5-methyl-4-[3-(trifluormethyl)phenyl]-4,7-dihydro-[1,2,4]triazolo[1,5-a]pyrimidin-2-yl}carbamoyl)benzoesäure

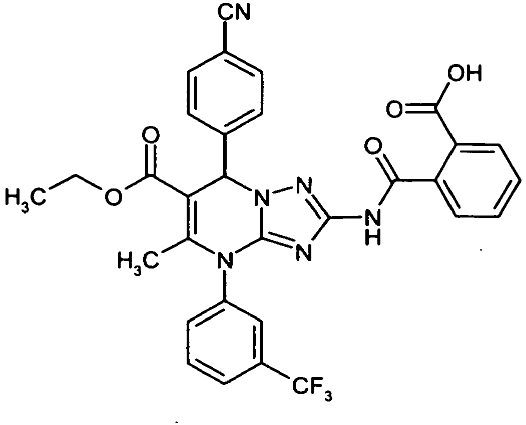

Zu einer Lösung von Ethyl 7-(4-cyanophenyl)-2-(1,3-dioxo-1,3-dihydro-2*H*-isoindol-2-yl)-5-methyl-4-[3-(trifluormethyl)phenyl]-4,7-dihydro[1,2,4]triazolo[1,5-a]pyrimidin-6-carboxylat (50.0 mg, 83.5 µmol; Beispiel 1) in Ethanol (6.25 ml) wurden Wasser (1 ml) und Kaliumcarbonat (23.1 mg, 167 µmol, 2 eq.) gegeben. Das Reaktionsgemisch wurde in einem geschlossenen, druckfesten Glasrohr auf 50°C erhitzt. Anschließend wurde das Reaktionsgemisch mit 1 N Salzsäure (670 µl, 670 µmol, 8 eq.) versetzt, im Vakuum eingeengt und der Rückstand mittels präparativer HPLC aufgereinigt (Gromsil C18-Säule, 10 µm, 250 x 30 mm; Eluent: Acetonitril-Wasser-0.1% TFA 10:90 → 100:0). Man erhielt das Produkt als Feststoff (45 mg, 87% d. Th.).

LC-MS (Methode 3): Rₜ = 3.66 min; MS (ESIpos): *m*/*z* (%) = 617.3 (100) [M+H]⁺; MS (ESIneg): *m*/*z* (%) = 615.2 (100) [M-H]⁻.

¹H-NMR (400 MHz, DMSO-d₆): δ = 1.05 (t, 3H), 2.15 (s, 3H), 4.00 (q, 2H), 6.45 (br. s, 1H), 7.30-8.20 (m, 12H), 10.80 (br. s, 1H), 12.85 (br. s, 1H).

### Beispiel 12

### 2-({6-Cyano-7-(4-cyanophenyl)-5-methyl-4-[3-(trifluormethyl)phenyl]-4,7-dihydro[1,2,4]triazolo-[1,5-a]pyrimidin-2-yl} carbamoyl)benzoesäure

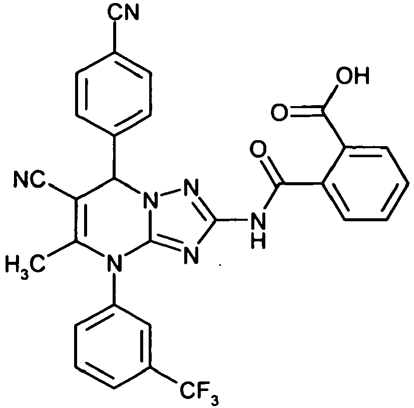

Zu einer Lösung von 7-(4-Cyanophenyl)-2-(1,3-dioxo-1,3-dihydro-2*H*-isoindol-2-yl)-methyl-4-[3-(trifluormethyl)phenyl]-4,7-dihydro[1,2,4]triazolo[1,5-a]pyrimidin-6-carbonitril (25.0 mg, 45.3 µmol; Beispiel 3) in Ethanol (3.4 ml) wurden Wasser (0.5 ml) und Kaliumcarbonat (8.1 mg, 59 µmol) gegeben. Das Reaktionsgemisch wurde in einem geschlossenen, druckfesten Glasrohr auf 55°C erhitzt. Bis zum vollständigen Umsatz wurde in zwei Einzelportionen weiteres Kaliumcarbonat (insgesamt 5 mg, 36 µmol) hinzugefügt und erneut auf 55°C erhitzt. Anschließend wurde das Reaktionsgemisch mit 1 N Salzsäure (180 µl, 180 µmol) versetzt, im Vakuum eingeengt und der Rückstand mittels präparativer HPLC aufgereinigt (Kromasil C18-Säule, 250 x 30 mm; Eluent: Acetonitril-Wasser-0.1% TFA 10:90 → 100:0). Man erhielt das Produkt als Feststoff (24.8 mg, 96% d. Th.).

LC-MS (Methode 4): Rₜ = 3.27 min; MS (ESIpos): *m*/*z* (%) = 570.1 (100) [M+H]⁺; MS (ESIneg): *m*/*z* (%) = 568.1 (100) [M-H]⁻.

¹H-NMR (400 MHz, DMSO-d₆): δ = 2.00 (s, 3H), 6.45 (br. s, 1H), 7.30-8.20 (m, 12H), 10.85 (br. s, 1H), 12.85 (br. s, 1H).

### Beispiel 13

### N-{(7R)-6-Cyano-7-(4-cyanophenyl)-5-methyl-4-[3-(trifluormethyl)phenyl]-4,7-dihydro[1,2,4]-triazolo[1,5-a]pyrimidin-2-yl}-1-fluorcyclopropancarboxamid

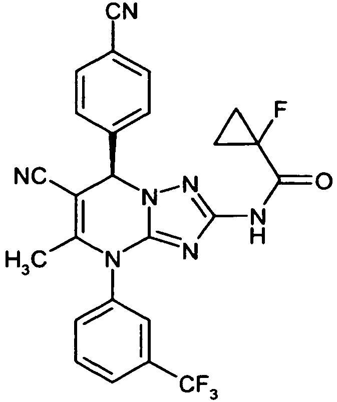

(7R)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde in zwei Portionen eine Lösung von 1-Fluorcyclopropancarbonsäurechlorid (20 mg, 165 µmol, 2.5 eq.) in abs. THF (1 ml) zugegeben und die Mischung 12 h lang gerührt. Das Reaktionsgemisch wurde dann im Vakuum eingeengt und direkt mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (29.5 mg, 89% d. Th.).

LC-MS (Methode 7): Rₜ = 2.03 min; MS (ESIpos): *m*/*z* (%) = 508.1 (100) [M+H]⁺; MS (ESIneg): *m*/*z* (%) = 506.2 (100) [M-H]⁻.

¹H-NMR (400 MHz, DMSO-d₆): δ = 1.10-1.35 (m, 4H), 1.95 (s, 3H), 6.45 (s, 1H), 7.75-7.95 (m, 7H), 8.15 (br. s, 1H), 10.60 (br. s, 1H).

### Beispiel 14

### N-{(7R)-6-Cyano-7-(4-cyanophenyl)-5-methyl-4-[3-(trifluormethyl)phenyl]-4,7-dihydro[1,2,4]-triazolo[1,5-a]pyrimidin-2-yl}-2,2-difluor-1-methylcyclopropancarboxamid

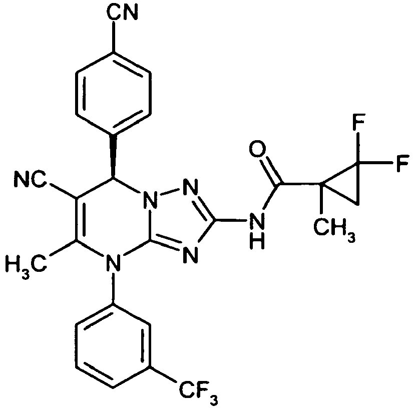

(7R)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde in zwei Portionen eine Lösung von 2,2-Difluor-1-methylcyclopropancarbonsäurechlorid (25 mg, 165 µmol, 2.5 eq.) in abs. THF (1 ml) zugegeben. Nachdem die Reaktionskontrolle mittels HPLC-Analytik weitgehenden Umsatz zeigte (3 h), wurde das Reaktionsgemisch im Vakuum eingeengt und direkt mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (26.8 mg, 76% d. Th.).

LC-MS (Methode 7): Rₜ = 2.10 min; MS (ESIpos): *m*/*z* (%) = 540.2 (100) [M+H]⁺; MS (ESIneg): *m*/*z* (%) = 538.2 (100) [M-H]⁻.

¹H-NMR (400 MHz, DMSO-d₆): δ = 1.30 (s, 3H), 1.40 (m, 1H), 1.90 (m, 1H), 2.00 (s, 3H), 6.45 (s, 1H), 7.75-7.95 (m, 7H), 8.20 (br. s, 1H), 10.70 (br. s, 1H).

### Beispiel 15

### N-{(7R)-6-Cyano-7-(4-cyanophenyl)-5-methyl-4-[3-(trifluormethyl)phenyl]-4,7-dihydro[1,2,4]-triazolo[1,5-a]pyrimidin-2-yl}-2,2-dimethylpropanamid

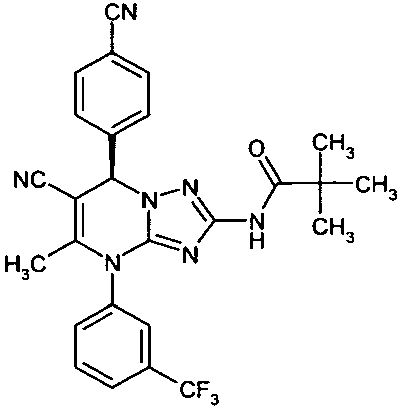

(7R)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde in zwei Portionen eine Lösung von 2,2-Dimethylpropanoylchlorid (20 mg, 164 µmol, 2.5 eq.) in abs. THF (1 ml) zugegeben. Nachdem die Reaktionskontrolle mittels HPLC-Analytik weitgehenden Umsatz zeigte (12h), wurde das Reaktionsgemisch im Vakuum eingeengt und direkt mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (26.9 mg, 81% d. Th.).

LC-MS (Methode 7): Rₜ = 2.10 min; MS (ESIpos): *m*/*z* (%) = 506.2 (100) [M+H]⁺; MS (ESIneg): *m*/*z* (%) = 504.3 (100) [M-H]⁻.

¹H-NMR (400 MHz, DMSO-d₆): δ = 1.05 (s, 9H), 1.95 (s, 3H), 6.40 (s, 1H), 7.75-7.95 (m, 7H), 8.20 (br. s, 1H), 9.80 (br. s, 1H).

### Beispiel 16

### N-{(7R)-6-Cyano-7-(4-cyanophenyl)-5-methyl-4-[3-(trifluormethyl)phenyl]-4,7-dihydro[1,2,4]-triazolo[1,5-a]pyrimidin-2-yl}-2-hydroxyacetamid

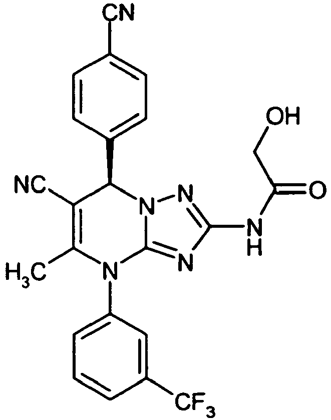

Zu einer Lösung von 2-((7*R*)-6-Cyano-7-(4-cyanophenyl)-5-methyl-4-[3-(trifluormethyl)phenyl]-4,7-dihydro[1,2,4]triazolo[1,5-a]pyrimidin-2-ylamino)-2-oxoethyl acetat (10.0 mg, 19.2 µmol; Beispiel 31) in THF (625 µl) wurde bei RT eine Lösung von Lithiumhydroxid (2.3 mg, 95.9 µmol, 5 eq.) in Wasser (125 µl) gegeben. Nach 1 h Rühren wurde vollständiger Umsatz festgestellt. Das Reaktionsgemisch wurde mit 1 N Salzsäure (153 µl, 8 eq.) versetzt und direkt mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (6.5 mg, 71 % d. Th.).

LC-MS (Methode 2): Rₜ = 2.06 min; MS (ESIpos): *m*/*z* (%) = 479.9 (100) [M+H]⁺; MS (ESIneg): *m*/*z* (%) = 477.9 (100) [M-H]⁻.

¹H-NMR (400 MHz, DMSO-d₆): δ = 1.95 (s, 3H), 3.85 (s, 2H), 6.45 (s, 1H), 7.75-7.95 (m, 7H), 8.20 (br. s, 1H), 10.05 (s, 1H).

### Beispiel 17

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}acetamid

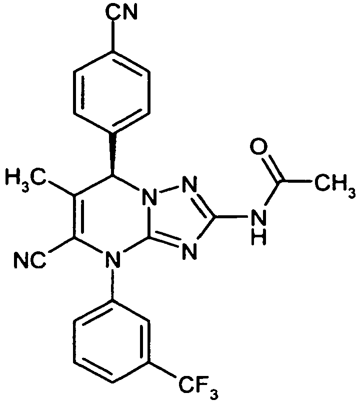

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (22 mg, 48 µmol) wurde unter Argonschutzgasatmosphäre in abs. Pyridin (5 ml) gelöst. Bei Raumtemperatur wurde Essigsäureanhydrid (98 mg, 960 µmol, 20 eq.) zugegeben. Nachdem die Reaktionskontrolle mittels DC-Analytik weitgehenden Umsatz zeigte (einige Stunden), wurde das Reaktionsgemisch im Vakuum eingeengt und direkt mittels präparativer HPLC gereinigt (Reprosil C18-Säule, 30 x 200 mm; Eluent: Acetonitril-Wasser-0.1% TFA). Nach Lyophilisation erhielt man das Produkt als Feststoff (20.6 mg, 93% d. Th.).

LC-MS (Methode 2): Rₜ = 2.19 min; MS (ESIpos): *m*/*z* (%) = 464.0 (100) [M+H]⁺; MS (ESIneg): *m*/*z* (%) = 462.1 (100) [M-H]⁻.

¹H-NMR (400 MHz, DMSO-d₆): δ = 1.85 (s, 3H), 2.00 (s, 3H), 6.45 (s, 1H), 7.70-7.95 (m, 7H), 8.20 (br. s, 1H), 10.40 (br. s, 1H).

### Beispiel 18

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}-2-methylpropanamid

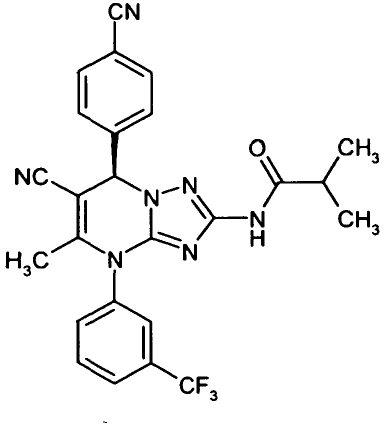

(7R)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde Isobutyrylchlorid (21 mg, 107 µmol, 3 eq.) zugegeben. Nach 12 h Rühren zeigte die Reaktionskontrolle mittels HPLC-Analytik weitgehenden Umsatz. Das Reaktionsgemisch wurde im Vakuum eingeengt und direkt mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (24.5 mg, 76% d. Th.).

LC-MS (Methode 2): Rₜ = 2.37 min; MS (ESIpos): m/z (%) = 492.1 (100) [M+H]⁺; MS (ESIneg): *m*/*z* (%) = 490.2 (100) [M-H]⁻.

¹H-NMR (400 MHz, DMSO-d₆): δ = 0.90 (d, 3H), 0.95 (d, 3H), 2.00 (s, 3H), 2.45 (m, 1H), 6.45 (s, 1H), 7.70-7.95 (m, 7H), 8.20 (br. s, 1H), 10.35 (br. s, 1H).

### Beispiel 19

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}propanamid

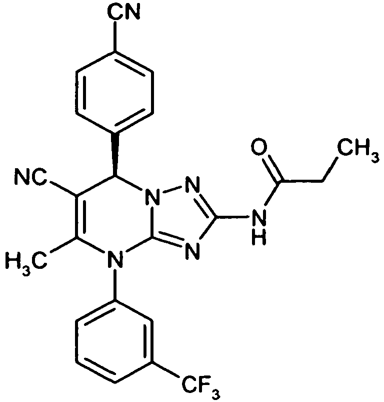

**(7*R*)-2-Amino-7-(4-cyanophenyl)-5-methyl-4-[3-(trifluormethyl)phenyl]-4,7-dihydro[1,2,4]tri**azolo[1,5-a]pyrimidin-6-carbonitril-Hydrochlorid (40 mg, 87 µmol) wurde unter Argonschutzgasatmosphäre in abs. Pyridin (2 ml) gelöst. Bei Raumtemperatur wurde Propionsäureanhydrid (227 mg, 1747 µmol, 20 eq.) zugegeben. Nach 12 h zeigte die Reaktionskontrolle mittels HPLC-Analytik weitgehenden Umsatz. Das Reaktionsgemisch wurde direkt mittels präparativer HPLC gereinigt (Reprosil C18-Säule, 30 x 250 mm; Eluent: Acetonitril-Wasser-0.1% TFA). Nach Lyophilisation erhielt man das Produkt als Feststoff (32.6 mg, 78% d. Th.).

LC-MS (Methode 2): Rₜ = 2.28 min; MS (ESIpos): *m*/*z* (%) = 478.1 (100) [M+H]⁺; MS (ESIneg): *m*/*z* (%) = 476.2 (100) [M-H]⁻.

¹H-NMR (400 MHz, DMSO-d₆): δ = 0.90 (t, 3H), 1.95 (s, 3H), 2.15 (q, 2H), 6.45 (s, 1H), 7.70-7.95 (m, 7H), 8.20 (br. s, 1H), 10.35 (br. s, 1H).

### Beispiel 20

### N-{(7R)-6-Cyano-7-(4-cyanophenyl)-5-methyl-4-[3-(trifluormethyl)phenyl]-4,7-dihydro[1,2,4]-triazolo[1,5-a]pyrimidin-2-yl}cyclopentancarboxamid

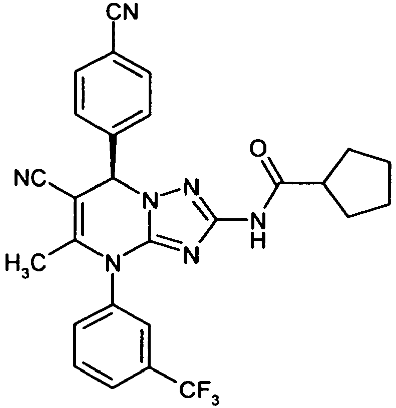

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde Cyclopentancarbonsäurechlorid (26 mg, 197 µmol, 3 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Reprosil C18-Säule, 30 x 250 mm; Eluent: Acetonitril-Wasser-0.1% TFA). Nach Lyophilisation erhielt man das Produkt als Feststoff (14.6 mg, 41% d. Th.).

LC-MS (Methode 7): Rₜ = 2.12 min; MS (ESIpos): *m*/*z* (%) = 518.2 (100) [M+H]⁺; MS (ESIneg): *m*/*z* (%) = 516.3 (100) [M-H]⁻.

¹H-NMR (400 MHz, DMSO-d₆): δ = 0.85-1.70 (m, 8H), 2.00 (s, 3H), 2.65 (m, 1H), 6.40 (s, 1H), 7.70-8.00 (m, 7H), 8.20 (br. s, 1H), 10.40 (br. s, 1H).

### Beispiel 21

### N-{(7R)-6-Cyano-7-(4-cyanophenyl-5-methyl-4-[3-(trifluormethyl)phenyl]-4,7-dihydro[1,2,4]triazolo[1,5-a]pyrimidin-2-yl}cyclobutancarboxamid

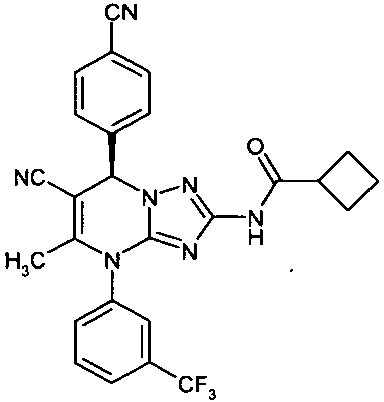

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde Cyclobutancarbonsäurechlorid (23 mg, 193 µmol, 3 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Reprosil C18-Säule, 30 x 250 mm; Eluent: Acetonitril-Wasser-0.1% TFA). Nach Lyophilisation erhielt man das Produkt als Feststoff (21.9 mg, 68% d. Th.).

LC-MS (Methode 2): Rₜ = 2.45 min; MS (ESIpos): *m*/*z* (%) = 504.1 (100) [M+H]⁺; MS (ESIneg): *m*/*z* (%) = 502.2 (100) [M-H]⁻.

¹H-NMR (400 MHz, DMSO-d₆): δ = 1.40-2.10 (m, 6H), 2.00 (s, 3H), 3.10 (m, 1H), 6.40 (s, 1H), 7.70-7.95 (m, 7H), 8.20 (br. s, 1H), 10.25 (br. s, 1H).

### Beispiel 22

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}cyclopropancarboxamid

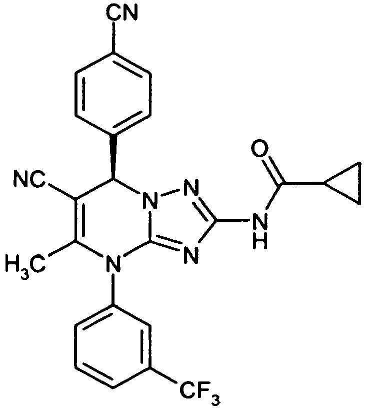

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde Cyclopropancarbonsäurechlorid (20 mg, 192 µmol, 3 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Reprosil C18-Säule, 30 x 250 mm; Eluent: Acetonitril-Wasser-0.1% TFA). Nach Lyophilisation erhielt man das Produkt als Feststoff (11.8 mg, 38% d. Th.).

LC-MS (Methode 2): Rₜ = 2.34 min; MS (ESIpos): *m*/*z* (%) = 490.0 (100) [M+H]⁺; MS (ESIneg): *m*/*z* (%) = 488.0 (100) [M-H]⁻.

¹H-NMR (400 MHz, DMSO-d₆): δ = 0.65 (m, 4H), 1.70 (m, 1H), 2.00 (s, 3H), 6.40 (s, 1H), 7.70-7.95 (m, 7H), 8.20 (br. s, 1H), 10.70 (br. s, 1H).

### Beispiel 23

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}cyclohexancarboxamid

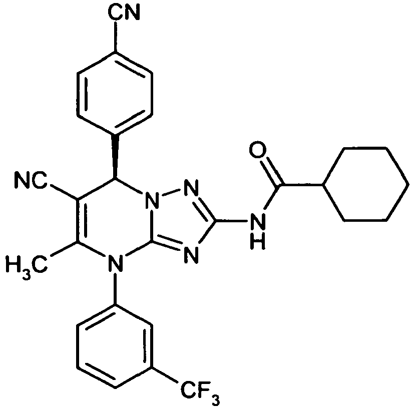

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (13 mg, 28 µmol) wurde unter Argonschutzgasatmosphäre in abs. Pyridin (0.65 ml) gelöst. Bei Raumtemperatur wurde Cyclohexancarbonsäurechlorid (8.3 mg, 57 µmol, 2 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Kromasil C18-Säule, 20 x 50 mm; Eluent: Acetonitril-Wasser-0.1% TFA). Nach Lyophilisation erhielt man das Produkt als Feststoff (12.5 mg, 83% d. Th.).

LC-MS (Methode 2): Rₜ = 2.59 min; MS (ESIpos): *m*/*z* (%) = 532.1 (100) [M+H]⁺; MS (ESIneg): *m*/*z* (%) = 530.2 (100) [M-H]⁻.

¹H-NMR (400 MHz, DMSO-d₆): δ = 1.00-1.30 (m, 5H), 1.50-1.70 (m, 5H), 2.00 (s, 3H), 2.20 (br. m, 1H), 6.40 (s, 1H), 7.70-7.95 (m, 7H), 8.20 (br. s, 1H), 10.30 (br. s, 1H).

### Beispiel 24

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}thiophen-2-carboxamid

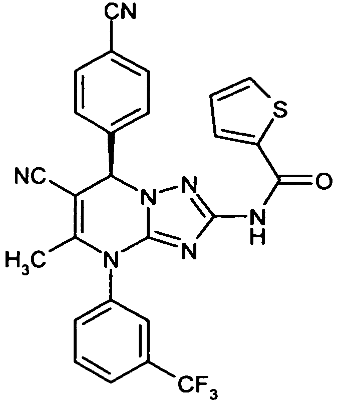

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde Thiophen-2-carbonsäurechlorid (29 mg, 197 µmol, 3 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Reprosil C18-Säule, 30 x 250 mm; Eluent: Acetonitril-Wasser-0.1% TFA). Nach Lyophilisation erhielt man das Produkt als Feststoff (9.6 mg, 28% d. Th.).

LC-MS (Methode 2): Rₜ = 2.49 min; MS (ESIpos): *m*/*z* (%) = 532.0 (100) [M+H]⁺; MS (ESIneg): *m*/*z* (%) = 530.1 (100) [M-H]⁻.

¹H-NMR (400 MHz, DMSO-d₆): δ = 2.00 (s, 3H), 6.50 (s, 1H), 7.10 (m, 1H), 7.75-8.00 (m, 9H), 8.20 (br. s, 1H), 10.85 (br. s, 1H).

### Beispiel 25

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}-3-methylbutanamid

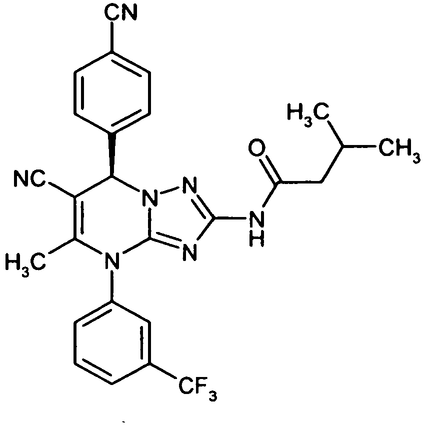

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde Isovalerylchlorid (24 mg, 197 µmol, 3 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Kromasil C18-Säule, 20 x 50 mm; Eluent: Acetonitril-Wasser-0.1% TFA). Nach Lyophilisation erhielt man das Produkt als Feststoff (22.4 mg, 68% d. Th.).

LC-MS (Methode 7): Rₜ = 2.08 min; MS (ESIpos): *m*/*z* (%) = 506.2 (100) [M+H]⁺; MS (ESIneg): *m*/*z* (%) = 504.3 (100) [M-H]⁻.

¹H-NMR (400 MHz, DMSO-d₆): δ = 0.80 (d, 6H), 1.90 (m, 1H), 2.00 (s, 3H), 2.05 (m, 2H), 6.40 (s, 1H), 7.70-7.95 (m, 7H), 8.20 (br. s, 1H), 10.35 (br. s, 1H).

### Beispiel 26

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}tetrahydrofuran-3-carboxamid

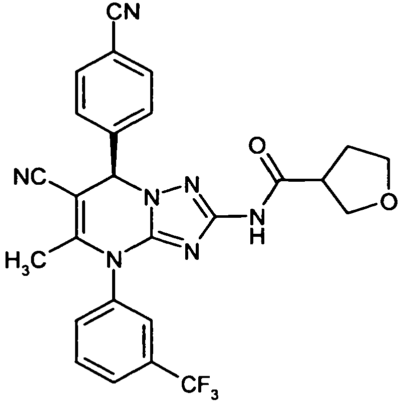

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde Tetrahydrofuran-3-carbonsäurechlorid (26 mg, 197 µmol, 3 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Reprosil C18-Säule, 250 x 30 mm; Eluent: Acetonitril-Wasser-0.1% TFA). Nach Lyophilisation erhielt man das Produkt als Feststoff (21.1 mg, 62% d. Th.).

LC-MS (Methode 2): Rₜ = 2.24 min; MS (ESIpos): *m*/*z* (%) = 520.1 (100) [M+H]⁺; MS (ESIneg): *m*/*z* (%) = 518.2 (100) [M-H]⁻.

¹H-NMR (400 MHz, DMSO-d₆): δ = 1.90 (m, 2H), 2.00 (s, 3H), 3.00 (m, 1H), 3.50-3.80 (m, 4H), 6.45 (s, 1H), 7.70-7.95 (m, 7H), 8.20 (br. s, 1H), 10.55 (br. s, 1H).

### Beispiel 27

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}-2-phenoxyacetamid

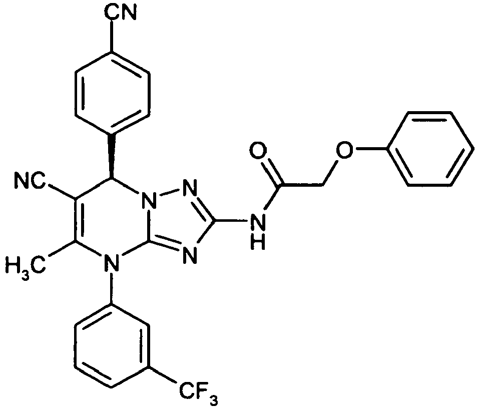

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde Phenoxyacetylchlorid (21 mg, 197 µmol, 3 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (20.9 mg, 56% d. Th.).

LC-MS (Methode 7): Rₜ = 2.17 min; MS (ESIpos): *m*/*z* (%) = 556.2 (100) [M+H]⁺; MS (ESIneg): *m*/*z* (%) = 554.3 (100) [M-H]⁻.

¹H-NMR (400 MHz, DMSO-d₆): δ = 2.00 (s, 3H), 4.55 (s, 2H), 6.45 (s, 1H), 6.70 (d, 2H), 6.80 (t, 1H), 7.15 (t, 2H), 7.70-7.95 (m, 7H), 8.20 (br. s, 1H), 10.70 (br. s, 1H).

### Beispiel 28

### N²-Acetyl-N-{(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}glycinamid

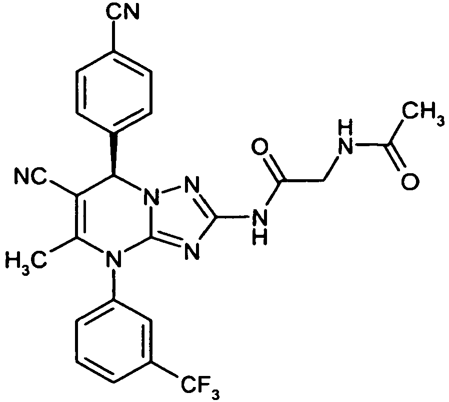

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (13 mg, 28 µmol) wurde unter Argonschutzgasatmosphäre in abs. Pyridin (2.0 ml) gelöst. Bei Raumtemperatur wurde Acetamidoessigsäurechlorid (8 mg, 57 µmol, 2 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer.HPLC gereinigt (Kromasil C18-Säule, 50 x 20 mm; Eluent: Acetonitril-Wasser-0.1% TFA). Nach Lyophilisation erhielt man das Produkt als Feststoff (9.6 mg, 65% d. Th.).

LC-MS (Methode 2): Rₜ = 2.06 min; MS (ESIpos): *m*/*z* (%) = 521.1 (100) [M+H]⁺; MS (ESIneg): *m*/*z* (%) = 519.2 (100) [M-H]⁻.

¹H-NMR (400 MHz, DMSO-d₆): δ = 1.80 (s, 3H), 2.00 (s, 3H), 3.65 (d, 2H), 6.45 (s, 1H), 7.70-7.95 (m, 8H), 8.20 (br. s, 1H), 10.45 (br. s, 1H).

### Beispiel 29

### N-{(7R)-6-Cyano-7-(4-cyanophenyl)-5-methyl-4-[3-(trifluonnethyl)phenyl]-4,7-dihydro[1,2,4]triazolo[1,5-a]pyrimidin-2-yl}benzamid

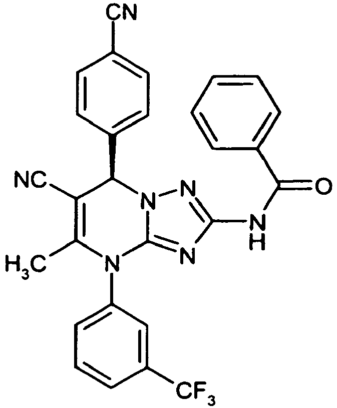

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (13.5 mg, 29 µmol) wurde unter Argonschutzgasatmosphäre in abs. Pyridin (1.0 ml) gelöst. Bei Raumtemperatur wurde Benzoesäureanhydrid (11 mg, 47 µmol, 1.6 eq.) zugegeben und die Mischung 12 h lang gerührt. Das Reaktionsgemisch wurde dann im Vakuum eingeengt und mittels präparativer HPLC gereinigt (Gromsil C18-Säule, 10 µm, 30 x 250 mm; Eluent: Acetonitril-Wasser-0.1% TFA). Nach Lyophilisation erhielt man das Produkt als Feststoff (3.5 mg, 23% d. Th.).

LC-MS (Methode 5): Rₜ = 1.27 min; MS (ESIpos): *m*/*z* (%) = 526.2 (100) [M+H]⁺; MS (ESIneg): *m*/*z* (%) = 524.4 (100) [M-H]⁻.

¹H-NMR (400 MHz, DMSO-d₆): δ = 2.00 (s, 3H), 6.45 (s, 1H), 7.40 (m, 2H), 7.55 (t, 1H), 7.70-7.95 (m, 9H), 8.20 (br. s, 1H), 10.80 (br. s, 1H).

### Beispiel 30

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}-2-[2-(2-methoxyethoxy)ethoxy]acetamid

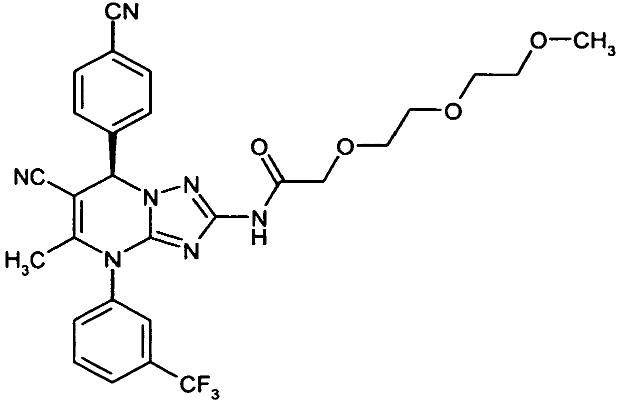

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (14 mg, 31 µmol) wurde unter Argonschutzgasatmosphäre in abs. Pyridin (0.7 ml) gelöst. Bei Raumtemperatur wurde 2-[2-(2-Methoxyethoxy)-ethoxy]essigsäurechlorid (12 mg, 61 µmol, 2 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (12.4 mg, 70% d. Th.).

LC-MS (Methode 2): Rₜ = 2.29 min; MS (ESIpos): *m*/*z* (%) = 582.2 (100) [M+H]⁺; MS (ESIneg): *m*/*z* (%) = 580.2 (100) [M-H]⁻.

¹H-NMR (400 MHz, DMSO-d₆): δ = 2.00 (s, 3H), 3.15 (s, 3H), 3.30-3.50 (m, 8H), 3.90 (s, 2H), 6.45 (s, 1H), 7.70-8.00 (m, 7H), 8.20 (br. s, 1H), 10.15 (br. s, 1H).

### Beispiel 31

### 2-({(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}amino)-2-oxoethyl acetat

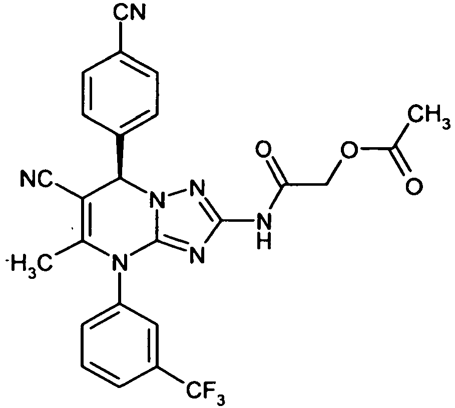

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (1.5 ml) gelöst. Bei Raumtemperatur wurde Acetoxyessigsäurechlorid (37 mg, 262 µmol, 4 eq.) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (19.6 mg, 57% d. Th.).

LC-MS (Methode 2): Rₜ = 2.30 min; MS (ESIpos): *m*/*z* (%) = 522.1 (100) [M+H]⁺; MS (ESIneg): *m*/*z* (%) = 520.2 (100) [M-H]⁻.

¹H-NMR (400 MHz, DMSO-d₆): δ = 1.95 (s, 3H), 2.00 (s, 3H), 4.45 (br. s, 2H), 6.45 (s, 1H), 7.70-7.95 (m, 7H), 8.20 (br. s, 1H), 10.70 (br. s, 1H).

### Beispiel 32

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl} isoxazol-5-carboxamid

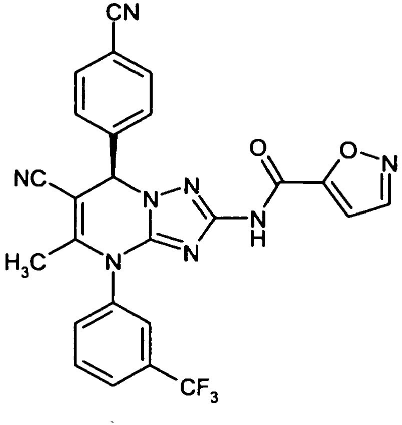

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (30 mg, 66 µmol) wurde unter Argonschutzgasatmosphäre in abs. Pyridin (2 ml) gelöst. Bei Raumtemperatur wurde Isoxazol-5-carbonsäurechlorid (26 mg, 197 µmol, 3 eq.) zugegeben und das Gemisch über Nacht gerührt. Nachdem die HPLC-Kontrolle weitgehenden Umsatz zeigte, wurde das Reaktionsgemisch im Vakuum eingeengt und mittels präparativer HPLC gereinigt (Gromsil C18-Säule, 30 x 250 mm; Eluent: Acetonitril-Wasser-0.1 % TFA). Nach Lyophilisation erhielt man das Produkt als Feststoff (5 mg, 15% d. Th.).

LC-MS (Methode 7): Rₜ = 1.92 min; MS (ESIpos): *m*/*z* (%) = 517.0 (100) [M+H]⁺; MS (ESIneg): *m*/*z* (%) = 515.1 (100) [M-H]⁻.

¹H-NMR (400 MHz, DMSO-d₆): δ = 2.00 (s, 3H), 6.50 (s, 1H), 7.25 (s, 1H), 7.70-7.95 (m, 7H), 8.20 (br. s, 1H), 8.70 (s, 1H), 11.3 5 (s, 1H).

### Beispiel 33

### tert.-Butyl [2-({(7R)-6-cyano-7-(4-cyanophenyl)-5-methyl-4-[3-(trifluormethyl)phenyl]-4,7-dihydro[1,2,4]triazolo[1,5-a]pyrimidin-2-yl}amino)-2-oxoethyl]carbamat

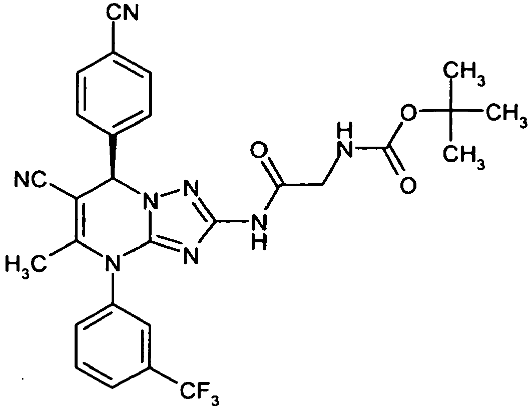

*N-*Boc-Gly-OH (5 mg, 28 µmol) und NMM (5.3 mg, 52 µmol, 2.4 eq.) wurden unter Argonschutzgasatmosphäre in abs. THF (1 ml) gelöst und bei -15°C mit Isobutylchlorformiat (4.2 mg, 31 µmol, 1.4 eq.) versetzt (Lösung A). Bei -78°C wurden (7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril-Hydrochlorid (10 mg, 22 µmol) und NMM (2.2 mg, 22 µmol, 1 eq.) hinzugefügt und die Mischung über Nacht unter langsamer Erwärmung auf RT gerührt. Da noch kein vollständiger Umsatz festzustellen war, wurde erneut eine Lösung vom Typ A, bestehend aus jeweils 285 µmol *N*-Boc-Gly-OH, Isobutylchlorformiat und NMM in abs. THF, bei -15°C zugegeben und die Mischung unter Erwärmung auf RT über 3 d gerührt. Das Reaktionsgemisch wurde dann direkt mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (6.3 mg, 50% d. Th.).

LC-MS (Methode 7): Rₜ = 2.01 min; MS (ESIpos): *m*/*z* (%) = 523.1 (100), 579.2 (80) [M+H]⁺; MS (ESIneg): *m*/*z* (%) = 577.3 (100) [M-H]⁻.

### Beispiel 34

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}glycinamid-Trifluoracetat

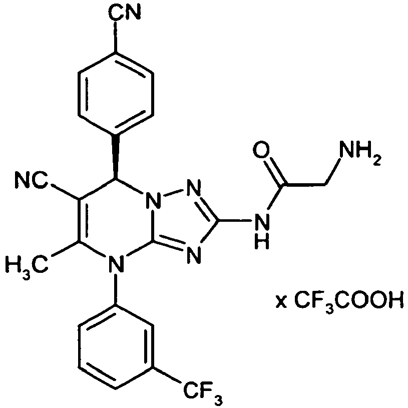

*tert*.-Butyl [2-((7*R*)-6-cyano-7-(4-cyanophenyl)-5-methyl-4-[3-(trifluormethyl)phenyl]-4,7-dihydro-[1,2,4]triazolo[1,5-a]pyrimidin-2-ylamino)-2-oxoethyl]carbamat (6.4 mg, 11 µmol) wurde unter Argonschutzgasatmosphäre in trockenem Dichlormethan (5 ml) gelöst. Bei Raumtemperatur wurde Trifluoressigsäure (1 ml) zugegeben und die Mischung für 40 min gerührt. Das Reaktionsgemisch wurde dann im Vakuum eingedampft und der Rückstand mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (∼6 mg, quant.).

LC-MS (Methode 2): Rₜ = 1.51 min; MS (ESIpos): *m*/*z* (%) = 479.1 (100) [M+H]⁺; MS (ESIneg): *m*/*z* (%) = 477.1 (100) [M-H]⁻.

¹H-NMR (400 MHz, DMSO-d₆): δ = 2.00 (s, 3H), 3.60 (br. s, 2H), 6.50 (s, 1H), 7.70-7.95 (m, 9H), 8.20 (br. s, 1H), 10.95 (s, 1H).

### Beispiel 35

### N-{(7S)-6-Cyano-7-[4-cyano-2-(methylsulfonyl)phenyl]-5-methyl-4-[3-(trifluormethyl)phenyl]-4,7-dihydro[1,2,4]triazolo[1,5-a]pyrimidin-2-yl}cyclopropancarboxamid

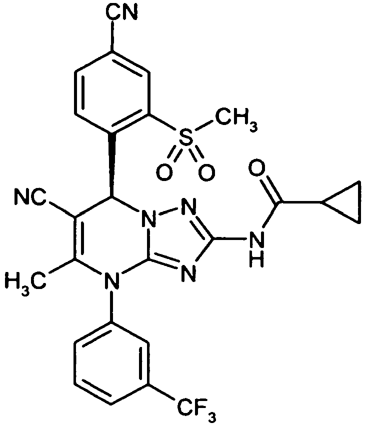

(7*S*)-2-Amino-7-[4-cyano-2-(methy)sulfonyl]phenyl]-5-methyl-4-[3-(trifluormethyl)phenyl]-4,7-dihydro[1,2,4]triazolo[1,5-a]pyrimidin-6-carbonitril-Hydrochlorid (16 mg, 30 µmol) wurde unter Argonschutzgasatmosphäre in abs. Pyridin (1 ml) gelöst. Bei Raumtemperatur wurde in zwei Portionen Cyclopropancarbonsäurechlorid (jeweils 3.7 mg, 36 µmol, 1.2 eq.) zugegeben. Nachdem die HPLC-Kontrolle weitgehenden Umsatz zeigte (12 h), wurde das Reaktionsgemisch im Vakuum eingeengt und mittels präparativer HPLC gereinigt (Macherey & Nagel Gravity C18-Säule, 21 x 300 mm; Eluent: Acetonitril-Wasser-0.1% TFA). Nach Lyophilisation erhielt man das Produkt als Feststoff (11.2 mg, 66% d. Th.).

LC-MS (Methode 5): Rₜ = 1.19 min; MS (ESIpos): *m*/*z* (%) = 568.0 (100) [M+H]⁺; MS (ESIneg): *m*/*z*(%) = 566.2 (100) [M-H]⁻.

¹H-NMR (400 MHz, DMSO-d₆): δ = 0.65 (m, 4H), 1.65 (m, 1H), 2.00 (s, 3H), 3.65 (s, 3H), 7.45 (s, 1H), 7.80-8.40 (m, 6H), 8.45 (s, 1H), 10.85 (br. s, 1H).

### Beispiel 36

### Methyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}carbamat

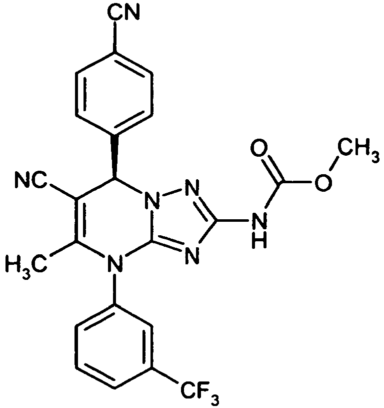

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril (30 mg, 71 µmol) wurde unter Argonschutzgasatmosphäre in einem Gemisch aus abs. THF (3 ml) und abs. Pyridin (57.5 µl) gelöst. Bei Raumtemperatur wurde in mehreren Portionen Pyridin (5 x 57 µl) und Chlorameisensäuremethylester (5 x 33.6 mg, 5 x 356 µmol; 25 eq.) zugegeben. Die Mischung wurde anschließend für 12 h auf 80°C erhitzt. Nachdem die HPLC-Analytik weitgehenden Umsatz zeigte, wurde das Reaktionsgemisch im Vakuum eingeengt und mittels präparativer HPLC gereinigt (Kromasil C18-Säule, 30 x 250 mm; Eluent: Acetonitril-Wasser-0.1% TFA). Nach Lyophilisation erhielt man das Produkt als Feststoff (31.1 mg, 91 % d. Th.).

LC-MS (Methode 5): Rₜ = 1.16 min; MS (ESIpos): *m*/*z* (%) = 480.3 (100) [M+H]⁺; MS (ESIneg): *m*/*z* (%) = 478.4 (100) [M-H]⁻.

¹H-NMR (500 MHz, DMSO-d₆): δ = 2.00 (s, 3H), 3.50 (s, 3H), 6.40 (s, 1H), 7.70-7.95 (m, 7H), 8.15 (br. s, 1H), 10.00 (s, 1H).

### Beispiel 37

### Methyl {6-cyano-7-[4-cyano-2-(methylsulfonyl)phenyl]-5-methyl-4-[3-(trifluormethyl)phenyl]-4,7-dihydro[1,2,4]triazolo[1,5-a]pyrimidin-2-yl}carbamat

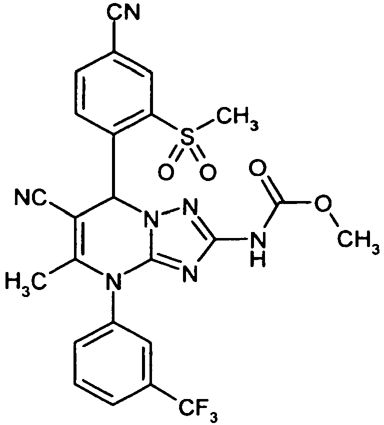

2-Amino-7-[4-cyano-2-(methylsulfonyl)phenyl]-5-methyl-4-[3-(trifluormethyl)phenyl]-4,7-dihydro[1,2,4]triazolo[1,5-a]pyrimidin-6-carbonitril (7 mg, 13.6 µmol) wurde unter Argonschutzgasatmosphäre in einem Gemisch aus abs. THF (0.6 ml) und abs. Pyridin (11 µl) gelöst. Bei Raumtemperatur wurde in mehreren Portionen abs. Pyridin (5 x 11 µl) und Chlorameisensäuremethylester (5 x 6.4 mg, 5 x 68.1 µmol; 25 eq.) zugegeben. Die Mischung wurde anschließend für 12 h auf 80°C erhitzt. Nachdem die HPLC-Analytik weitgehenden Umsatz zeigte, wurde das Reaktionsgemisch im Vakuum eingeengt und mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (5.4 mg, 69% d. Th.).

LC-MS (Methode 7): Rₜ= 1.91 min; MS (ESIpos): *m*/*z* (%) = 558.1 (100) [M+H]⁺; MS (ESIneg): *m*/*z* (%) = 556.1 (100) [M-H]⁻.

¹H-NMR (400 MHz, DMSO-d₆): δ = 2.00 (s, 3H), 3.50 (s, 3H), 3.65 (s, 3H), 7.45 (s, 1H), 7.80-8.45 (m, 7H), 10.20 (s, 1H).

### Beispiel 38

### Benzyl {(7R)-6-cyano-7-(4-cyanophenyl-5-methyl-4-[3-(trifluormethyl)phenyl]-4,7-dihydro-[1,2,4]triazolo[1,5-a]pyrimidin-2-yl)carbamat

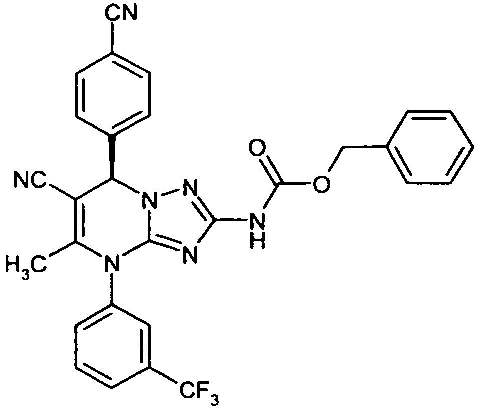

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril (400 mg, 874 µmol) wurde unter Argonschutzgasatmosphäre in abs. Pyridin (15 ml) vorgelegt. Bei 0°C wurde in mehreren Portionen Chlorameisensäurebenzylester (3 x 460 mg, 3 x 2621 µmol; 9 eq.) zugegeben und die Mischung anschließend für 12 h unter Erwärmung auf RT gerührt. Nachdem die HPLC-Analytik weitgehenden Umsatz zeigte, wurde das Reaktionsgemisch im Vakuum eingeengt und mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (500 mg, quant.).

LC-MS (Methode 7): Rₜ = 2.23 min; MS (ESIpos): *m*/*z* (%) = 556.1 (100) [M+H]⁺; MS (ESIneg): *m*/*z* (%) = 554.1 (100) [M-H]⁻.

¹H-NMR (400 MHz, DMSO-d₆): δ = 1.95 (s, 3H), 5.00 (s, 2H), 6.40 (s, 1H), 7.20-7.35 (m, 5H), 7.70-7.95 (m, 7H), 8.15 (br. s, 1H), 10.15 (s, 1H).

### Beispiel 39

### Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}methylcarbamat

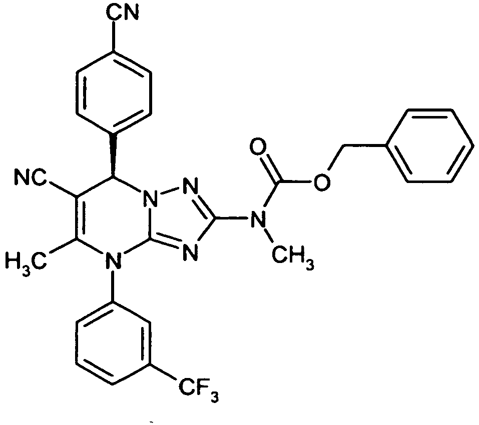

Zu einer Lösung von Benzyl {(7*R*)-6-cyano-7-(4-cyanophenyl)-5-methyl-4-[3-(trifluormethyl)-phenyl]-4,7-dihydro[1,2,4]triazolo[1,5-a]pyrimidin-2-yl}carbamat (15 mg, 27 µmol) in DMF (2 ml) wurden Kaliumcarbonat (6.0 mg, 43 µmol, 1.6 eq.), 18-Krone-6 (11.4 mg, 43 µmol, 1.5 eq.) und Methyliodid (6.1 mg, 53 µmol, 2.0 eq.) gegeben. Das Reaktionsgemisch wurde für 12 h bei RT gerührt, dann im Vakuum eingeengt und mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (13.3 mg, 86% d. Th.).

LC-MS (Methode 5): Rₜ = 1.41 min; MS (ESIpos): *m*/*z* (%) = 570.1 (100) [M+H]⁺; MS (ESIneg): *m*/*z* (%) = 569.1 (70) [M-H]⁻.

### Beispiel 40

### Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}ethylcarbamat

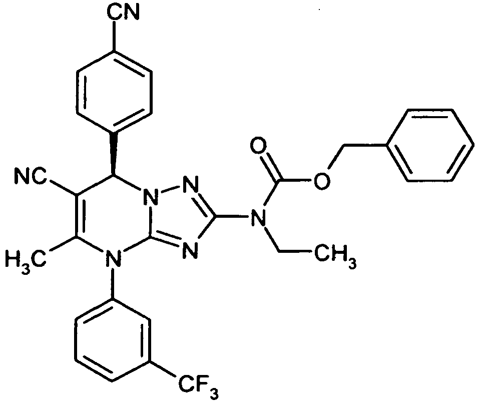

Zu einer Lösung von Benzyl {(7*R*)-6-cyano-7-(4-cyanophenyl)-5-methyl-4-[3-(trifluormethyl)-phenyl]-4,7-dihydro[1,2,4]triazolo[1,5-a]pyrimidin-2-yl}carbamat (20mg, 36 µmol) in DMF (2 ml) wurden Kaliumcarbonat (8 mg, 58 µmol, 1.6 eq.), 18-Krone-6 (15.2 mg, 58 µmol, 1.6 eq.) und Iodethan (9 mg, 58 µmol, 1.6 eq.) gegeben. Das Reaktionsgemisch wurde für 12 h bei RT gerührt und dann im Vakuum eingeengt. Der Rückstand wurde mit Essigsäure (4 mg, 72 µmol, 2 eq.) angesäuert und anschließend mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (19.6 mg, 93% d. Th.).

LC-MS (Methode 2): Rₜ = 2.79 min; MS (ESIpos): *m*/*z* (%) = 584.1 (100) [M+H]⁺; MS (ESIneg): *m*/*z* (%) = 582.1 (100) [M-H]⁻.

¹H-NMR (400 MHz, DMSO-d₆): δ = 0.95 (t, 3H), 2.00 (s, 3H), 3.45 (m, 2H), 5.00 (d, 1H), 5.05 (d, 1H), 6.50 (s, 1H), 7.15-7.40 (m, 5H), 7.70-7.95 (m, 7H), 8.15 (br. s, 1H).

### Beispiel 41

### Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}(cyanomethyl)carbamat

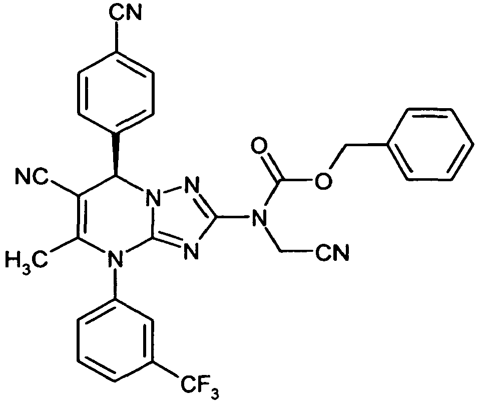

Zu einer Lösung von Benzyl {(7*R*)-6-cyano-7-(4-cyanophenyl)-5-methyl-4-[3-(trifluormethyl)-phenyl]-4,7-dihydro[1,2,4]triazolo[1,5-a]pyrimidin-2-yl}carbamat (30 mg, 54 µmol) in DMF (2 ml) wurden Kaliumcarbonat (15.7 mg, 113 µmol, 2.1 eq.), 18-Krone-6 (30 mg, 113 µmol, 2.1 eq.) und Iodacetonitril (19 mg, 113 µmol, 2.1 eq.) gegeben. Das Reaktionsgemisch wurde für 12 h bei RT gerührt und dann im Vakuum eingeengt. Der Rückstand wurde mit Essigsäure angesäuert und anschließend mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (25 mg, 78% d. Th.).

LC-MS (Methode 5): Rₜ = 1.40 min; MS (ESIpos): *m*/*z* (%) = 434.0 (100), 550.9 (20), 595.0 (30) [M+H]⁺; MS (ESIneg): *m*/*z* (%) = 593.8 (100) [M-H]⁻.

¹H-NMR (400 MHz, DMSO-d₆): δ = 2.00 (s, 3H), 4.55 (d, 2H), 5.10 (d, 2H), 6.55 (s, 1H), 7.15-7.35 (m, 5H), 7.70-7.95 (m, 7H), 8.15 (br. s, 1H).

### Beispiel 42

### Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}propylcarbamat

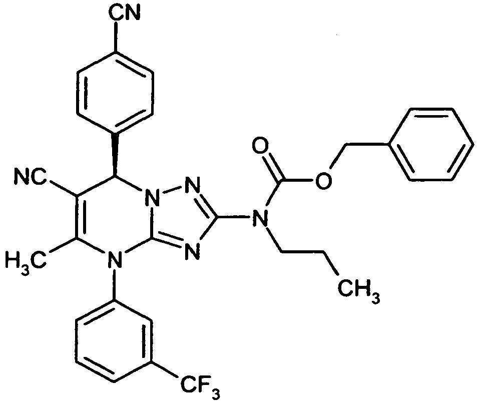

Zu einer Lösung von Benzyl {(7*R*)-6-cyano-7-(4-cyanophenyl)-5-methyl-4-[3-(trifluormethyl)-phenyl]-4,7-dihydro[1,2,4]triazolo[1,5-a]pyrimidin-2-yl}carbamat (30 mg, 54 µmol) in DMF (2 ml) wurden Kaliumcarbonat (15.7 mg, 113 µmol, 2.1 eq.), 18-Krone-6 (30 mg, 113 µmol, 2.1 eq.) und *n*-Propyliodid (19.2 mg, 113 µmol, 2.1 eq.) gegeben. Das Reaktionsgemisch wurde für 12 h bei RT gerührt und dann im Vakuum eingeengt. Der Rückstand wurde mit Essigsäure angesäuert und anschließend mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (25 mg, 77% d. Th.).

LC-MS (Methode 5): Rₜ = 1.50 min; MS (ESIpos): *m*/*z* (%) = 598.1 (100) [M+H]⁺; MS (ESIneg): *m*/*z* (%) = 596.2 (100) [M-H]⁻.

¹H-NMR (400 MHz, DMSO-d₆): δ = 0.70 (t, 3H), 1.35 (m, 2H), 2.00 (s, 3H), 3.45 (m, 2H), 5.00 (d, 1H), 5.05 (d, 1H), 6.50 (s, 1H), 7.15-7.40 (m, 5H), 7.70-7.95 (m, 7H), 8.15 (br. s, 1H).

### Beispiel 43

### Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}(2-methylpropyl)carbamat

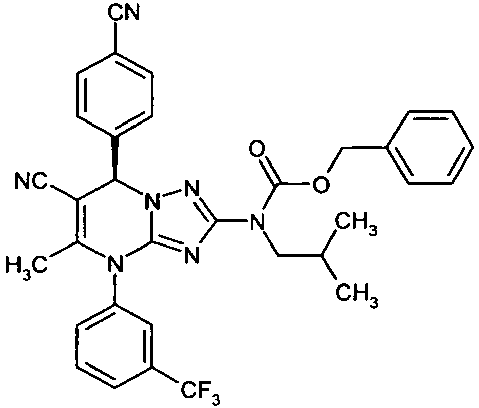

Zu einer Lösung von Benzyl {(7*R*)-6-cyano-7-(4-cyanophenyl)-5-methyl-4-[3-(trifluormethyl)-phenyl]-4,7-dihydro[1,2,4]triazolo[1,5-a]pyrimidin-2-yl}carbamat (30 mg, 54 µmol) in DMF (2 ml) wurden Kaliumcarbonat (15.7 mg, 113 µmol, 2.1 eq.), 18-Krone-6 (30 mg, 113 µmol, 2.1 eq.) und 1-Iod-2-methylpropan (21 mg, 113 µmol, 2.1 eq.) gegeben. Das Reaktionsgemisch wurde für 12 h bei RT gerührt und dann im Vakuum eingeengt. Der Rückstand wurde mit Essigsäure (3 eq.) angesäuert und anschließend mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (25 mg, 76% d. Th.).

LC-MS (Methode 5): Rₜ = 1.54 min; MS (ESIpos): *m*/*z* (%) = 612.1 (100) [M+H]⁺; MS (ESIneg): *m*/*z* (%) = 610.0 (100) [M-H]⁻.

¹H-NMR (400 MHz, DMSO-d₆): δ = 0.70 (m, 6H), 1.65 (m, 1H), 2.00 (s, 3H), 3.25 (d, 2H), 4.95 (d, 1H), 5.05 (d, 1H), 6.55 (s, 1H), 7.15-7.40 (m, 5H), 7.70-7.95 (m, 7H), 8.15 (br. s, 1H).

### Beispiel 44

### Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}(cyclopropylmethyl)carbamat

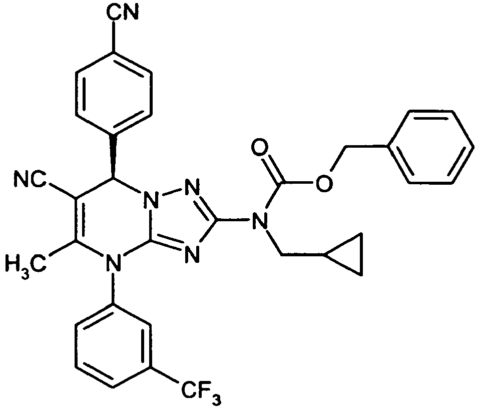

Zu einer Lösung von Benzyl {(7*R*)-6-cyano-7-(4-cyanophenyl)-5-methyl-4-[3-(trifluormethyl)-phenyl]-4,7-dihydro[1,2,4]triazolo[1,5-a]pyrimidin-2-yl}carbamat (30 mg, 54 µmol) in DMF (2 ml) wurden Kaliumcarbonat (15.7 mg, 113 µmol, 2.1 eq.), 18-Krone-6 (30 mg, 113 µmol, 2.1 eq.) und 1-(Brommethyl)cyclopropan (15.3 mg, 113 µmol, 2.1 eq.) gegeben. Das Reaktionsgemisch wurde für 12 h bei RT gerührt und dann im Vakuum eingeengt. Der Rückstand wurde mit Essigsäure angesäuert und anschließend mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (22 mg, 66% d. Th.).

LC-MS (Methode 5): Rₜ = 1.50 min; MS (ESIpos): *m*/z (%) = 610.1 (100) [M+H]⁺; MS (ESIneg): *m*/*z* (%) = 608.9 (100) [M-H]⁻.

¹H-NMR (400 MHz, DMSO-d₆): δ = 0.25 (m, 2H), 0.85 (m, 1H), 2.00 (s, 3H), 3.30 (m, 4H), 5.00 (d, 1H), 5.05 (d, 1H), 6.50 (s, 1H), 7.15-7.40 (m, 5H), 7.70-7.95 (m, 7H), 8.15 (br. s, 1H).

### Beispiel 45

### tert.-Butyl N-[(benzyloxy)carbonyl]-N-{(7R)-6-cyano-7-(4-cyanophenyl)-5-methyl-4-[3-trifluormethyl)phenyl]-4,7-dihydro[1,2,4]triazolo[1,5-a]pyrimidin-2-yl}glycinat

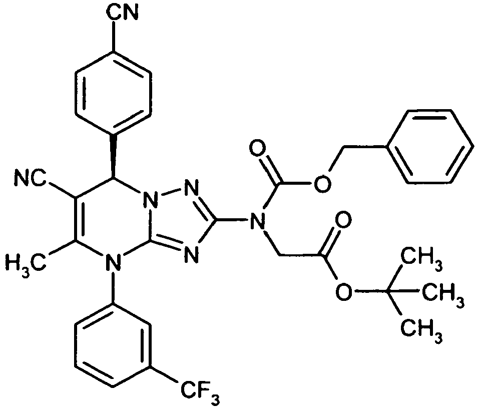

Zu einer Lösung von Benzyl {(7*R*)-6-cyano-7-(4-cyanophenyl)-5-methyl-4-[3-(trifluormethyl)-phenyl]-4,7-dihydro[1,2,4]triazolo[1,5-a]pyrimidin-2-yl}carbamat (20 mg, 36 µmol) in trockenem DMF (5 ml) wurden Kaliumcarbonat (8.0 mg, 58 µmol, 1.6 eq.) und Bromessigsäure-*tert*.-butylester (11.2 mg, 58 µmol, 1.6 eq.) gegeben. Das Reaktionsgemisch wurde für 12 h bei RT gerührt, dann im Vakuum eingeengt und der Rückstand mittels präparativer HPLC gereinigt (Kromasil C18-Säule, 5 µm, 50 x 20 mm; Eluent: Acetonitril-Wasser-0.1% TFA). Nach Lyophilisation erhielt man das Produkt als Feststoff (25 mg, 76% d. Th.).

LC-MS (Methode 7): Rₜ = 6.61 min; MS (ESIpos): *m*/*z* (%) = 670.2 (100) [M+H]⁺; MS (ESIneg): *m*/*z* (%) = 668.2 (90) [M-H]⁻.

¹H-NMR (400 MHz, DMSO-d₆): δ = 1.15 (s, 9H), 2.00 (s, 3H), 4.10 (d, 1H), 4.15 (d, 1H), 5.10 (s, 2H), 6.45 (s, 1H), 7.20-7.30 (m, 5H), 7.70-7.95 (m, 7H), 8.15 (br. s, 1H).

### Beispiel 46

### Ethyl {(7R)-6-cyano-7-(4-cyanophenyl)-5-methyl-4-[3-(trifluormethyl)phenyl]-4,7-dihydro[1,2,4]-triazolo[1,5-a]pyrimidin-2-yl}carbamat

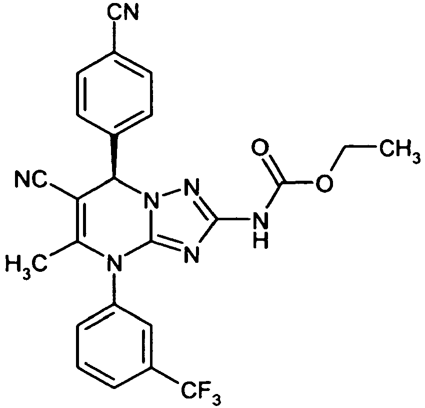

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril (25 mg, 59 µmol) wurde unter Argonschutzgasatmosphäre in einem Gemisch aus abs. THF (2.5 ml) und abs. Pyridin (48 ml) gelöst. Bei Raumtemperatur wurde Chlorameisensäureethylester (32 mg, 297 µmol, 5 eq.) zugegeben und die Mischung für 12 h auf 80°C erhitzt. Nachdem die HPLC-Analytik weitgehenden Umsatz zeigte, wurde das Reaktionsgemisch im Vakuum eingeengt und mittels präparativer HPLC gereinigt (Reprosil C18-Säule, 30 x 250 mm; Eluent: Acetonitril-Wasser-0.1% TFA). Nach Lyophilisation erhielt man das Produkt als Feststoff (25 mg, 86% d. Th.).

LC-MS (Methode 7): Rₜ = 1.97 min; MS (ESIpos): *m*/*z* (%) = 494.2 (100) [M+H]⁺; MS (ESIneg): *m*/*z* (%) = 492.3 (100) [M-H]⁻.

¹H-NMR (400 MHz, DMSO-d₆): δ = 1.10 (t, 3H), 2.00 (s, 3H), 3.95 (q, 2H), 6.40 (s, 1H), 7.75-7.95 (m, 7H), 8.20 (br. s, 1H), 9.95 (s, 1H).

### Beispiel 47

### 1-Methylethyl {(7R)-6-cyano-7-(4-cyanophenyl)-5-methyl-4-[3-(trifluormethyl)phenyl]-4,7-dihydro[1,2,4]triazolo[1,5-a]pyrimidin-2-yl}carbamat

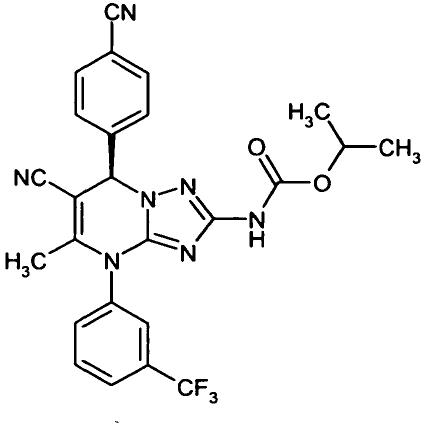

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril (25 mg, 59 µmol) wurde unter Argonschutzgasatmosphäre in einem Gemisch aus abs. THF (2.5 ml) und abs. Pyridin (48 µl, 593 µmol, 10 eq.) gelöst. Bei Raumtemperatur wurde Chlorameisensäureisopropylester (36 mg, 297 µmol, 5 eq.) zugegeben und die Mischung für 12 h auf 80°C erhitzt. Nachdem die HPLC-Analytik weitgehenden Umsatz zeigte, wurde das Reaktionsgemisch im Vakuum eingeengt und mittels präparativer HPLC gereinigt (Reprosil C18-Säule, 30 x 250 mm; Eluent: Acetonitril-Wasser-0.1% TFA). Nach Lyophilisation erhielt man das Produkt als Feststoff (23.2 mg, 77% d. Th.).

LC-MS (Methode 7): Rₜ = 2.08 min; MS (ESIpos): *m*/*z* (%) = 508.3 (100) [M+H]⁺; MS (ESIneg): *mlz* (%) = 506.3 (100) [M-H]⁻.

¹H-NMR (400 MHz, DMSO-d₆): δ = 1.10 (dd, 6H), 1.95 (s, 3H), 4.70 (hept, 1H), 6.40 (s, 1H), 7.75-7.95 (m, 7H), 8.20 (br. s, 1H), 9.85 (s, 1H).

### Beispiel 48

### Ethyl 7-(4-cyanophenyl)-2-[(methoxycarbonyl)amino]-5-methyl-4-[3-(trifluormethyl)phenyl]-4,7-dihydro[1,2,4]triazolo[1,5-a]pyrimidin-6-carboxylat

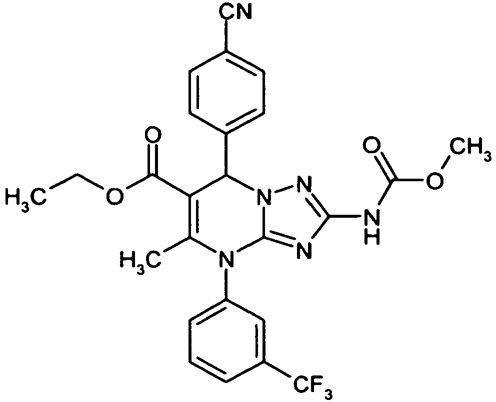

2-Amino-7-(4.cyanophenyl)-5-methyl-4-[3-(trifluormethyl)phenyl]-4,7-dihydro[1,2,4]triazolo[1,5-a]pyrimidin-6-carbonsäureethylester (10 mg, 21 µmol) wurde unter Argonschutzgasatmosphäre in einem Gemisch aus abs. THF (3 ml) und abs. Pyridin (3 ml) gelöst. Bei 0°C wurde in zwei Portionen Chlorameisensäuremethylester (17 mg, 180 µmol, 8.5 eq.) zugegeben und die Mischung für 12 h unter Erwärmung auf RT gerührt. Nachdem die HPLC-Analytik weitgehenden Umsatz zeigte, wurde das Reaktionsgemisch im Vakuum eingeengt und mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (10.8 mg, 96% d. Th.).

LC-MS (Methode 2): Rₜ = 2.59 min; MS (ESIpos): *m*/*z* (%) = 527.2 (100) [M+H]⁺; MS (ESIneg): *m*/*z* (%) = 525.1 (100) [M-H]⁻.

### Beispiel 49

### Ethyl 2-[(benzyloxy)carbonyl]amino-7-(4-cyanophenyl)-5-methyl-4-[3-(trifluormethyl)phenyl]-4,7-dihydro[1,2,4]triazolo[1,5-a]pyrimidin-6-carboxylat

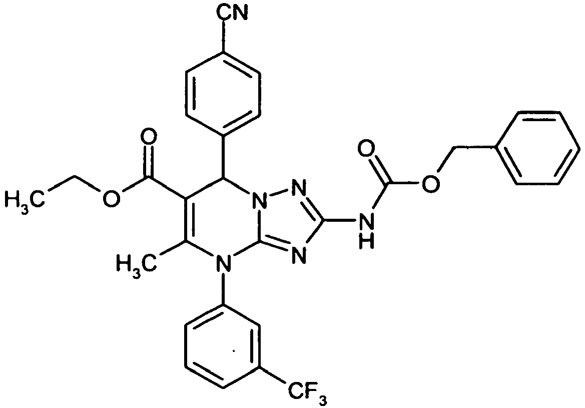

2-Amino-7-(4-cyanophenyl)-5-methyl-4-[3-(trifluormethyl)phenyl]-4,7-dihydro[1,2,4]triazolo[1,5-a]pyrimidin-6-carbonsäureethylester (130 mg, 278 µmol) wurde unter Argonschutzgasatmosphäre in abs. Pyridin (3.6 ml) gelöst. Bei 0°C wurde in vier Portionen Chlorameisensäurebenzylester (379 mg, 2.2 mmol, 8 eq.) zugegeben und die Mischung für 12 h unter Erwärmung auf RT gerührt. Nachdem die HPLC-Analytik weitgehenden Umsatz zeigte, wurde das Reaktionsgemisch im Vakuum eingeengt, der Rückstand in Acetonitril aufgenommen, mit 1 N Salzsäure angesäuert und dann mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (146.3 mg, 87% d. Th.).

LC-MS (Methode 4): Rₜ = 3.94 min; MS (ESIpos): *m*/*z* (%) = 603.2 (100) [M+H]⁺; MS (ESIneg): *m*/*z* (%) = 602.3 (100) [M-H]⁻.

¹H-NMR (400 MHz, DMSO-d₆): δ = 1.00 (t, 3H), 2.15 (s, 3H), 3.95 (q, 2H), 5.00 (s, 2H), 6.40 (s, 1H), 7.20-7.35 (m, 5H), 7.70-7.95 (m, 7H), 8.15 (br. s, 1H), 10.05 (s, 1H).

### Beispiel 50

### Ethyl 7-(4-cyanophenyl)-2-[(ethoxycarbonyl)amino]-5-methyl-4-[3-(trifluormethyl)phenyl]-4,7-dihydro[1,2,4]triazolo[1,5-a]pyrimidin-6-carboxylat

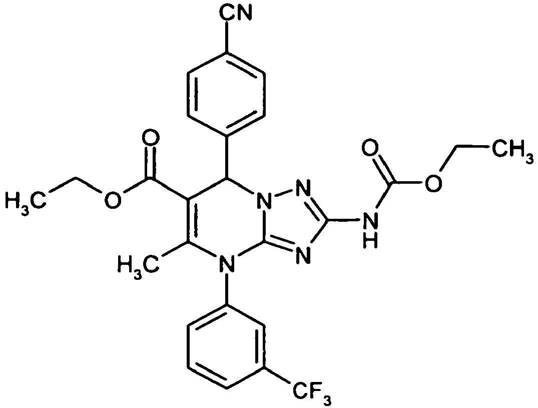

Ethyl 2-[(benzyloxy)carbonyl]amino-7-(4-cyanophenyl)-5-methyl-4-[3-(trifluormethyl)phenyl]-4,7-dihydro[1,2,4]triazolo[1,5-a]pyrimidin-6-carboxylat (125 mg, 207 µmol) wurde unter Argonschutzgasatmosphäre in einem Gemisch aus THF (0.9 ml), Wasser (0.6 ml) und Ethanol (3.8 ml) gelöst. Nach Zugabe von festem Lithiumhydroxid (14.9 mg, 620 µmol, 3 eq.) wurde das Reaktionsgemisch 1.5 h lang auf 55°C erhitzt. Im Eisbad wurde mit 1 N Salzsäure (4.1 ml) angesäuert und dann mittels präparativer HPLC gereinigt (Kromasil C18-Säule, 30 x 250 mm; Eluent: Acetonitril-Wasser-0.1% TFA). Nach Lyophilisation erhielt man das Produkt als Feststoff (4.8 mg, 5% d. Th.).

LC-MS (Methode 2): Rₜ = 2.70 min; MS (ESIpos): *m*/*z* (%) = 541.1 (100) [M+H]⁺; MS (ESIneg): *m*/*z* (%) = 539.2 (100) [M-H]⁻.

¹H-NMR (400 MHz, DMSO-d₆): δ = 1.00 (t, 3H), 1.10 (t, 3H), 2.15 (s, 3H), 3.95 (2q, 4H), 6.40 (s, 1H), 7.70-7.95 (m, 7H), 8.15 (br. s, 1H), 9.85 (s, 1H).

### Beispiel 51

### 1-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}-3-methylhamstoff

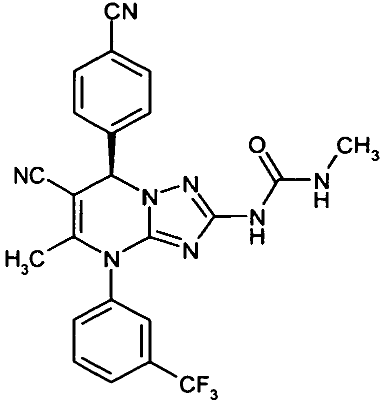

(7*R*)-2-Amino-7-(4-cyanophenyl)-5-methyl-4-[3-(trifluormethyl)phenyl]-4,7-dihydro[1,2,4]triazolo[1,5-a]pyrimidin-6-carbonitril (13 mg, 28 µmol) wurde unter Argonschutzgasatmosphäre in abs. Dichlormethan (2 ml) vorgelegt. Bei -78°C wurden Chlorameisensäure-4-nitrophenylester (28.6 mg, 142 µmol, 5 eq.), Triethylamin (14.7 mg, 145 µmol, 5.1 eq.) und DMAP (3.5 mg, 28 µmol) hinzugefügt. Nach 2 h Rühren bei -78°C ließ man das Reaktionsgemisch zunächst auf -20°C (2 h) und schließlich auf 0°C (2 h) auftauen. Danach wurde erneut auf -78°C heruntergekühlt und eine 2 M Lösung von Methylamin in THF (102 µl, 7.2 eq.) zugegeben. Die Mischung wurde anschließend auf -10°C erwärmt und über Nacht gerührt. Das Reaktionsgemisch wurde dann mit 2 M Lösung von Methylamin in THF (1 ml) versetzt, im Vakuum eingeengt und der Rückstand mittels präparativer HPLC gereinigt (Reprosil C18-Säule, 30 x 250 mm; Eluent: Acetonitril-Wasser-0.1% TFA). Nach Lyophilisation erhielt man das Produkt als Feststoff (5.9 mg, 43% d. Th.).

LC-MS (Methode 2): Rₜ = 2.25 min; MS (ESIpos): *m*/*z* (%) = 479.0 (100) [M+H]⁺; MS (ESIneg): *m*/*z* (%) = 477.1 (100) [M-H]⁻.

### Beispiel 52

### 1-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}-3-ethylharnstoff

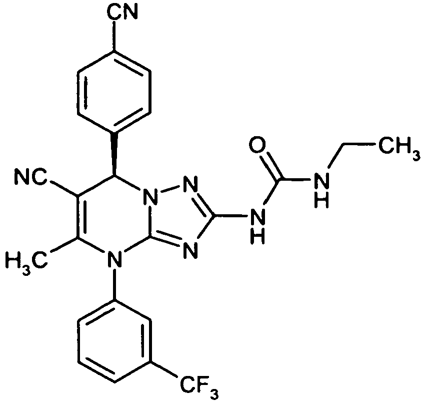

(7*R*)-2-Amino-7-(4-cyanophenyl)-5-methyl-4-[3-(trifluormethyl)phenyl]-4,7-dihydro[1,2,4]triazolo[1,5.a]pyrimidin-6-carbonitril (15 mg, 33 µmol) wurde unter Argonschutzgasatmosphäre in abs. Pyridin (2 ml) gelöst und mit festem Natriumsulfat (Spatelspitze) sowie Ethylisocyanat (46.6 mg, 655 µmol, 20 eq.) versetzt. Das Reaktionsgemisch wurde anschließend in einem Mikrowellenreaktor erhitzt (2.5 h bei 100°C, dann 2 h bei 110°C und schließlich 2 h bei 125°C). Nach Abkühlen wurde das Reaktionsgemisch filtriert, das Filtrat im Vakuum eingeengt und der Rückstand mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (7.8 mg, 48% d. Th.).

LC-MS (Methode 2): Rₜ = 2.34 min; MS (ESIpos): *m*/*z* (%) = 493.2 (100) [M+H]⁺; MS (ESIneg): *m*/*z* (%) = 491.2 (100) [M-H]⁻.

¹H-NMR (400 MHz, DMSO-d₆): δ = 0.80 (t, 3H), 2.00 (s, 3H), 2.95 (m, 2H), 6.40 (s, 1H), 7.15 (t, 1H), 7.75-7.95 (m, 7H), 8.20 (br. s, 1H), 9.35 (s, 1H).

### Beispiel 53

### 1-{(7R)-6-Cyano-7-(4-cyanophenyl)-5-methyl-4-(3-(trifluormethyl)phenyl]-4,7-dihydro[1,2,4]triazolo[1,5-a]pyrimidin-2-yl}-3-(2-hydroxyethyl)harnstoff

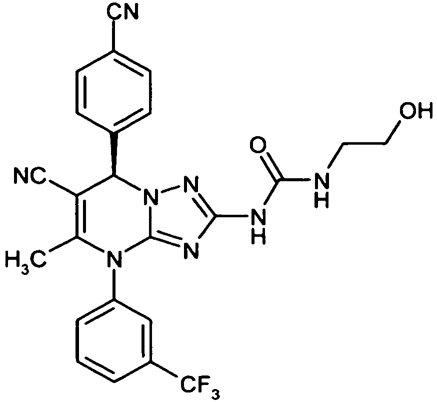

Unter einer Argonschutzgasatmosphäre wurden bei 0°C zu einer Lösung von Benzyl {(7*R*)-6-cyano-7-(4-cyanophenyl)-5-methyl-4-[3-(trifluormethyl)phenyl]-4,7-dihydro[1,2,4]triazolo[1,5-a]pyrimidin-2-yl}carbamat (10 mg, 18 µmol) in trockenem Dichlormethan (1 ml) Chlorameisensäure-4-nitrophenylester (7.3 mg, 36 µmol, 2 eq.), Triethylamin (3.1 mg, 40 µmol, 2.2 eq.) sowie DMAP (0.2 mg, 1.8 µmol, 0.1 eq.) gegeben und die Mischung für 12 h bei 4°C gerührt. Danach wurde bei 0°C 2-Aminoethanol (8.5 mg, 139 µmol, 10 eq.) hinzugefügt und die Mischung erneut für 12 h bei 4°C gerührt. Das Reaktionsgemisch wurde dann im Vakuum eingeengt und mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (1.9 mg, 27% d. Th.).

LC-MS (Methode 5): Rₜ = 1.08 min; MS (ESIpos): *m*/*z* (%) = 509.2 (100) [M+H]⁺; MS (ESIneg): *m*/*z* (%) = 507.5 (100) [M-H]⁻.

### Beispiel 54

### N-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}methansulfonamid

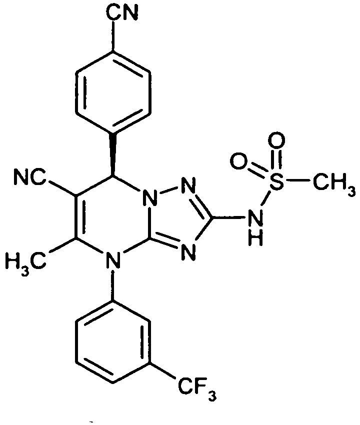

Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}(methylsulfonyl)carbamat (16.0 mg, 11 µmol) wurde unter einer Argonschutzgasatmosphäre in entgastem Methanol (2 ml) gelöst. Nach Zugabe einer katalytischen Menge von Palladium auf Aktivkohle (10%-ig) wurde unter einer Wasserstoffatmosphäre (~1 atm) für 0.5 h bei RT hydriert. Das Reaktionsgemisch wurde danach filtriert, das Filtrat im Vakuum eingeengt und der Rückstand mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (8.5 mg, 71% d. Th.).

LC-MS (Methode 5): Rₜ = 1.17 min; MS (ESIpos): *m*/*z* (%) = 500.1 (100) [M+H]⁺; MS (ESIneg): *m*/*z* (%) = 499.6 (100) [M-H]⁻.

¹H-NMR (400 MHz, DMSO-d₆): δ = 2.00 (s, 3H), 3.00 (s, 3H), 6.45 (s, 1H), 7.75-7.95 (m, 7H), 8.20 (br. s, 1H), 10.75 (s, 1H).

### Beispiel 55

### (7R)-7-(4-Cyanophenyl)-5-methyl-2-(methylamino)-4-[3-(trifluormethyl)phenyl]-4,7-dihydro-[1,2,4]triazolo[1,5-a]pyrimidin-6-carbonitril

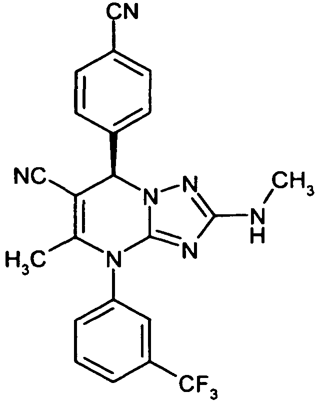

Benzyl {(7*R*)-6-cyano-7-(4-cyanophenyl)-5-methyl-4-[3-(trifluormethyl)phenyl]-4,7-dihydro-[1,2,4]triazolo[1,5-a]pyrimidin-2-yl}methylcarbamat (13.0 mg, 23 µmol) wurde unter einer Argonschutzgasatmosphäre in entgastem Methanol (2 ml) gelöst. Nach Zugabe einer katalytischen Menge von Palladium auf Aktivkohle (10%-ig) wurde unter einer Wasserstoffatmosphäre (∼1 atm) für 2 h bei RT hydriert. Das Reaktionsgemisch wurde danach filtriert, das Filtrat im Vakuum eingeengt und der Rückstand mittels präparativer HPLC gereinigt (Kromasil C18-Säule, 50 x 20 mm; Eluent: Acetonitril-Wasser-0.1% TFA). Nach Lyophilisation erhielt man das Produkt als Feststoff (8.2 mg, 83% d. Th.).

LC-MS (Methode 7): Rₜ = 1.92 min; MS (ESIpos): *m*/*z* (%) = 436.3 (100) [M+H]⁺; MS (ESIneg): *m*/*z* (%) = 434.2 (100) [M-H]⁻.

¹H-NMR (400 MHz, DMSO-d₆): δ = 2.00 (s, 3H), 2.45 (s, 3H), 6.00 (br. s, 1H), 6.20 (s, 1H), 7.75-7.95 (m, 7H), 8.15 (br. s, 1H).

### Beispiel 56

### (7R)-7-(4-Cyanophenyl)-2-(ethylamino)-5-methyl-4-[3-(trifluormethyl)phenyl]-4,7-dihydro[1,2,4]-triazolo[1,5-a]pyrimidin-6-carbonitril

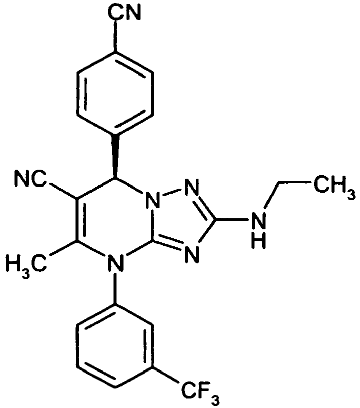

Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}(ethyl)carbamat (17.0 mg, 29 µmol) wurde unter einer Argonschutzgasatmosphäre in entgastem Methanol (2 ml) gelöst. Nach Zugabe von Palladium auf Aktivkohle (10%-ig; 2 mg) wurde unter einer Wasserstoffatmosphäre (∼1 atm) für 1 h bei RT hydriert. Das Reaktionsgemisch wurde danach filtriert, das Filtrat im Vakuum eingeengt und der Rückstand mittels präparativer HPLC gereinigt (Kromasil C18-Säule, 50 x 20 mm; Eluent: Acetonitril-Wasser-0.1% TFA). Nach Lyophilisation erhielt man das Produkt als Feststoff (10.9 mg, 83% d. Th.).

LC-MS (Methode 2): Rₜ = 2.40 min; MS (ESIpos): *m*/*z* (%) = 450.1 (100) [M+H]⁺; MS (ESIneg): *m*/*z* (%) = 448.0 (100) [M-H]⁻.

¹H-NMR (400 MHz, DMSO-d₆): δ = 0.95 (t, 3H), 1.95 (s, 3H), 2.85 (m, 2H), 6.05 (br. s, 1H), 6.20 (s, 1H), 7.70-7.95 (m, 7H), 8.15 (br. s, 1H).

### Beispiel 57

### (7R)-2-[(Cyanomethyl)amino]-7-(4-cyanophenyl)-5-methyl-4-[3-(trifluormethyl)phenyl]-4,7-dihydro[1,2,4]triazolo[1,5-a]pyrimidin-6-carbonitril

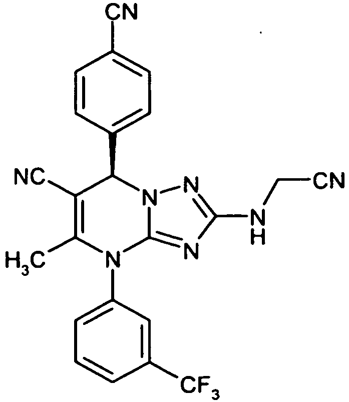

Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}(cyanomethyl)carbamat (10.0 mg, 17 µmol) wurde unter einer Argonschutzgasatmosphäre in entgastem Methanol (3 ml) gelöst. Nach Zugabe von Palladium auf Aktivkohle (10%-ig; 2 mg) wurde unter einer Wasserstoffatmosphäre (~1 atm) für 0.5 h bei RT hydriert. Das Reaktionsgemisch wurde danach filtriert, das Filtrat im Vakuum eingeengt und der Rückstand mittels präparativer HPLC gereinigt (Kromasil C18-Säule, 50 x 20 mm; Eluent: Acetonitril-Wasser-0.1% TFA). Nach Lyophilisation erhielt man das Produkt als Feststoff(7.4 mg, 96% d. Th.).

LC-MS (Methode 7): Rₜ = 1.98 min; MS (ESIpos): *m*/*z* (%) = 461.1 (100) [M+H]⁺; MS (ESIneg): m/z (%) = 459.1 (100) [M-H]⁻.

¹H-NMR (400 MHz, DMSO-d₆): δ = 1.95 (s, 3H), 3.90 (m, 2H), 6.30 (s, 1H), 6.90 (br. t, 1H), 7.70-7.95 (m, 7H), 8.15 (br. s, 1H).

### Beispiel 58

### (7R)-7-(4-Cyanophenyl)-5-methyl-2-(propylamino)-4-[3-(trifluormethyl)phenyl]-4,7-dihydro-[1,2,4]triazolo[1,5-a]pyrimidin-6-carbonitril

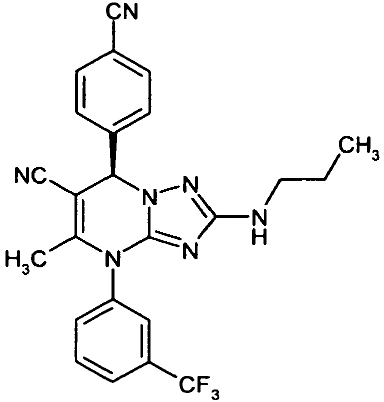

Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}(propyl)carbamat (17.0mg, 28 µmol) wurde unter einer Argonschutzgasatmosphäre in entgastem Methanol (5 ml) gelöst. Nach Zugabe von Palladium auf Aktivkohle (10%-ig; 5 mg) wurde unter einer Wasserstoffatmosphäre (∼1 atm) für 0.5 h bei RT hydriert. Das Reaktionsgemisch wurde danach filtriert, das Filtrat im Vakuum eingeengt und der Rückstand mittels präparativer HPLC gereinigt (Kromasil C18-Säule, 50 x 20 mm; Eluent: Acetonitril-Wasser-0.1% TFA). Nach Lyophilisation erhielt man das Produkt als Feststoff (10.7 mg, 81% d. Th.).

LC-MS (Methode 7): Rₜ = 2.19 min; MS (ESIpos): m/z (%) = 464.2 (100) [M+H]⁺; MS (ESIneg): *m*/*z* (%) = 462.2 (100) [M-H]⁻.

¹H-NMR (400 MHz, DMSO-d₆): δ = 0.70 (t, 3H), 1.35 (m, 2H), 1.95 (s, 3H), 2.75 (m, 2H), 6.10 (br. s, 1H), 6.20 (s, 1H), 7.70-7.95 (m, 7H), 8.15 (br. s, 1H).

### Beispiel 59

### (7R)-7-(4-Cyanophenyl)-5-methyl-2-[(2-methylpropyl)amino]-4-[3-(trifluormethyl)phenyl]-4,7-dihydro[1,2,4]triazolo[1,5-a]pyrimidin-6-carbonitril

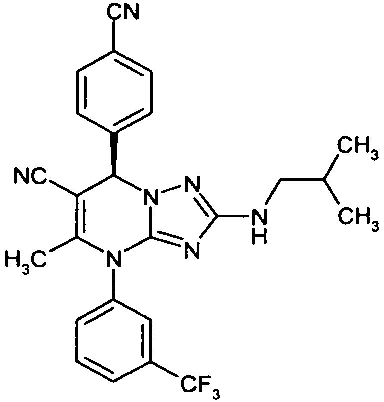

Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}(2-methylpropyl)carbamat (25.0 mg, 41 µmol) wurde unter einer Argonschutzgasatmosphäre in entgastem Methanol (5 ml) gelöst. Nach Zugabe von Palladium auf Aktivkohle (10%-ig; 5 mg) wurde unter einer Wasserstoffatmosphäre (∼1 atm) für 0.75 h bei RT hydriert. Das Reaktionsgemisch wurde danach filtriert, das Filtrat im Vakuum eingeengt und der Rückstand mittels präparativer HPLC gereinigt (Kromasil C18-Säule, 50 x 20 mm; Eluent: Acetonitril-Wasser-0.1% TFA). Nach Lyophilisation erhielt man das Produkt als Feststoff (13.6 mg, 70% d. Th.).

LC-MS (Methode 7): Rₜ = 2.32 min; MS (ESIpos): *m*/*z* (%) = 478.1 (100) [M+H]⁺; MS (ESIneg): *m*/*z* (%) = 476.2 (100) [M-H]⁻.

¹H-NMR (400 MHz, DMSO-d₆): δ = 0.70 (m, 6H), 1.65 (m, 1H), 1.95 (s, 3H), 2.65 (m, 2H), 6.15 (br. s, 1H), 6.20 (s, 1H), 7.65-7.95 (m, 7H), 8.15 (br. s, 1H).

### Beispiel 60

### (7R)-7-(4-Cyanophenyl)-2-[(cyclopropylmethyl)amino]-5-methyl-4-[3-(trifluormethyl)phenyl]-4,7-dihydro[1,2,4]triazolo[1,5-a]pyrimidin-6-carbonitril

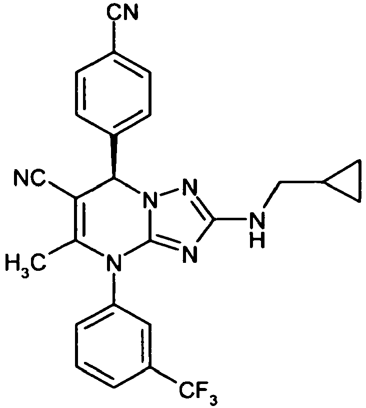

Benzyl {(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}(cyclopropylmethyl)carbamat (20.0 mg, 33 µmol) wurde unter einer Argonschutzgasatmosphäre in entgastem Methanol (5 ml) gelöst. Nach Zugabe von Palladium auf Aktivkohle (10%-ig; 5 mg) wurde unter einer Wasserstoffatmosphäre (∼1 atm) für 1.75 h bei RT hydriert. Das Reaktionsgemisch wurde danach filtriert, das Filtrat im Vakuum eingeengt und der Rückstand mittels präparativer HPLC gereinigt (Kromasil C18-Säule, 50 x 20 mm; Eluent: Acetonitril-Wasser-0.1% TFA). Nach Lyophilisation erhielt man das Produkt als Feststoff (10.9 mg, 70% d. Th.).

LC-MS (Methode 7): Rₜ = 2.21 min; MS (ESIpos): *m*/*z* (%) = 476.1 (100) [M+H]⁺; MS (ESIneg): *m*/*z* (%) = 474.2 (100) [M-H]⁻.

¹H-NMR (400 MHz, DMSO-d₆): δ = 0.05 (m, 2H), 0.25 (m, 2H), 0.80 (m, 1H), 1.95 (s, 3H), 2.65-2.80 (m, 2H), 6.15 (br. s, 1H), 6.20 (s, 1H), 7.65-7.95 (m, 7H), 8.15 (br. s, 1H).

### Beispiel 61

### tert.-Butyl N-{(7R)-6-cyano-7-(4-cyanophenyl)-5-methyl-4-[3-(trifluormethyl)phenyl]-4,7-dihydro-[1,2,4]triazolo[1,5-a]pyrimidin-2-yl}glycinat

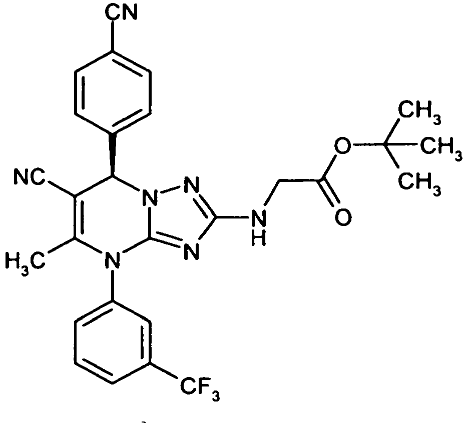

*tert*.-Butyl *N*-[(benzyloxy)carbonyl]-*N*-{(7R)-6-cyano-7-(4-cyanophenyl)-5-methyl-4-[3-(trifluormethyl)phenyl]-4,7-dihydro[1,2,4]triazolo[1,5-a]pyrimidin-2-yl}glycinat (23.0 mg, 34 µmol) wurde unter einer Argonschutzgasatmosphäre in entgastem Methanol (2.3 ml) gelöst. Nach Zugabe von Palladium auf Aktivkohle (10%-ig; 15.2 mg) wurde unter einer Wasserstoffatmosphäre (∼1 atm) für 0.5 h bei RT hydriert. Das Reaktionsgemisch wurde danach filtriert, das Filtrat im Vakuum eingeengt und der Rückstand mittels präparativer HPLC gereinigt (Kromasil C18-Säule, 50 x 20 mm; Eluent: Acetonitril-Wasser-0.1% TFA). Nach Lyophilisation erhielt man das Produkt als Feststoff (21.2 mg, quant.).

LC-MS (Methode 7): Rₜ = 2.25 min; MS (ESIpos): *m*/*z* (%) = 480.3 (100), 536.3 (80) [M+H]⁺; MS (ESIneg): *m*/*z* (%) = 534.3 (100) [M-H]⁻.

¹H-NMR (400 MHz, DMSO-d₆): δ = 1.15 (s, 9H), 1.95 (s, 3H), 3.45 (m, 2H), 6.20 (s, 1H), 6.45 (br. t, 1H), 7.65-7.95 (m, 7H), 8.15 (br. s, 1H).

### Beispiel 62

### N-{(7R)-6-Cyano-7-(4-cyanophenyl)-5-methyl-4-[3-(trifluormethyl)phenyl]-4,7-dihydro[1,2,4]-triazolo[1,5-a]pyrimidin-2-yl}glycin

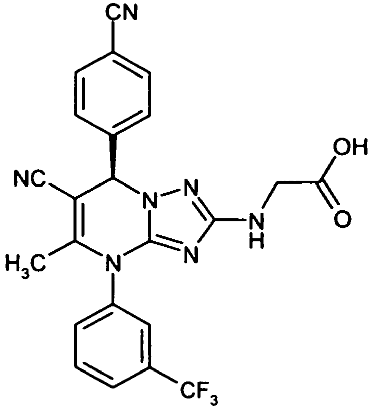

*tert*.-Butyl *N*-{(7*R*)-6-cyano-7-(4-cyanophenyl)-5-methyl-4-[3-(trifluormethyl)phenyl]-4,7-dihydro-[1,2,4]triazolo[1,5-a]pyrimidin-2-yl}glycinat (18.0 mg, 34 µmol) wurde unter Argonschutzgasatmosphäre in trockenem Dichlormethan (3.1 ml) gelöst. Bei Raumtemperatur wurde Trifluoressigsäure (1.6 ml) zugegeben und die Mischung für 45 min gerührt. Anschließend wurde erneut Trifluoressigsäure (2 ml) zugegeben und für weitere 30 min gerührt. Das Reaktionsgemisch wurde danach im Vakuum eingedampft und der Rückstand mittels präparativer HPLC gereinigt (Gromsil C18-Säule, 30 x 250 mm; Eluent: Acetonitril-Wasser-0.1% TFA). Nach Lyophilisation erhielt man das Produkt als Feststoff (15.2 mg, 94% d. Th.).

LC-MS (Methode 7): Rₜ = 1.78 min; MS (ESIpos): *m*/*z* (%) = 480.2 (100) [M+H]⁺; MS (ESIneg): *m*/*z* (%) = 478.2 (100) [M-H]⁻.

¹H-NMR (400 MHz, DMSO-d₆): δ = 1.95 (s, 3H), 3.55 (s, 2H), 6.20 (s, 1H), 6.40 (br. t, 1H), 7.65-7.95 (m, 7H), 8.15 (br. s, 1H), 12.30 (br. s, 1H).

### Beispiel 63

### N²-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}glycinamid

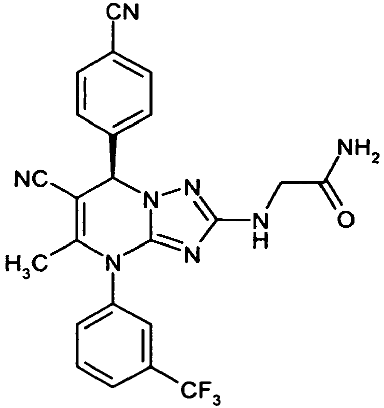

Unter Argonschutzgasatmosphäre wurde eine Lösung von *N*-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}glycin (10.5 mg, 22 µmol) in DMF (1 ml) bei 0°C mit HATU (25 mg, 66 µmol, 3 eq.) und Triethylamin (11 mg, 110 µmol, 5 eq.) versetzt. Nachdem das Reaktionsgemisch auf RT aufgetaut war, wurde erneut auf 0°C abgekühlt und anschließend eine 0.5 M Lösung von Ammoniak in Dioxan (0.44 ml, 219 µmol, 10 eq.) zugegeben. Die Mischung wurde für 2 h unter allmählicher Erwärmung auf RT gerührt. Das Reaktionsgemisch wurde danach im Vakuum eingedampft und der Rückstand mittels präparativer HPLC gereinigt (Kromasil C18-Säule, 50 x 20 mm; Eluent: Acetonitril-Wasser-0.1% TFA). Nach Lyophilisation erhielt man das Produkt als Feststoff (9.2 mg, 88% d. Th.).

LC-MS (Methode 5): Rₜ = 1.07 min; MS (ESIpos): *m*/*z* (%) = 479.2 (100) [M+H]⁺; MS (ESIneg): *m*/*z* (%) = 477.5 (100) [M-H]⁻.

¹H-NMR (400 MHz, DMSO-d₆): δ = 1.95 (s, 3H), 3.40 (d, 2H), 6.25 (s, 1H), 6.90 (br. s, 1H), 7.05 (br. s, 1H), 7.65-7.95 (m, 8H), 8.15 (br. s, 1H).

### Beispiel 64

### N²-{(7R)-6-Cyano-7-(4-cyanophenyl)-5-methyl-4-[3-(trifluormethyl)phenyl]-4,7-dihydro[1,2,4]triazolo[1,5-a]pyrimidin-2-yl}-N,N-dimethylglycinamid

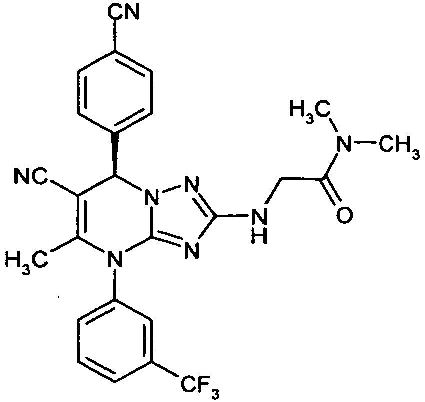

Die Titelverbindung wurde als Nebenprodukt bei der Herstellung der Verbindung aus Beispiel 63 erhalten und im Zuge der dort beschriebenen HPLC-Aufreinigung abgetrennt. Nach Lyophilisation der entsprechenden Fraktionen wurde 1 mg (9% d. Th.) als Feststoff erhalten.

LC-MS (Methode 5): Rₜ = 1.15 min; MS (ESIpos): *m*/*z* (%) = 507.2 (100) [M+H]⁺; MS (ESIneg): *m*/*z* (%) = 505.1 (100) [M-H]⁻.

¹H-NMR (400 MHz, DMSO-d₆): δ = 1.95 (s, 3H), 2.75 (s, 3H), 2.85 (s, 3H), 3.65 (d, 1H), 3.70 (d, 1H), 5.80 (br. s, 1H), 6.25 (s, 1H), 7.65-7.95 (m, 7H), 8.15 (br. s, 1H).

### Beispiel 65

### (rac)-7-(4-Cyanophenyl)2-(methoxymethyl)-5-methyl-4-[3-(trifluormethyl)phenyl]-4,7-dihydro-[1,2,4]triazolo[1,5-a]pyrimidin-6-carbonitril

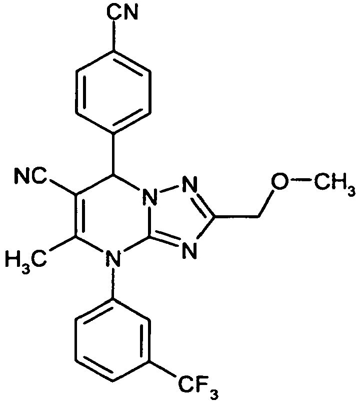

(rac)-7-(4-Cyanophenyl)-2-(methoxymethyl)-5-methyl-4,7-dihydro[1,2,4]triazolo[1,5-a]pyrimidin-6-carbonitril (35 mg, 114 µmol) wurde unter Argonschutzgasatmosphäre in abs. Dichlormethan (30 ml) für 1 h mit Molekularsieb (0.5 g, 4 Å) gerührt. Anschließend wurden 3-(Trifluormethyl)-phenylboronsäure (65 mg, 343 µmol, 3 eq.), wasserfreies Kupfer(II)acetat (62.3 mg, 343 µmol, 3 eq.), abs. Pyridin (25 µl) sowie Triethylamin (35 mg, 343 µmol, 3 eq.) zugegeben und die Mischung 12 h lang bei RT gerührt. Danach wurden erneut Molekularsieb (0.5 g, 4 Å), 3-(Trifluormethyl)phenylboronsäure (22 mg, 114 µmol, 1 eq.), wasserfreies Kupfer(II)acetat (21 mg, 114 µmol, 1 eq.) und abs. Pyridin (829 µl) hinzugefügt und die Mischung weitere 3 d bei RT gerührt. Das Reaktionsgemisch wurde dann über Kieselgur filtriert, der Filterrückstand mit Dichlormethan und Pyridin nachgewaschen und das Filtrat zur Trockene eingeengt. Der Rückstand wurde mittels präparativer HPLC gereinigt (Gromsil C18-Säule, 10 µm; Eluent: Acetonitril/Wasser + 0.1% TFA 10:90 → 90:10). Das Produkt wurde als farbloser Feststoff erhalten (4.4 mg, 9% d. Th.).

LC-MS (Methode 8): Rₜ = 2.18 min; MS (ESIpos): *m*/*z* (%) = 451.3 (100) [M+H]⁺; MS (ESIneg): *m*/*z* (%) = 449.1 (100) [M-H]⁻.

¹H-NMR (400 MHz, DMSO-d₆): δ = 2.00 (s, 3H), 3.15 (s, 2H), 4.15 (s, 3H), 6.50 (s, 1H), 7.70-8.00 (m, 7H), 8.15 (br. s, 1H).

### Beispiel 66

### (rac)-7-(4-Cyanophenyl)-5-methyl-2-thiophen-2-yl-4-[3-(trifluormethyl)phenyl]-4,7-dihydro-[1,2,4]triazolo[1,5-a]pyrimidin-6-carbonitril

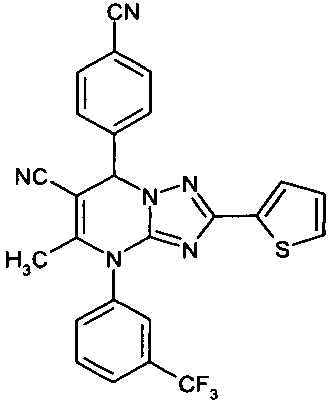

(*rac*)-7-(4-Cyanophenyl)-5-methyl-2-thiophen-2-yl-4,7-dihydro[1,2,4]triazolo[1,5-a]pyrimidin-6-carbonitril (70 mg, 230 µmol) wurde unter Argonschutzgasatmosphäre in abs. Dichlormethan (60 ml) für 1 h mit Molekularsieb (0.5 g, 4 Å) gerührt. Anschließend wurden 3-(Trifluormethyl)-phenylboronsäure (131 mg, 690 µmol, 3 eq.), wasserfreies Kupfer(II)acetat (125 mg, 690 µmol, 3 eq.), abs. Pyridin (1668 µl) sowie Triethylamin (70 mg, 690 µmol, 3 eq.) zugegeben und die Mischung 12 h lang bei RT gerührt. Danach wurden erneut Molekularsieb (0.5 g, 4 Å), 3-(Trifluormethyl)phenylboronsäure (44 mg, 230 µmol, 1 eq.), wasserfreies Kupfer(II)acetat (42 mg, 230 µmol, 1 eq.) und abs. Pyridin (500 µl) hinzugefügt und die Mischung weitere 3 d bei RT gerührt. Das Reaktionsgemisch wurde dann über Kieselgur filtriert, der Filterrückstand mit Dichlormethan und Pyridin nachgewaschen und das Filtrat zur Trockene eingeengt. Der Rückstand wurde mittels präparativer HPLC gereinigt (Gromsil C18-Säule, 10 µm; Eluent: Acetonitril/Wasser + 0.1% TFA 10:90 → 90:10). Das Produkt wurde als farbloser Feststoff erhalten (40.1 mg, 36% d. Th.).

LC-MS (Methode 8): Rₜ = 2.52 min; MS (ESIpos): m/z (%) = 489.2 (100) [M+H]⁺; MS (ESIneg): *m*/*z* (%) = 487.1 (100) [M-H]⁻.

¹H-NMR (400 MHz, DMSO-d₆): δ = 2.00 (s, 3H), 6.55 (s, 1H), 7.05 (m, 1H), 7.35 (m, 1H), 7.55 (m, 1H), 7.80-8.00 (m, 7H), 8.20 (br. s, 1H).

### Beispiel 67

### (rac)-7-(4-Cyanophenyl)-5-methyl-2-(1-methylethyl)-4-[3-(trifluormethyl)phenyl]-4,7-dihydro-[1,2,4]triazolo[1,5-a]pyrimidin-6-carbonitril

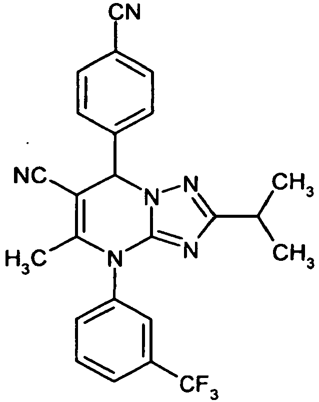

(rac)-7-(4-Cyanophenyl)-5-methyl-2-(1-methylethyl)-4,7-dihydro[1,2,4]triazolo[1,5-a]pyrimidin-6-carbonitril (63 mg, 207 µmol) wurde unter Argonschutzgasatmosphäre in abs. Dichlormethan (50 ml) für 1 h mit Molekularsieb (0.5 g, 4 Å) gerührt. Anschließend wurden 3-(Trifluormethyl)-phenylboronsäure (118 mg, 621 µmol, 3 eq.), wasserfreies Kupfer(II)acetat (113 mg, 621 µmol, 3 eq.), abs. Pyridin (1390 µl) sowie Triethylamin (63 mg, 621 µmol, 3 eq.) zugegeben und die Mischung 12 h lang bei RT gerührt. Danach wurden erneut Molekularsieb (0.5 g, 4 Å), 3-(Trifluormethyl)phenylboronsäure (39 mg, 207 µmol, 1 eq.), wasserfreies Kupfer(II)acetat (38 mg, 207 µmol, 1 eq.) und abs. Pyridin (500 µl) hinzugefügt und die Mischung weitere 3 d bei RT gerührt. Das Reaktionsgemisch wurde dann über Kieselgur filtriert, der Filterrückstand mit Dichlormethan und Pyridin nachgewaschen und das Filtrat zur Trockene eingeengt. Der Rückstand wurde mittels präparativer HPLC gereinigt (Gromsil C18-Säule, 10 µm; Eluent: Acetonitril/Wasser + 0.1% TFA 10:90 → 90:10). Das Produkt wurde als farbloser Feststoff erhalten (29.8 mg, 64% d. Th.).

LC-MS (Methode 8): Rₜ = 2.44 min; MS (ESIpos): *mlz* (%) = 449.3 (100) [M+H]⁺; MS (ESIneg): *m*/*z* (%) = 447.1 (70) [M-H]⁻.

¹H-NMR (400 MHz, DMSO-d₆): δ = 1.0 (m, 6H), 1.95 (s, 3H), 2.70 (m, 1H), 6.45 (s, 1H), 7.70-8.00 (m, 7H), 8.15 (br. s, 1H).

### Beispiel 68

### (rac)-7-(4-Cyanophenyl)-2-methoxy-5-methyl-4-[3-(trifluormethyl)phenyl]-4,7-dihydro[1,2,4]-triazolo[1,5-a]pyrimidin-6-carbonitril

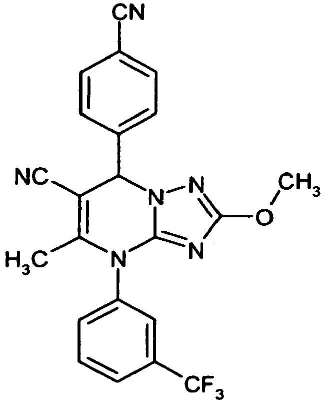

(*rac*)-7-(4-Cyanophenyl)-2-methoxy-5-methyl-4,7-dihydro[1,2,4]triazolo[1,5-a]pyrimidin-6-carbonitril (50 mg, 171 µmol) wurde unter Argonschutzgasatmosphäre in abs. Dichlormethan (7 ml) für 1 h mit Molekularsieb (0.5 g, 4 Å) gerührt. Anschließend wurden 3-(Trifluormethyl)phenylboronsäure (97 mg, 513 µmol, 3 eq.), wasserfreies Kupfer(II)acetat (93 mg, 513 µmol, 3 eq.), abs. Pyridin (8 ml) sowie Triethylamin (72 µl, 513 µmol, 3 eq.) zugegeben und die Mischung 48 h lang bei RT gerührt. Danach wurde erneut wasserfreies Kupfer(II)acetat (31 mg, 171 µmol, 1 eq.) sowie 2,6-Lutidin (60 µl, 513 µmol, 3 eq.) hinzugefügt und die Mischung unter einer Atmosphäre von trockener Luft für weitere 7 d bei RT gerührt. Danach wurde das Reaktionsgemisch zur Trockene eingeengt. Der Rückstand wurde in Essigsäureethylester (10 ml) suspendiert und filtriert. Das Filtrat wurde mit Wasser (5 ml) und gesättigter Natriumchlorid-Lösung gewaschen, über Natriumsulfat getrocknet, filtriert und im Vakuum eingeengt. Der Rückstand wurde mittels präparativer HPLC gereinigt (Reprosil C18-Säule, 10 µm; Eluent: Acetonitril/Wasser+0.1%TFA 10:90 → 90:10). Der so erhaltene braune Feststoff wurde durch Filtration über eine kleine Kieselgel-Kartusche mit einem Eluentengemisch aus Cyclohexan und Essigsäureethylester (2:1) entfärbt und anschließend lyophilisiert. Das Produkt wurde als Feststoff erhalten (10.8 mg, 15% d. Th.).

LC-MS (Methode 5): Rₜ = 1.26 min; MS (ESIpos): *m*/*z* (%) = 437.1 (100) [M+H]⁺; MS (ESIneg): m/z (%) = 435.2 (100) [M-H]⁻.

¹H-NMR (400 MHz, DMSO-d₆): δ = 1.95 (s, 3H), 3.65 (s, 3H), 6.35 (s, 1H), 7.70-8.00 (m, 7H), 8.15 (br. s, 1H).

### Beispiel 69

### (rac)-4-(6-Acetyl-5-methyl-4-[3-(trifluormethyl)phenyl]-4,7-dihydrotetrazolo[1,5-a]pyrimidin-7-yl)benzonitril

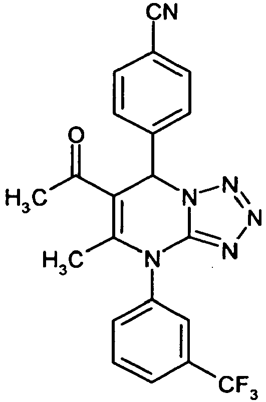

(rac)-4-(6-Acetyl-5-methyl-4,7-dihydrotetrazolo[1,5-a]pyrimidin-7-yl)benzonitril (50 mg, 178 µmol) wurde unter Argonschutzgasatmosphäre in abs. Dichlormethan (3 ml) für 1 h mit Molekularsieb (0.5 g, 4 Å) gerührt. Anschließend wurden 3-(Trifluormethyl)phenylboronsäure (101.6 mg, 535 µmol, 3 eq.), wasserfreies Kupfer(II)acetat (97.2 mg, 535 µmol, 3 eq.), abs. Pyridin (115 µl, 1.43 mmol, 8 eq.) sowie Triethylamin (54.2 mg, 535 µmol, 3 eq.) zugegeben und die Mischung 12 h lang bei RT gerührt. Danach wurden erneut Molekularsieb (0.5 g, 4 Å), 3-(Trifluormethyl)phenylboronsäure (33 mg, 178 µmol, 1 eq.), wasserfreies Kupfer(II)acetat (32 mg, 178 µmol, 1 eq.), Triethylamin (18.1 mg, 178 µmol, 1 eq.) sowie abs. Pyridin (57.5 µl, 715 µmol, 4 eq.) hinzugefügt und die Mischung weitere 12 h bei RT gerührt. Das Reaktionsgemisch wurde dann über Kieselgur filtriert, der Filterrückstand mit Dichlormethan und Methanol nachgewaschen und das Filtrat zur Trockene eingeengt. Der Rückstand wurde mittels präparativer HPLC gereinigt (Gromsil C18-Säule, 10 µm; Eluent: Acetonitril/Wasser + 0.1% TFA 10:90 → 90:10). Das Produkt wurde als farbloser Feststoff erhalten (14.6 mg, 19% d. Th.).

LC-MS (Methode 2): Rₜ = 2.47 min; MS (ESIpos): *m*/*z* (%) = 425.1 (100) [M+H]⁺; MS (ESIneg): *m*/*z* (%) = 423.1 (100) [M-H]⁻.

¹H-NMR (400 MHz, DMSO-d₆): δ = 2.10 (s, 3H), 2.20 (s, 3H), 7.10 (s, 1H), 7.80-8.00 (m, 7H), 8.20 (br. s, 1H).

### Beispiel 70

### (rac)-7-(4-Cyanophenyl)-5-methyl-4-[3-(trifluormethyl)phenyl]-4,7-dihydrotetrazolo[1,5-a]pyrimidin-6-carbonsäureethylester

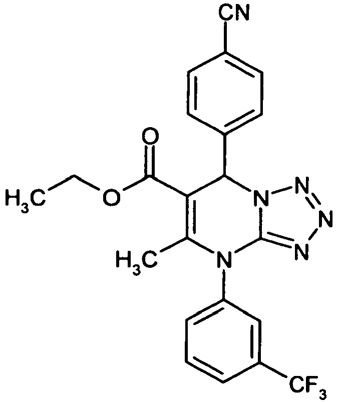

(rac)-7-(4-Cyanophenyl)-5-methyl-4,7-dihydrotetrazolo[1,5-a]pyrimidin-6-carbonsäureethylester (600 mg, 1.9 mmol) wurde unter Argonschutzgasatmosphäre in abs. Dichlormethan (450 ml) für 1 h mit Molekularsieb (0.5 g, 4 Å) gerührt. Anschließend wurden 3-(Trifluormethyl)phenylboronsäure (1.10 g, 5.8 mmol, 3 eq.), wasserfreies Kupfer(II)acetat (1.05 g, 5.8 mmol, 3 eq.), abs. Pyridin (1.25 ml, 15.5 mmol, 8 eq.) sowie Triethylamin (0.59 g, 5.8 mmol, 3 eq.) zugegeben und die Mischung 12 h lang bei RT gerührt. Danach wurden erneut Molekularsieb (0.5 g, 4 Å), 3-(Trifluormethyl)phenylboronsäure (0.37 g, 1.9 mmol, 1 eq.), wasserfreies Kupfer(II)acetat (0.35 g, 1.9 mmol, 1 eq.) sowie abs. Pyridin (0.63 ml, 7.73 mmol, 4 eq.) hinzugefügt und die Mischung weitere 12 h bei RT gerührt. Das Reaktionsgemisch wurde dann über Kieselgur filtriert, der Filterrückstand mit Dichlormethan und Methanol nachgewaschen und das Filtrat zur Trockene eingeengt. Der Rückstand wurde mittels präparativer HPLC gereinigt (Gromsil C18-Säule, 10 µm Eluent: Acetonitril/Wasser + 0.1% TFA 10:90 → 90:10). Das Produkt wurde als farbloser, amorpher Feststoff erhalten (404.6 mg, 46% d. Th.).

LC-MS (Methode 2): Rₜ = 2.36 min; MS (ESIpos): *m*/*z* (%) = 455.1 (100) [M+H]⁺; MS (ESIneg) *mlz* (%) = 453.2 (100) [M-H]⁻.

¹H-NMR (400 MHz, DMSO-d₆): δ = 1.00 (t, 3H), 2.20 (s, 3H), 4.00 (q, 2H), 6.90 (s, 1H), 7.80-8.00 (m, 7H), 8.20 (br. s, 1H).

### Beispiel 71

### (7R)-7-(4-Cyanophenyl)-5-methyl-4-[3-(trifluormethyl)phenyl]-4,7-dihydrotetrazolo[1,5-a]pyrimidin-6-carbonsäureethylester

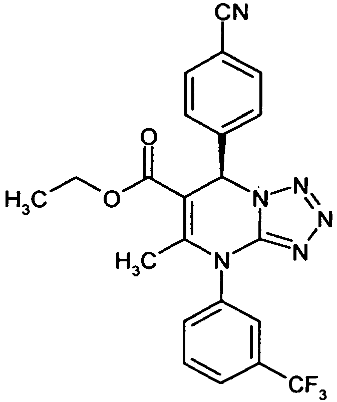

(rac)-7-(4-Cyanophenyl)-5-methyl-4-[3-(trifluormethyl)phenyl]-4,7-dihydrotetrazolo[1,5-a]pyrimidin-6-carbonsäureethylester (443 mg, 0.98 mmol) wurde durch präparative HPLC-Chromatographie an chiraler Phase in die Enantiomere aufgetrennt [stationäre Phase: Daicel Chiralpak IB 5 µm; Säulendimension: 250 x 20 mm; Probenvorbereitung: Lösung in 320 ml Methanol/MTBE (3:13); Injektionsvolumen: 0.9 ml; Eluent: MTBE/Methanol 9:1; Fluss: 15 ml/min; Temperatur: 30°C; Detektion: 220 nm]. Es wurden 215 mg (97% d. Th., >99.5% ee) des 7R-Enantiomeren als farbloser, amorpher Feststoff erhalten.

HPLC [stationäre Phase: Daicel Chiralpak IB 5 µm; Säulendimension: 250 x 4.6 mm; Eluent: MTBE/Methanol 9:1; Fluss: 1 ml/min; Temperatur: 25°C; Detektion: 220 nm]: Rₜ = 4.13 min. (7S-Enantiomer: Rₜ = 3.59 min.).

Die Absolutkonfiguration der Titelverbindung wurde durch Einkristall-Röntgenstrukturanalyse belegt.

Für weitere analytische Daten siehe die racemische Verbindung (Beispiel 70).

### Beispiel 72

### (7R)-7-(4-Cyanophenyl)-5-methyl-4-[3-(trifluormethyl)phenyl]-4,7-dihydrotetrazolo[1,5-a]pyrimidin-6-carbonsäure

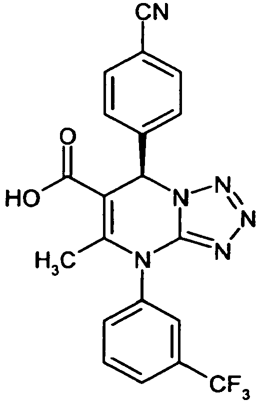

Die Reaktion wurde unter Argon durchgeführt. Zu einer Lösung von (7*R*)-7-(4-Cyanophenyl)-5-methyl-4-[3-(trifluormethyl)phenyl]-4,7-dihydrotetrazolo[1,5-a]pyrimidin-6-carbonsäureethylester (100 mg, 220 µmol) in einem Ethanol/THF/Wasser-Gemisch (4:1:0.6, 5.6 ml) wurde festes Lithiumhydroxid (15.8 mg, 660 µmol, 3 eq.) gegeben. Die Lösung wurde für 1.5 h auf 55°C erhitzt. Anschließend wurde bei 0°C mit 1 N Salzsäure (0.66 ml) angesäuert und dann direkt mittels präparativer HPLC aufgereinigt (Kromasil C18-Säule; Eluent: Acetonitril/Wasser+0.1%TFA 10:90 → 90:10). Das Produkt wurde als farbloser, amorpher Feststoff erhalten (67 mg, 71 % d. Th.).

LC-MS (Methode 2): Rₜ = 2.36 min; MS (ESIpos): *m*/*z* (%) = 427.1 (100) [M+H]⁺; MS (ESIneg): *m*/*z* (%) = 425.0 (80) [M-H]⁻.

¹H-NMR (400 MHz, DMSO-d₆): δ = 2.20 (s, 3H), 6.85 (s, 1H), 7.75-8.00 (m, 7H), 8.20 (br. s, 1H), 12.85 (br. s, 1H).

### Beispiel 73

### (7R)-7-(4-Cyanophenyl)-5-methyl-4-[3-(trifluormethyl)phenyl]-4,7-dihydrotetrazolo[1,5-a]pyrimidin-6-carboxamid

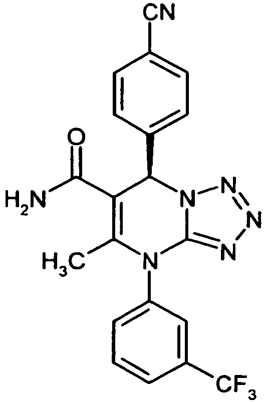

Die Reaktion wurde unter Argon durchgeführt. (7R)-7-(4-Cyanophenyl)-5-methyl-4-[3-(trifluormethyl)phenyl]-4,7-dihydrotetrazolo[1,5-a]pyrimidin-6-carbonsäure (62 mg, 145 µmol) und Ammoniumchlorid (116.7 mg, 2.2 mmol, 15 eq.) wurden in trockenem DMF (3.5 ml) bei °C vorgelegt und mit HATU (415 mg, 1.8 mmol, 7.5 eq.) sowie Triethylamin (147 mg, 1.45 mmol, 10 eq.) versetzt. Das Gemisch wurde über 12 h unter allmählicher Erwärmung auf RT gerührt. Danach wurde das Reaktionsgemisch eingeengt, der Rückstand in Acetonitril (mit 0.1% TFA) aufgenommen und dann mittels präparativer HPLC gereinigt (Gromsil C18-Säule, 10 µm; Eluent: Acetonitril/Wasser + 0.1% TFA 10:90 → 90:10). Das Produkt wurde als farbloser, amorpher Feststoff erhalten (36 mg, 58% d. Th.).

LC-MS (Methode 4): Rₜ = 2.86 min; MS (ESIpos): *m*/*z* (%) = 426.1 (100) [M+H]⁺; MS (ESIneg): *m*/*z* (%) = 424.2 (60) [M-H]⁻.

¹H-NMR (400 MHz, DMSO-d₆): δ = 1.90 (s, 3H), 6.90 (s, 1H), 7.30 (br. s, 1H), 7.55 (br. s, 1H), 7.70-7.95 (m, 7H), 8.10 (br. s, 1H).

### Beispiel 74

### (rac)-7-(4-Cyanophenyl)-5-methyl-4-[3-(trifluormethyl)phenyl]-4,7-dihydrotetrazolo[1,5-a]pyrimidin-6-carbonitril

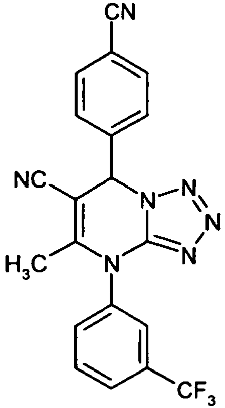

Die Reaktion wurde unter Argon durchgeführt. (rac)-7-(4-Cyanophenyl)-5-methyl-4-[3-(trifluormethyl)phenyl]-4,7-dihydrotetrazolo[1,5-a]pyrimidin-6-carboxamid (10 mg, 24 µmol; hergestellt analog zu Beispiel 72/73 ausgehend von Beispiel 70) wurde in trockenem THF (0.6 ml) vorgelegt, mit Methoxycarbonylsulfamoyl-triethylammoniumhydroxid (Burgess-Reagens; 22 mg, 94 µmol, 4 eq.) versetzt und 1 h bei RT gerührt. Die HPLC-Kontrolle zeigte vollständigen Umsatz an. Das Reaktionsgemisch wurde daraufhin eingeengt und der Rückstand über präparative HPLC gereinigt (Gromsil C18-Säule, 10 µm; Eluent: Acetonitril/Wasser + 0.1% TFA 10:90 → 90:10). Die Titelverbindung wurde als Feststoff erhalten (8.3 mg, 87% d. Th.).

LC-MS (Methode 2): Rₜ = 2.57 min; MS (ESIpos): *m*/*z* (%) = 408.1 (100) [M+H]⁺; MS (ESIneg): *m*/*z* (%) = 406.1 (100) [M-H]⁻.

HR-MS (Methode 10): C₂₀H₁₃N₇F₃ [M+H]⁺ gef. 408.1183, ber. 408.1179.

¹H-NMR (400 MHz, DMSO-d₆): δ = 2.05 (s, 3H), 6.90 (s, 1H), 7.85 (br. m, 3H), 8.00 (br. s, 4H), 8.25 (br. s, 1H).

### Beispiel 75

### (7R)-7-(4-Cyanophenyl)-5-methyl-4-[3-(trifluormethyl)phenyl]-4,7-dihydrotetrazolo[1,5-a]pyrimidin-6-carbonitril

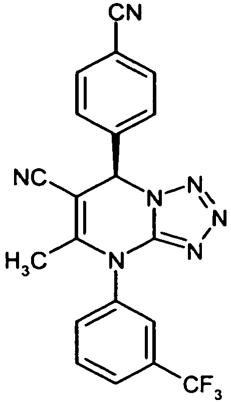

Die Reaktion wurde unter Argon durchgeführt. (7R)-7-(4-Cyanophenyl)-5-methyl-4-[3-(trifluormethyl)phenyl]-4,7-dihydrotetrazolo[1,5-a]pyrimidin-6-carboxamid (34 mg, 80 µmol) wurde in trockenem THF (1.9 ml) vorgelegt, mit Methoxycarbonylsulfamoyl-triethylammoniumhydroxid (Burgess-Reagens; 76 mg, 320 µmol, 4 eq.) versetzt und 1 h bei RT gerührt. Die HPLC-Kontrolle zeigte vollständigen Umsatz an. Das Reaktionsgemisch wurde daraufhin eingeengt und der Rückstand über präparative HPLC gereinigt (Gromsil C18-Säule, 10 µm; Eluent: Acetonitril/Wasser + 0.1 % TFA 10:90 → 90:10). Die Titelverbindung wurde als Feststoff erhalten (29 mg, 89% d. Th.).

LC-MS (Methode 3): Rₜ = 3.62 min; MS (ESIpos): *mlz* (%) = 408.3 (30) [M+H]⁺; MS (ESIneg): *m*/*z* (%) = 406.2 (100) [M-H]⁻.

### Beispiel 76

### 4-(6-(2-Methoxyethyl)-7-oxo-4-[3-(trifluormethyl)phenyl]-5,6,7,8-tetrahydro-4H pyrrolo[3,4-d]-tetrazolo[1,5-a]pyrimidin-8-yl)benzonitril

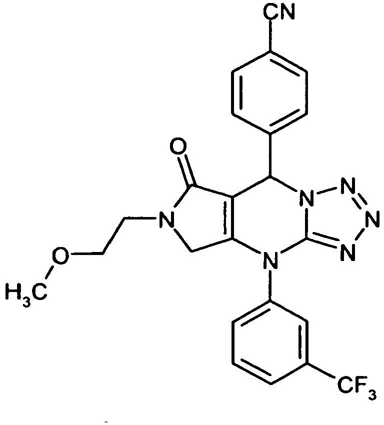

Ethyl 5-(brommethyl)-7-(4-cyanophenyl)-4-[3-(trifluormethyl)phenyl]-4,7-dihydrotetrazolo[1,5-a]-pyrimidin-6-carboxylat (35.3 mg, 66.1 µmol) wurde in Aceton (1.0ml) vorgelegt. Es wurde 2-Methoxyethylamin (12.4 mg, 4 µl, 165 µmol, 2.5 eq.) zugetropft und anschließend über Nacht bei RT gerührt. Nach Zugabe von weiterem 2-Methoxyethylamin (9.9 mg, 132 µmol, 2 eq.) wurde nochmals für 5 h bei RT gerührt. Das Reaktionsgemisch wurde dann im Vakuum eingedampft und der Rückstand mittels präparativer HPLC gereinigt (Kromasil C18-Säule, 30 x 250 mm; Eluent: Acetonitril-Wasser-0.1% TFA). Nach Lyophilisation erhielt man das Produkt als Feststoff (13 mg, 41% d. Th.).

LC-MS (Methode 4): Rₜ = 3.13 min; MS (ESIpos): *m*/*z* (%) = 482.1 (100) [M+H]⁺; MS (ESIneg): *m*/*z* (%) = 480.2 (100) [M-H]⁻.

¹H-NMR (400 MHz, DMSO-d₆): δ = 3.15 (s, 3H), 3.30-3.50 (m, 4H), 4.15 (dd, 2H), 6.90 (s, 1H), 7.75-8.15 (m, 7H), 8.30 (br. s, 1H).

### Beispiel 77

### (rac)-6-(4-Cyanophenyl)-8-methyl-9-[3-(trifluormethyl)phenyl]-6,9-dihydropyrimido[2,1-f]purin-7-carbonsäureethylester

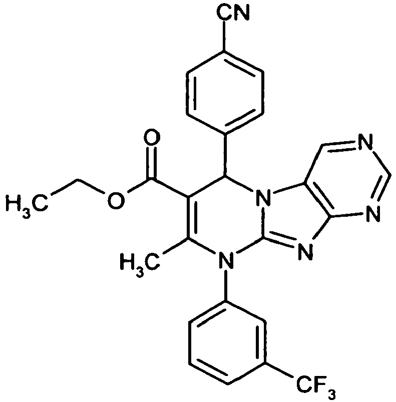

(*rac*)-Ethyl 6-(4-cyanophenyl)-8-methyl-6,9-dihydropyrimido[2,1-f]purin-7-carboxylat (30 mg, 0.08 mmol), 3-(Trifluormethyl)phenylboronsäure (31 mg, 0.17 mmol, 2 eq.), wasserfreies Kupfer-(II)acetat (30 mg, 0.17 mmol, 2 eq.) und Molekularsieb (100 mg, 4 Å) wurden vorgelegt. Unter Argonatmosphäre wurden abs. Dichlormethan (5 ml), Pyridin (27 µl, 0.33 mmol, 4 eq.) und Triethylamin (23 µl, 0.17 mmol, 2 eq.) zugegeben. Nach 12 h Rühren wurde über Kieselgur filtriert und das Filtrat im Vakuum eingeengt. Der Rückstand wurde mittels präparativer HPLC gereinigt (Gromsil C18-Säule, 30 x 250 mm; Eluent: Acetonitril-Wasser-0.1% TFA). Das Produkt wurde nach Lyophilisation als Feststoff erhalten (5.6 mg, 13% d. Th.).

LC-MS (Methode 2): R, = 2.41 min; MS (ESIpos): *m*/*z* (%) = 505.2 (100) [M+H]⁺; MS (ESIneg): m/z (%) = 503.2 (100) [M-H]⁻.

¹H-NMR (400 MHz, DMSO-d₆): δ = 1.1 (t, 3H), 2.2 (s, 3H), 4.05 (m, 2H), 6.9 (s, 1H), 7.85-8.3 (br. m, 8H), 8.7 (br. m, 2H).

### Beispiel 78

### (rac)-9-(4-Cyanophenyl)-7-methyl-6-[3-(trifluormethyl)phenyl]-6,9-dihydropyrimido[1,2-e]purin-8-carbonsäureethylester

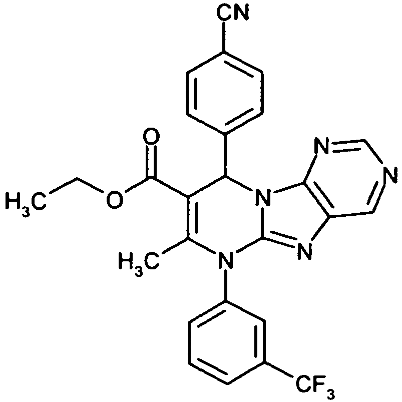

(*rac*)-Ethyl 9-(4-cyanophenyl)-7-methyl-6,9-dihydropyrimido[1,2-e]purin-8-carboxylat (20 mg, 55 µmol), 3-(Trifluormethyl)phenylboronsäure (21 mg, 0.11 mmol, 2 eq.), wasserfreies Kupfer(II)-acetat (20 mg, 0.11 mmol, 2 eq.) und Molekularsieb (50 mg, 4 Å) wurden vorgelegt. Unter Argonatmosphäre wurden abs. Dichlormethan (2 ml), Pyridin (18 µl, 0.22 mmol, 4 eq.) und Triethylamin (15 µl, 0.11 mmol, 2 eq.) zugegeben. Nach 12 h Rühren wurde über Kieselgur filtriert und das Filtrat im Vakuum eingeengt. Der Rückstand wurde mittels präparativer HPLC gereinigt (Gromsil C18-Säule, 30 x 250 mm; Eluent: Acetonitril-Wasser-0.1% TFA). Das Produkt wurde nach Lyophilisation als Feststoff erhalten (5.6 mg, 19% d. Th.).

LC-MS (Methode 2): Rₜ = 2.64 min; MS (ESIpos): m/z (%) = 505.1 (100) [M+H]⁺; MS (ESIneg): *m*/*z* (%) = 503.1 (100) [M-H]⁻.

¹H-NMR (400 MHz, DMSO-d₆): δ = 1.15 (t, 3H), 2.25 (s, 3H), 4.05 (m, 2H), 6.7 (s, 1H), 7.85-8.3 (br. m, 8H), 8.7-8.75 (br. m, 2H).

### Beispiel 79

### (rac)-Ethyl 7-(4-cyanophenyl)-5-methyl-4-[3-(trifluormethyl)phenyl]-2-{[3-(trifluormethyl)-phenyl]amino}-4,7-dihydro[1,2,4]triazolo[1,5-a]pyrimidin-6-carboxylat

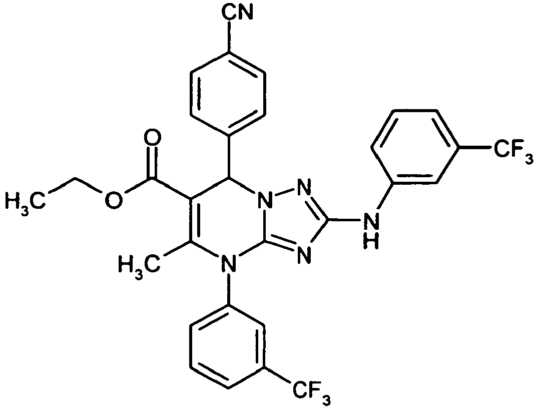

(*rac*)-Ethyl 2-amino-7-(4-cyanophenyl)-5-methyl-4,7-dihydro[1,2,4]triazolo[1,5-a]pyrimidin-6-carboxylat (20 mg, 61 µmol), 3-(Trifluormethyl)phenylboronsäure (23 mg, 0.123 mmol, 2 eq.), wasserfreies Kupfer(II)acetat (22 mg, 0.123 mmol, 2 eq.) und Molekularsieb (80 mg, 4 Å) wurden vorgelegt. Unter Argonatmosphäre wurden abs. Dichlormethan (2 ml), Pyridin (10 µl, 0.123 mmol, 2 eq.) und Triethylamin (17 µl, 0.123 mmol, 2 eq.) zugegeben. Nach 12 h Rühren wurde über Kieselgur filtriert und das Filtrat im Vakuum eingeengt. Der Rückstand wurde mittels präparativer HPLC gereinigt (Gromsil C18-Säule, 30 x 250 mm; Eluent: Acetonitril-Wasser-0.1% TFA). Das Produkt wurde nach Lyophilisation als Feststoff erhalten (8.5 mg, 8% d. Th.).

LC-MS (Methode 1): Rₜ = 3.03 min; MS (ESIpos): *m*/*z* (%) = 613.3 (100) [M+H]⁺; MS (ESIneg): *m*/*z* (%) = 611.3 (100) [M-H]⁻.

¹H-NMR (400 MHz, DMSO-d₆): δ = 1.0 (t, 3H), 2.2 (s, 3H), 4.0 (q, 2H), 6.45 (s, 1H), 7.05 (m, 1H), 7.35 (m, 1H), 7.45 (m, 1H), 7.75-8.0 (br. m, 8H), 8.2 (br. m, 1H), 9.65 (s, 1H).

### Beispiel 80

### (rac)-Diethyl 7-(4-cyanophenyl)-5-methyl-4-[3-(trifluormethyl)phenyl]-4,7-dihydro[1,2,4]triazolo[1,5-a]pyrimidin-2,6-dicarboxylat

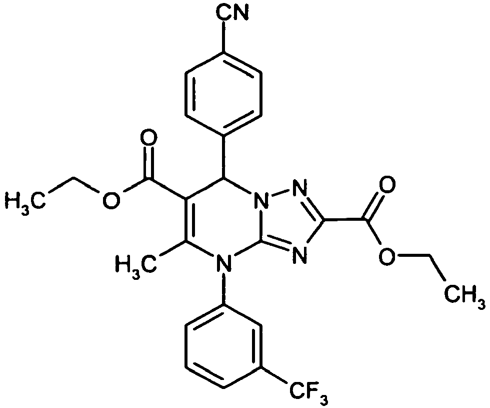

(*rac*)-Diethyl 7-(4-cyanophenyl)-5-methyl-4,7-dihydro[1,2,4]triazolo[1,5-a]pyrimidin-2,6-dicarboxylat (200 mg, 0.52 mmol), 3-(Trifluormethyl)phenylboronsäure (199 mg, 1.05 mmol, 2 eq.), wasserfreies Kupfer(II)acetat (190 mg, 1.05 mmol, 2 eq.) und Molekularsieb (500 mg, 4 Å) wurden vorgelegt. Unter Argonschutzgasatmosphäre wurden abs. Dichlormethan (5 ml), Pyridin (170 µl, 2.10 mmol, 4 eq.) und Triethylamin (146 µl, 1.05 mmol, 2 eq.) zugegeben. Nach 24 h Rühren wurde über Kieselgur filtriert, der Filterrückstand mit Dichlormethan nachgewaschen und das Filtrat im Vakuum eingeengt. Der Rückstand wurde mittels präparativer HPLC gereinigt (Gromsil C18-Säule, 30 x 250 mm; Eluent: Acetonitril-Wasser-0.1% TFA). Das Produkt wurde nach Lyophilisation als Feststoff erhalten (32.7 mg, 12% d. Th.).

LC-MS (Methode 4): Rₜ = 3.83 min; MS (ESIpos): *m*/*z* (%) = 526.2 (100) [M+H]⁺; MS (ESIneg): *m*/*z* (%) = 524.3 (100) [M-H]⁻.

¹H-NMR (400 MHz, DMSO-d₆): δ = 1.0 (t, 3H), 1.2 (t, 3H), 2.15 (s, 3H), 4.0 (q, 2H), 4.2 (m, 2H), 6.6 (s, 1H), 7.75-7.95 (m, 7H), 8.15 (br. s, 1H).

### Beispiel 81

### (rac)-Ethyl 7-(4-cyanophenyl)-2,5-dimethyl-4-[3-(trifluormethyl)phenyl]-4,7-dihydro[1,2,4]triazolo[1,5-a]pyrimidin-6-carboxylat

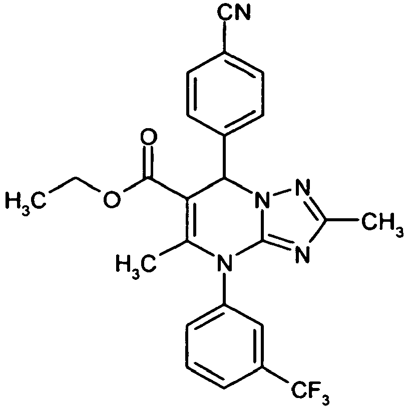

(*rac*)-Ethyl 7-(4-cyanophenyl)-2,5-dimethyl-4,7-dihydro[1,2,4]triazolo[[1,5-a]pyrimidin-6-carboxylat (50 mg, 0.15 mmol), 3-(Trifluormethyl)phenylboronsäure (88 mg, 0.46 mmol, 3 eq.), wasserfreies Kupfer(II)acetat (140 mg, 0.77 mmol, 5 eq.) und Molekularsieb (80 mg, 4 Å) wurden vorgelegt. Unter Argonschutzgasatmosphäre wurden abs. Dichlormethan (4 ml), Pyridin (125 µl, 1.55 mmol, 10 eq.) und Triethylamin (108 µl, 0.77 mmol, 5 eq.) zugegeben. Nach 24 h Rühren wurde über Kieselgur filtriert und das Filtrat im Vakuum eingeengt. Der Rückstand wurde mittels präparativer HPLC gereinigt (Gromsil C18-Säule, 30 x 250 mm; Eluent: Acetonitril-Wasser-0.05% TFA). Das Produkt wurde nach Lyophilisation als Feststoff erhalten (4.5 mg, 6% d. Th.).

LC-MS (Methode 1): Rₜ = 2.47 min; MS (ESIpos): *m*/z (%) = 468.3 (100) [M+H]⁺; MS (ESIneg): *m*/z (%) = 466.2 (100) [M-H]⁻.

¹H-NMR (400 MHz, DMSO-d₆): δ = 1.0 (t, 3H), 2.0 (s, 3H), 2.2 (s, 3H), 4.0 (q, 2H), 6.4 (s, 1H), 7.65 (m, 2H), 7.8-7.9 (m, 5H), 8.1 (br. s, 1H).

### Beispiel 82

### (rac)-Ethyl 7-(4-cyanophenyl)-2-methoxy-5-methyl-4-[3-(trifluormethyl)phenyl]-4,7-dihydro-[1,2,4]triazolo[1,5-a]pyrimidin-6-carboxylat

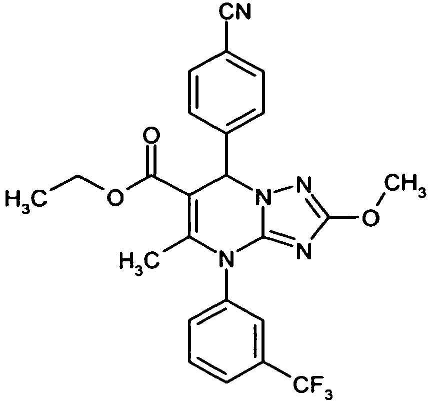

(*rac*)-Ethyl 7-(4-cyanophenyl-2-methoxy-5-methyl-4,7-dihydro[1,2,4]triazolo[1,5-a]pyrimidin-6-carboxylat (185 mg, 0.55 mmol), 3-(Trifluormethyl)phenylboronsäure (207 mg, 1.09 mmol, 2 eq.), wasserfreies Kupfer(n)acetat (198 mg, 1.09 mmol, 2 eq.) und Molekularsieb (80 mg, 4 Å) wurden vorgelegt. Unter Argonschutzgasatmosphäre wurden abs. Dichlormethan (12 ml), Pyridin (176 µl, 2.18 mmol, 4 eq.) und Triethylamin (152 µl, 1.09 mmol, 2 eq.) zugegeben. Nach 24 h Rühren wurde über Kieselgur filtriert und das Filtrat im Vakuum eingeengt. Der Rückstand wurde mittels präparativer HPLC gereinigt (Gromsil C18-Säule, 30 x 250 mm; Eluent: Acetonitril-Wasser-0.05% TFA). Das Produkt wurde nach Lyophilisation als Feststoff erhalten (103 mg, 39% d. Th.).

LC-MS (Methode 1): Rₜ = 2.57 min; MS (ESIpos): m/z (%) = 484.3 (100) [M+H]⁺; MS (ESIneg): *m*/*z* (%) = 482.3 (100) [M-H]⁻.

¹H-NMR (400 MHz, DMSO-d₆): δ = 1.05 (t, 3H), 2.15 (s, 3H), 3.65 (s, 3H), 4.0 (q, 2H), 6.35 (s, 1H), 7.65 (m, 2H), 7.8-7.9 (m, 5H), 8.1 (br. s, 1H).

### Beispiel 83

### (rac)-Ethyl 7-(4-cyanophenyl)-5-methyl-4-[3-(trifluormethyl)phenyl]-4,7-dihydro[1,2,4]triazolo-[1,5-a]pyrimidin-6-carboxylat

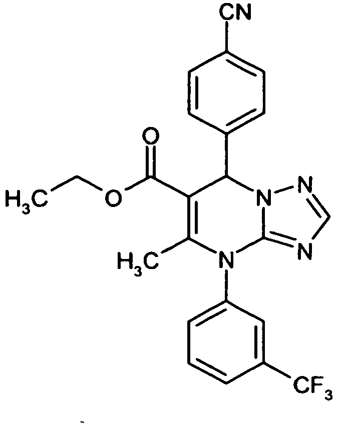

(*rac*)-Ethyl 7-(4-cyanophenyl)-5-methyl-4,7-dihydro[1,2,4]triazolo[1,5-a]pyrimidin-6-carboxylat (20 mg, 64 µmol), 3-(Trifluormethyl)phenylboronsäure (25 mg, 0.13 mmol, 2 eq.), wasserfreies Kupfer(II)acetat (23 mg, 0.13 mmol, 2 eq.) und Molekularsieb (100 mg, 4 Å) wurden vorgelegt. Unter Argonschutzgasatmosphäre wurden abs. Dichlormethan (2 ml), Pyridin (10 µl, 0.13 mmol, 2 eq.) und Triethylamin (18 µl, 0.13 mmol, 2 eq.) zugegeben. Nach 24 h Rühren wurde über Kieselgur filtriert und das Filtrat im Vakuum eingeengt. Der Rückstand wurde mittels präparativer HPLC gereinigt (Gromsil C18-Säule, 30 x 250 mm; Eluent: Acetonitril-Wasser-0.05% TFA). Das Produkt wurde nach Lyophilisation als Feststoff erhalten (10.5 mg, 36% d. Th.).

LC-MS (Methode 3): Rₜ = 3.8 min; MS (ESIpos): *m*/*z* (%) = 454.2 (100) [M+H]⁺; MS (ESIneg): *m*/*z* (%).= 452.2 (100) [M-H]⁻.

¹H-NMR (400 MHz, DMSO-d₆): δ = 1.05 (t, 3H), 2.2 (s, 3H), 4.0 (q, 2H), 6.55 (s, 1H), 7.6 (s, 1H), 7.7 (m, 2H), 7.8-7.9 (m, 5H), 8.1 (br. s, 1H).

### Beispiel 84

### (7R)-6-Cyano-7-(4-cyanophenyl)-N,5-dimethyl-4-[3-(trifluormethyl)phenyl]-4,7-dihydro[1,2,4]-triazolo[1,5-a]pyrimidin-2-sulfonamid

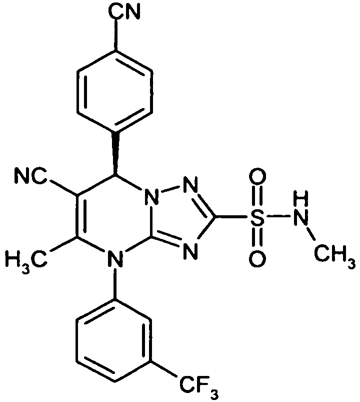

Unter Argonatmosphäre wurde abs. THF (3 ml) 30 min lang mit Molekularsieb (4 Å, 30 mg) gerührt. (7*R*)-6-Cyano-7-(4-cyanophenyl)-5-methyl-4-[3-(trifluormethyl)phenyl]-4,7-dihydro[1,2,4]-triazolo[1,5-a]pyrimidin-2-sulfonylchlorid (63%-ig, 50 mg, 62 µmol), Methylamin (2 M Lösung in THF; 94 µl, 0.19 mmol, 3 eq.) und Triethylamin (9 µl, 62 µmol, 1 eq.) wurden hinzugegeben und die Mischung 12 h lang bei RT gerührt. Die Reaktionslösung wurde dann im Vakuum eingeengt und der Rückstand mittels präparativer HPLC gereinigt (Reprosil C18-Säule, 30 x 250 mm; Eluent: Acetonitril-Wasser-0.1% TFA). Das Produkt wurde nach Lyophilisation als Feststoff erhalten (23 mg, 73% d. Th.).

LC-MS (Methode 9): Rₜ = 1.07 min; MS (ESIpos): *m*/*z* (%) = 500.3 (100) [M+H]⁺; MS (ESIneg): *m*/*z* (%) = 498.3 (100) [M-H]⁻.

¹H-NMR (400 MHz, DMSO-d₆): δ = 2.0 (s, 3H), 2.45 (d, 3H), 6.65 (s, 1H), 7.85 (m, 3H), 7.9-8.05 (m, 5H), 8.2 (br. s, 1 H).

### Beispiel 85

### (7R)-6-Cyano-7-(4-cyanophenyl)-5-methyl-4-[3-(trifluormethyl)phenyl]-4,7-dihydro[1,2,4]triazolo-[1,5-a]pyrimidin-2-sulfonamid

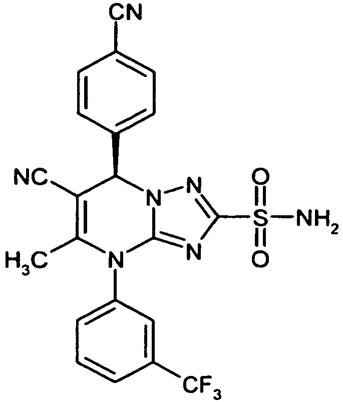

Unter Argonatmosphäre wurde abs. Dioxan (5ml) 30 min lang mit Molekularsieb (4 Å, 30 mg) gerührt. (7*R*)-6-Cyano-7-(4-cyanophenyl)-5-methyl-4-[3-(trifluormethyl)phenyl]-4,7-dihydro[1,2,4]-triazolo[1,5-a]pyrimidin-2-sulfonylchlorid (63%-ig, 60 mg, 75 µmol) wurde hinzugegeben und die Lösung auf 7°C abgekühlt. Dann wurde 15 min lang trockenes Ammoniakgas eingeleitet und die Mischung anschließend auf 0°C gekühlt. Triethylamin (11 µl, 75 µmol, 1 eq.) wurde zugesetzt und der Ansatz dann 12 h unter allmählicher Erwärmung auf RT gerührt. Das Lösungsmittel wurde danach im Vakuum abdestilliert und der Rückstand mittels präparativer HPLC gereinigt (Reprosil C18-Säule, 30 x 250 mm; Eluent: Acetonitril-Wasser-0.1% TFA). Das Produkt wurde nach Lyophilisation als Feststoff erhalten (33 mg, 90% d. Th.).

LC-MS (Methode 9): Rₜ = 1.02 min; MS (ESIpos): *m*/*z* (%) = 486.3 (100) [M+H]⁺; MS (ESIneg): *m*/*z* (%) = 484.3 (100) [M-H]⁻.

¹H-NMR (400 MHz, DMSO-d₆): δ = 2.0 (s, 3H), 6.6 (s, 1H), 7.75 (s, 2H), 7.85 (m, 3H), 7.9-8.05 (m, 4H), 8.25 (br. s, 1H).

### Beispiel 86

### (7R)-6-Cyano-7-(4-cyanophenyl)-N-ethyl-5-methyl-4-[3-(trifluormethyl)phenyl]-4,7-dihydro-[1,2,4]triazolo[1,5-a]pyrimidin-2-sulfonamid

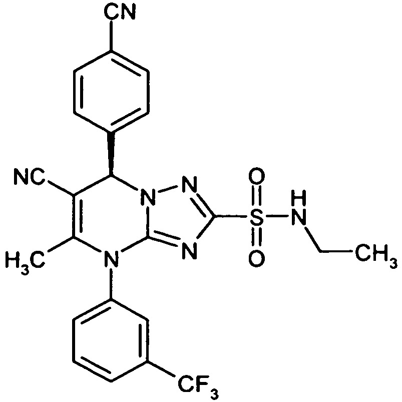

Unter Argonatmosphäre wurde abs. THF (3 ml) für 30 min mit Molekularsieb (4 Å, 30 mg) gerührt. (7*R*)-6-Cyano-7-(4-cyanopheny-5-methyl-4-[3-trifluormethyl)phenyl]-4,7-dihydro[1,2,4]-triazolo[1,5-a]pyrimidin-2-sulfonylchlorid (63%-ig, 50 mg, 62 µmol), Ethylamin (2 M Lösung in THF; 94 µl, 0.19 mmol, 3 eq.) und Triethylamin (9 µl, 62 µmol, 1 eq.) wurden hinzugegeben und die Mischung 12 h lang bei RT gerührt. Die Reaktionslösung wurde dann im Vakuum eingeengt und der Rückstand mittels präparativer HPLC gereinigt (Reprosil C18-Säule, 30 × 250 mm; Eluent: Acetonitril-Wasser-0.1% TFA). Das Produkt wurde nach Lyophilisation als Feststoff erhalten (29.9 mg, 93% d. Th.).

LC-MS (Methode 9): Rₜ = 1.24 min; MS (ESIpos): *m*/*z* (%) = 514.0 (100) [M+H]⁺; MS (ESIneg): *m*/*z* (%) = 512.9 (100) [M-H]⁻.

¹H-NMR (400 MHz, DMSO-d₆): δ = 0.9 (t, 3H), 2.0 (s, 3H), 2.8 (m, 2H), 6.65 (s, 1H), 7.85 (m, 3H), 7.9-8.0 (m, 4H), 8.05 (t, 1H), 8.2 (br. s, 1H).

### Beispiel 87

### (7R)-6-Cyano-7-(4-cyanophenyl)-N-cyclopropyl-5-methyl-4-[3-(trifluormethyl)phenyl]-4,7-dihydro[1,2,4]triazolo[1,5-a]pyrimidin-2-sulfonamid

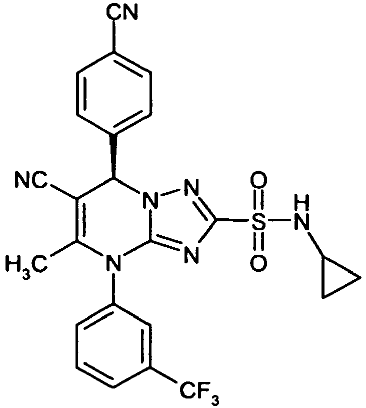

Unter Argonatmosphäre wurde abs. THF (3 ml) für 30 min mit Molekularsieb (4 Å, 30 mg) gerührt. (7*R*)-6-Cyano-7-(4-cyanophenyl)-5-methyl-4-[3-(trifluormethyl)phenyl]-4,7-dihydro[1,2,4]-triazolo[1,5-a]pyrimidin-2-sulfonylchlorid (63%-ig, 50 mg, 62 µmol), Cyclopropylamin (13 µl, 0.19 mmol, 3 eq.) und Triethylamin (9 µl, 63 µmol, 1 eq.) wurden hinzugegeben und die Mischung 12 h lang bei RT gerührt. Die Reaktionslösung wurde dann im Vakuum eingeengt und der Rückstand mittels präparativer HPLC gereinigt (Reprosil C18-Säule, 30 x 250 mm; Eluent: Acetonitril-Wasser-0.1% TFA). Das Produkt wurde nach Lyophilisation als Feststoff erhalten (27 mg, 82% d. Th.).

LC-MS (Methode 9): Rₜ = 1.11 min; MS (ESIpos): *m*/*z* (%) = 526.3 (100) [M+H]⁺; MS (ESIneg): *mlz* (%) = 524.3 (100) [M-H]⁻.

¹H-NMR (400 MHz, DMSO-d₆): δ = 0.3-0.45 (m, 4H), 2.0 (s, 3H), 2.25 (m, 1H), 6.65 (s, 1H), 7.85 (m, 3H), 7.9-8.0 (m, 4H), 8.25 (br. s, 1H), 8.4 (d, 1H).

### Beispiel 88

### (7R)-7-(4-Cyanophenyl)-5-methyl-4-[3-(trifluormethyl)phenyl]-4,7-dihydro[1,2,4]triazolo[1,5-a]-pyrimidin-6-carbonitril

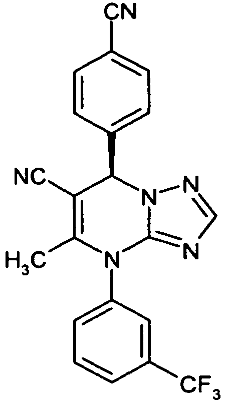

Die Titelverbindung wurde als Nebenprodukt bei der Herstellung von (7*R*)-6-Cyano-7-(4-cyanophenyl)5-methyl-4-[3-(trifluormethyl)phenyl]-4,7-dihydro[1,2,4]triazolo[1,5-a]pyrimidin-2-sulfonylchlorid (Beispiel 29A) erhalten. Das Produkt wurde mittels präparativer HPLC (Kromasil 5µ-Säule, 50 x 20 mm; Eluent: Acetonitril-Wasser-0.1% TFA) isoliert und nach Lyophilisation der entsprechenden Fraktionen als Feststoff erhalten (3 mg, 4% d. Th.).

LC-MS (Methode 9): Rₜ = 1.11 min; MS (ESIpos): *m*/*z* (%) = 407.3 (100) [M+H]⁺; MS (ESIneg): *m*/*z* (%) = 405.3 (100) [M-H]⁻.

¹H-NMR (400 MHz, DMSO-d₆): δ = 2.0 (s, 3H), 6.55 (s, 1H), 7.70 (s, 1H), 7.75 (m, 2H), 7.85 (m, 1H), 7.95 (m, 4H), 8.15 (br. s, 1H).

### Beispiel 89

### (rac)-Ethyl 5-(4-cyanophenyl)-7-methyl-8-[3-(trifluormethyl)phenyl]-5,8-dihydroimidazo[1,2-a]-pyrimidin-6-carboxylat

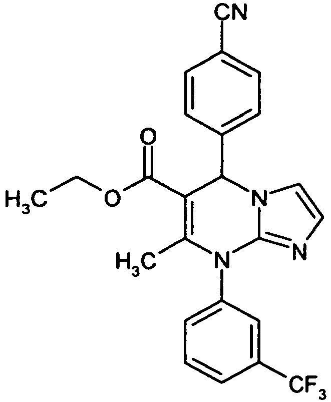

(*rac*)-Ethyl 5-(4-cyanophenyl)-7-methyl-5,8-dihydroimidazo[1,2-a]pyrimidin-6-carboxylat (152 mg, 0.49 mmol), 3-(Trifluormethyl)phenylboronsäure (187 mg, 0.1 mmol, 2 eq.), wasserfreies Kupfer(II)acetat (181 mg, 0.1 mmol, 2 eq.) und Molekularsieb (200 mg, 4 Å) wurden vorgelegt. Unter Argonschutzgasatmosphäre wurden abs. Dichlormethan (5 ml), Pyridin (80 µl, 0.1 mmol, 2 eq.) und Triethylamin (137 µl, 0.1 mmol, 2 eq.) hinzugegeben. Nach 12 h Rühren wurde nochmals wasserfreies Kupfer(II)acetat (181 mg, 0.1 mmol, 2 eq.) sowie Triethylamin (137 µl, 0.1 mmol, 2 eq.) hinzugefügt und die Mischung für weitere 48 h gerührt. Danach wurde über Kieselgur filtriert und das Filtrat im Vakuum eingeengt. Der Rückstand wurde mittels präparativer HPLC gereinigt (Gromsil C18-Säule, 30 x 250 mm; Eluent: Acetonitril-Wasser-0.05% TFA). Das Produkt wurde nach Lyophilisation als Feststoff erhalten (26 mg, 12% d. Th.).

LC-MS (Methode 11): Rₜ = 2.48 min; MS (ESIpos): *m*/*z* (%) = 453.1 (100) [M+H]⁺; MS (ESIneg): *m*/*z* (%) = 451.2 (100) [M-H]⁻.

¹H-NMR (400 MHz, DMSO-d₆): δ = 1.05 (t, 3H), 2.2 (s, 3H), 4.0 (q, 2H), 6.5 (s, 1H), 6.75 (br. s, 1H), 6.95 (s, 1H), 7.7 (m, 2H), 7.8-7.95 (m, 5H), 8.1 (s, 1H).

### Beispiel 90

### Ethyl (5R)-5-(4-cyanophenyl)-7-methyl-8-[3-(trifluonnethyl)phenyll-5,8-dihydroimidazo[1,2-a]-pyrimidin-6-carboxylat

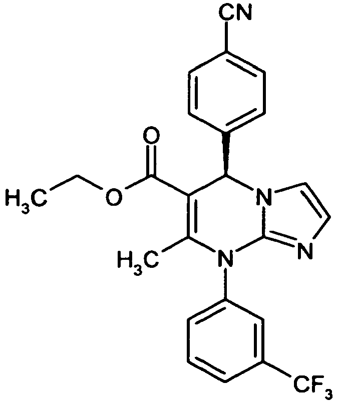

(*rac*)-Ethyl 5-(4-cyanophenyl)-7-methyl-8-[3(trifluormethyl)phenyl]-5,8-dihydroimidazo[1,2-a]-pyrimidin-6-carboxylat (210 mg) wurde durch präparative HPLC-Chromatographie an chiraler Phase in die Enantiomere aufgetrennt [stationäre Phase: Daicel Chiralpak AS-H, 5 µ, 250 x 20 mm; Probenvorbereitung: Lösung in 7 ml Isopropanol; Fluss: 15 ml/min; Detektion: 260 nm; Injektionsvolumen: 1 ml; Temperatur: 40°C; Eluent: Isopropanol/Isohexan 1:1]. Die Titelverbindung wurde als Feststoff erhalten (85 mg, 81% d. Th.). Der Enantiomerenüberschuss (ee-Wert) wurde chromatographisch bestimmt [Säule: Daicel Chiralpak AS-H, 5µ, 250 x 4.6 mm; Eluent: Isopropanol/Isohexan 7:3; Fluss: 1 ml/min; Temperatur: 30°C; Detektion: 215 nm; Rₜ = 5.46 min; ee >99.5%].

LC-MS (Methode 1): Rₜ = 2.33 min; MS (ESIpos): *m*/z (%) = 453.1 (100) [M+H]⁺; MS (ESIneg): *m*/*z* (%) = 451.2 (100) [M-H]⁻.

¹H-NMR (500 MHz, CDCl₃): δ = 1.15 (t, 3H), 2.25 (s, 3H), 4.10 (q, 2H), 6.40 (s, 1H), 6.50 (s, 1H), 6.75 (s, 1H), 7.40 (m, 2H), 7.60-7.80 (m, 6H).

### Beispiel 91

### (rac)-3-Amino-5-(4-cyanophenyl)-7-methyl-8-[3-(trifluormethyl)phenyl]-5,8-dihydro[1,2,4]triazolo[4,3-a]pyrimidin-6-carbonitril-Hydrochlorid

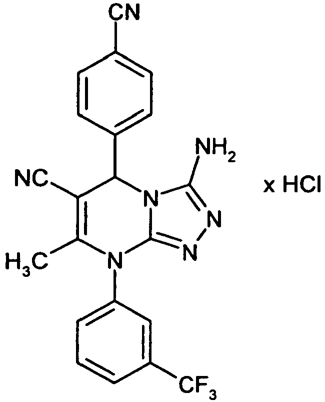

(*rac*)-4-(4-Cyanophenyl)-2-hydrazinyl-6-methyl-1-[3-(trifluormethyl)phenyl]-1,4-dihydropyrimidin-5-carbonitril-Trifluoracetat (10mg, 23.1 µmol) wurde unter Argonatmosphäre in abs. Methanol (2.5 ml) mit Molekularsieb (4 Å,10 mg) vorgelegt. Bromcyan (12 µl, 115 µmol, 5 eq.) wurde zugegeben und die Mischung 12 h lang bei RT gerührt. Die Reaktionslösung wurde danach im Vakuum eingeengt und der Rückstand mittels präparativer HPLC gereinigt (Kromasil C18-Säule, 20 x 50 mm; Eluent: Acetonitril-Wasser-0.1% TFA). Nach Lyophilisation wurde das Produkt in Form der freien Base als Feststoff erhalten (4 mg, 48% d. Th.). Die Substanz wurde in 0.5 ml einer 4 N Lösung von Chlorwasserstoff in Dioxan suspendiert und das Gemisch dann wieder im Vakuum zur Trockene eingeengt. Diese Prozedur wurde noch einmal wiederholt. Der Rückstand wurde schließlich mit Wasser versetzt und erneut lyophilisiert.

LC-MS (Methode 9): Rₜ = 0.90 min; MS (ESIpos): *m*/*z* (%) = 422.3 (100) [M+H]⁺; MS (ESIneg): *m*/*z* (%) = 420.3 (100) [M-H]⁻.

¹H-NMR (400 MHz, DMSO-d₆): δ = 1.95 (s, 3H), 3.5 (m, 2H), 6.15 (s, 1H), 7.65 (m, 2H), 7.85-8.00 (m, 5H), 8.10 (br. s, 1H).

### Beispiel 92

### N-{6-Cyano-5-(4-cyanophenyl)-7-methyl-8-[3-(trifluormethyl)phenyl]-5,8-dihydro[1,2,4]triazolo-[4,3-a]pyrimidin-3-yl}cyclopropancarboxamid

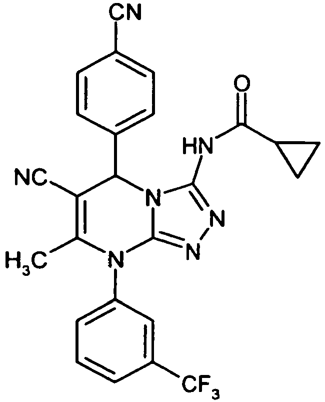

3-Amino-5-(4-cyanophenyl)-7-methyl-8-[3-(trifluormethyl)phenyl]-5,8-dihydro[1,2,4]triazolo-[4,3-a]pyrimidin-6-carbonitril-Hydrochlorid (5 mg, 11 µmol) wurde unter Argonschutzgasatmosphäre in abs. Pyridin (0.5 ml) gelöst. Bei Raumtemperatur wurde Cyclopropancarbonsäurechlorid (2.3 mg, 21.8 µmol, 2 eq.) in abs. THF (50 µl) zugegeben. Nach 12 h zeigte die Reaktionskontrolle mittels HPLC-Analytik weitgehenden Umsatz. Das Reaktionsgemisch wurde im Vakuum eingeengt und mittels präparativer HPLC gereinigt (Kromasil C18-Säule 5 µm, 20 × 50mm; Eluent: Acetonitril-Wasser-0.1% TFA). Nach Lyophilisation wurde das Produkt als Feststoff erhalten (2.2 mg, 41% d. Th.).

LC-MS (Methode 9): Rₜ = 0.98 min; MS (ESIpos): *m*/*z* (%) = 490.2 (100) [M+H]⁺; MS (ESIneg): *mlz* (%) = 488.2 (100) [M-H]⁻.

¹H-NMR (400 MHz, DMSO-d₆): δ = 0.65-1.00 (br. m, 4H), 1.45 (m, 1H), 1.95 (s, 3H), 6.10 (s, 1H), 7.60 (m, 2H), 7.85 (m, 1H), 7.95 (m, 4H), 8.20 (br. s, 1H), 10.50 (br. s, 1H).

### B. Bewertung der pharmakologischen Wirksamkeit

Die pharmakologische Wirkung der erfindungsgemäßen Verbindungen kann in den nachstehend beschriebenen Assays gezeigt werden:

### Abkürzungen:

- AMC: 7-Amido-4-methylcumarin
- BNP: brain natriuretic peptide
- BSA: bovine serum albumin
- HEPES: *N*-(2-Hydroxyethyl)piperazin-*N*'-2-ethansulfonsäure
- HNE: humane neutrophile Elastase
- IC: Inhibitionskonzentration
- MeOSuc: Methoxysuccinyl
- NADP: Nikotinamid-Adenin-Dinukleotid-Phosphat
- v/v: Volumen zu Volumen-Verhältnis (einer Lösung)
- w/v: Gewicht zu Volumen-Verhältnis (einer Lösung)

### B-1. In vitro HNE-Inhibitionstest

Die Wirkstärke der erfindungsgemäßen Verbindungen wird in einem in *vitro*-Hemmtest ermittelt. Die HNE-vermittelte amidolytische Spaltung eines geeigneten Peptidsubstrates führt hierbei zu einer Fluoreszenzlichtzunahme. Die Signalintensität des Fluoreszenzlichtes ist direkt proportional zur Enzymaktivität. Die Wirkkonzentration einer Testverbindung, bei der die Hälfte des Enzyms inhibiert ist (50% Signalintensität des Fluoreszenzlichtes), wird als IC₅₀-Wert angegeben.

### Ausführung:

In einer 384 Loch-Mikrotiterplatte werden in einem Testvolumen von insgesamt 50 µl Testpuffer (0.1 M HEPES pH 7.4, 0.5 M NaCl, 0.1% w/v BSA, 1% v/v DMSO), Enzym (80 pM HNE; Fa. Serva, Heidelberg) und Substrat (20 µM MeOSuc-Ala-Ala-Pro-Val-AMC; Fa. Bachem, Weil am Rhein) bei An- und Abwesenheit der Testsubstanz zwei Stunden bei 37°C inkubiert. Die Fluoreszenzlichtintensität der Testansätze wird gemessen (Ex. 380 nm, Em. 460 nm). Die IC₅₀-Werte werden durch eine Auftragung der Fluoreszenzlichtintensität gegenüber der Wirkstofflconzentration ermittelt.

Für die erfindungsgemäßen Verbindungen repräsentative IC₅₀-Werte (bei einer HNE-Konzentration von 80 pM) sind in der folgenden Tabelle A wiedergegeben:

**Tabelle A: Hemmung der humanen neutrophilen Elastase (HNE)**

| **Ausführungsbeispiel Nr.** | **IC₅₀ [nM]** |
|---|---|
| 5 | < 0.3 |
| 9 | < 0.3 |
| 23 | < 0.3 |
| 35 | < 0.3 |
| 50 | < 0.3 |
| 53 | < 0.3 |
| 56 | < 0.3 |
| 67 | 85.0 |
| 78 | 3.0 |
| 90 | 6.5 |

### B-2. Tiermodell der pulmonalen arteriellen Hypertonie

Die Monocrotalin-induzierte pulmonale Hypertonie der Ratte ist ein weit verbreitetes Tiermodell für die pulmonale arterielle Hypertonie. Das Pyrrolizidin-Alkaloid Monocrotalin wird nach subkutaner Injektion in der Leber zum toxischen Monocrotalinpyrrol metabolisiert und führt innerhalb weniger Tage zu einer Endothelschädigung im Lungenkreislauf, gefolgt von einem Remodeling der kleinen pulmonalen Arterien (Mediahypertrophie, *de novo*-Muskularisierung). Eine einmalige subkutane Injektion ist ausreichend, um bei Ratten innerhalb von 4 Wochen eine ausgeprägte pulmonale Hypertonie zu induzieren [Cowan et al., Nature Med. 6, 698-702 (2000)].

Für das Modell werden männliche Sprague-Dawley-Ratten verwendet. An Tag 0 erhalten die Tiere eine subkutane Injektion von 60 mg/kg Monocrotalin. Die Behandlung der Tiere beginnt erst frühestens 14 Tage nach der Monocrotalin-Injektion und erstreckt sich über einen Zeitraum von mindestens 14 Tagen. Am Studienende erfolgen hämodynamische Untersuchungen der Tiere sowie eine Ermittlung der arteriellen und zentralvenösen Sauerstoffsättigung. Für die hämodynamische Messung werden die Ratten initial mit Pentobarbital (60 mg/kg) anästhesiert. Anschließend werden die Tiere tracheotomiert und künstlich beatmet (Frequenz: 60 Atemzüge/min; Verhältnis Inspiration zu Exspiration: 50:50; positiver endexspiratorischer Druck: 1 cm H₂O; Atemzugvolumen: 10 ml/kg Körpergewicht; FIO₂: 0.5). Die Narkose wird durch lsofluran-Inhalationsnarkose aufrechterhalten. Der systemische Blutdruck wird in der linken *A. carotis* mittels eines Millar-Microtip-Katheters ermittelt. Ein Polyethylenkatheter wird über die rechte *V. jugularis* in den rechten Ventrikel vorgeschoben zur Bestimmung des rechten Ventrikeldruckes. Das Herzminutenvolumen wird mittels Thermodilution ermittelt. Im Anschluß an die Hämodynamik wird das Herz entnommen und das Verhältnis rechter zu linker Ventrikel inklusive Septum bestimmt. Weiterhin werden Plasmaproben zur Bestimmung von Biomarkern (zum Beispiel proBNP) und Plasma-Substanzspiegeln gewonnen.

### B-3 . Tiermodell des akuten Lungenversagens

Elastase-induziertes Lungenversagen an Maus, Ratte oder Hamster ist ein weit verbreitetes Tiermodell für akutes Lungenversagen (auch: "acute lung injury", "acute respiratory distress syndrome") [Tremblay et al., Chest 121,582-588 (2002); Kuraki et al., Am. J. Resp. Crit. Care Med. 166, 596-500 (2002)]. Die Behandlung der Tiere erfolgt 1 Stunde vor orotrachealer Instillation der humanen neutrophilen Elastase (HNE). 2 Stunden nach der orotrachealen HNE-Instillation wird eine bronchoalveolare Lavage durchgeführt und der Hämoglobingehalt sowie das Differentialzellbild in der Lavage bestimmt.

### B-4. Tiermodell des Lungenemphysems

Elastase-induziertes Lungenemphysem an Maus, Ratte oder Hamster ist ein weit verbreitetes Tiermodell für Lungenemphysem [Sawada et al., Exp. Lung Res. 33, 277-288 (2007)]. Die Tiere erhalten eine orotracheale Instillation porciner Pankreas-Elastase. Die Behandlung der Tiere beginnt am Tag der Instillation der porcinen Pankreas-Elastase und erstreckt sich über einen Zeitraum von 3 Wochen. Am Studienende wird die Lungen-Compliance bestimmt und eine Alveolarmorphometrie durchgeführt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 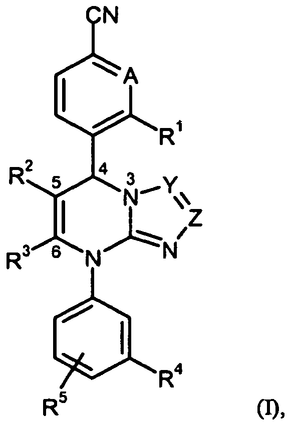 in welcher
A für C-R⁶ oder N steht, worin
R⁶ Wasserstoff, Fluor oder Chlor bedeutet,
Y für C-R⁷ oder N steht, worin
R⁷ Wasserstoff, (C₁-C₆)-Alkyl, Amino oder eine Gruppe der Formel -NH-C(=O)-R⁸ oder -NH-SO₂-R⁸ bedeutet, worin
R⁸ (C₁-C₆)-Alkyl, (C₃-C₆)-Cycloalkyl oder Phenyl darstellt,
wobei (C₁-C₆)-Alkyl mit Hydroxy, (C₁-C₄)-Alkoxy, (C₃-C₆)-Cycloalkyl oder Phenyl und bis zu dreifach mit Fluor substituiert sein kann
und wobei
die genannten Cycloalkyl-Gruppen bis zu zweifach, gleich oder verschieden, mit (C₁-C₄)-Alkyl und bis zu zweifach mit Fluor
sowie
die genannten Phenyl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy
substituiert sein können,
Z für C-R⁹ oder N steht, worin
R⁹ Wasserstoff, (C₁-C₆)-Alkyl, (C₁-C₆)-Alkoxy, (C₁-C₆)-Alkoxycarbonyl, (C₃-C₆)-Cycloalkyl, Phenyl oder 5- oder 6-gliedriges Heteroaryl bedeutet,
wobei (C₁-C₆)-Alkyl mit Hydroxy, (C₁₋C₄)-Alkoxy, (C₃₋C₆)-Cycloalkyl oder Phenyl und bis zu dreifach mit Fluor substituiert sein kann
und wobei
die genannten Cycloalkyl-Gruppen bis zu zweifach, gleich oder verschieden, mit (C₁-C₄)-Alkyl und bis zu zweifach mit Fluor
sowie
die genannten Phenyl- und Heteroaryl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy
substituiert sein können,
oder
R⁹ eine Gruppe der Formel -SO₂-NR¹⁰R¹¹ oder -NR¹²R¹³ bedeutet, worin
R¹⁰ und R¹¹ gleich oder verschieden sind und unabhängig voneinander Wasserstoff, (C₁-C₆)-Alkyl, (C₃-C₆)-Cycloalkyl, Phenyl oder 5- oder 6-gliedriges Heteroaryl darstellen,
R¹² Wasserstoff, (C₁-C₆)-Alkyl, (C₃-C₆)-Cycloalkyl, Phenyl oder (C₁-C₆)-Alkylsulfonyl darstellt,
wobei (C₁-C₆)-Alkyl mit Cyano, Hydroxy, (C₁-C₄)-Alkoxy, Hydroxycarbonyl, (C₁-C₄)-Alkoxycarbonyl, Aminocarbonyl, mono-(C₁-C₄)-alkylaminocarbonyl, Di-(C₁-C₄)-alkylaminocarbonyl, (C₃-C₆)-Cycloalkyl oder Phenyl und bis zu dreifach mit Fluor substituiert sein kann
und wobei
die genannten Cycloalkyl-Gruppen bis zu zweifach, gleich oder verschieden, mit (C₁-C₄)-Alkyl und bis zu zweifach mit Fluor
sowie
die genannten Phenyl-Gruppen bis zu zweifach, gleich oder verschieden, mit Fluor, Chlor, Cyano, (C₁)-C₄)-Alkyl, Difluormethyl, Trifluormethyl, (C₁-C₄)-Alkoxy, Difluormethoxy und Trifluormethoxy
substituiert sein können,
R¹³ Wasserstoff, (C₁-C₆)-Alkyl oder eine Gruppe der Formel -C(=O)-R¹⁴, -C(=O)-O-R¹⁵ oder -C(=O)-NR¹⁶R¹⁷ darstellt, worin
R¹⁴ Wasserstoff, (C₁-C₈)-Alkyl, (C₃-C₆)-Cycloalkyl, 4- bis 6-gliedriges Heterocyclyl, Phenyl oder 5- oder 6-gliedriges Heteroaryl bedeutet,
wobei (C₁-C₈)-Alkyl mit Hydroxy, (C₁-C₄)-Alkoxy, Benzyloxy, Phenoxy, (C₁-C₄)-Acyloxy, Amino, Mono-(C₁-C₄)-alkylamino, Di-(C₁-C₄)-alkylamino, (C₁-C₄)-Acyl-amino, (C₁-C₄)-Alkoxycarbonylamino, (C₃-C₆)-Cycloalkyl oder Phenyl und bis zu dreifach mit Fluor substituiert sein kann und bis zu zwei CH₂-Gruppen in (C₁-C₈)-Alkyl, soweit in einer chemisch stabilen Verbindung resultierend, gegen ein Sauerstoffatom ausgetauscht sein können
und wobei
die genannten Cycloalkyl- und Heterocyclyl-Gruppen bis zu zweifach, gleich oder verschieden, mit (C₁-C₄)-Alkyl und bis zu zweifach mit Fluor
sowie
die genannten Phenyl- und Heteroaryl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy, Trifluormethoxy und Hydroxycarbonyl
substituiert sein können,
R¹⁵ (C₁-C₆)-Alkyl, welches mit (C₃-C₆)-Cycloalkyl oder Phenyl substituiert sein kann, bedeutet,
R¹⁶ und R¹⁷ gleich oder verschieden sind und unabhängig voneinander Wasserstoff oder (C₁-C₆)-Alkyl, welches mit Hydroxy oder (C₁-C₄)-Alkoxy substituiert sein kann, bedeuten,
oder
R¹² und R¹³ zusammen mit dem Stickstoffatom, an das sie gebunden sind, einen 5- oder 6-gliedrigen Heterocyclus bilden, welcher ein weiteres Ring-Heteroatom aus der Reihe N, O oder S enthalten kann und der bis zu zweifach, gleich oder verschieden, mit (C₁-C₄)-Alkyl, Hydroxy, (C₁-C₄)-Alkoxy und Oxo substituiert und mit einem Phenyl-Ring anelliert sein kann,
oder worin
R⁷ und R⁹, sofern beide vorhanden, miteinander verknüpft sind und zusammen mit den Kohlenstoffatomen, an die sie gebunden sind, einen anellierten Phenyl-, Pyridyl- oder Pyrimidyl-Ring bilden, welcher jeweils bis zu zweifach, gleich oder verschieden, mit Fluor, Chlor, Cyano, (C₁-C₄)-Alkyl, Difluormethyl, Trifluormethyl, (C₁₋C₄)-Alkoxy, Difluormethoxy und Trifluormethoxy substituiert sein kann,
R¹ für Wasserstoff, Halogen, Cyano, Nitro, (C₁-C₆)-Alkyl, Difluormethyl, Trifluormethyl, (C₁-C₆)-Alkoxy, Difluormethoxy, Trifluormethoxy, Amino, Mono- oder Di-(C₁-C₆)-alkylaminooder
für eine Gruppe der Formel -NH-C(=O)-R¹⁸, -NH-C(=O)-NHR¹⁸, -NH-SO₂-R¹⁹ oder -S(O)ₙ-R²⁰ steht, worin
R¹⁸ Wasserstoff oder (C₁-C₆)-Alkyl bedeutet,
R¹⁹ (C₁-C₆)-Alkyl bedeutet,
R²⁰ (C₁-C₆)-Alkyl, das mit Hydroxy, (C₁-C₄)-Alkoxy, Amino, Mono- oder Di-(C₁-C₄)-alkylamino, Hydroxycarbonyl, Aminocarbonyl, (C₃-C₆)-Cycloalkyl oder Phenyl und bis zu dreifach mit Fluor substituiert sein kann, oder (C₂-C₆)-Alkenyl, (C₃-C₆)-Cycloalkyl oder Phenyl bedeutet,
wobei die genannten Cycloalkyl-Gruppen bis zu zweifach, gleich oder verschieden, mit (C₁-C₄)-Alkyl, Hydroxy und (C₁-C₄)-Alkoxy
und
die genannten Phenyl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy
substituiert sein können,
und
n die Zahl 0, 1 oder 2 bedeutet,
R² für Cyano oder eine Gruppe der Formel -C(=O)-R²¹, -C(=O)-O-R²¹ oder -C(=O)-NH-R²¹ steht, worin
R²¹ Wasserstoff, (C₁-C₆)-Alkyl, (C₃-C₆)-Alkenyl oder (C₃-C₆)-Cycloalkyl bedeutet,
wobei (C₁-C₆)-Alkyl und (C₃-C₆)-Cycloalkyl ihrerseits bis zu zweifach, gleich oder verschieden, mit Hydroxy, (C₁-C₄)-Alkoxy, Hydroxycarbonyl, (C₁-C₄)-Alkoxycarbonyl, Amino, Mono- und Di-(C₁-C₄)-alkylamino substituiert sein können und in (C₁-C₆)-Alkyl und (C₃-C₆)-Cycloalkyl jeweils eine CH₂-Gruppe, soweit in einer chemisch stabilen Verbindung resultierend, gegen ein O-Atom ausgetauscht sein kann,
R³ für Methyl oder Ethyl steht
oder
R² und R³ miteinander verknüpft sind und zusammen eine anellierte Gruppe der Formel 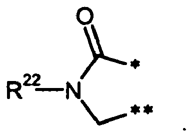
* die Verknüpfungsstelle mit der in Formel (I) bezeichneten 5-Position des Dihydropyrimidin-Rings
und
** die Verknüpfungsstelle mit der in Formel (I) bezeichneten 6-Position des Dihydropyrimidin-Rings bedeuten
und
R²² Wasserstoff, (C₁-C₆)-Alkyl oder (C₃-C₆)-Cycloalkyl bedeutet,
wobei (C₁-C₆)-Alkyl mit Hydroxy, (C₁-C₄)-Alkoxy, Aminocarbonyl, Aminocarbonylamino, (C₁-C₄)-Acylamino oder (C₃-C₆)-Cycloalkyl substituiert sein kann,
R⁴ für Nitro oder Trifluormethyl steht
R⁵ für Wasserstoff, Fluor oder Chlor steht,
sowie ihre Salze, Solvate und Solvate der Salze.

2. Verbindung der Formel (I) nach Anspruch 1, in welcher
A für CH steht,
Y für C-R⁷ oder N steht,
Z für C-R⁹ oder N steht,
wobei mindestens eines der beiden Ringglieder Y und Z für N steht
und worin
R⁷ Wasserstoff, Amino oder eine Gruppe der Formel -NH-C(=O)-R⁸ oder -NH-SO₂-R⁸ bedeutet, worin
R⁸ (C₁-C₄)-Alkyl oder (C₃-C₆)-Cycloalkyl darstellt,
wobei (C₁-C₄)-Alkyl mit Hydroxy, (C₁-C₄)-Alkoxy oder (C₃-C₆)-Cycloalkyl und bis zu dreifach mit Fluor substituiert sein kann
und
die genannten Cycloalkyl-Gruppen bis zu zweifach, gleich oder verschieden, mit (C₁-C₄)-Alkyl und bis zu zweifach mit Fluor substituiert sein können,
und
R⁹ Wasserstoff, (C₁-C₄)-Alkyl, (C₁-C₄)-Alkoxy, (C₁-C₄)-Alkoxycarbonyl, (C₃-C₆)-Cycloalkyl oder 5- oder 6-gliedriges Heteroaryl bedeutet,
wobei (C₁-C₄)-Alkyl mit Hydroxy, (C₁-C₄)-Alkoxy oder (C₃-C₆)-Cycloalkyl und bis zu dreifach mit Fluor substituiert sein kann
und wobei
die genannten Cycloalkyl-Gruppen bis zu zweifach, gleich oder verschieden, mit (C₁-C₄)-Alkyl und bis zu zweifach mit Fluor
sowie
die genannte Heteroaryl-Gruppe bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy
substituiert sein können,
oder
R⁹ eine Gruppe der Formel -SO₂-NR¹⁰R¹¹ oder -NR¹²R¹³ bedeutet, worin
R¹⁰ und R¹¹ gleich oder verschieden sind und unabhängig voneinander Wasserstoff, (C₁-C₄)-Alkyl oder (C₃-C₆)-Cycloalkyl darstellen,
R¹² Wasserstoff, (C₁-C₄)-Alkyl, (C₃-C₆)-Cycloalkyl oder (C₁-C₄)-Alkylsulfonyl darstellt,
wobei (C₁-C₄)-Alkyl mit Cyano, Hydroxy, (C₁-C₄)-Alkoxy, Hydroxycarbonyl, (C₁-C₄)-Atkoxycarbonyl, Aminocarbonyl, Mono-(C₁-C₄)-alkylaminocarbonyl, Di-(C₁-C₄)-alkylaminocarbonyl oder (C₃-C₆)-Cycloalkyl und bis zu dreifach mit Fluor substituiert sein kann
und
die genannten Cycloalkyl-Gruppen bis zu zweifach, gleich oder verschieden, mit (C₁-C₄)-Alkyl und bis zu zweifach mit Fluor substituiert sein können,
R¹³ Wasserstoff, (C₁-C₄)-Alkyl oder eine Gruppe der Formel -C(=O)-R¹⁴, -C(=O)-O-R¹⁵ oder -C(=O)-NR¹⁶R¹⁷ darstellt, worin
R¹⁴ (C₁-C₆)-Alkyl, (C₃-C₆)-Cycloalkyl, 4- bis 6-gliedriges Heterocyclyl oder 5- oder 6-gliedriges Heteroaryl bedeutet,
wobei (C₁-C₆)-Alkyl mit Hydroxy, (C₁-C₄)-Alkoxy, (C₁-C₄)-Acyloxy, Amino, Mono-(C₁-C₄)-alkylamino, Di-(C₁-C₄)-alkylamino, (C₁-C₄)-Acylamino, (C₁-C₄)-Alkoxycarbonylamino oder (C₃-C₆)-Cycloalkyl und bis zu dreifach mit Fluor substituiert sein kann
und wobei
die genannten Cycloalkyl- und Heterocyclyl-Gruppen bis zu zweifach, gleich oder verschieden, mit (C₁-C₄)-Alkyl und bis zu zweifach mit Fluor
sowie
die genannte Heteroaryl-Gruppe bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy
substituiert sein können,
R¹⁵ (C₁-C₄)-Alkyl, welches mit (C₃-C₆)-Cycloalkyl oder Phenyl substituiert sein kann, bedeutet,
und
R¹⁶ und R¹⁷ gleich oder verschieden sind und unabhängig voneinander Wasserstoff oder (C₁-C₄)-Alkyl, welches mit Hydroxy oder (C₁-C₄)-Alkoxy substituiert sein kann, bedeuten,
R¹ für Wasserstoff, Fluor, Chlor, Nitro, Methyl, Difluormethyl, Trifluormethyl oder eine Gruppe der Formel -SO₂-R²⁰ steht, worin
R²⁰ (C₁-C₄)-Alkyl, das mit Hydroxy, Methoxy oder Ethoxy oder bis zu dreifach mit Fluor substituiert sein kann, bedeutet,
R² für Cyano oder eine Gruppe der Formel -C(=O)-R²¹ oder -C(=O)-O-R²¹ steht, worin
R²¹ Methyl, Ethyl oder 2-Hydroxyethyl bedeutet,
R³ für Methyl steht,
R⁴ für Trifluormethyl steht
und
R⁵ für Wasserstoff steht,
sowie ihre Salze, Solvate und Solvate der Salze.

3. Verbindung der Formel (I) nach Anspruch 1 oder 2, in welcher
A für CH steht,
Y für C-R⁷ oder N steht,
Z für C-R⁹ oder N steht,
wobei entweder Y für C-R⁷ und Z für N oder Y für N und Z für C-R⁹ stehen
und worin
R⁷ Amino oder eine Gruppe der Formel -NH-C(=O)-R⁸ bedeutet, worin
R⁸ (C₁-C₄)-Alkyl oder (C₃C₆)-Cycloalkyl darstellt,
wobei (C₁-C₄)-Alkyl mit Hydroxy, Methoxy, Ethoxy oder (C₃-C₆)-Cycloalkyl und bis zu dreifach mit Fluor substituiert sein kann
und
die genannten Cycloalkyl-Gruppen bis zu zweifach mit Methyl und bis zu zweifach mit Fluor substituiert sein können,
und
R⁹ (C₁-C₄)-Alkyl, (C₁-C₄)-Alkoxy oder (C₃-C₆)-Cycloalkyl bedeutet,
wobei (C₁-C₄)-Alkyl mit Hydroxy, Methoxy, Ethoxy oder (C₃-C₆)-Cycloalkyl und bis zu dreifach mit Fluor substituiert sein kann
und
die genannten Cycloalkyl-Gruppen bis zu zweifach mit Methyl und bis zu zweifach mit Fluor substituiert sein können,
oder
R⁹ eine Gruppe der Formel -SO₂-NR¹⁰R¹¹ oder -NR¹²R¹³ bedeutet, worin
R¹⁰ und R¹¹ gleich oder verschieden sind und unabhängig voneinander Wasserstoff, Methyl, Ethyl oder Cyclopropyl darstellen,
R¹² Wasserstoff, (C₁-C₄)-Alkyl, (C₃-C₆)-Cycloalkyl oder (C₁-C₄)-Alkylsulfonyl darstellt,
wobei (C₁-C₄)-Alkyl mit Cyano, Hydroxy, (C₁-C₄)-Alkoxy, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl oder (C₃-C₆)-Cycloalkyl und bis zu dreifach mit Fluor substituiert sein kann
und
die genannten Cycloalkyl-Gruppen bis zu zweifach mit Methyl und bis zu zweifach mit Fluor substituiert sein können,
R¹³ Wasserstoff oder eine Gruppe der Formel -C(=O)-R¹⁴ oder -C(=O)-NR¹⁶R¹⁷ darstellt, worin
R¹⁴ (C₁-C₄)-Alkyl, (C₃-C₆)-Cycloalkyl oder 5- oder 6-gliedriges Heteroaryl bedeutet,
wobei (C₁-C₄)-Alkyl mit Hydroxy, (C₁-C₄)-Alkoxy, Amino, Mono-(C₁-C₄)-alkylamino, Di-(C₁-C₄)-alkylamino, (C₁-C₄)-Acylamino oder (C₃-C₆)-Cycloalkyl und bis zu dreifach mit Fluor substituiert sein kann
und wobei
die genannten Cycloalkyl-Gruppen bis zu zweifach mit Methyl und bis zu zweifach mit Fluor
sowie
die genannte Heteroaryl-Gruppe bis zu zweifach, gleich oder verschieden, mit Fluor, Cyano, Methyl, Difluormethyl, Trifluormethyl, Methoxy und Trifluormethoxy
substituiert sein können,
und
R¹⁶ und R¹⁷ gleich oder verschieden sind und unabhängig voneinander Wasserstoff oder (C₁-C₄)-Alkyl, welches mit Hydroxy, Methoxy oder Ethoxy substituiert sein kann, bedeuten,
R¹ für Wasserstoff, Nitro, Trifluormethyl, Methylsulfonyl oder Trifluormethylsulfonyl steht,
R² für Cyano, Acetyl, Ethoxycarbonyl oder (2-Hydroxyethoxy)carbonyl steht,
R³ für Methyl steht,
R⁴ für Trifluormethyl steht
und
R⁵ für Wasserstoff steht,
sowie ihre Salze, Solvate und Solvate der Salze.

4. Verbindung der Formel (I) nach Anspruch 1, 2 oder 3, in welcher
A für CH steht,
Y für N steht,
Z für C-R⁹ steht, worin
R⁹ eine Gruppe der Formel -NHR¹² oder -NH-C(=O)-R¹⁴ bedeutet, worin
R¹² eine Gruppe der Formel -CH₂-C(=O)-OH oder -CH₂-C(=O)-NH₂ darstellt
und
R¹⁴ (C₃-C₆)-Cycloalkyl, das bis zu zweifach mit Methyl und bis zu zweifach mit Fluor substituiert sein kann, oder (C₁-C₄)-Alkyl darstellt,
R¹ für Wasserstoff oder Methylsulfonyl steht,
R² für Cyano steht,
R³ für Methyl steht,
R⁴ für Trifluormethyl steht
und
R⁵ für Wasserstoff steht,
sowie ihre Salze, Solvate und Solvate der Salze.

5. Verfahren zur Herstellung von Verbindungen der Formel (I), wie in den Ansprüchen 1 bis 4 definiert, **dadurch gekennzeichnet, dass** man zunächst eine Verbindung der Formel (II) 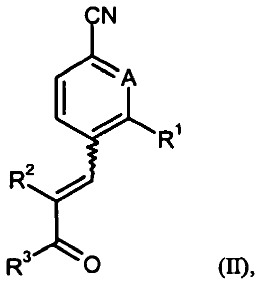 in welcher A, R¹, R² und R³ jeweils die in den Ansprüchen 1 bis 4 angegebenen Bedeutungen haben,
in Gegenwart einer Base mit einer Verbindung der Formel (III) 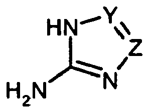 in welcher Y und Z die in den Ansprüchen 1 bis 4 angegebenen Bedeutungen haben,
zu einem Intermediat der Formel (IV) 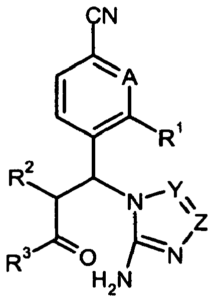 in welcher A, Y, Z, R¹, R² und R³ jeweils die oben angegebenen Bedeutungen haben,
umsetzt, dieses dann *in situ* oder in einem separaten, säurekatalysierten Reaktionsschritt zu einer Verbindung der Formel (V) 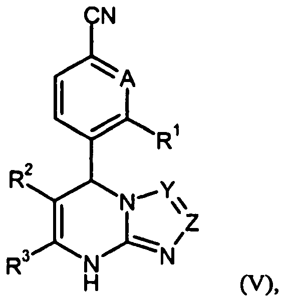 in welcher A, Y, Z, R¹, R² und R³ jeweils die oben angegebenen Bedeutungen haben, cyclisiert und die Verbindung (V) anschließend in Gegenwart eines Kupfer(II)-Katalysators und einer Base mit einer Phenylboronsäure der Formel (VI) 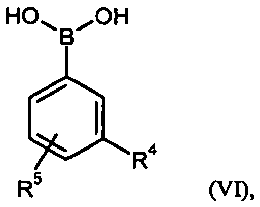 in welcher R⁴ und R⁵ die in den Ansprüchen 1 bis 4 angegebenen Bedeutungen haben, zu einer Verbindung der Formel (I) kuppelt
und gegebenenfalls die so erhaltenen Verbindungen der Formel (I) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

6. Verbindung, wie in einem der Ansprüche 1 bis 4 definiert, zur Behandlung und/oder Prävention von Krankheiten.

7. Verbindung, wie in einem der Ansprüche 1 bis 4 definiert, zur Verwendung in einem Verfahren zur Behandlung und/oder Prävention von pulmonaler arterieller Hypertonie (PAH) und anderen Formen der pulmonalen Hypertonie (PH), chronisch-obstruktiven Lungenerkrankungen (COPD), akuter Lungenschädigung (ALI), akutem Atemwegssyndrom (ARDS), Bronchiektasie, Bronchiolitis obliterans, Lungenemphysem, alpha-1-Antitrypsin-Defizienz (AATD) und zystischer Fibrose (CF).

8. Verwendung einer Verbindung, wie in einem der Ansprüche 1 bis 4 definiert, zur Herstellung eines Arzneimittels zur Behandlung und/oder Prävention von pulmonaler arterieller Hypertonie (PAH) und anderen Formen der pulmonalen Hypertonie (PH), chronisch-obstruktiven Lungenerkrankungen (COPD), akuter Lungenschädigung (ALI), akutem Atemwegssyndrom (ARDS), Bronchiektasie, Bronchiolitis obliterans, Lungenemphysem, alpha-1-Antitrypsin-Defizienz (AATD) und zystischer Fibrose (CF).

9. Arzneimittel enthaltend eine Verbindung, wie in einem der Ansprüche 1 bis 4 definiert, in Kombination mit einem oder mehreren inerten, nicht-toxischen, pharmazeutisch geeigneten Hilfsstoffen.

10. Arzneimittel enthaltend eine Verbindung, wie in einem der Ansprüche 1 bis 4 definiert, in Kombination mit einem oder mehreren weiteren Wirkstoffen ausgewählt aus der Gruppe bestehend aus Kinase-Inhibitoren, Stimulatoren und Aktivatoren der löslichen Guanylatcyclase, Prostacyclin-Analoga, Endothelinrezeptor-Antagonisten, Phosphodiesterase-Inhibitoren, beta-adrenerge Rezeptor-Agonisten, Anticholinergika und Glucocorticoide.

11. Arzneimittel nach Anspruch 9 oder 10 zur Behandlung und/oder Prävention von pulmonaler arterieller Hypertonie (PAH) und anderen Formen der pulmonalen Hypertonie (PH), chronisch-obstruktiven Lungenerkrankungen (COPD), akuter Lungenschädigung (ALI), akutem Atemwegssyndrom (ARDS), Bronchiektasie, Bronchiolitis obliterans, Lungenemphysem, alpha-1-Antitrypsin-Defizienz (AATD) und zystischer Fibrose (CF).

## Claims

1. Compound of the formula (I) 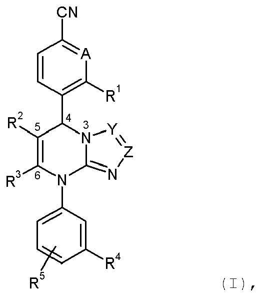 in which
A represents C-R⁶ or N in which
R⁶ represents hydrogen, fluorine or chlorine,
Y represents C-R⁷ or N in which
R⁷ represents hydrogen, (C₁-C₆)-alkyl, amino or a group of the formula -NH-C(-O)-R⁸ or -NH-SO₂-R⁸ in which
R⁸ represents (C₁-C₆)-alkyl, (C₃-C₆)-cycloalkyl or phenyl,
where (C₁-C₆)-alkyl may be substituted by hydroxyl, (C₁-C₄)-alkoxy, (C₃-C₆)-cycloalkyl or phenyl and up to three times by fluorine
and where
the cycloalkyl groups mentioned may be substituted up to two times by identical or different substituents from the group consisting of (C₁-C₄)-alkyl and up to two times by fluorine
and
the phenyl groups mentioned may be substituted up to two times by identical or different substituents from the group consisting of fluorine, chlorine, cyano, (C₁-C₄)-alkyl, difluoromethyl, trifluoromethyl, (C₁-C₉)-alkoxy, difluoromethoxy and trifluoromethoxy
Z represents C-R⁹ or N in which
R⁹ represents hydrogen, (C₁-C₆)-alkyl, (C₁-C₆)-alkoxy, (C₁-C₆)-alkoxycarbonyl, (C₃-C₆)-cycloalkyl, phenyl or 5- or 6-membered heteroaryl,
where (C₁-C₆)-alkyl may be substituted by hydroxyl, (C₁-C₄)-alkoxy, (C₃-C₆)-cycloalkyl or phenyl and up to three times by fluorine
and where
the cycloalkyl groups mentioned may be substituted up to two times by identical or different substituents from the group consisting of (C₁-C₄)-alkyl and up to two times by fluorine
and
the phenyl and heteroaryl groups mentioned may be substituted up to two times by identical or different substituents from the group consisting of fluorine, chlorine, cyano, (C₁-C₄)-alkyl, difluoromethyl, trifluoromethyl, (C₁-C₄)-alkoxy, difluoromethoxy and trifluoromethoxy,
or
R⁹ represents a group of the formula -SO₂-NR¹⁰R¹¹ or -NR¹²R¹³ in which
R¹⁰ and R¹¹ are identical or different and independently of one another represent hydrogen, (C₁-C₆)-alkyl, (C₃-C₆)-cycloalkyl, phenyl or 5- or 6-membered heteroaryl,
R¹² represents hydrogen, (C₁-C₆)-alkyl, (C₃-C₆)-cycloalkyl, phenyl or (C₁-C₆)-alkylsulfonyl,
where (C₁-C₆)-alkyl may be substituted by cyano, hydroxyl, (C₁-C₄)-alkoxy, hydroxycarbonyl, (C₁-C₄)-alkoxycarbonyl, aminocarbonyl, mono-(C₁-C₄)-alkylaminocarbonyl, di-(C₁-C₄)-alkylaminocarbonyl, (C₃-C₆)-cycloalkyl or phenyl and up to three times by fluorine
and where
the cycloalkyl groups mentioned may be substituted up to two times by identical or different substituents from the group consisting of (C₁-C₄)-alkyl and up to two times by fluorine
and
the phenyl groups mentioned may be substituted up to two times by identical or different substituents from the group consisting of fluorine, chlorine, cyano, (C₁-C₄)-alkyl, difluoromethyl, trifluoromethyl, (C₁-C₄)-alkoxy, difluoromethoxy and trifluoromethoxy,
R¹³ represents hydrogen, (C₁-C₆)-alkyl or a group of the formula -C(=O)-R¹⁴, -C(=O)-O-R¹⁵ or -C(=O)-NR¹⁶R¹⁷ in which
R¹⁴ represents hydrogen, (C₁-C₈)-alkyl, (C₃-C₆)-cycloalkyl, 4-to 6-membered heterocyclyl, phenyl or 5- or 6-membered heteroaryl,
where (C₁-C₈)-alkyl may be substituted by hydroxyl, (C₁-C₄)-alkoxy, benzyloxy, phenoxy, (C₁-C₄)-acyloxy, amino, mono-(C₁-C₄)-alkylamino, di-(C₁-C₄)-alkylamino, (C₁-C₄)-acylamino, (C₁-C₄)-alkoxycarbonylamino, (C₃-C₆)-cycloalkyl or phenyl and up to three times by fluorine and up to two CH₂ groups in (C₁-C₈)-alkyl may be exchanged for an oxygen atom provided this results in a stable compound
and where
the cycloalkyl and heterocyclyl groups mentioned may be substituted up to two times by identical or different substituents from the group consisting of (C₁-C₄)-alkyl and up to two times by fluorine
and
the phenyl and heteroaryl groups mentioned may be substituted up to two times by identical or different substituents from the group consisting of fluorine, chlorine, cyano, (C₁-C₄)-alkyl, difluoromethyl, trifluoromethyl, (C₁-C₄)-alkoxy, difluoromethoxy, trifluoromethoxy and hydroxycarbonyl,
R¹⁵ represents (C₁-C₆)-alkyl which may be substituted by (C₃-C₆)-cycloalkyl or phenyl,
R¹⁶ and R¹⁷ are identical or different and independently of one another represent hydrogen or (C₁-C₆)-alkyl which may be substituted by hydroxyl or (C₁-C₄)-alkoxy,
or
R¹² and R¹³ together with the nitrogen atom to which they are attached form a 5- or 6-membered heterocycle which may contain a further ring heteroatom from the group consisting of N, O and S and which may be substituted up to two times by identical or different substituents from the group consisting of (C₁-C₄)-alkyl, hydroxyl, (C₁-C₄)-alkoxy and oxo and may be fused to a phenyl ring,
or in which
R⁷ and R⁹, if both are present, are attached to one another and together with the carbon atoms to which they are attached form a fused phenyl, pyridyl or pyrimidyl ring which may in each case be substituted up to two times by identical or different substituents from the group consisting of fluorine, chlorine, cyano, (C₁-C₄)-alkyl, difluoromethyl, trifluoromethyl, (C₁-C₄)-alkoxy, difluoromethoxy and trifluoromethoxy,
R¹ represents hydrogen, halogen, cyano, nitro, (C₁-C₆)-alkyl, difluoromethyl, trifluoromethyl, (C₁-C₆)-alkoxy, difluoromethoxy, trifluoromethoxy, amino, mono- or di-(C₁-C₆)-alkylamino
or represents a group of the formula -NH-C(=O)-R¹⁸, -NH-C(=O)-NHR¹⁸, -NH-SO₂-R¹⁹ or -S(O)ₙ-R²⁰ in which
R¹⁸ represents hydrogen or (C₁-C₆)-alkyl,
R¹⁹ represents (C₁-C₆)-alkyl,
R²⁰ represents (C₁-C₆)-alkyl which may be substituted by hydroxyl, (C₁-C₉)-alkoxy, amino, mono- or di-(C₁-C₄)-alkylamino, hydroxycarbonyl, aminocarbonyl, (C₃-C₆)-cycloalkyl or phenyl and up to three times by fluorine, or (C₂-C₆)-alkenyl, (C₃-C₆)-cycloalkyl or phenyl,
where the cycloalkyl groups mentioned may be substituted up to two times by identical or different substituents from the group consisting of (C₁-C₄)-alkyl, hydroxyl and (C₁-C₄)-alkoxy
and
the phenyl groups mentioned may be substituted up to two times by identical or different substituents from the group consisting of fluorine, chlorine, cyano, (C₁-C₄)-alkyl, difluoromethyl, trifluoromethyl, (C₁-C₄)-alkoxy, difluoromethoxy and trifluoromethoxy,
and
n represents the number 0, 1 or 2,
R² represents cyano or a group of the formula - C(=O)-R²¹, -C(=O)-O-R²¹ or -C(=O)-NH-R²¹ in which
R²¹ represents hydrogen, (C₁-C₆)-alkyl, (C₃-C₆)-alkenyl or (C₃-C₆)-cycloalkyl,
where (C₁-C₆)-alkyl and (C₃-C₆)-cycloalkyl for their part may be substituted up to two times by identical or different substituents from the group consisting of hydroxyl, (C₁-C₉)-alkoxy, hydroxycarbonyl, (C₁-C₉)-alkoxycarbonyl, amino, mono- and di-(C₁-C₉)-alkylamino and in (C₁-C₆)-alkyl and (C₃-C₆)-cycloalkyl in each case one CH₂ group may be replaced by an oxygen atom provided this results in a chemically stable compound,
R³ represents methyl or ethyl
or
R² and R³ are attached to one another and together form a fused group of the formula 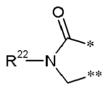 in which
* denotes the point of attachment to the 5-position, marked in formula (I), of the dihydropyrimidine ring
and
** denotes the point of attachment to the 6-position, marked in formula (I), of the dihydropyrimidine ring
and
R²² represents hydrogen, (C₁-C₆)-alkyl or (C₃-C₆)-cycloalkyl,
where (C₁-C₆)-alkyl may be substituted by hydroxyl, (C₁-C₉)-alkoxy, aminocarbonyl, aminocarbonylamino, (C₁-C₄)-acylamino or (C₃-C₆)-cycloalkyl,
R⁴ represents nitro or trifluoromethyl
and
R⁵ represents hydrogen, fluorine or chlorine,
and its salts, solvates and solvates of the salts.

2. Compound of the formula (I) according to Claim 1 in which
A represents CH,
Y represents C-R⁷ or N,
Z represents C-R⁹ or N,
where at least one of the two ring members Y and Z represents N
and in which
R⁷ represents hydrogen, amino or a group of the formula -NH-C(=O)-R⁸ or -NH-SO₂-R⁸ in which
R⁸ represents (C₁-C₄)-alkyl or (C₃-C₆)-cycloalkyl,
where (C₁-C₄)-alkyl may be substituted by hydroxyl, (C₁-C₄)-alkoxy or (C₃-C₆)-cycloalkyl and up to three times by fluorine
and
the cycloalkyl groups mentioned may be substituted up to two times by identical or different substituents from the group consisting of (C₁-C₄)-alkyl and up to two times by fluorine,
and
R⁹ represents hydrogen, (C₁-C₄)-alkyl, (C₁-C₄)-alkoxy, (C₁-C₄)-alkoxycarbonyl, (C₃-C₆)-cycloalkyl or 5- or 6-membered heteroaryl,
where (C₁-C₄)-alkyl may be substituted by hydroxyl, (C₁-C₄)-alkoxy or (C₃-C₆)-cycloalkyl and up to three times by fluorine
and where
the cycloalkyl groups mentioned may be substituted up to two times by identical or different substituents from the group consisting of (C₁-C₄)-alkyl and up to two times by fluorine
and
the heteroaryl group mentioned may be substituted up to two times by identical or different substituents from the group consisting of fluorine, chlorine, cyano, (C₁-C₄)-alkyl, difluoromethyl, trifluoromethyl, (C₁-C₉)-alkoxy, difluoromethoxy and trifluoromethoxy,
or
R⁹ represents a group of the formula -SO₂-NR¹⁰R¹¹ or -NR¹²R¹³ in which
R¹⁰ and R¹¹ are identical or different and independently of one another represent hydrogen, (C₁-C₄)-alkyl or (C₃-C₆)-cycloalkyl,
R¹² represents hydrogen, (C₁-C₄)-alkyl, (C₃-C₆)-cycloalkyl or (C₁-C₉)-alkylsulfonyl,
where (C₁-C₄)-alkyl may be substituted by cyano, hydroxyl, (C₁-C₄)-alkoxy, hydroxycarbonyl, (C₁-C₄)-alkoxycarbonyl, aminocarbonyl, mono-(C₁-C₄)-alkylaminocarbonyl, di-(C₁-C₄)-alkylaminocarbonyl or (C₃-C₆)-cycloalkyl and up to three times by fluorine
and
the cycloalkyl groups mentioned may be substituted up to two times by identical or different substituents from the group consisting of (C₁-C₄)-alkyl and up to two times by fluorine,
R¹³ represents hydrogen, (C₁-C₄)-alkyl or a group of the formula -C(=O)-R¹⁹, -C(=O)-O-R¹⁵ or -C(=O)-NR¹⁶R¹⁷ in which
R¹⁴ represents (C₁-C₆)-alkyl, (C₃-C₆)-cycloalkyl, 4- to 6-membered heterocyclyl or 5- or 6-membered heteroaryl,
where (C₁-C₆)-alkyl may be substituted by hydroxyl, (C₁-C₄)-alkoxy, (C₁-C₄)-acyloxy, amino, mono-(C₁-C₄)-alkylamino, di-(C₁-C₄)-alkylamino, (C₁-C₄)-acylamino, (C₁-C₄)-alkoxycarbonylamino or (C₃-C₆)-cycloalkyl and up to three times by fluorine
and where
the cycloalkyl and heterocyclyl groups mentioned may be substituted up to two times by identical or different substituents from the group consisting of (C₁-C₄)-alkyl and up to two times by fluorine
and
the heteroaryl group mentioned may be substituted up to two times by identical or different substituents from the group consisting of fluorine, chlorine, cyano, (C₁-C₄)-alkyl, difluoromethyl, trifluoromethyl, (C₁-C₄)-alkoxy, difluoromethoxy and trifluoromethoxy,
R¹⁵ represents (C₁-C₄)-alkyl which may be substituted by (C₃-C₆)-cycloalkyl or phenyl,
and
R¹⁶ and R¹⁷ are identical or different and independently of one another represent hydrogen or (C₁-C₄)-alkyl which may be substituted by hydroxyl or (C₁-C₄)-alkoxy,
R¹ represents hydrogen, fluorine, chlorine, nitro, methyl, difluoromethyl, trifluoromethyl or a group of the formula -SO₂-R²⁰ in which
R²⁰ represents (C₁-C₄)-alkyl which may be substituted by hydroxyl, methoxy or ethoxy or up to three times by fluorine,
R² represents cyano or a group of the formula - C(=O)-R²¹ or -C(=O)-O-R²¹ in which
R²¹ represents methyl, ethyl or 2-hydroxyethyl,
R³ represents methyl,
R⁴ represents trifluoromethyl
and
R⁵ represents hydrogen,
and its salts, solvates and solvates of the salts.

3. Compound of the formula (I) according to Claim 1 or 2 in which
A represents CH,
Y represents C-R⁷ or N,
Z represents C-R⁹ or N,
where either Y represents C-R⁷ and Z represents N or Y represents N and Z represents C-R⁹
and in which
R⁷ represents amino or a group of the formula -NH-C(-O)-R⁸ in which
R⁸ represents (C₁-C₄)-alkyl or (C₃-C₆)-cycloalkyl,
where (C₁-C₄)-alkyl may be substituted by hydroxyl, methoxy, ethoxy or (C₃-C₆)-cycloalkyl and up to three times by fluorine
and
the cycloalkyl groups mentioned may be substituted up to two times by methyl and up to two times by fluorine,
and
R⁹ represents (C₁-C₄)-alkyl, (C₁-C₄)-alkoxy or (C₃-C₆)-cycloalkyl,
where (C₁-C₄)-alkyl may be substituted by hydroxyl, methoxy, ethoxy or (C₃-C₆)-cycloalkyl and up to three times by fluorine
and
the cycloalkyl groups mentioned may be substituted up to two times by methyl and up to two times by fluorine,
or
R⁹ represents a group of the formula -SO₂-NR¹⁰R¹¹ or -NR,¹²R¹¹ in which
R¹⁰ and R¹¹ are identical or different and independently of one another represent hydrogen, methyl, ethyl or cyclopropyl,
R¹² represents hydrogen, (C₁-C₄)-alkyl, (C₃-C₆)-cycloalkyl or (C₁-C₄)-alkylsulfonyl,
where (C₁-C₄)-alkyl may be substituted by cyano, hydroxyl, (C₁-C₄)-alkoxy, hydroxycarbonyl, (C₁-C₄)-alkoxycarbonyl, aminocarbonyl, mono-(C₁-C₄)-alkylaminocarbonyl, di-(C₁-C₄)-alkylaminocarbonyl or (C₃-C₆)-cycloalkyl and up to three times by fluorine
and
the cycloalkyl groups mentioned may be substituted up to two times by methyl and up to two times by fluorine,
R¹³ represents hydrogen or a group of the formula -C(=O)-R¹⁴ or -C(=O)-NR¹⁶R¹⁷ in which
R¹⁴ represents (C₁-C₄)-alkyl, (C₃-C₆)-cycloalkyl or 5- or 6-membered heteroaryl,
where (C₁-C₄)-alkyl may be substituted by hydroxyl, (C₁-C₄)-alkoxy, amino, mono-(C₁-C₄)-alkylamino, di-(C₁-C₄)-alkylamino, (C₁-C₄)-acylamino or (C₃-C₆)-cycloalkyl and up to three times by fluorine
and where
the cycloalkyl groups mentioned may be substituted up to two times by methyl and up to two times by fluorine
and
the heteroaryl group mentioned may be substituted up to two times by identical or different substituents from the group consisting of fluorine, cyano, methyl, difluoromethyl, trifluoromethyl, methoxy and trifluoromethoxy,
and
R¹⁶ and R¹⁷ are identical or different and independently of one another represent hydrogen or (C₁-C₄)-alkyl which may be substituted by hydroxyl, methoxy or ethoxy,
R¹ represents hydrogen, nitro, trifluoromethyl, methylsulfonyl or trifluoromethylsulfonyl,
R² represents cyano, acetyl, ethoxycarbonyl or (2-hydroxyethoxy)carbonyl,
R³ represents methyl,
R⁴ represents trifluoromethyl
and
R⁵ represents hydrogen,
and its salts, solvates and solvates of the salts.

4. Compound of the formula (I) according to Claim 1, 2 or 3 in which
A represents CH,
Y represents N,
Z represents C-R⁹ in which
R⁹ represents a group of the formula -NHR¹² or -NH-C(=O)-R¹⁴ in which
R¹² represents a group of the formula - CH₂-C(=O)-OH or -CH₂-C(=O)-NH₂
and
R¹⁴ represents (C₃-C₆)-cycloalkyl which may be substituted up to two times by methyl and up to two times by fluorine, or (C₁-C₄)-alkyl,
R¹ represents hydrogen or methylsulfonyl,
R² represents cyano,
R³ represents methyl,
R⁴ represents trifluoromethyl
and
R⁵ represents hydrogen, and its salts, solvates and solvates of the salts.

5. Process for preparing compounds of the formula (I) as defined in Claims 1 to 4, **characterized in that** initially a compound of the formula (II) 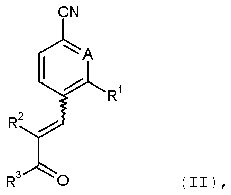 in which A, R¹, R² and R³ each have the meanings given in Claims 1 to 4,
is reacted in the presence of a base with a compound of the formula (III) 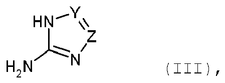 in which Y and Z have the meanings given in Claims 1 to 4,
to give an intermediate of the formula (IV) 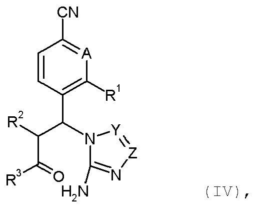 in which A, Y, Z, R¹, R² and R³ each have the meanings given above,
this is then cyclized *in situ* or in a separate acid-catalyzed reaction step to give a compound of the formula (V) 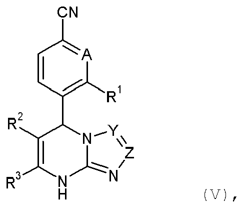 in which A, Y, Z, R¹, R² and R³ each have the meanings given above,
and the compound (V) is then coupled in the presence of a copper(II) catalyst and a base with a phenylboronic acid of the formula (VI) 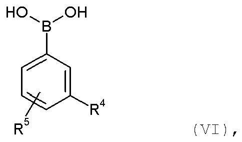 in which R⁴ and R⁵ have the meanings given in Claims 1 to 4,
to give a compound of the formula (I) and the compounds of the formula (I) obtained in this manner are, if appropriate, separated by methods known to the person skilled in the art into their enantiomers and/or diastereomers and/or converted with the appropriate (i) solvents and/or (ii) bases or acids into their solvates, salts and/or solvates of the salts.

6. Compound as defined in any of Claims 1 to 4 for the treatment and/or prevention of diseases.

7. Compound as defined in any of Claims 1 to 4 for use in a method for the treatment and/or prevention of pulmonary arterial hypertension (PAH) and other forms of pulmonary hypertension (PH), of chronic-obstructive pulmonary diseases (COPD), of acute lung injury (ALI), of acute respiratory distress syndrome (ARDS), of bronchiectasis, Bronchiolitis obliterans, of pulmonary emphysema, of alpha-1 antitrypsin deficiency (AATD) and of cystic fibrosis (CF).

8. Use of a compound as defined in any of Claims 1 to 4 for preparing a medicament for the treatment and/or prevention of pulmonary arterial hypertension (PAH) and other forms of pulmonary hypertension (PH), of chronic-obstructive pulmonary diseases (COPD), of acute lung injury (ALI), of acute respiratory distress syndrome (ARDS), of bronchiectasis, Bronchiolitis obliterans, of pulmonary emphysema, of alpha-1 antitrypsin deficiency (AATD) and of cystic fibrosis (CF).

9. Medicament comprising a compound as defined in any of Claims 1 to 4 in combination with one or more inert non-toxic pharmaceutically acceptable auxiliaries.

10. Medicament comprising a compound as defined in any of Claims 1 to 4 in combination with one or more further active compounds selected from the group of the kinase inhibitors, stimulators and activators of soluble guanylate cyclase, prostacyclin analogs, endothelin receptor antagonists, phosphodiesterase inhibitors, beta-adrenergic receptor agonists, anticholinergics and glucocorticoids.

11. Medicament according to Claim 9 or 10 for the treatment and/or prevention of pulmonary arterial hypertension (PAH) and other forms of pulmonary hypertension (PH), or chronic-obstructive pulmonary diseases (COPD), of acute lung injury (ALI), of acute respiratory distress syndrome (ARDS), of bronchiectasis, Bronchiolitis obliterans, of pulmonary emphysema, of alpha-1 antitrypsin deficiency (AATD) and of cystic fibrosis (CF).

## Revendications

1. Composé de formule (I) 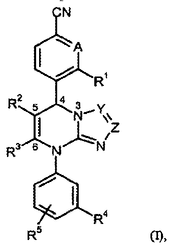 dans laquelle
A représente C-R⁶ ou N, où
R⁶ signifie hydrogène, fluor ou chlore,
Y représente C-R⁷ ou N, où
R⁷ signifie hydrogène, (C₁-C₆)-alkyle, amino ou un groupe de formule -NH-C(=O)-R⁸ ou -NH-SO₂-R⁸, dans laquelle
R⁸ représente (C₁-C₆)-alkyle, (C₃-C₆)-cycloalkyle ou phényle, (C₁-C₆)-alkyle pouvant être substitué par hydroxy, (C₁-C₄)-alcoxy, (C₃-C₆)-cycloalkyle ou phényle et jusqu'à trisubstitué par fluor et les groupes cycloalkyle mentionnés pouvant être jusqu'à disubstitués, de manière identique ou différente, par (C₁-C₉)-alkyle et jusqu'à disubstitués par fluor et
les groupes phényle mentionnés pouvant être jusqu'à disubstitués, de manière identique ou différente, par fluor, chlore, cyano, (C₁-C₄)-alkyle, difluorométhyle, trifluorométhyle, (C₁-C₄)-alcoxy, difluorométhoxy et trifluorométhoxy,
Z représente C-R⁹ ou N, où
R⁹ signifie hydrogène, (C₁-C₆)-alkyle, (C₁-C₆)-alcoxy, (C₁-C₆)-alcoxycarbonyle, (C₃-C₆)-cycloalkyle, phényle ou hétéroaryle de 5 ou 6 chaînons,
(C₁-C₆)-alkyle pouvant être substitué par hydroxy, (C₁-C₄)-alcoxy, (C₃-C₆)-cycloalkyle ou phényle et jusqu'à trisubstitué par fluor et les groupes cycloalkyle mentionnés pouvant être jusqu'à disubstitués, de manière identique ou différente, par (C₁-C₄)-alkyle et jusqu'à disubstitués par fluor et les groupes phényle et hétéroaryle mentionnés pouvant être jusqu'à disubstitués, de manière identique ou différente, par fluor, chlore, cyano, (C₁-C₄)-alkyle, difluorométhyle, trifluorométhyle, (C₁-C₄)-alcoxy, difluorométhoxy et trifluorométhoxy, ou
R⁹ signifie un groupe de formule -SO₂-NR¹⁰R¹¹ ou -NR¹²R¹³, dans laquelle
R¹⁰ et R¹¹ sont identiques ou différents et représentent, indépendamment l'un de l'autre, hydrogène, (C₁-C₆)-alkyle, (C₃-C₆)-cycloalkyle, phényle ou hétéroaryle de 5 ou 6 chaînons,
R¹² représente hydrogène, (C₁-C₆)-alkyle ou (C₃-C₆)-cycloalkyle, phényle ou (C₁-C₆)-alkylsulfonyle,
(C₁-C₆)-alkyle pouvant être substitué par cyano, hydroxy, (C₁-C₄)-alcoxy, hydroxycarbonyle, (C₁-C₄)-alcoxycarbonyle, aminocarbonyle, mono-(C₁-C₄)-alkylaminocarbonyle, di-(C₁-C₄)-alkylaminocarbonyle, (C₃-C₆)-cycloalkyle ou phényle et jusqu'à trisubstitué par fluor et
les groupes cycloalkyle mentionnés pouvant être jusqu'à disubstitués, de manière identique ou différente, par (C₁-C₄)-alkyle et jusqu'à disubstitués par fluor et
les groupes phényle mentionnés pouvant être jusqu'à disubstitués, de manière identique ou différente, par fluor, chlore, cyano, (C₁-C₄)-alkyle, difluorométhyle, trifluorométhyle, (C₁-C₄)-alcoxy, difluorométhoxy et trifluorométhoxy,
R¹³ représente hydrogène, (C₁-C₆)-alkyle ou un groupe de formule -C(=O)-R¹⁴, -C(=O)-O-R¹⁵ ou -C(=O)-NR¹⁶R¹⁷, dans laquelle
R¹⁴ signifie hydrogène, (C₁-C₈)-alkyle, (C₃-C₆)-cycloalkyle, hétérocyclyle de 4 à 6 chaînons, phényle ou hétéroaryle de 5 ou 6 chaînons, (C₁-C₈)-alkyle pouvant être substitué par hydroxy, (C₁-C₄)-alcoxy, benzyloxy, phénoxy, (C₁-C₉)-acyloxy, amino, mono-(C₁-C₄)-alkylamino, di-(C₁-C₄)-alkylamino, (C₁-C₄)-acylamino, (C₁-C₄)-alcoxycarbonylamino, (C₃-C₆)-cycloalkyle ou phényle et jusqu'à trisubstitué par fluor et jusqu'à deux groupes CH₂ dans (C₁-C₈)-alkyle pouvant être remplacés par un atome d'oxygène, pour autant qu'on obtienne un composé chimiquement stable et
les groupes cycloalkyle et hétérocycle mentionnés pouvant être jusqu'à disubstitués, de manière identique ou différente, par (C₁-C₄)-alkyle et jusqu'à disubstitués par fluor et
les groupes phényle et hétéroaryle mentionnés pouvant être jusqu'à disubstitués, de manière identique ou différente, par fluor, chlore, cyano, (C₁-C₄)-alkyle, difluorométhyle, trifluorométhyle, (C₁-C₉)-alcoxy, difluorométhoxy, trifluorométhoxy et hydroxycarbonyle,
R¹⁵ signifie (C₁-C₆)-alkyle, qui peut être substitué par (C₃-C₆)-cycloalkyle ou phényle,
R¹⁶ et R¹⁷ peuvent être identiques ou différents et signifient, indépendamment l'un de l'autre, hydrogène ou (C₁-C₆)-alkyle, qui peut être substitué par hydroxy ou (C₁-C₄)-alcoxy, ou
R¹² et R¹³ ensemble avec l'atome d'azote auquel ils sont liés, forment un hétérocycle de 5 ou 6 chaînons, qui peut contenir un autre hétéroatome de cycle de la série N, O ou S et qui peut être jusqu'à disubstitué, de manière identique ou différente, par (C₁-C₄)-alkyle, hydroxy, (C₁-C₄)-alcoxy et oxo et fusionné avec un cycle phényle,
ou dans laquelle
R⁷ et R⁹, pour autant qu'ils soient tous les deux présents, sont liés l'un à l'autre et forment, ensemble avec les atomes de carbone auxquels ils sont liés, un cycle phényle, pyridyle ou pyrimidyle fusionné, qui peut être à chaque fois jusqu'à disubstitué, de manière identique ou différente, par fluor, chlore, cyano, (C₁-C₄)-alkyle, difluorométhyle, trifluorométhyle, (C₁-C₄)-alcoxy, difluorométhoxy et trifluorométhoxy,
R¹ représente hydrogène, halogène, cyano, nitro, (C₁-C₆)-alkyle, difluorométhyle, trifluorométhyle, (C₁-C₆)-alcoxy, difluorométhoxy, trifluorométhoxy, amino, mono-(C₁-C₆)-alkylamino ou di-(C₁-C₆)-alkylamino ou
représente un groupe de formule -NH-C(=O)-R¹⁸, -NH-C(=O)-NHR¹⁸, -NH-SO₂-R¹⁹ ou -S(O)ₙ-R²⁰, dans laquelle
R¹⁸ signifie hydrogène ou (C₁-C₆)-alkyle,
R¹⁹ signifie (C₁-C₆)-alkyle,
R²⁰ signifie (C₁-C₆)-alkyle, qui peut être substitué par hydroxy, (C₁-C₄)-alcoxy, amino, mono-(C₁-C₄)-alkylamino ou di-(C₁-C₉)-alkylamino, hydroxycarbonyle, aminocarbonyle, (C₃-C₆)-cycloalkyle ou phényle et jusqu'à trisubstitué par fluor, ou (C₂-C₆)-alcényle, (C₃-C₆)-cycloalkyle ou phényle, les groupes cycloalkyle mentionnés pouvant être jusqu'à disubstitués, de manière identique ou différente, par (C₁-C₄)-alkyle, hydroxy et (C₁-C₄)-alcoxy et
les groupes phényle mentionnés pouvant être jusqu'à disubstitués, de manière identique ou différente, par fluor, chlore, cyano, (C₁-C₄)-alkyle, difluorométhyle, trifluorométhyle, (C₁-C₄)-alcoxy, difluorométhoxy et trifluorométhoxy, et
n vaut le nombre 0, 1 ou 2,
R² représente cyano ou un groupe de formule -C(=O)-R²¹, -C(=O)-O-R²¹ ou -C(=O)-NH-R²¹, dans laquelle
R²¹ signifie hydrogène, (C₁-C₆)-alkyle, (C₃-C₆)-alcényle ou (C₃-C₆)-cycloalkyle, (C₁-C₆)-alkyle et (C₃-C₆)-cycloalkyle pouvant à leur tour être jusqu'à disubstitués, de manière identique ou différente, par hydroxy, (C₁-C₄)-alcoxy, hydroxycarbonyle, (C₁-C₄)-alcoxycarbonyle, amino, mono-(C₁-C₄)-alkylamino et di-(C₁-C₄)-alkylamino et, dans (C₁-C₆)-alkyle et (C₃-C₆)-cycloalkyle, à chaque fois un groupe CH₂ pouvant être remplacé par un atome O pour autant qu'on obtienne un composé chimiquement stable,
R³ représente méthyle ou éthyle ou
R² et R³ sont liés l'un à l'autre et forment ensemble un groupe fusionné de formule 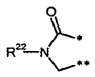
* signifiant le site de liaison avec la position désignée par 5 du cycle dihydropyrimidine dans la formule (I) et
** signifiant le site de liaison avec la position désignée par 6 du cycle dihydropyrimidine dans la formule (I) et
R²² signifiant hydrogène, (C₁-C₆)-alkyle ou (C₃-C₆)-cycloalkyle, (C₁-C₆)-alkyle pouvant être substitué par hydroxy, (C₁-C₄)-alcoxy, aminocarbonyle, aminocarbonylamino, (C₁-C₄)-acylamino ou (C₃-C₆)-cycloalkyle,
R⁴ représente nitro ou trifluorométhyle et
R⁵ représente hydrogène, fluor ou chlore, ainsi que ses sels, solvates et les solvates des sels.

2. Composé de formule (I) selon la revendication 1, dans laquelle
A représente CH,
Y représente C-R⁷ ou N,
Z représente C-R⁹ ou N, au moins un des chaînons de cycle Y et Z représentant N et où
R⁷ signifie hydrogène, amino ou un groupe de formule -NH-C(=O)-R⁸ ou -NH-SO₂-R⁸, dans laquelle
R⁸ signifie (C₁-C₄)-alkyle ou (C₃-C₆)-cycloalkyle, (C₁-C₄)-alkyle pouvant être substitué par hydroxy, (C₁-C₄)-alcoxy ou (C₃-C₆)-cycloalkyle et jusqu'à trisubstitué par fluor et
les groupes cycloalkyle mentionnés pouvant être jusqu'à disubstitués, de manière identique ou différente, par (C₁-C₄)-alkyle et jusqu'à disubstitués par fluor,
et
R⁹ signifie hydrogène, (C₁-C₄)-alkyle, (C₁-C₄)-alcoxy, (C₁-C₄)-alcoxycarbonyle, (C₃-C₆)-cycloalkyle ou hétéroaryle de 5 ou 6 chaînons, (C₁-C₄)-alkyle pouvant être substitué par hydroxy, (C₁-C₄)-alcoxy ou (C₃-C₆)-cycloalkyle et jusqu'à trisubstitué par fluor et où
les groupes cycloalkyle mentionnés peuvent être jusqu'à disubstitués, de manière identique ou différente, par (C₁-C₄)-alkyle et jusqu'à disubstitués par fluor
et
les groupes hétéroaryle mentionnés peuvent être jusqu'à disubstitués, de manière identique ou différente, par fluor, chlore, cyano, C₁-C₄)-alkyle, difluorométhyle, trifluorométhyle, (C₁-C₄)-alcoxy, difluorométhoxy et trifluorométhoxy, ou
R⁹ signifie un groupe de formule -SO₂-NR¹⁰R¹¹ ou -NR¹²R¹³, dans laquelle
R¹⁰ et R¹¹ sont identiques ou différents et représentent, indépendamment l'un de l'autre, hydrogène, (C₁-C₄)-alkyle ou (C₃-C₆)-cycloalkyle,
R¹² représente hydrogène, (C₁-C₄)-alkyle, (C₃-C₆)-cycloalkyle ou (C₁-C₄)-alkylsulfonyle,
(C₁-C₉)-alkyle pouvant être substitué par cyano, hydroxy, (C₁-C₄)-alcoxy, hydroxycarbonyle, (C₁-C₄)-alcoxycarbonyle, aminocarbonyle, mono-(C₁-C₄)-alkylaminocarbonyle, di-(C₁-C₄)-alkylaminocarbonyle ou (C₃-C₆)-cycloalkyle et jusqu'à trisubstitué par fluor et
les groupes cycloalkyle mentionnés pouvant être jusqu'à disubstitués, de manière identique ou différente, par (C₁-C₄)-alkyle et jusqu'à disubstitués par fluor,
R¹³ représente hydrogène, (C₁-C₄)-alkyle ou un groupe de formule -C(=O)-R¹⁴, -C(=O)-O-R¹⁵ ou -C(=O)-NR¹⁶R¹⁷, dans laquelle
R¹⁴ signifie (C₁-C₆)-alkyle, (C₃-C₆)-cycloalkyle, hétérocyclyle de 4 à 6 chaînons ou hétéroaryle de 5 ou 6 chaînons,
(C₁-C₆)-alkyle pouvant être substitué par hydroxy, (C₁-C₄)-alcoxy, (C₁-C₄)-acyloxy, amino, mono-(C₁-C₄)-alkylamino, di-(C₁-C₄)-alkylamino, (C₁-C₄)-acylamino, (C₁-C₄)-alcoxycarbonylamino ou (C₃-C₆)-cycloalkyle et jusqu'à trisubstitué par fluor et
où
les groupes cycloalkyle et hétérocycle mentionnés peuvent être jusqu'à disubstitués, de manière identique ou différente, par (C₁-C₄)-alkyle et jusqu'à disubstitués par fluor et les groupes hétéroaryle mentionnés peuvent être jusqu'à disubstitués, de manière identique ou différente, par fluor, chlore, cyano, (C₁-C₄)-alkyle, difluorométhyle, trifluorométhyle, (C₁-C₄)-alcoxy, difluorométhoxy et trifluorométhoxy,
R¹⁵ signifie (C₁-C₄)-alkyle, qui peut être substitué par (C₃-C₆)-cycloalkyle ou phényle, et
R¹⁶ et R¹⁷ sont identiques ou différents et signifient, indépendamment l'un de l'autre, hydrogène ou (C₁-C₄)-alkyle, qui peut être substitué par hydroxy ou (C₁-C₄)-alcoxy,
R¹ représente hydrogène, fluor, chlore, nitro, méthyle, difluorométhyle, trifluorométhyle ou un groupe de formule -SO₂-R²⁰, dans laquelle
R²⁰ signifie (C₁-C₄)-alkyle, qui peut être substitué par hydroxy, méthoxy ou éthoxy ou jusqu'à trisubstitué par fluor,
R² représente cyano ou un groupe de formule -C(-O)-R²¹ ou -C(-O)-O-R²¹, dans laquelle
R²¹ signifie méthyle, éthyle ou 2-hydroxyéthyle,
R³ représente méthyle,
R⁴ représente trifluorométhyle et
R⁵ représente hydrogène,
ainsi que ses sels, solvates et les solvates des sels.

3. Composé de formule (I) selon la revendication 1 ou 2, dans laquelle
A représente CH,
Y représente C-R⁷ ou N,
Z représente C-R⁹ ou N, Y représentant C-R⁷ et Z représentant N ou alors Y représentant N et Z représentant C-R⁹ et où
R⁷ signifie amino ou un groupe de formule -NH-C(=O)-R⁸, dans laquelle
R⁸ représente (C₁-C₉)-alkyle ou (C₃-C₆)-cycloalkyle, (C₁-C₄)-alkyle pouvant être substitué par hydroxy, méthoxy, éthoxy ou (C₃-C₆)-cycloalkyle et jusqu'à trisubstitué par fluor et
les groupes cycloalkyle mentionnés pouvant être jusqu'à disubstitués par méthyle et jusqu'à disubstitués par fluor,
et
R⁹ signifie (C₁-C₄)-alkyle, (C₁-C₄)-alcoxy ou (C₃-C₆)-cycloalkyle, (C₁-C₉)-alkyle pouvant être substitué par hydroxy, méthoxy, éthoxy ou (C₃-C₆)-cycloalkyle et jusqu'à trisubstitué par fluor
et
les groupes cycloalkyle mentionnés pouvant être jusqu'à disubstitués par méthyle et jusqu'à disubstitués par fluor, ou
R⁹ signifie un groupe de formule -SO₂-NR¹⁰R¹¹ ou -NR¹²R¹³, dans laquelle
R¹⁰ et R¹¹ sont identiques ou différents et représentent, indépendamment l'un de l'autre hydrogène, méthyle, éthyle ou cyclopropyle,
R¹² représente hydrogène, (C₁-C₄)-alkyle, (C₃-C₆)-cycloalkyle ou (C₁-C₄)-alkylsulfonyle, (C₁-C₄)-alkyle pouvant être substitué par cyano, hydroxy, (C₁-C₉)-alcoxy, hydroxycarbonyle, (C₁-C₄)-alcoxycarbonyle, aminocarbonyle, mono-(C₁-C₄)-alkylaminocarbonyle, di-(C₁-C₄)-alkylaminocarbonyle ou (C₃-C₆)-cycloalkyle et jusqu'à trisubstitué par fluor et
les groupes cycloalkyle mentionnés pouvant être jusqu'à disubstitués par méthyle et jusqu'à disubstitués par fluor,
R¹³ représente hydrogène ou un groupe de formule -C(=O)-R¹⁴ ou -C(=O)-NR¹⁶R¹⁷, dans laquelle
R¹⁴ signifie (C₁-C₉)-alkyle, (C₃-C₆)-cycloalkyle ou hétéroaryle de 5 ou 6 chaînons,
(C₁-C₄)-alkyle pouvant être substitué par hydroxy, (C₁-C₄)-alcoxy, amino, mono-(C₁-C₄)-alkylamino, di-(C₁-C₄)-alkylamino, (C₁-C₄)-acylamino ou (C₃-C₆)-cycloalkyle et jusqu'à trisubstitué par fluor et
où
les groupes cycloalkyle mentionnés peuvent être jusqu'à disubstitués par méthyle et jusqu'à disubstitués par fluor,
et
les groupes hétéroaryle mentionnés peuvent être jusqu'à disubstitués, de manière identique ou différente, par fluor, cyano, méthyle, difluorométhyle, trifluorométhyle, méthoxy et trifluorométhoxy,
et
R¹⁶ et R¹⁷ sont identiques ou différents et signifient, indépendamment l'un de l'autre, hydrogène ou (C₁-C₄)-alkyle, qui peut être substitué par hydroxy, méthoxy ou éthoxy,
R¹ représente hydrogène, nitro, trifluorométhyle, méthylsulfonyle ou trifluorométhylsulfonyle,
R² représente cyano, acétyle, éthoxycarbonyle ou (2-hydroxyéthoxy)carbonyle,
R³ représente méthyle,
R⁴ représente trifluorométhyle et
R⁵ représente hydrogène,
ainsi que ses sels, solvates et les solvates des sels.

4. Composé de formule (I) selon la revendication 1, 2 ou 3, dans laquelle
A représente CH,
Y représente N,
Z représente C-R⁹, où
R⁹ signifie un groupe de formule -NHR¹² ou -NH-C(=O)-R¹⁴, dans laquelle
R¹² représente un groupe de formule -CH₂-C(=O)-OH ou -CH₂-C(=O)-NH₂ et
R¹⁴ représente (C₃-C₆)-cycloalkyle, qui peut être jusqu'à disubstitué par méthyle et jusqu'à disubstitué par fluor, ou (C₁-C₄)-alkyle,
R¹ représente hydrogène ou méthylsulfonyle,
R² représente cyano,
R³ représente méthyle,
R⁴ représente trifluorométhyle et
R⁵ représente hydrogène,
ainsi que ses sels, solvates et les solvates des sels.

5. Procédé pour la préparation de composés de formule (I), tels que définis dans les revendications 1 à 4, **caractérisé en ce qu'**on transforme d'abord un composé de formule (II) 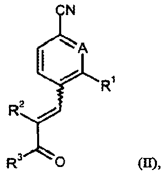 dans laquelle A, R¹ R² et R³ présentent à chaque fois les significations indiquées dans les revendications 1 à 4,
en présence d'une base avec un composé de formule (III) 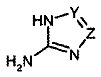 dans laquelle Y et Z présentent les significations indiquées dans les revendications 1 à 4, en un
intermédiaire de formule (IV) 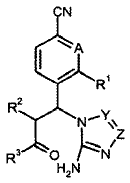 dans laquelle A, Y, Z, R¹ R² et R³ présentent à chaque fois les significations indiquées ci-dessus,
on cyclise alors celui-ci in situ ou dans une étape de réaction séparée, catalysée par un acide, en un composé de formule (V) 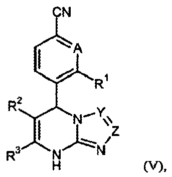 dans laquelle A, Y, Z, R¹ R² et R³ présentent à chaque fois les significations indiquées ci-dessus,
et on couple ensuite le composé (V) en présence d'un catalyseur de cuivre (II) et d'une base avec un acide phénylboronique de formule (VI) 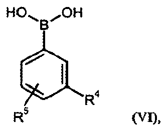 dans laquelle R⁴ et R⁵ présentent les significations indiquées dans les revendications 1 à 4 en un composé de formule (I)
et on sépare le cas échéant les composés de formule (I) ainsi obtenus selon des procédés connus par l'homme du métier en leurs énantiomères et/ou diastéréo-isomères et/ou on les transforme avec (i) des solvants et/ou (ii) des bases ou acides correspondants en leurs solvates, sels et/ou solvates des sels.

6. Composé, tel que défini dans l'une quelconque des revendications 1 à 4, destiné au traitement et/ou à la prévention de maladies.

7. Composé, tel que défini dans l'une quelconque des revendications 1 à 4, destiné à une utilisation dans un procédé pour le traitement et/ou la prévention de l'hypertonie artérielle pulmonaire (HAP) et d'autres formes de l'hypertonie pulmonaire (HP), des maladies pulmonaires obstructives chroniques (MPOC), du syndrome respiratoire aigu (ALI - acute lung injury), du syndrome de détresse respiratoire aiguë (ARDS), de la bronchectasie, de la bronchiolite oblitérante, de l'emphysème pulmonaire, de la déficience en alpha-1-antitrypsine (AATD) et de la fibrose kystique (CF).

8. Utilisation d'un composé, tel que défini dans l'une quelconque des revendications 1 à 4, pour la préparation d'un médicament destiné au traitement et/ou à la prévention de l'hypertonie artérielle pulmonaire (HAP) et d'autres formes de l'hypertonie pulmonaire (HP), des maladies pulmonaires obstructives chroniques (MPOC), du syndrome respiratoire aigu (ALI - acute lung injury), du syndrome de détresse respiratoire aiguë (ARDS), de la bronchectasie, de la bronchiolite oblitérante, de l'emphysème pulmonaire, de la déficience en alpha-1-antitrypsine (AATD) et de la fibrose kystique (CF).

9. Médicament contenant un composé, tel que défini dans l'une quelconque des revendications 1 à 4, en combinaison avec un ou plusieurs adjuvants inertes, non toxiques, pharmaceutiquement appropriés.

10. Médicament contenant un composé tel que défini dans l'une quelconque des revendications 1 à 4 en combinaison avec une ou plusieurs autres substances actives choisies dans le groupe constitué par les inhibiteurs de kinase, les stimulateurs et les activateurs de la guanylate cyclase soluble, les analogues de prostacycline, les antagonistes du récepteur de l'endothéline, les inhibiteurs de la phosphodiestérase, les agonistes du récepteur bêta-adrénergique, les anticholinergiques et les glucocorticoïdes.

11. Médicament selon la revendication 9 ou 10 pour le traitement et/ou la prévention de l'hypertonie artérielle pulmonaire (HAP) et d'autres formes de l'hypertonie pulmonaire (HP), des maladies pulmonaires obstructives chroniques (MPOC), du syndrome respiratoire aigu (ALI - acute lung injury), du syndrome de détresse respiratoire aiguë (ARDS), de la bronchectasie, de la bronchiolite oblitérante, de l'emphysème pulmonaire, de la déficience en alpha-1-antitrypsine (AATD) et de la fibrose kystique (CF).
